# EUROPEAN PATENT SPECIFICATION

(11) **EP 2 576 046 B1**
(45) Date of publication and mention of the grant of the patent: **19.11.2014**
(21) Application number: 11726004.2
(22) Date of filing: 24.05.2011
(51) Int. Cl.: C07C 2/84, C07C 2/06, C10G 50/00, B01J 35/06

(54) **Method for producing ethylene from methane using a polycrystalline nanowire catalyst**
Verfahren zur Herstellung von Ethylen aus Methan unter Verwendung von einem Nanodraht-Katalysator
Procédé de préparation d'éthylene à partir de méthane par utilisation d'un catalyseur nanofil

(30) Priority: 21.12.2010 US 201061425631 P; 24.05.2010 US 347774 P
(43) Date of publication of application: 10.04.2013
(73) Proprietor: Siluria Technologies, Inc., San Francisco, California 94158 (US)
(72) Inventor: SCHER, Erik, C., San Francisco, California 94110 (US); ZURCHER, Fabio, R., Brisbane, California 94005 (US); CIZERON, Joel, M., Sunnyvale, California 94087 (US); SCHAMMEL, Wayne, P., San Francisco, California 94158 (US); TKACHENKO, Alex, San Francisco, California 94111 (US); GAMORAS, Joel, Vallejo, California 94590 (US); KARSHTEDT, Dmitry, San Francisco, California 94116 (US); NYCE, Greg, Pleasanton, California 94566 (US)
(74) Representative: Grünecker, Kinkeldey, Stockmair & Schwanhäusser
(86) International application number: PCT/US2011/037821
(87) International publication number: WO 2011/149996

(56) References cited:
- EP-A1- 1 632 467
- WO-A1-2008/022147
- WO-A1-2011/050359
- WO-A2-2004/033488
- WO-A2-2005/067683
- WO-A2-2008/005055
- US-A- 5 897 945
- AURÉLIE DÉBART ET AL: "Alpha-MnO2 Nanowires: A Catalyst for the O2 Electrode in Rechargeable Lithium Batteries", ANGEWANDTE CHEMIE. INTERNATIONAL EDITION, WILEY VCH VERLAG, WEINHEIM, vol. 47, no. 24, 2 June 2008 (2008-06-02), pages 4521-4524, XP002624173, ISSN: 1433-7851, DOI: 10.1002/ANIE.200705648 [retrieved on 2008-05-06]
- Heinz Zimmermann ET AL: "Ethylene" In: "Ullmann's Encyclopedia of Industrial Chemistry", 15 April 2009 (2009-04-15), Wiley-VCH Verlag GmbH & Co. KGaA, Weinheim, Germany, XP055007506, ISBN: 978-3-52-730673-2 page 62 table 17
- NELTNER B ET AL: "Production of Hydrogen Using Nanocrystalline Protein-Templated Catalysts on M13 Phage", ACS NANO, AMERICAN CHEMICAL SOCIETY, UNITED STATES, vol. 4, no. 6, 22 June 2010 (2010-06-22), pages 3227-3235, XP002615564, ISSN: 1936-0851, DOI: 10.1021/NN100346H [retrieved on 2010-06-07]
- Brian Neltner: "Hybrid Bio-Templated Catalysts", PhD Thesis, 1 June 2010 (2010-06-01), XP055007170, Retrieved from the Internet: URL:http://brianneltner.com/Brian%Neltner% -%Dissertation.pdf [retrieved on 2011-09-14]

## Description

### BACKGROUND

### Technical Field

This invention relates a method for the preparation of ethylene from methane comprising contacting a mixture comprising oxygen and methane at a temperature below 900 °C with an inorganic catalytic polycrystalline nanowire and a method for the preparation of a downstream product of ethylene, the method comprising converting methane into ethylene in the presence of an inorganic polycrystalline catalytic nanowire and further oligomerizing the ethylene to prepare a downstream product of ethylene.

### Description of the Related Art

Catalysis is the process in which the rate of a chemical reaction is either increased or decreased by means of a catalyst. Positive catalysts increase the speed of a chemical reaction, while negative catalysts slow it down. Substances that increase the activity of a catalyst are referred to as promoters or activators, and substances that deactivate a catalyst are referred to as catalytic poisons or deactivators. Unlike other reagents, a catalyst is not consumed by the chemical reaction, but instead participates in multiple chemical transformations. In the case of positive catalysts, the catalytic reaction generally has a lower rate-limiting free energy change to the transition state than the corresponding uncatalyzed reaction, resulting in an increased reaction rate at the same temperature. Thus, at a given temperature, a positive catalyst tends to increase the yield of desired product while decreasing the yield of undesired side products. Although catalysts are not consumed by the reaction itself, they may be inhibited, deactivated or destroyed by secondary processes, resulting in loss of catalytic activity.

Catalysts are generally characterized as either heterogeneous or homogeneous. Heterogeneous catalysts exist in a different phase than the reactants (*e.g*. a solid metal catalyst and gas phase reactants), and the catalytic reaction generally occurs on the surface of the heterogeneous catalyst. Thus, for the catalytic reaction to occur, the reactants must diffuse to and/or adsorb onto the catalyst surface. This transport and adsorption of reactants is often the rate limiting step in a heterogeneous catalysis reaction. Heterogeneous catalysts are also generally easily separable from the reaction mixture by common techniques such as filtration or distillation.

In contrast to a heterogeneous catalyst, a homogenous catalyst exists in the same phase as the reactants (*e.g*., a soluble organometallic catalyst and solvent-dissolved reactants). Accordingly, reactions catalyzed by a homogeneous catalyst are controlled by different kinetics than a heterogeneously catalyzed reaction. In addition, homogeneous catalysts can be difficult to separate from the reaction mixture.

While catalysis is involved in any number of technologies, one particular area of importance is the petrochemical industry. At the foundation of the modern petrochemical industry is the energy-intensive endothermic steam cracking of crude oil. Cracking is used to produce nearly all the fundamental chemical intermediates in use today. The amount of oil used for cracking and the volume of green house gases (GHG) emitted in the process are quite large: cracking consumes nearly 10% of the total oil extracted globally and produces 200M metric tons of CO₂ equivalent every year (Ren, T, Patel, M. Res. Conserv. Recycl. 53:513, 2009). There remains a significant need in this field for new technology directed to the conversion of unreactive petrochemical feedstocks (*e.g*. paraffins, methane, ethane, etc.) into reactive chemical intermediates (*e.g.* olefins), particularly with regard to highly selective heterogeneous catalysts for the direct oxidation of hydrocarbons.

While there are multistep paths to convert methane to certain specific chemicals using first; high temperature steam reforming to syngas (a mixture of H₂ and CO), followed by stochiometry adjustment and conversion to either methanol or, via the Fischer-Tropsch (F-T) synthesis, to liquid hydrocarbon fuels such as diesel or gasoline, this does not allow for the formation of certain high value chemical intermediates. This multi-step indirect method also requires a large capital investment in facilities and is expensive to operate, in part due to the energy intensive endothermic reforming step. (For instance, in methane reforming, nearly 40% of methane is consumed as fuel for the reaction.) It is also inefficient in that a substantial part of the carbon fed into the process ends up as the GHG CO₂, both directly from the reaction and indirectly by burning fossil fuels to heat the reaction. Thus, to better exploit the natural gas resource, direct methods that are more efficient, economical and environmentally responsible are required.

One of the reactions for direct natural gas activation and its conversion into a useful high value chemical, is the oxidative coupling of methane ("OCM") to ethylene: 2CH₄+O₂ → C₂H₄ + 2H₂O. *See, e.g.,* Zhang, Q., Journal ofNatural Gas Chem., 12:81, 2003; Olah, G. "Hydrocarbon Chemistry", Ed. 2, John Wiley & Sons (2003). This reaction is exothermic (ΔH = -67kcals/mole) and has only been shown to occur at very high temperatures (>700°C). Although the detailed reaction mechanism is not fully characterized, experimental evidence suggests that free radical chemistry is involved. (Lunsford, J. Chem. Soc., Chem. Comm., 1991; H. Lunsford, Angew. Chem., Int. Ed. Engl., 34:970, 1995). In the reaction, methane (CH₄) is activated on the catalyst surface, forming methyl radicals which then couple in the gas phase to form ethane (C₂H₆), followed by dehydrogenation to ethylene (C₂H₄). Several catalysts have shown activity for OCM, including various forms of iron oxide, V₂O₅, MoO₃, Co₃O₄, Pt-Rh, Li/ZrO₂, Ag-Au, Au/Co₃O₄, Co/Mn, CeO₂, MgO, La₂O₃, Mn₃O₄, Na₂WO₄, MnO, ZnO, and combinations thereof, on various supports. A number of doping elements have also proven to be useful in combination with the above catalysts.

Since the OCM reaction was first reported over thirty years ago, it has been the target of intense scientific and commercial interest, but the fundamental limitations of the conventional approach to C-H bond activation appear to limit the yield of this attractive reaction. Specifically, numerous publications from industrial and academic labs have consistently demonstrated characteristic performance of high selectivity at low conversion of methane, or low selectivity at high conversion (J.A. Labinger, Cat. Lett., 1:371, 1988). Limited by this conversion/selectivity threshold, no OCM catalyst has been able to exceed 20-25% combined C₂ yield (*i.e*. ethane and ethylene), and all such yields are reported at extremely high temperatures (>800C). This lack of progress with conventional heterogeneous catalysts and reactors during the last third of a century suggests that conventional approaches have reached the limit of their performance.

In this regard, it is believed that the low yield of desired products (*i.e.* C₂H₄ and C₂H₆) is caused by the unique homogeneous/heterogeneous nature of the reaction. Specifically, due to the high reaction temperature, a majority of methyl radicals escape the catalyst surface and enter the gas phase. There, in the presence of oxygen and hydrogen, multiple side reactions are known to take place (J.A. Labinger, Cat. Lett., 1:371, 1988). The non-selective over-oxidation of hydrocarbons to CO and CO₂ (*e.g*., complete oxidation) is the principal competing fast side reaction. Other undesirable products (*e.g.* methanol, formaldehyde) have also been observed and rapidly react to form CO and CO₂.

In order to dramatically increase the yield of OCM, a catalyst optimized for the activation of the C-H bond of methane at lower temperatures (*e.g*. 500-900°C) is required. While the above discussion has focused on the OCM reaction, numerous other catalytic reactions (as discussed in greater detail below) would significantly benefit from catalytic optimization. Accordingly, there remains a need in the art for improved catalysts and, more specifically, a need for novel approaches to catalyst design for improving the yield of, for example, the OCM reaction and other catalyzed reactions. The present invention fulfills these needs and provides further related advantages.

### BRIEF SUMMARY

The present invention provides a method for the preparation of ethylene from methane comprising contacting a mixture comprising oxygen and methane at a temperature below 900 °C with a catalytic nanowire,
wherein the catalytic nanowire is an inorganic catalytic polycrystalline nanowire having a ratio of effective length to actual length of less than one and an aspect ratio of greater than ten as measured by TEM in bright field mode at 5 keV, and comprising one or more elements from any of Groups 1 through 7, lanthanides, actinides or combinations thereof in the form of oxides, hydroxides, oxyhydroxides, sulfates, carbonates, oxide carbonates, oxalates, phosphates, hydrogenphosphates, dihydrogenphosphates, oxyhalides, hydroxihalides, oxyhydroxides, oxysulfates or combinations thereof.

The present invention also provides a method for the preparation of a downstream product of ethylene, the method comprising converting methane into ethylene in the presence of a catalytic nanowire and further oligomerizing the ethylene to prepare a downstream product of ethylene,
wherein the catalytic nanowire is an inorganic catalytic polycrystalline nanowire having a ratio of effective length to actual length of less than one and an aspect ratio of greater than ten as measured by TEM in bright field mode at 5 keV, and comprising one or more elements from any of Groups 1 through 7, lanthanides, actinides or combinations thereof in the form of oxides, hydroxides, oxyhydroxides, sulfates, carbonates, oxide carbonates, oxalates, phosphates, hydrogenphosphates, dihydrogenphosphates, oxyhalides, hydroxihalides, oxyhydroxides, oxysulfates or combinations thereof.

Preferred embodiments of the invention are set forth in the dependent claims.

### BRIEF DESCRIPTION OF THE SEVERAL VIEWS OF THE DRAWINGS

In the drawings, the sizes and relative positions of elements in the drawings are not necessarily drawn to scale. For example, the various elements and angles are not drawn to scale, and some of these elements are arbitrarily enlarged and positioned to improve drawing legibility. Further, the particular shapes of the elements as drawn are not intended to convey any information regarding the actual shape of the particular elements, and have been selected solely for ease of recognition in the drawings.
Figure 1 schematically depicts a first part of an OCM reaction at the surface of a metal oxide catalyst.
Figure 2 shows a high throughput work flow for synthetically generating and testing libraries of nanowires.
Figures 3A and 3B illustrate a nanowire.
Figures 4A and 4B illustrate a nanowire.
Figures 5A and 5B illustrate a plurality of nanowires.
Figure 6 illustrates a filamentous bacteriophage.
Figure 7 is a flow chart of a nucleation process for forming a metal oxide nanowire.
Figure 8 is a flow chart of a sequential nucleation process for forming a nanowire in a core/shell configuration.
Figure 9 illustrates a number of downstream products of ethylene.
Figure 10 depicts a representative process for preparing a lithium doped MgO nanowire.
Figure 11 presents the X-ray diffraction patterns of Mg(OH)2 nanowires and MgO nanowires.
Figure 12 shows a number of MgO nanowires each synthesized in the presence of a different phage sequence.
Figure 13 depicts a representative process for growing a core/shell structure of ZrO₂/La₂O₃ nanowires with Strontium dopant.
Figure 14 is a gas chromatograph showing the formation of OCM products at 700 °C when passed over a Sr doped La₂O₃ nanowire.
Figure 15A -15C are graphs showing methane conversion, C2 selectivity, and C2 yield, in an OCM reaction catalyzed by Sr doped La₂O₃ nanowires vs. the corresponding bulk material in the same reaction temperature range.
Figure 16A -16B are graphs showing the comparative results of C2 selectivities in an OCM reaction catalyzed by Sr doped La₂O₃ nanowire catalysts prepared by different synthetic conditions.
Figure 17 is a graph comparing ethane and propane conversions in ODH reactions catalyzed by either Li doped MgO phage-based nanowires or Li doped MgO bulk catalyst.
Figure 18 is a TEM image showing La₂O₃ nanowires prepared under non-template-directed conditions.
Figure 19 depicts OCM and ethylene oligomerization modules.
Figure 20 shows methane conversion, C2 selectivity and C2 yield in a reaction catalyzed by a representative nanowire at a CH₄/O₂ ratio of 4.
Figure 21 shows methane conversion, C2 selectivity and C2 yield in a reaction catalyzed by a representative nanowire at a CH₄/O₂ ratio of 5.5.
Figure 22 is a graph showing methane conversion, C2 selectivity and C2 yield in a reaction catalyzed by Mg/Na doped La₂O₃ nanowires.

### DETAILED DESCRIPTION OF THE INVENTION

In the following description, certain specific details are set forth in order to provide a thorough understanding of various embodiments. Unless the context requires otherwise, throughout the specification and claims which follow, the word "comprise" and variations thereof, such as, "comprises" and "comprising" are to be construed in an open, inclusive sense, that is, as "including, but not limited to."

Reference throughout this specification to "one embodiment" or "an embodiment" means that a particular feature, structure or characteristic described in connection with the embodiment is included in at least one embodiment. Thus, the appearances of the phrases "in one embodiment" or "in an embodiment" in various places throughout this specification are not necessarily all referring to the same embodiment. Furthermore, the particular features, structures, or characteristics may be combined in any suitable manner in one or more embodiments. Also, as used in this specification and the appended claims, the singular forms "a," "an," and "the" include plural referents unless the content clearly dictates otherwise. It should also be noted that the term "or" is generally employed in its sense including "and/or" unless the content clearly dictates otherwise.

As discussed above, heterogeneous catalysis takes place between several phases. Generally, the catalyst is a solid, the reactants are gases or liquids and the products are gases or liquids. Thus, a heterogeneous catalyst provides a surface that has multiple active sites for adsorption of one more gas or liquid reactants. Once adsorbed, certain bonds within the reactant molecules are weakened and dissociate, creating reactive fragments of the reactants, *e.g*., in free radical forms. One or more products are generated as new bonds between the resulting reactive fragments form, in part, due to their proximity to each other on the catalytic surface.

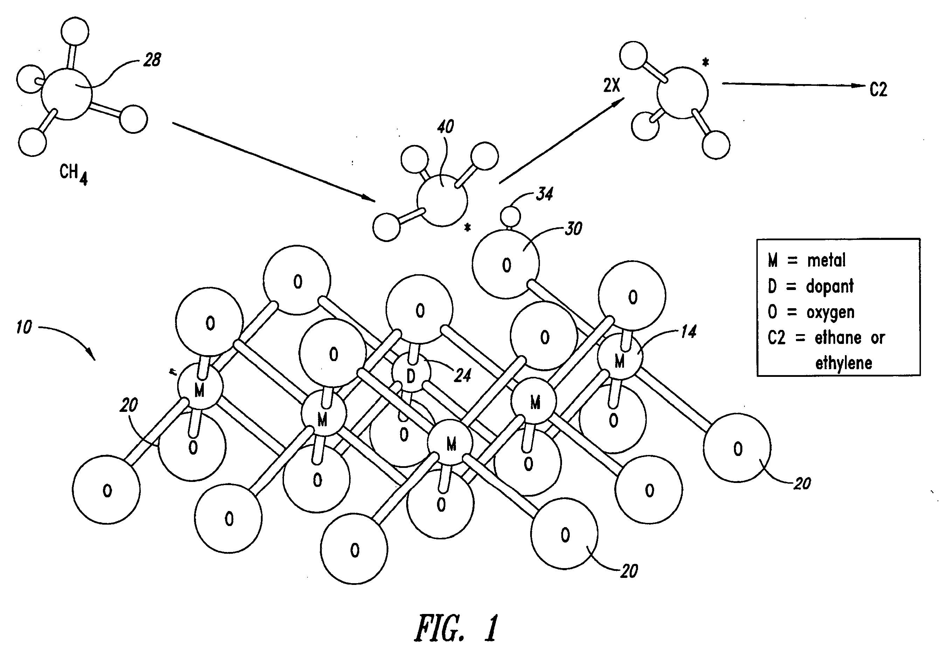

As an example, Figure 1 shows schematically the first part of an OCM reaction that takes place on the surface of a metal oxide catalyst 10 which is followed by methyl radical coupling in the gas phase. A crystal lattice structure of metal atoms 14 and oxygen atoms 20 are shown, with an optional dopant 24 incorporated into the lattice structure. In this reaction, a methane molecule 28 comes into contact with an active site (*e.g.,* surface oxygen 30) and becomes activated when a hydrogen atom 34 dissociates from the methane molecule 28. As a result, a methyl radical 40 is generated on or near the catalytic surface. Two methyl radicals thus generated can couple in the gas phase to create ethane and/or ethylene, which are collectively referred to as the "C2" coupling products.

It is generally recognized that the catalytic properties of a catalyst strongly correlate to its surface morphology. Typically, the surface morphology can be defined by geometric parameters such as: (1) the number of surface atoms (*e.g.,* the surface oxygen of Figure 1) that coordinate to the reactant; and (2) the degree of coordinative unsaturation of the surface atoms, which is the coordination number of the surface atoms with their neighboring atoms. For example, the reactivity of a surface atom decreases with decreasing coordinative unsaturation. For example, for the dense surfaces of a face-centered crystal, a surface atom with 9 surface atom neighbors will have a different reactivity than one with 8 neighbors. Additional surface characteristics that may contribute to the catalytic properties include, for example, crystal dimensions, lattice distortion, surface reconstructions, defects, grain boundaries. *See*, *e.g*., Van Santen R.A. et al New Trends in Materials Chemistry 345-363 (1997).

Catalysts in nano-size dimensions have substantially increased surface areas compared to their counterpart bulk materials. The catalytic properties are expected to be enhanced as more surface active sites are exposed to the reactants. Typically in traditional preparations, a top-down approach (*e.g*., milling) is adopted to reduce the size of the bulk material. However, the surface morphologies of such catalysts remain largely the same as those of the parent bulk material.

Described herein are nanowires with controllable or tunable surface morphologies. In particular, nanowires synthesized by a "bottom up" approach, by which inorganic polycrystalline nanowires are nucleated from solution phase in the presence of a template, *e.g*., a linear or anisotropic shaped biological template. By varying the synthetic conditions, nanowires having different compositions and/or different surface morphologies are generated.

In contrast to a bulk catalyst of a given elemental composition, which is likely to have a particular corresponding surface morphology, diverse nanowires with different surface morphologies can be generated despite having the same elemental composition. In this way, morphologically diverse nanowires can be created and screened according to their catalytic activity and performance parameters in any given catalytic reaction. Advantageously, the nanowires disclosed herein and methods of producing the same have general applicability to a wide variety of heterogeneous catalyses, including without limitation: oxidative coupling of methane (*e.g.*, Figure 1), oxidative dehydrogenation of alkanes to their corresponding alkenes, selective oxidation of alkanes to alkenes and alkynes, oxidation of carbon monoxide, dry reforming of methane, selective oxidation of aromatics, Fischer-Tropsch reaction, and hydrocarbon cracking.

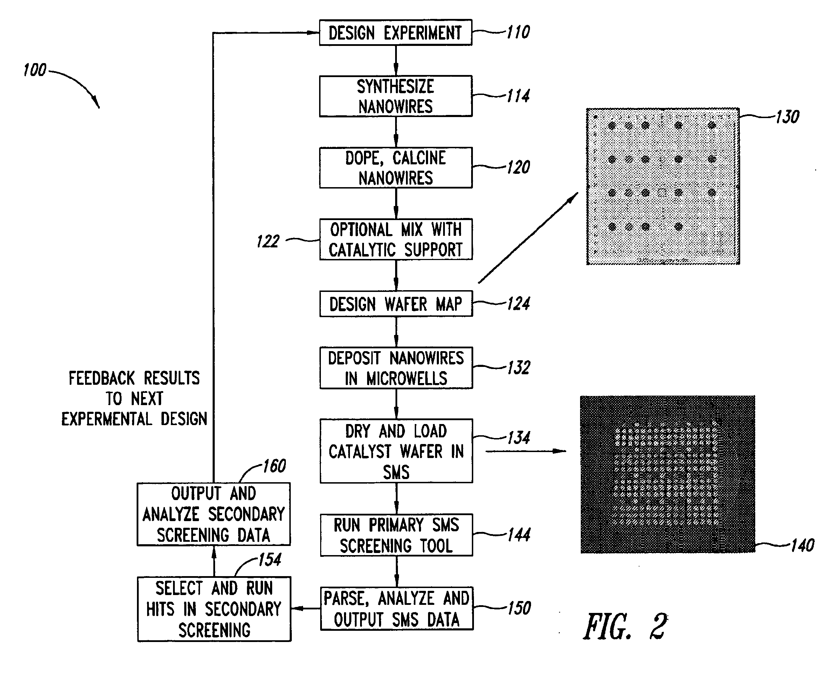

Figure 2 schematically shows a high throughput work flow for synthetically generating libraries of morphologically or compositionally diverse nanowires and screening for their catalytic properties. An initial phase of the work flow involves a primary screening, which is designed to broadly and efficiently screen a large and diverse set of nanowires that logically could perform the desired catalytic transformation. For example, certain doped bulk metal oxides (*e.g.*, Li/MgO and Sr/La₂O₃) are known catalysts for the OCM reaction. Therefore, nanowires of various metal oxide compositions and/or surface morphologies can be prepared and evaluated for their catalytic performances in an OCM reaction.

More specifically, the work flow 100 begins with designing synthetic experiments based on solution phase template formations (block 110). The synthesis, subsequent treatments and screenings can be manual or automated. As will be discussed in more detail herein, by varying the synthetic conditions, nanowires can be prepared with various surface morphologies and/or compositions in respective microwells (block 114). The nanowires are subsequently calcined and then optionally doped (block 120). Optionally, the doped and calcined nanowires are further mixed with a catalyst support (block 122). Beyond the optional support step, all subsequent steps are carried out in a "wafer" format, in which nanowire catalysts are deposited in a quartz wafer that has been etched to create an ordered array of microwells. Each microwell is a self-contained reactor, in which independently variable processing conditions can be designed to include, without limitation, respective choices of elemental compositions, catalyst support, reaction precursors, templates, reaction durations, pH values, temperatures, ratio between reactants, gas flows, and calcining conditions (block 124). Due to design contrasts of some wafers, calcining and other temperature variables can be identical in all microwells. A wafer map 130 can be created to correlate the processing conditions to the nanowire in each microwell. A library of diverse nanowires can be generated in which each library member corresponds to a particular set of processing conditions and corresponding compositional and/or morphological characteristics.

Nanowires obtained under various synthetic conditions are thereafter deposited in respective microwells of a wafer (140) for evaluating their respective catalytic properties in a given reaction (blocks 132 and 134). The catalytic performance of each library member can be screened serially by several known primary screening technologies, including scanning mass spectroscopy (SMS) (Symyx Technologies Inc., Santa Clara, California). The screening process is fully automated, and the SMS tool can determine if a nanowire is catalytically active or not, as well as its relative strength as a catalyst at a particular temperature. Typically, the wafer is placed on a motion control stage capable of positioning a single well below a probe that flows the feed of the starting material over the nanowire surface and removes reaction products to a mass spectrometer and/or other detector technologies (blocks 134 and 140). The individual nanowire is heated to a preset reaction temperature, *e.g*., using a CO₂ IR laser from the backside of the quartz wafer and an IR camera to monitor temperature and a preset mixture of reactant gases. The SMS tool collects data with regard to the consumption of the reactant(s) and the generation of the product(s) of the catalytic reaction in each well (block 144), and at each temperature and flow rate.

The SMS data obtained as described above provide information on relative catalytic properties among all the library members (block 150). In order to obtain more quantitative data on the catalytic properties of the nanowires, possible hits that meet certain criteria are subjected to a secondary screening (block 154). Typically, secondary screening technologies include a single, or alternatively multiple channel fixed-bed or fluidized bed reactors (as described in more detail herein). In parallel reactor systems or multi-channel fixed-bed reactor system, a single feed system supplies reactants to a set of flow restrictors. The flow restrictors divide the flows evenly among parallel reactors. Care is taken to achieve uniform reaction temperature between the reactors such that the various nanowires can be differentiated solely based on their catalytic performances. The secondary screening allows for accurate determination of catalytic properties such as selectivity, yield and conversion.(block 160). These results serve as a feedback for designing further nanowire libraries. Additional description of SMS tools in a combinatorial approach for discovering catalysts can be found in, *e.g*., Bergh, S. et al. Topics in Catalysts 23:1-4, 2003.

Thus, as described herein, compositional and morphologically diverse nanowires can be rationally synthesized to meet catalytic performance criteria. These and other aspects of the present disclosure are described in more detail below.

### Definitions

As used herein, and unless the context dictates otherwise, the following terms have the meanings as specified below.

"Catalyst" means a substance which alters the rate of a chemical reaction. A catalyst may either increase the chemical reaction rate (*i.e*. a "positive catalyst") or decrease the reaction rate (*i.e*. a "negative catalyst"). Catalysts participate in a reaction in a cyclic fashion such that the catalyst is cyclically regenerated. "Catalytic" means having the properties of a catalyst.

"Nanoparticle" means a particle having at least one diameter on the order of nanometers (*e.g.* between about 1 and 100 nanometers).

"Nanowire" means a nanowire structure having at least one diameter on the order of nanometers (*e.*g. between about 1 and 100 nanometers) and an aspect ratio greater than 10:1. The "aspect ratio" of a nanowire is the ratio of the actual length (L) of the nanowire to the diameter (D) of the nanowire. Aspect ratio is expressed as L:D.

"Polycrystalline nanowire" means a nanowire having multiple crystal domains. Polycrystalline nanowires generally have different morphologies (*e.g*. bent vs. straight) as compared to the corresponding "single-crystalline" nanowires.

"Effective length" of a nanowire means the shortest distance between the two distal ends of a nanowire as measured by transmission electron microscopy (TEM) in bright field mode at 5 keV. "Average effective length" refers to the average of the effective lengths of individual nanowires within a plurality of nanowires.

"Actual length" of a nanowire means the distance between the two distal ends of a nanowire as traced through the backbone of the nanowire as measured by TEM in bright field mode at 5 keV. "Average actual length" refers to the average of the actual lengths of individual nanowires within a plurality of nanowires.

The "diameter" of a nanowire is measured in an axis perpendicular to the axis of the nanowire's actual length (*i.e.* perpendicular to the nanowires backbone). The diameter of a nanowire will vary from narrow to wide as measured at different points along the nanowire backbone. As used herein, the diameter of a nanowire is the most prevalent (*i.e*. the mode) diameter.

The "ratio of effective length to actual length" is determined by dividing the effective length by the actual length. A nanowire having a "bent morphology" will have a ratio of effective length to actual length of less than one as described in more detail herein. A straight nanowire will have a ratio of effective length to actual length equal to one as described in more detail herein.

"Inorganic" means a substance comprising a metal element. Typically, an inorganic can be one or more metals in its elemental state, or more preferably, a compound formed by a metal ion (Mⁿ⁺, wherein n 1, 2, 3, 4, 5, 6 or 7) and an anion (X^{m-}, m is 1, 2, 3 or 4) which balance and neutralize the positive charges of the metal ion through electrostatic interactions. Non-limiting examples of inorganic compounds include oxides, hydroxides, halides, nitrates, sulfates, carbonates, acetates, oxalates, and combinations thereof, of metal elements. Other non-limiting examples of inorganic compounds include Li₂CO₃, LiOH, Li₂O, LiCl, LiBr, LiI, Li₂C₂O₄, Li₂SO₄, Na₂CO₃, NaOH, Na₂O, NaCl, NaBr, NaI, Na₂C₂O₄, Na₂SO₄, K₂CO₃, KOH, K₂O, KCl, KBr, KI, K₂C₂O₄, K₂SO₄, CsCO₃, CsOH, Cs₂O, CsCl, CsBr, Csl, CsC₂O₄, CsSO₄, Be(OH)₂, BeCO₃, BeO, BeCl₂, BeBr₂, BeI₂, BeC₂O₄. BeSO₄, Mg(OH)₂, MgCO₃, MgO, MgCl₂, MgBr₂, MgI₂, MgC₂O₄. MgO₄, Ca(OH)₂, CaO, CaCl₂, CaBr₂, CaI₂, Ca(OH)₂, CaC₂O₄, CaSO₄, Y₂O₃, Y₂(CO₃)₃, Y(OH)₃, YCl₃, YBr₃, Yl₃, Y₂(C₂O₄)₃, Y₂(SO₄)₃, Zr(OH)₄, ZrO(OH)₂, ZrO2, ZrCl₄, ZrBr₄, ZrI₄, Zr(C₂O₄)₂, Zr(SO₄)₂, Ti(OH)₄, TiO(OH)₂, Ti02, TiCl₄, TiBr₄, TiI₄, Ti(C₂O₄)₂, Ti(SO₄)₂,BaO, Ba(OH)₂, BaCO₃, BaCl₂, BaBr₂, BaI₂, BaC₂O₄, BaSO₄, La(OH)₃, La₂O₃, LaCl₃, LaBr₃, LaI₃, La₂(C₂O₄)₃, La₂(SO₄)₃, Ce(OH)₄, CeO₂, Ce₂O₃, CeCl₄, CeBr₄, CeI₄, Ce(C₂O₄)₂, Ce(SO₄)₂, ThO₂, ThCl₄, ThBr₄, ThI₄, Th(OH)₄, Th(C₂O₄)₂, Th(SO₄)₂, Sr(OH)₂, SrCO₃, SrO, SrCl₂, SrBr₂, SrI₂, SrC₂O₄, SrSO₄, Sm₂O₃, SmCl₃, SmBr₃, SmI₃, Sm(OH)₃, Sm₂(CO₃)₃, Sm₂(C₂O₃)₃, Sm₂(SO₄)₃, LiCa₂Bi₃O₄Cl₆, Na₂WO₄, K/SrCoO₃, K/Na/SrCoO₃, Li/SrCoO₃, SrCoO₃, molybdenum oxides, molybdenum hydroxides, molybdenum chlorides, molybdenum bromides, molybdenum iodides, molybdenum oxalates, molybdenum sulfates, manganese oxides, manganese chlorides, manganese bromides, manganese iodides, manganese hydroxides, manganese oxalates, manganese sulfates, manganese tugstates, vanadium oxides, vanadium chlorides, vanadium bromides, vanadium iodides, vanadium hydroxides, vanadium oxalates, vanadium sulfates, tungsten oxides, tungsten chlorides, tungsten bromides, tungsten iodides, tungsten hydroxides, tungsten oxalates, tungsten sulfates, neodymium oxides, neodymium chlorides, neodymium bromides, neodymium iodides, neodymium hydroxides, neodymium oxalates, neodymium sulfates, europium oxides, europium chlorides, europium bromides, europium iodides, europium hydroxides, europium oxalates, europium sulfates rhenium oxides, rhenium chlorides, rhenium bromides, rhenium iodides, rhenium hydroxides, rhenium oxalates, rhenium sulfates, chromium oxides, chromium chlorides, chromium bromides, chromium iodides, chromium hydroxides, chromium oxalates, chromium sulfates, and potassium molybdenum oxides.

"Salt" means a compound comprising negative and positive ions. Salts are generally comprised of metallic cations and non-metallic counter ions. Under appropriate conditions, *e.g.*, the solution also comprises a template, the metal ion (Mⁿ⁺) and the anion (X^{m-}) bind to the template to induce nucleation and growth of a nanowire of MₘXₙ on the template. "Anion precursor" thus is a compound that comprises an anion and a cationic counter ion, which allows the anion (X^{m-}) dissociate from the cationic counter ion in a solution. Specific examples of the metal salt and anion precursors are described in further detail herein.

"Oxide" refers to a metal compound comprising oxygen. Examples of oxides include, but are not limited to, metal oxides (MₓO_{y}), metal oxyhalide (MₓO_{y}X_{z}), metal oxynitrates (MₓO_{y}(NO₃)_{z}), metal phosphates (Mₓ(PO₄)_{y}), metal oxide carbonates (MₓO_{y}(CO₃)_{z}), metal carbonates and the like, wherein x, y and z are numbers from 1 to 100.

"Crystal domain" means a continuous region over which a substance is crystalline.

"Single-crystalline nanowires" means a nanowire having a single crystal domain.

"Template" is any synthetic and/or natural material that provides at least one nucleation site where ions can nucleate and grow to form nanoparticles. In certain embodiments, the templates can be a multi-molecular biological structure comprising one or more biomolecules. Typically, the biological template comprises multiple binding sites that recognize certain ions and allow for the nucleation and growth of the same. Non-limiting examples of biological templates include bacteriophages, amyloid fibers, viruses and capsids.

"Biomolecule" refers to any organic molecule of a biological origin. Biomolecule includes modified and/or degraded molecules of a biological origin. Non-limiting examples of biomolecules include peptides, proteins (including cytokines, growth factors, etc.), nucleic acids, polynucleotides, amino acids, antibodies, enzymes, and single-stranded or double-stranded nucleic acid, including any modified and/or degraded forms thereof.

"Amyloid fibers" refers to proteinaceous filaments of about 1-25 nm in diameter.

A "bacteriophage" or "phage" is any one of a number of viruses that infect bacteria. Typically, bacteriophages consist of an outer protein coat or "major coat protein" enclosing genetic material. A non-limiting example of a bacteriophage is the M13 bacteriophage. Non-limiting examples of bacteriophage coat proteins include the pIII, pV, pVIII, etc. protein as described in more detail below.

A "capsid" is the protein shell of a virus. A capsid comprises several oligomeric structural subunits made of proteins.

"Nucleation" refers to the process of forming a solid from solubilized particles, for example forming a nanowire *in situ* by converting a soluble precursor (*e.g*. metal and hydroxide ions) into nanocrystals in the presence of a template.

"Nucleation site" refers to a site on a template, for example a bacteriophage, where nucleation of ions may occur. Nucleation sites include, for example, amino acids having carboxylic acid (-COOH), amino (-NH₃⁺ or-NH₂), hydroxyl (-OH), and/or thiol (-SH) functional groups.

A "peptide" refers to two or more amino acids joined by peptide (amide) bonds. The amino-acid building blocks (subunits) include naturally occurring α-amino acids and/or unnatural amino acids, such as β-amino acids and homoamino acids. An unnatural amino acid can be a chemically modified form of a natural amino acid. Peptides can be comprised of 2 or more, 5 or more, 10 or more, 20 or more, or 40 or more amino acids.

"Peptide sequence" refers to the sequence of amino acids within a peptide or protein.

"Protein" refers to a natural or engineered macromolecule having a primary structure characterized by peptide sequences. In addition to the primary structure, proteins also exhibit secondary and tertiary structures that determine their final geometric shapes.

"Polynucleotide" means a molecule comprised of two or more nucleotides connected via an internucleotide bond (*e.g*. a phosphate bond). Polynucleotides may be comprised of both ribose and/or deoxy ribose nucleotides. Examples of nucleotides include guanosine, adenosine, thiamine, and cytosine, as well as unnatural analogues thereof.

"Nucleic acid" means a macromolecule comprised of polynucleotides. Nucleic acids may be both single stranded and double stranded, and, like proteins, can exhibit secondary and tertiary structures that determine their final geometric shapes.

"Nucleic acid sequence" of "nucleotide sequence" refers to the sequence of nucleotides within a polynucleotide or nucleic acid.

"Anisotropic" means having an aspect ratio greater than one.

"Anisotropic biomolecule" means a biomolecule, as defined herein, having an aspect ratio greater than 1. Non-limiting examples of anisotropic biomolecules include bacteriophages, amyloid fibers, and capsids.

"Turnover number" is a measure of the number of reactant molecules a catalyst can convert to product molecules per unit time.

"Dopant" or "doping agent" is an impurity added to or incorporated within a catalyst to optimize catalytic performance (*e.g*. increase or decrease catalytic activity). As compared to the undoped catalyst, a doped catalyst may increase or decrease the selectivity, conversion, and/or yield of a reaction catalyzed by the catalyst.

"Atomic percent" (at%) or "atomic ratio" when used in the context of nanowire dopants refers to the ratio of the total number of dopant atoms to the total number of non-oxygen atoms in the nanowire. For example, the atomic percent of dopant in a lithium doped Mg₆MnO₈ nanowire is determined by calculating the total number of lithium atoms and dividing by the sum of the total number of magnesium and manganese atoms and multiplying by 100 *(i.e.,* atomic percent of dopant = [Li atoms/(Mg atoms + Mn atoms)] x 100)

"Group 1" elements include lithium (Li), sodium (Na), potassium (K), rubidium (Rb), cesium (Cs), and francium (Fr).

"Group 2" elements include beryllium (Be), magnesium (Mg), calcium (Ca), strontium (Sr), barium (Ba), and radium (Ra).

"Group 3" elements include scandium (Sc) and yttrium (Y).

"Group 4" elements include titanium (Ti), zirconium (Zr), halfnium (Hf), and rutherfordium (Rf).

"Group 5" elements include vanadium (V), niobium (Nb), tantalum (Ta), and dubnium (Db).

"Group 6" elements include chromium (Cr), molybdenum (Mo), tungsten (W), and seaborgium (Sg).

"Group 7" elements include manganese (Mn), technetium (Tc), rhenium (Re), and bohrium (Bh).

"Group 8" elements include iron (Fe), ruthenium (Ru), osmium (Os), and hassium (Hs).

"Group 9" elements include cobalt (Co), rhodium (Rh), iridium (Ir), and meitnerium (Mt).

"Group 10" elements include nickel (Ni), palladium (Pd), platinum (Pt) and darmistadium (Ds).

"Group 11" elements include copper (Cu), silver (Ag), gold (Au), and roentgenium (Rg).

"Group 12" elements include zinc (Zn), cadmium (Cd), mercury (Hg), and copernicium (Cn).

"Lanthanides" include lanthanum (La), cerium (Ce), praseodymium (Pr), neodymium (Nd), promethium (Pm), samarium (Sm), europium (Eu), gadolinium (Gd), terbium (Tb), dysprosium (Dy), holmium (Ho), erbium (Er), thulium (Tm), yitterbium (Yb), and lutetium (Lu).

"Actinides" include actinium (Ac), thorium (Th), protactinium (Pa), uranium (U), neptunium (Np), plutonium (Pu), americium (Am), curium (Cm), berklelium (Bk), californium (Cf), einsteinium (Es), fermium (Fm), mendelevium (Md), nobelium (No), and lawrencium (Lr).

"Metal element" or "metal" is any element, except hydrogen, selected from Groups 1through XII, lanthanides, actinides, aluminum (Al), gallium (Ga), indium (In), tin (Sn), thallium (TI), lead (Pb), and bismuth (Bi). Metal elements include metal elements in their elemental form as well as metal elements in an oxidized or reduced state, for example, when a metal element is combined with other elements in the form of compounds comprising metal elements. For example, metal elements can be in the form of hydrates, salts, oxides, as well as various polymorphs thereof.

"Semi-metal element" refers to an element selected from boron (B), silicon (Si), germanium (Ge), arsenic (As), antimony (Sb), tellurium (Te), and polonium (Po).

"Non-metal element" refers to an element selected from carbon (C), nitrogen (N), oxygen (O), fluorine (F), phosphorus (P), sulfur (S), chlorine (CI), selenium (Se), bromine (Br), iodine (I), and astatine (At).

"Conversion" means the mole fraction (*i.e.,* percent) of a reactant converted to a product or products.

"Selectivity" refers to the percent of converted reactant that went to a specified product, *e.g*., C2 selectivity is the % of methane that formed ethane and ethylene, C3 selectivity is the % of methane that formed propane and propylene, CO selectivity is the percent of methane that formed CO.

"Yield" is a measure of (*e.g*. percent) of product obtained relative to the theoretical maximum product obtainable. Yield is calculated by dividing the amount of the obtained product in moles by the theoretical yield in moles. Percent yield is calculated by multiplying this value by 100.

"Bulk catalyst" or "bulk material" means a catalyst prepared by traditional techniques, for example by milling or grinding large catalyst particles to obtain smaller/higher surface area catalyst particles. Bulk materials are prepared with minimal control over the size and/or morphology of the material.

"Alkane" means a straight chain or branched, noncyclic or cyclic, saturated aliphatic hydrocarbon. Alkanes include linear, branched and cyclic structures. Representative straight chain alkyls include methyl, ethyl, n-propyl, n-butyl, n-pentyl, n-hexyl; while branched alkyls include isopropyl, sec-butyl, isobutyl, tert-butyl, isopentyl. Representative cyclic alkyls include cyclopropyl, cyclobutyl, cyclopentyl, cyclohexyl. "Alkene" means a straight chain or branched, noncyclic or cyclic, unsaturated aliphatic hydrocarbon having at least one carbon-carbon double bond. Alkenes include linear, branched and cyclic structures. Representative straight chain and branched alkenes include ethylenyl, propylenyl, 1-butenyl, 2-butenyl, isobutylenyl, 1-pentenyl, 2-pentenyl, 3-methyl-1-butenyl, 2-methyl-2-butenyl, and 2,3-dimethyl-2-butenyl. Cyclic alkenes include cyclohexene and cyclopentene.

"Alkyne" means a straight chain or branched, noncyclic or cyclic, unsaturated aliphatic hydrocarbon having at least one carbon-carbon triple bond. Alkynes include linear, branched and cyclic structures. Representative straight chain and branched alkynes include acetylenyl, propynyl, 1-butynyl, 2-butynyl, 1-pentynyl, 2-pentynyl, 3-methyl-1-butynyl. Representative cyclic alkynes include cycloheptyne.

"Aromatic" means a carbocyclic moiety having a cyclic system of conjugated p orbitals forming a delocalized conjugated π system and a number of π electrons equal to 4n + 2 with n = 0, 1, 2, 3. Representative examples of aromatics include benzene and naphthalene and toluene.

"Carbon-containing compounds" are compounds which comprise carbon. Non-limiting examples of carbon-containing compounds include hydrocarbons, CO and CO₂.

### Nanowires

### 1. Structure/Physical Characteristics

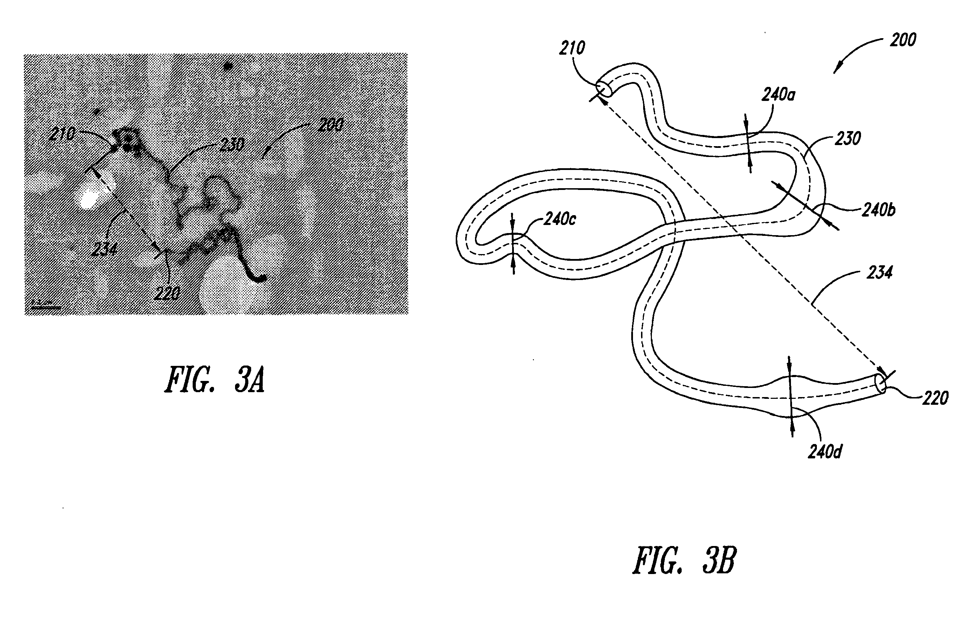

Figure 3A is a TEM image of a polycrystalline nanowire 200 having two distal ends 210 and 220. As shown, an actual length 230 essentially traces along the backbone of the nanowire 200, whereas an effective length 234 is the shortest distance between the two distal ends. The ratio of the effective length to the actual length is an indicator of the degrees of twists, bends and/or kinks in the general morphology of the nanowire. Figure 3B is a schematic representation of the nanowire 200 of Figure 3A. Typically, the nanowire is not uniform in its thickness or diameter. At any given location along the nanowire backbone, a diameter (240a, 240b, 240c, 240d) is the longest dimension of a cross section of the nanowire, *i.e*., is perpendicular to the axis of the nanowire backbone).

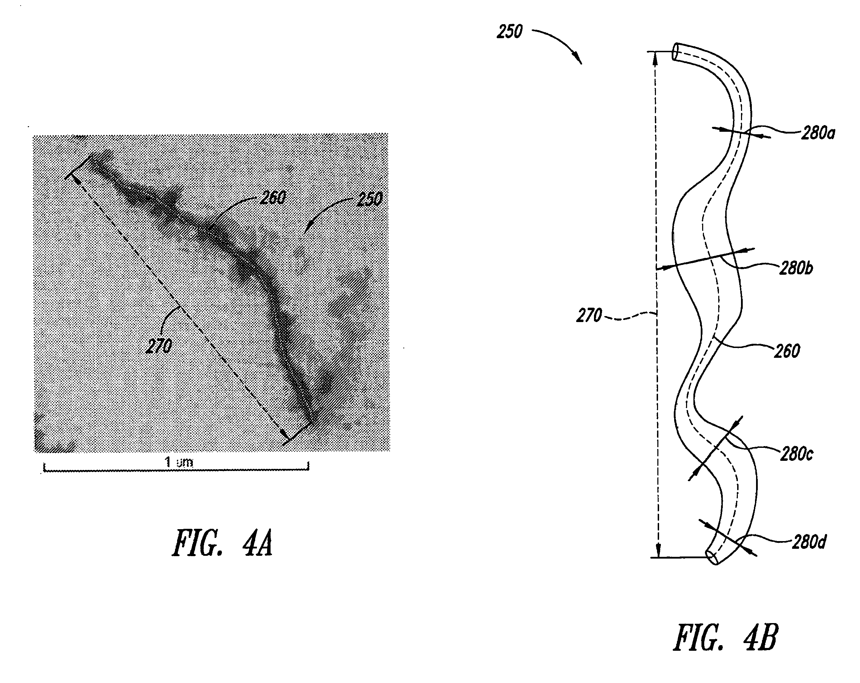

Compared to nanowire 200 of Figure 3A, nanowire 250 of Figure 4A has a different morphology and does not exhibit as many twists, bends and kinks, which suggests a different underlying crystal structure and different number of defects and/or stacking faults. As shown, for nanowire 250, the ratio of the effective length 270 and the actual length 260 is greater than the ratio of the effective length 234 and the actual length 240 of nanowire 200 of Figure 3A. Figure 4B is a schematic representation of the nanowire 250, which shows nonuniform diameters (280a, 280b, 280c and 280d).

As noted above, nanowires having a "bent" morphology (*i.e.* "bent nanowires") are provided. A "bent' morphology means that the bent nanowires comprise various twists, bends and/or kinks in their general morphology as illustrated generally in Figure 3A and 3B and discussed above. Bent nanowires have a ratio of effective length to actual length of less than one. Accordingly, the present disclosure provides nanowires having a ratio of effective length to actual length of less than one.

Preferably , the nanowires have a ratio of effective length to actual length of between 0.9 and 0.1, between 0.8 and 0.2, between 0.7 and 0.3, or between 0.6 and 0.4. Or, the ratio of effective length to actual length is less than 0.9, less than 0.8, less than 0.7, less than 0.6, less than 0.5, less than 0.4, less than 0.3, less than 0.2 or less than 0.1. Alternatively, the ratio of effective length to actual length is less than 1.0 and more than 0.9, less than 1.0 and more than 0.8, less than 1.0 and more than 0.7, less than 1.0 and more than 0.6, less than 1.0 and more than 0.5, less than 1.0 and more than 0.4, less than 1.0 and more than 0.3, less than 1.0 and more than 0.2, or less than 1.0 and more than 0.1.

The ratio of effective length to actual length of a nanowire having a bent morphology may vary depending on the angle of observation. For example, one-skilled in the art will recognize that the same nanowire, when observed from different perspectives, can have a different effective length as determined by TEM. In addition, not all nanowires having a bent morphology will have the same ratio of effective length to actual length. Accordingly, in a population (*i.e*. plurality) of nanowires having a bent morphology, a range of ratios of effective length to actual length is expected. Although the ratio of effective length to actual length may vary from nanowire to nanowire, nanowires having a bent morphology will always have a ratio of effective length to actual length of less than one from any angle of observation.

The actual lengths of the nanowires disclosed herein may vary. For example the nanowires can have an actual length of between 100 nm and 100 µm. Or, the nanowires have an actual length of between 100 nm and 10 µm. Alternatively, the nanowires have an actual length of between 200 nm and 10 µm, between 500 nm and 5 µm, greater than 5 µm, between 800 nm and 1000 nm. Preferably, the nanowires have an actual length of 900 nm. As noted below, the actual length of the nanowires is determined by TEM in bright field mode at 5 keV.

The diameter of the nanowires may be different at different points along the nanowire backbone. However, the nanowires comprise a mode diameter (*i.e*. the most frequently occurring diameter). As used herein, the diameter of a nanowire refers to the mode diameter. The nanowires may have a diameter of between 1 nm and 500 nm, between 1 nm and 100 nm, between 7 nm and 100 nm, between 7 nm and 50 nm, between 7 nm and 25 nm, or between 7 nm and 15 nm. Alternatively, the diameter is greater than 500 nm. As noted below, the diameter of the nanowires may be determined by TEM in bright field mode at 5 keV.

The nanowires have an aspect ratio of greater than 10:1. Preferably, the nanowires have an aspect ratio greater than 20:1, greater than 50:1, or greater than 100:1.

The nanowires comprise a solid core, or the nanowires comprise a hollow core.

The morphology of a nanowire (including length, diameter, and other parameters) can be determined by transmission electron microscopy (TEM). Transmission electron microscopy (TEM) is a technique whereby a beam of electrons is transmitted through an ultra thin specimen, interacting with the specimen as it passes through. An image is formed from the interaction of the electrons transmitted through the specimen. The image is magnified and focused onto an imaging device, such as a fluorescent screen, on a layer of photographic film or detected by a sensor such as a CCD camera. TEM techniques are well known to those of skill in the art.

A TEM image of nanowires may be taken in bright field mode at 5 keV (*e.g*., as shown in Figures 3A and 4A).

The nanowires of the present disclosure can be further characterized by powder x-ray diffraction (XRD). XRD is a technique capable of revealing information about the crystallographic structure, chemical composition, and physical properties of materials, including nanowires. XRD is based on observing the scattered intensity of an X-ray beam hitting a sample as a function of incident and scattered angle, polarization, and wavelength or energy.

Crystal structure, composition, and phase, including the crystal domain size of the nanowires, can be determined by XRD. The nanowires comprise multiple crystal domains (*i.e*. polycrystalline). For example, the average crystal domain of the nanowires is less than 100 nm, less than 50 nm, less than 30 nm, less than 20 nm, less than 10 nm, less than 5nm, or less than 2 nm.

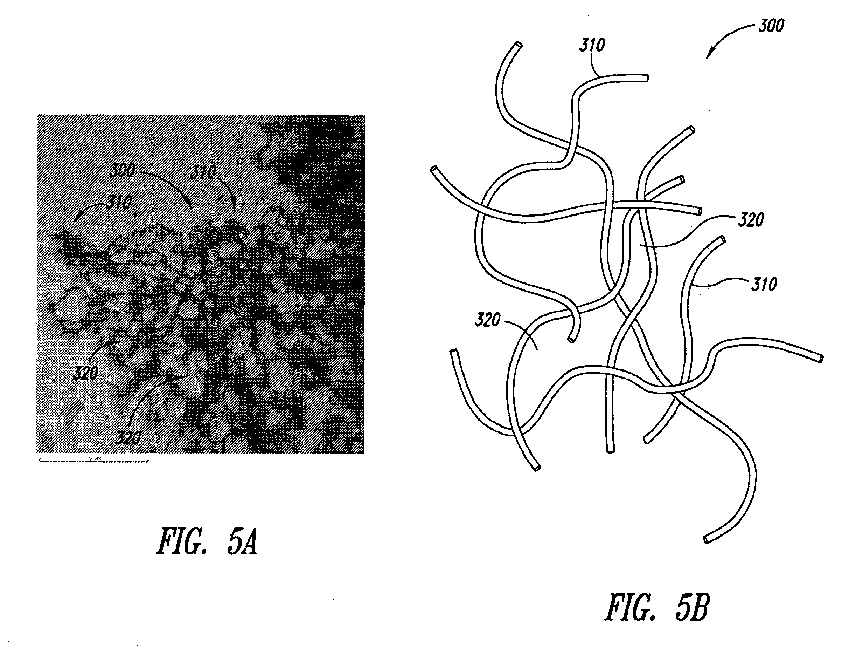

Typically, a catalytic material described herein comprises a plurality of nanowires. The plurality of nanowires can form a mesh of randomly distributed and, to various degrees, interconnected nanowires. Figure 5A is a TEM image of a nanowire mesh 300 comprising a plurality of nanowires 310 and a plurality of pores 320. Figure 5B is a schematic representation of the nanowire mesh 300 of Figure 5A.

The total surface area per gram of a nanowire or plurality of nanowires may have an effect on the catalytic performance. Pore size distribution may affect the nanowires catalytic performance as well. Surface area and pore size distribution of the nanowires or plurality of nanowires can be determined by BET (Brunauer, Emmett, Teller) measurements. BET techniques utilize nitrogen adsorption at various temperatures and partial pressures to determine the surface area and pore sizes of catalysts. BET techniques for determining surface area and pore size distribution are well known in the art.

The nanowires have a surface area of between 0.0001 and 3000 m²/g, between 0.0001 and 2000 m²/g, between 0.0001 and 1000 m²/g, between 0.0001 and 500 m²/g, between 0.0001 and 100 m²/g, between 0.0001 and 50 m²/g, between 0.0001 and 20 m²/g, between 0.0001 and 10 m²/g or between 0.0001 and 5 m²/g.

Alternatively, the nanowires have a surface area of between 0.001 and 3000 m²/g, between 0.001 and 2000 m²/g, between 0.001 and 1000 m²/g, between 0.001 and 500 m²/g, between 0.001 and 100 m²/g, between 0.001 and 50 m²/g, between 0.001 and 20 m²/g, between 0.001 and 10 m²/g or between 0.001 and 5 m²/g.

Or, the nanowires have a surface area of between 2000 and 3000 m²/g, between 1000 and 2000 m²/g, between 500 and 1000 m²/g, between 100 and 500 m²/g, between 10 and 100 m²/g, between 5 and 50 m²/g, between 2 and 20 m²/g or between 0.0001 and 10 m²/g.

Alternatively, the nanowires have a surface area of greater than 2000 m²/g, greater than 1000 m²/g, greater than 500 m²/g, greater than 100 m²/g, greater than 50 m²/g, greater than 20 m²/g, greater than 10 m²/g, greater than 5 m²/g, greater than 1 m²/g, greater than 0.0001 m²/g.

### 2. Chemical Composition

As noted above, disclosed herein are nanowires useful as catalysts. The catalytic nanowires may have any number of compositions and morphologies. The nanowires are inorganic and polycrystalline. The nanowires may a ratio of effective length to actual length of less than one and an aspect ratio of greater than ten as measured by TEM in bright field mode at 5 keV. The nanowires comprise one or more elements from any of Groups 1 through 7, lanthanides, actinides or combinations thereof.

The nanowires comprise one or more metal elements from any of Groups I-7, lanthanides, actinides or combinations thereof, for example, the nanowires may be mono-metallic, bi-metallic, trimetallic (*i.e*. contain one, two, three metal elements). The metal elements are present in the nanowires in the form of oxides, hydroxides, oxyhydroxides, sulfates, carbonates, oxide carbonates, oxalates, phosphates, hydrogenphosphates, dihydrogenphosphates, oxyhalides, hydroxihalides, oxyhydroxides, oxysulfates or combinations thereof.

In certain examples, metal oxides may be hygroscopic and may change forms once exposed to air. Accordingly, although the nanowires are often referred to as metal oxides, in certain embodiments the nanowires also comprise hydrated oxides, oxyhydroxides, hydroxides or combinations thereof.

The nanowires may comprise one or more metal elements from Group I, one or more metal elements from Group 2, one or more metal elements from Group 3, one or more metal elements from Group 4, one or more metal elements from Group 5, one or more metal elements from Group 6, one or more metal elements from Group 7, one or more metal elements from the lanthanides, or one or more metal elements from the actinides.

Preferably, the nanowires comprise one or more metal elements from any of Groups I-7, lanthanides, actinides or combinations thereof in the form of an oxide, one or more metal elements from Group 1 in the form of an oxide, one or more metal elements from Group 2 in the form of an oxide, one or more metal elements from Group 3 in the form of an oxide, one or more metal elements from Group 4 in the form of an oxide, one or more metal elements from Group 5 in the form of an oxide, one or more metal elements from Group 6 in the form of an oxide, one or more metal elements from Group 7 in the form of an oxide, one or more metal elements from the lanthanides in the form of an oxide, one or more metal elements from the actinides in the form of an oxide.

The nanowires may comprise oxides, hydroxides, sulfates, carbonates, oxide carbonates, oxalates, phosphates (including hydrogenphosphates and dihydrogenphosphates), oxyhalides, hydroxihalides, oxyhydroxides, oxysulfates or combinations thereof of one or more metal elements from any of Groups I-7, lanthanides, actinides or combinations thereof. Alternatively, the nanowires comprise oxides, hydroxides, sulfates, carbonates, oxide carbonates, oxalates or combinations thereof of one or more metal elements from any of Groups I-7, lanthanides, actinides or combinations thereof. Preferably, the nanowires comprise oxides, hydroxides , or oxide carbonates. More preferably, the nanowires comprise Li₂CO₃, LiOH, Li₂O, Li₂C₂O₄, Li₂SO₄, Na₂CO₃, NaOH, Na₂O, Na₂C₂O₄, Na₂SO₄, K₂CO₃, KOH, K₂O, K₂C₂O₄, K₂SO₄, CsCO₃, CsOH, Cs₂O, CsC₂O₄, CsSO₄, Be(OH)₂, BeCO₃, BeO, BeC₂O₄. BeSO₄, Mg(OH)₂, MgCO₃, MgO, MgC₂O₄, MgSO₄, Ca(OH)₂, CaO, Ca(OH)₂, CaC₂O₄, CaSO₄, Y₂O₃, Y₂(CO₃)₃, Y(OH)₃, Y₂(C₂O₄)₃, Y₂(SO₄)₃, Zr(OH)₄, ZrO(OH)₂, ZrO2, Zr(C₂O₄)₂, Zr(SO₄)₂, Ti(OH)₄, TiO(OH)₂, Ti02, Ti(C₂O₄)₂, Ti(SO₄)₂,BaO, Ba(OH)₂, BaCO₃, BaC₂O₄, BaSO₄, La(OH)₃, La₂O₃, La₂(C₂O₄)₃, La₂(SO₄)₃, Ce(OH)₄, CeO₂, Ce₂O₃, Ce(C₂O₄)₂, Ce(SO₄)₂, ThO₂, Th(OH)₄, Th(C₂O₄)₂, Th(SO₄)₂, Sr(OH)₂, SrCO₃, SrO, SrC₂O₄, SrSO₄, Sm₂O₃, Sm(OH)₃, Sm₂(CO₃)₃, Sm₂(C₂O₃)₃, Sm₂(SO₄)₃, LiCa₂Bi₃O₄Cl₆, NaMnO₄, Na₂WO₄, NaMn/WO₄, CoWO₄, CuWO₄, K/SrCoO₃, K/Na/SrCoO₃, Na/SrCoO₃, Li/SrCoO₃, SrCoO₃, Mg₆MnO₈, LiMn₂O₄, Li/Mg₆MnO₈, Na₁₀Mn/W₅O₁₇, Mg₃Mn₃B₂O₁₀, Mg₃(BO₃)₂, molybdenum oxides, molybdenum hydroxides, molybdenum oxalates, molybdenum sulfates, Mn₂O₃, Mn₃O₄, manganese oxides, manganese hydroxides, manganese oxalates, manganese sulfates, manganese tungstates, vanadium oxides, vanadium hydroxides, vanadium oxalates, vanadium sulfates, tungsten oxides, tungsten hydroxides, tungsten oxalates, tungsten sulfates, neodymium oxides, neodymium hydroxides, neodymium oxalates, neodymium sulfates, europium oxides, europium hydroxides, europium oxalates, europium sulfates, praseodymium oxides, praseodymium hydroxides, praseodymium oxalates, praseodymium sulfates, rhenium oxides, rhenium hydroxides, rhenium oxalates, rhenium sulfates, chromium oxides, chromium hydroxides, chromium oxalates, chromium sulfates, potassium molybdenum oxides/silicon oxide or combinations thereof.

Further preferably, the nanowires comprise Li₂O, Na₂O, K₂O, Cs₂O, BeO MgO, CaO, ZrO(OH)₂, ZrO2, TiO₂, TiO(OH)₂, BaO, Y₂O₃, La₂O₃, CeO₂, Ce₂O₃, ThO₂, SrO, Sm₂O₃, Nd₂O₃, Eu₂O₃, Pr₂O₃, LiCa₂Bi₃O₄Cl₆, NaMnO₄, Na₂WO₄, Na/Mn/WO₄, Na/MnWO₄, Mn/WO4, K/SrCoO₃, K/Na/SrCoO₃, K/SrCoO₃, Na/SrCoO₃, Li/SrCoO₃, SrCoO₃, Mg₆MnO₈, Na/B/Mg₆MnO₈, Li/B/Mg₆MnO₈, Zr₂Mo₂O₈, molybdenum oxides, Mn₂O₃, Mn₃O₄, manganese oxides, vanadium oxides, tungsten oxides, neodymium oxides, rhenium oxides, chromium oxides, or combinations thereof.

In still other aspects, the nanowires comprise lanthanide containing perovskites. A perovskite is any material with the same type of crystal structure as calcium titanium oxide (CaTiO₃). Examples of perovskites within the context of the present disclosure include, but are not limited to, LaCoO₃ and La/SrCoO₃.

In other embodiments, the nanowires comprise TiO₂, Sm₂O₃, V₂O₅, MoO₃, BeO, MnO₂, MgO, La₂O₃, Nd₂O₃, Eu₂O₃, ZrO₂, SrO, Na₂WO₄, Mn/WO4, BaCO₃, Mn₂O₃, Mn₃O₄, Mg₆MnO₈, Na/B/Mg₆MnO₈, Li/B/Mg₆MnO₈, NaMnO₄, CaO or combinations thereof. In further embodiments, the nanowires comprise MgO, La₂O₃, Nd2O3, Na₂WO₄, Mn/WO4, Mn₂O₃, Mn₃O₄, Mg₆MnO₈, Na/B/Mg₆MnO₈, Li/B/Mg₆MnO₈ or combinations thereof.

In some embodiments, the nanowires comprises Mg, Ca, La, W, Mn, Mo, Nd, Sm, Eu, Pr, Zr or combinations thereof, and in other embodiments the nanowire comprises MgO, CaO, La₂O₃, Na₂WO₄, Mn₂O₃, Mn₃O₄, Nd₂O₃, Sm₂O₃, Eu₂O₃, Pr₂O₃, Mg₆MnO₈, NaMnO₄, Na/Mn/W/O, Na/MnWO₄, MnWO₄ or combinations thereof.

In more specific embodiments, the nanowires comprise MgO. In other specific embodiments, the nanowires comprise La₂O₃. In other specific embodiments, the nanowires comprise Na₂WO₄ and may optionally further comprise Mn/WO₄. In other specific embodiments, the nanowires comprise Mn₂O₃. In other specific embodiments, the nanowires comprise Mn₃O₄. In other specific embodiments, the nanowires comprise Mg₆MnO₈. In other specific embodiments, the nanowires comprise NaMnO₄. In other specific embodiments, the nanowires comprise Nd₂O₃. In other specific embodiments, the nanowires comprise Eu₂O₃. In other specific embodiments, the nanowires comprise Pr₂O₃.

In certain embodiments, the nanowires comprise an oxide of a group 2 element. For example, in some embodiments, the nanowires comprise an oxide of magnesium. In other embodiments, the nanowires comprise an oxide of calcium. In other embodiments, the nanowires comprise an oxide of strontium. In other embodiments, the nanowires comprise an oxide of barium.

In certain other embodiments, the nanowires comprise an oxide of a group 3 element. For example, in some embodiments, the nanowires comprise an oxide of yttrium. In other embodiments, the nanowires comprise an oxide of scandium.

In yet other certain embodiments, the nanowires comprise an oxide of an early lanthanide element. For example, in some embodiments, the nanowires comprise an oxide of lanthanum. In other embodiments, the nanowires comprise an oxide of cerium. In other embodiments, the nanowires comprise an oxide of praseodymium. In other embodiments, the nanowires comprise an oxide of neodymium. In other embodiments, the nanowires comprise an oxide of promethium. In other embodiments, the nanowires comprise an oxide of samarium. In other embodiments, the nanowires comprise an oxide of europium. In other embodiments, the nanowires comprise an oxide of gandolinium.

In certain other embodiments, the nanowires comprise a lanthanide in the form of an oxide carbonate. For example, the nanowires may comprise Ln₂O₂(CO₃), where Ln represents a lanthanide. Examples in this regard include: La, Ce, Pr, Nd, Pm, Sm, Eu, Gd, Tb, Dy, Ho, Er, Tm, Yb and Lu oxide carbonates. In other embodiments, the nanowires comprise an oxide carbonate of one or more elements from any of Groups 1 through 7, lanthanides, actinides or combinations thereof. Accordingly in one embodiment the nanowires comprise Li, Na, K, Rb, Cs, Fr, Be, Mg, Ca, Sr, Ba, Ra, Sc, Y, Ti, Zr, Hf, V, Nb, Ta, Cr, Mo, W, Mn, Tc or Re oxide carbonate. In other embodiments, the nanowires comprise Ac, Th or Pa oxide carbonate. An oxide carbonate may be represented by the following formula: MₓO_{y}(CO₃)_{z}, wherein M is a metal element from any of Groups 1 through 7, lanthanides or actinides and x, y and z are intergers such that the overall charge of the metal oxide carbonate is neutral.

In other embodiments, the nanowires comprise TiO₂, Sm₂O₃, V₂O₅, MoO₃, BeO, MnO₂, MgO, La₂O₃, ZrO₂, SrO, Na₂WO₄, BaCO₃, Mn₂O₃, Mn₃O₄, Mg₆MnO₈, Na/B/Mg₆MnO₈, Li/B/Mg₆MnO₈, Zr₂Mo₂O₈, NaMnO₄, CaO or combinations thereof and further comprise one or more dopants comprised of metal elements, semi-metal elements, non-metal elements or combinations thereof. In some further embodiments, the nanowires comprise MgO, La₂O₃, Na₂WO₄, Mn₂O₃, Mn₃O₄, Mg₆MnO₈, Zr₂Mo₂O₈, NaMnO₄ or combinations thereof, and the nanowires further comprise Li, Sr, Zr, Ba, Mn or Mn/WO₄.

In some embodiments, the nanowires or a catalytic material comprising a plurality of the nanowires comprise a combination of one or more of metal elements from any of Groups I-7, lanthanides or actinides and one or more of metal elements, semi-metal elements or non-metal elements. For example in one embodiment, the nanowires comprise the combinations of Li/Mg/O, Ba/Mg/O, Zr/La/O, Ba/La/O, Sr/La/O, Zr/V/P/O, Mo/V/Sb/O, V₂O₅/Al₂O₃, Mo/V/O, V/Ce/O, V/Ti/P/O, V₂O₅/TiO₂, V/P/O/TiO₂, V/P/O/Al₂O₃, V/Mg/O, V₂O₅/ZrO₂, Mo/V/Te/O, V/Mo/O/Al₂O₃, Ni/V/Sb/O, Co/V/Sb/O, Sn/V/Sb/O, Bi/V/Sb/O, Mo/V/Te/Nb/O, Mo/V/Nb/O, V₂O₅/MgO/SiO₂, V/Co, MoO₃/Al₂O₃, Ni/Nb/O, NiO/Al₂O₃, Ga/Cr/Zr/P/O, MoO₃/Cl/SiO₂/TiO₂, Co/Cr/Sn/W/O, Cr/Mo/O, MoO₃/Cl/SiO₂/TiO₂, Co/Ca, NiO/MgO, MoO₃/Al₂O₃, Nb/P/Mo/O, Mo/V/Te/Sb//Nb/O, La/Na/Al/O, Ni/Ta/Nb/O, Mo/Mn/V/W/O, Li/Dy/Mg/O, Sr/La/Nd/O, Co/Cr/Sn/W/O, MoO₃/SiO₂/TiO₂, Sm/Na/P/O, Sm/Sr/O, Sr/La/Nd/O, Co/P/O/TiO₂, La/Sr/Fe/Cl/O, La/Sr/Cu/Cl/O, Y/Ba/Cu/O, Na/Ca/O, V₂O₅/ZrO₂, V/Mg/O, Mn/V/Cr/W/O/Al₂O₃, V₂O₅/K/SiO₂, V₂O₅/Ca/TiO₂, V₂O₅/K/TiO₂, V/Mg/Al/O, V/Zr/O, V/Nb/O, V₂O₅/Ga₂O₃, V/Mg/Al/O, V/Nb/O, V/Sb/O, V/Mn/O, V/Nb/O/Sb₂O₄, V/Sb/O/TiO₂, V₂O₅/Ca, V₂O₅/K/Al₂O₃, V₂O₅/TiO₂, V₂O₅/MgO/TiO₂, V₂O₅/ZrO₂, V/Al/F/O, V/Nb/O/TiO₂, Ni/V/O, V₂O₅/SmVO₄, V/W/O, V₂O₅/Zn/Al₂O₃, V₂O₅/CeO₂, V/Sm/O, V₂O₅/TiO₂/SiO₂, Mo/Li/O/Al₂O₃, Mg/Dy/Li/Cl/O, Mg/Dy/Li/Cl/O, Ce/Ni/O, Ni/Mo/O/V, Ni/Mo/O/V/N, Ni/Mo/O Sb/O/N, MoO₃/Cl/SiO₂/TiO₂, Co/Mo/O, Ni/Ti/O, Ni/Zr/O, Cr/O, MoO₃/Al₂O₃, Mn/P/O, MoO₃/K/ZrO₂, Na/W/O, Mn/Na/W/O, Mn/Na//W/O/SiO₂, Na/W/O/SiO₂, Mn/Mo/O, Nb₂O₅/TiO₂, Co/W/O, Ni/Mo/O, Ga/Mo/O, Mg/MoN/O, Cr₂O₃/Al₂O₃, Cr/Mo/Cs/O/Al₂O₃, Co/Sr/O/Ca, Ag/Mo/P/O, MoO₃/SmVO₄, Mo/Mg/Al/O, MoO₃/K/SiO₂/TiO₂, Cr/Mo/O/Al₂O₃, MoO₃/Al₂O₃, Ni/Co/Mo/O, Y/Zr/O, Y/Hf, Zr/Mo/Mn/O, Mg/Mn/O, Li/Mn/O, Mg/Mn/B/O, Mg/B/O, Na/B/Mg/Mn/O, Li/B/Mg/Mn/O, Mn/Na/P/O, Na/Mn/Mg/O, Zr/Mo/O, Mn/W/O or Mg/Mn/O.

In a specific embodiment, the nanowires comprise the combinations of Li/Mg/O, Ba/Mg/O, Zr/La/O, Ba/La/O, Sr/La/O, Sr/Nd/O, La/O, Nd/O, Eu/O, Mg/La/O, Mg/Nd/O, Na/La/O, Na/Nd/O, Sm/O, Mn/Na/W/O, Mg/Mn/O, Na/B/Mg/Mn/O, Li/B/Mg/Mn/O, Zr/Mo/O or Na/Mn/Mg/O. For example, in some embodiments the nanowires comprise the combinations of Li/MgO, Ba/MgO, Sr/La₂O₃, Ba/La₂O₃, Mn/Na₂WO₄, Mn/Na₂WO₄/SiO₂, Mn₂O₃/Na₂WO₄, Mn₃O₄/Na₂WO₄, Li/B/Mg₆MnO₈, Na/B/Mg₆MnO₈ or NaMnO₄/MgO. In certain embodiments, the nanowire comprises Li/MgO, Ba/MgO, Sr/La₂O₃, Mg/Na/La₂O₃, Sr/Nd₂O₃, or Mn/Na₂WO₄.

In some other specific embodiments, the nanowires comprise the combination of Li/MgO. In other specific embodiments, the nanowires comprise the combination of Ba/MgO. In other specific embodiments, the nanowires comprise the combination of Sr/La₂O₃. In other specific embodiments, the nanowires comprise the combination of Ba/La₂O₃. In other specific embodiments, the nanowires comprise the combination of Mn/Na₂WO₄. In other specific embodiments, the nanowires comprise the combination of Mn/Na₂WO₄/SiO₂. In other specific embodiments, the nanowires comprise the combination of Mn₂O₃/Na₂WO₄. In other specific embodiments, the nanowires comprise the combination of Mn₃O₄/Na₂WO₄. In other specific embodiments, the nanowires comprise the combination of Mn/WO₄/Na₂WO₄. In other specific embodiments, the nanowires comprise the combination of Li/B/Mg₆MnO₈. In other specific embodiments, the nanowires comprise the combination of Na/B/Mg₆MnO₈. In other specific embodiments, the nanowires comprise the combination of NaMnO₄/MgO.

Polyoxyometalates (POM) are a class of metal oxides that range in structure from the molecular to the micrometer scale. The unique physical and chemical properties of POM clusters, and the ability to tune these properties by synthetic means have attracted significant interest from the scientific community to create "designer" materials. For example, heteropolyanions such as the well-known Keggin [XM₁₂O₄₀]⁻ and Wells-Dawson [X₂M1₈O₆₂]⁻ anions (where M= W or Mo; and X = a tetrahedral template such as but not limited to Si, Ge, P) and isopolyanions with metal oxide frameworks with general formulas [MOₓ]ₙ where M = Mo, W, V, and Nb and x = 4-7 are ideal candidates for OCM/ODH catalysts. Accordingly, in one embodiment the nanowires comprise [XM₁₂O₄₀]⁻ or [X₂M1₈O₆₂]⁻ anions (where M= W or Mo; and X = a tetrahedral template such as but not limited to Si, Ge, P) and isopolyanions with metal oxide frameworks with general formulas [MOₓ]ₙ where M = Mo, W, V, and Nb and x = 4-7. In some embodiments, X is P or Si.

These POM clusters have "lacunary" sites that can accommodate divalent and trivalent first row transition metals, the metal oxide clusters acting as ligands. These lacunary sites are essentially "doping" sites, allowing the dopant to be dispersed at the molecular level instead of in the bulk which can create pockets of unevenly dispersed doped material. Because the POM clusters can be manipulated by standard synthetic techniques, POMs are highly modular and a wide library of materials can be prepared with different compositions, cluster size, and dopant oxidation state. These parameters can be tuned to yield desired OCM/ODH catalytic properties. Accordingly, preferably, the nanowire comprises one or more POM clusters. Such nanowires find utility as catalysts, for example, in the OCM and ODH reactions.

Silica doped sodium manganese tungstate (NaMn/WO₄/SiO₂) is a promising OCM catalyst. The NaMn/WO₄/SiO₂ system is attractive due to its high C2 selectivity and yield. Unfortunately, good catalytic activity is only achievable at temperatures greater than 800 °C and although the exact active portion of the catalyst is still subject to debate, it is thought that sodium plays an important role in the catalytic cycle. In addition, the NaMn/WO₄/SiO₂ catalyst surface area is relatively low < 2m²/g. Manganese tungstate (Mn/WO₄) nanorods (*i.e*., straight nanowires) can be used to model a NaMn/WO₄/SiO₂ based nanowire OCM catalyst. The Mn/WO₄ nanorods are prepared hydrothermally and the size can be tuned based on reaction conditions with dimensions of 25 - 75 nm in diameter to 200 -800 nm in length. The as-prepared nano-rods have higher surface areas than the NaMn/WO₄/SiO₂ catalyst systems. In addition, the amount of sodium, or other elements, can precisely doped into the Mn/WO₄ nanorod material to target optimal catalytic activity. Nanorod tungstate based materials can be expanded to but, not limited to, CoWO₄ or CuWO₄ materials which may serve as base materials for OCM/ODH catalysis. In addition to straight nanowires, the above discussion applies to the disclosed nanowires having a bent morphology as well. The nanowires of the disclosure may be analyzed by inductively coupled plasma mass spectrometry (ICP-MS) to determine the element content of the nanowires. ICP-MS is a type of mass spectrometry that is highly sensitive and capable of the determination of a range of metals and several non-metals at concentrations below one part in 10¹². ICP is based on coupling together an inductively coupled plasma as a method of producing ions (ionization) with a mass spectrometer as a method of separating and detecting the ions. ICP-MS methods are well known in the art.

In some embodiments, the nanowire comprises a combination of two or more metal compounds, for example metal oxides. For example, in some embodiments, the nanowire comprises Mn₂O₃/Na₂WO₄, Mn₃O₄/Na₂WO₄ MnWO₄/Na₂WO₄/Mn₂O₃, MnWO₄/Na₂WO₄/Mn₃O₄ or NaMnO₄/MgO.

### 3. Catalytic Materials

As noted above, the present disclosure describes a catalytic material comprising a plurality of nanowires. The catalytic material may comprise a support or carrier. The support is preferably porous and has a high surface area.The support may be active (*i.e.* has catalytic activity), or inactive (*i.e.* non-catalytic). It may comprise an inorganic oxide, Al₂O₃, SiO₂, TiO₂, MgO, ZrO₂, ZnO, LiAlO₂, MgAl₂O₄, MnO, MnO₂, Mn₃O₄, La₂O₃, AIP04, SiO₂/Al₂O₃, activated carbon, silica gel, zeolites, activated clays, activated Al₂O₃, diatomaceous earth, magnesia, aluminosilicates, calcium aluminate, support nanowires or combinations thereof. Preferably, the support comprises silicon, for example SiO₂. Or, the support comprises magnesium, for example MgO, zirconium, for example ZrO₂, lanthanum, for example La₂O₃, hafnium, for example HfO₂, aluminum, for example Al₂O₃, gallium, for example Ga₂O₃.

Alternatively, the support material comprises an inorganic oxide, Al₂O₃, SiO₂, TiO₂, MgO, ZrO₂, HfO2, CaO, ZnO, LiAlO₂, MgAl₂O₄, MnO, MnO₂, Mn₂O₄, Mn₃O₄, La₂O₃, activated carbon, silica gel, zeolites, activated clays, activated Al₂O₃, diatomaceous earth, magnesia, aluminosilicates, calcium aluminate, support nanowires or combinations thereof. For example, the support material may comprise SiO₂, ZrO₂, CaO, La₂O₃ or MgO.

Also, a nanowire may serve as a support for another nanowire. For example, a nanowire may be comprised of non-catalytic metal elements and adhered to or incorporated within the support nanowire is a catalytic nanowire. For example, the support nanowires are comprised of SiO₂, MgO, TiO₂, ZrO₂, Al₂O₃, or ZnO. Preparation of nanowire supported nanowire catalysts (*i.e.*, core/shell nanowires) is discussed in more detail below. The optimum amount of nanowire present on the support depends, *inter alia,* on the catalytic activity of the nanowire. The amount may range from 1 to 100 parts by weight nanowires per 100 parts by weight of support or from 10 to 50 parts by weight nanowires per 100 parts by weight of support. Alternatively, the amount of nanowire present on the support ranges from 100-200 parts of nanowires per 100 parts by weight of support, or 200-500 parts of nanowires per 100 parts by weight of support, or 500-1000 parts of nanowires per 100 parts by weight of support.

Typically, heterogeneous catalysts are used either in their pure form or blended with inert materials, such as silica, alumina, etc. The blending with inert materials is used in order to reduce and/or control large temperature non-uniformities within the reactor bed often observed in the case of strongly exothermic (or endothermic) reactions. In the case of complex multistep reactions, such as the reaction to convert methane into ethylene (OCM), typical blending materials can selectively slow down or quench one or more of the reactions of the system and promote unwanted side reactions. For example, in the case of the oxidative coupling of methane, silica and alumina can quench the methyl radicals and thus prevent the formation of ethane. Accordingly, the catalytic activity of the catalytic material can be tuned by blending two or more catalysts and/or catalyst support materials. The blended catalytic material may comprise a catalytic nanowire as described herein and a bulk catalyst material and/or inert support material.

The blended catalytic materials comprise metal oxides, hydroxides, oxy-hydroxides, carbonates, oxalates of the groups 1-16, lanthanides, actinides or combinations thereof. For example, the blended catalytic materials may comprise a plurality of inorganic catalytic polycrystalline nanowires, as disclosed herein, and any one or more of straight nanowires, nanoparticles, bulk materials and inert support materials. Bulk materials are defined as any material in which no attempt to control the size and/or morphology was performed during its synthesis. The catalytic materials may be undoped or may be doped with any of the dopants described herein.

### 4. Preparation of Catalytic Materials

The catalytic materials can be prepared according to any number of methods known in the art. For example, the catalytic materials can be prepared after preparation of the individual components by mixing the individual components in their dry form, e.g. blend of powders, and optionally, ball milling can be used to reduce particle size and/or increase mixing. Each component can be added together or one after the other to form layered particles. Alternatively, the individual components can be mixed prior to calcination, after calcination or by mixing already calcined components with uncalcined components. The catalytic materials may also be prepared by mixing the individual components in their dry form and optionally pressing them together into a "pill" followed by calcination to above 400 °C.

In other examples, the catalytic materials are prepared by mixing the individual components with one or more solvents into a suspension or slurry, and optional mixing and/or ball milling can be used to maximize uniformity and reduce particle size. Examples of slurry solvents useful in this context include, but are not limited to: water, alcohols, ethers, carboxylic acids, ketones, esters, amides, aldehydes, amines, alkanes, alkenes, alkynes, or aromatics . Alternatively, the individual components are deposited on a supporting material such as silica, alumina, magnesia, activated carbon, or by mixing the individual components using a fluidized bed granulator. Combinations of any of the above methods may also be used.

The catalytic materials may optionally comprise a dopant as described in more detail below. In this respect, doping material(s) may be added during preparation of the individual components, after preparation of the individual components but before drying of the same, after the drying step but before calcinations or after calcination. If more than one doping material is used, each dopant can be added together or one after the other to form layers of dopants.

Doping material(s) may also be added as dry components and optionally ball milling can be used to increase mixing. Alternatively, doping material(s) are added as a liquid (e.g. solution, suspension, slurry) to the dry individual catalyst components or to the blended catalytic material. The amount of liquid may optionally be adjusted for optimum wetting of the catalyst, which can result in optimum coverage of catalyst particles by doping material. Mixing and/or ball milling can also be used to maximize doping coverage and uniform distribution. Alternatively, doping material(s) are added as a liquid (e.g. solution, suspension, slurry) to a suspension or slurry of the catalyst in a solvent. Mixing and/or ball milling can be used to maximize doping coverage and uniform distribution. Incorporation of dopants can also be achieved using any of the methods described elsewhere herein.

As noted below, an optional calcination step usually follows an optional drying step at T < 200C (typically 60-120C) in a regular oven or in a vacuum oven. Calcination may be performed on the individual components of the catalytic material or on the blended catalytic material. Calcination is generally performed in an oven/furnace at a temperature higher than the minimum temperature at which at least one of the components decomposes or undergoes a phase transformation and can be performed in inert atmosphere (e.g. N₂, Ar, He) oxidizing atmosphere (air, O₂) or reducing atmosphere (H₂, H₂/N₂, H₂/Ar). The atmosphere may be a static atmosphere or a gas flow and may be performed at ambient pressure, at p < 1atm, in vacuum or at p > 1atm. High pressure treatment (at any temperature) may also be used to induce phase transformation including amorphous to crystalline.

Calcination is generally performed in any combination of steps comprising ramp up, dwell and ramp down. For example, ramp to 500 °C, dwell at 500 °C for 5h, ramp down to RT. Another example includes ramp to 100 °C, dwell at 100 °C for 2h, ramp to 300 °C, dwell at 300 °C for 4h, ramp to 550 °C, dwell at 550 °C for 4h, ramp down to RT. Calcination conditions (pressure, atmosphere type) can be changed during the calcination. In some embodiments, calcination is performed before preparation of the blended catalytic material (i.e., individual components are calcined), after preparation of the blended catalytic material but before doping, after doping of the individual components or blended catalytic material. Calcination may also be performed multiple times, e.g. after catalyst preparation and after doping.

The catalytic materials may incorporated into a reactor bed for performing any number of catalytic reactions (*e.g*., OCM, ODH). In this regard, the catalytic material may be packed neat (without diluents) or diluted with an inert material (*e.g*., sand, silica, alumina) The catalyst components may be packed uniformly forming a homogeneous reactor bed.

The particle size of the individual components within a catalytic material may also alter the catalytic activity, and other properties, of the same. Accordingly, in one embodiment, the catalyst is milled to a target average particle size or the catalyst powder is sieved to select a particular particle size. In some aspects, the catalyst powder may be pressed into pellets and the catalyst pellets can be optionally milled and or sieved to obtain the desired particle size distribution.

Alternatively, the catalysts are packed in bands forming a layered reactor bed. Each layer is composed by either a catalyst of a particular type, morphology or size or a particular blend of catalysts.

Described is also a catalytic material comprising one or more different catalysts. The catalysts may be a nanowire as disclosed herein and a different catalyst for example a bulk catalysts. Mixture of two or more nanowire catalysts are also contemplated. The catalytic material may comprise a catalyst, for example a nanowire catalyst, having good OCM activity and a catalyst having good activity in the ODH reaction. Either one or both of these catalysts may be nanowires as disclosed herein.

### 5. Dopants

The disclosure describes nanowires comprising a dopant (*i.e.,* doped nanowires). As noted above, dopants or doping agents are impurities added to or incorporated within a catalyst to optimize catalytic performance (*e.g*., increase or decrease catalytic activity). As compared to the undoped catalyst, a doped catalyst may increase or decrease the selectivity, conversion, and/or yield of a catalytic reaction. In one embodiment, nanowire dopants comprise one or more metal elements, semi-metal elements, non-metal elements or combinations thereof. The dopant may be present in any form and may be derived from any suitable source of the element (e.g., chlorides, nitrates). In some embodiments, the nanowire dopant is in elemental form. In other embodiments, the nanowire dopant is in reduced or oxidized form. In other embodiments, the nanowire dopant comprises an oxide, hydroxide, carbonate, nitrate, acetate, sulfate, formate, oxynitrate, halide, oxyhalide or hydroxyhalide of a metal element, semi-metal element or non-metal element or combinations thereof.

In one embodiment, the nanowires comprise one or more metal elements selected from Groups I-7, lanthanides, actinides or combinations thereof in the form of an oxide and further comprise one or more dopants, wherein the one or more dopants comprise metal elements, semi-metal elements, non-metal elements or combinations thereof. In another embodiment, the nanowires comprise one or more metal elements selected from group 1 in the form of an oxide and further comprise one or more dopants, wherein the one or more dopants comprise metal elements, semi-metal elements, non-metal elements or combinations thereof. In another embodiment, the nanowires comprise one or more metal elements selected from group 2 in the form of an oxide and further comprise one or more dopants, wherein the one or more dopants comprise metal elements, semi-metal elements, non-metal elements or combinations thereof. In another embodiment, the nanowires comprise one or more metal elements selected from group 3 in the form of an oxide and further comprise one or more dopants, wherein the one or more dopants comprise metal elements, semi-metal elements, non-metal elements or combinations thereof. In another embodiment, the nanowires comprise one or more metal elements selected from group 4 in the form of an oxide and further comprise one or more dopants, wherein the one or more dopants comprise metal elements, semi-metal elements, non-metal elements or combinations thereof. In another embodiment, the nanowires comprise one or more metal elements selected from group V in the form of an oxide and further comprise one or more dopants, wherein the one or more dopants comprise metal elements, semi-metal elements, non-metal elements or combinations thereof. In another embodiment, the nanowires comprise one or more metal elements selected from group 6 in the form of an oxide and further comprise one or more dopants, wherein the one or more dopants comprise metal elements, semi-metal elements, non-metal elements or combinations thereof. In another embodiment, the nanowires comprise one or more metal elements selected from group 7 in the form of an oxide and further comprise one or more dopants, wherein the one or more dopants comprise metal elements, semi-metal elements, non-metal elements or combinations thereof. In another embodiment, the nanowires comprise one or more metal elements selected from lanthanides in the form of an oxide and further comprise one or more dopants, wherein the one or more dopants comprise metal elements, semi-metal elements, non-metal elements or combinations thereof. In another embodiment, the nanowires comprise one or more metal elements selected from actinides in the form of an oxide and further comprise one or more dopants, wherein the one or more dopants comprise metal elements, semi-metal elements, non-metal elements or combinations thereof.

For example, in one embodiment, the nanowire dopant comprises Li, Li₂CO₃, LiOH, Li₂O, LiCl, LiNO₃, Na, Na₂CO₃, NaOH, Na₂O, NaCl, NaNO₃, K, K₂CO₃, KOH, K₂O, KCI, KNO₃, Rb, Rb₂CO₃, RbOH, Rb₂O, RbCl, RbNO₃, Mg, MgCO₃, Mg(OH)₂, MgO, MgCl₂, Mg(NO₃)₂, Ca, CaO, CaCO₃, Ca(OH)₂, CaCl₂, Ca(NO₃)₂, Sr, SrO, SrCO₃, Sr(OH)₂, SrCl₂, Sr(NO₃)₂, Ba, BaO, BaCO₃, Ba(OH)₂, BaCl₂, Ba(NO₃)₂, La, La₂O₃, La(OH)₃, LaCl₃, La(NO₃)₂, Nb, Nb₂O₃, Nb(OH)₃, NbCl₃, Nb(NO₃)₂, Sm, Sm₂O₃, Sm(OH)₃, SmCl₃, Sm(NO₃)₂, Eu, Eu₂O₃, Eu(OH)₃, EuCl₃, Eu(NO₃)₂, Gd, Gd₂O₃, Gd(OH)₃, GdCl₃, Gd(NO₃)₂, Ce, Ce(OH)₄, CeO₂, Ce₂O₃, CeCl₄, Ce(NO₃)₂, Th, ThO₂, ThCl₄, Th(OH)₄, Zr, ZrO₂, ZrCl₄, Zr(OH)₄, ZrOCl₂, ZrO(NO₃)₂, P, phosphorous oxides, phosphorous chlorides, phosphorous carbonates, Ni, nickel oxides, nickel chlorides, nickel carbonates, nickel hydroxides, Nb, niobium oxides, niobium chlorides, niobium carbonates, niobium hydroxides, Au, gold oxides, gold chlorides, gold carbonates, gold hydroxides, Mo, molybdenum oxides, molybdenum chlorides, molybdenum carbonates, molybdenum hydroxides, tungsten chlorides, tungsten carbonates, tungsten hydroxides, Cr, chromium oxides, chromium chlorides, chromium hydroxides, Mn, manganese oxides, manganese chlorides, manganese hydroxides, Zn, ZnO, ZnCl₂, Zn(OH)₂, B, borates, BCl₃, N, nitrogen oxides, nitrates, La, Ce, Pr, Nd, Pm, Sm, Eu, Gd, Tb, Dy, Ho, Er, Tm, Yb, Lu, In, Y, Sc, Al, Cu, Cs, Ga, Hf, Fe, Ru, Rh, Be, Co, Sb, V, Ag, Te, Pd, Tb, Ir, Rb or combinations thereof. In other embodiments, the nanowire dopant comprises Na, Eu, In, Nd, Sm, Ce, Gd, Y, Sc or combinations thereof.

In other embodiments, the nanowire dopant comprises Li, Li₂O, Na, Na₂O, K, K₂O, Mg, MgO, Ca, CaO, Sr, SrO, Ba, BaO, La, La₂O₃, Ce, CeO₂, Ce₂O₃, Th, ThO₂, Zr, ZrO₂, P, phosphorous oxides, Ni, nickel oxides, Nb, niobium oxides, Au, gold oxides, Mo, molybdenum oxides, Cr, chromium oxides, Mn, manganese oxides, Zn, ZnO, B, borates, N, nitrogen oxides or combinations thereof. In other embodiments, the nanowire dopant comprises Li, Na, K, Mg, Ca, Sr, Ba, La, Ce, Th, Zr, P, Ni, Nb, Au, Mo, Cr, Mn, Zn, B, N or combinations thereof. In other embodiments, the nanowire dopant comprises Li₂O, Na₂O, K₂O, MgO, CaO, SrO, BaO, La₂O₃, CeO₂, Ce₂O₃, ThO₂, ZrO₂, phosphorous oxides, nickel oxides, niobium oxides, gold oxides, molybdenum oxides, chromium oxides, manganese oxides, ZnO, borates, nitrogen oxides or combinations thereof. In further embodiments, the dopant comprises Sr or Li. In other specific embodiments, the nanowire dopant comprises La, Ce, Pr, Nd, Pm, Sm, Eu, Gd, Tb, Dy, Ho, Er, Tm, Yb, Lu, In, Y, Sc or combinations thereof. In other specific embodiments, the nanowire dopant comprises Li, Na, K, Mg, Ca, Ba, Sr, Eu, Sm, Co or Mn.

In certain embodiments, the dopant comprises an element from group 1. In some embodiments, the dopant comprises lithium. In some embodiments, the dopant comprises sodium. In some embodiments, the dopant comprises potassium. In some embodiments, the dopant comprises rubidium. In some embodiments, the dopant comprises caesium.

In some embodiments the nanowires comprise a lanthanide element and are doped with a dopant from group 1, group 2, or combinations thereof. For example, in some embodiments, the nanowires comprise a lanthanide element and are doped with lithium. In other embodiments, the nanowires comprise a lanthanide element and are doped with sodium. In other embodiments, the nanowires comprise a lanthanide element and are doped with potassium. In other embodiments, the nanowires comprise a lanthanide element and are doped with rubidium. In other embodiments, the nanowires comprise a lanthanide element and are doped with caesium. In other embodiments, the nanowires comprise a lanthanide element and are doped with beryllium. In other embodiments, the nanowires comprise a lanthanide element and are doped with magnesium. In other embodiments, the nanowires comprise a lanthanide element and are doped with calcium. In other embodiments, the nanowires comprise a lanthanide element and are doped with strontium. In other embodiments, the nanowires comprise a lanthanide element and are doped with barium.

In some embodiments the nanowires comprise a transition metal tungstate (e.g., Mn/W) and are doped with a dopant from group 1, group 2, or combinations thereof. For example, in some embodiments, the nanowires comprise a transition metal tungstate and are doped with lithium. In other embodiments, the nanowires comprise a transition metal tungstate and are doped with sodium. In other embodiments, the nanowires comprise a transition metal tungstate and are doped with potassium. In other embodiments, the nanowires comprise a transition metal tungstate and are doped with rubidium. In other embodiments, the nanowires comprise a transition metal tungstate and are doped with caesium. In other embodiments, the nanowires comprise a transition metal tungstate and are doped with beryllium. In other embodiments, the nanowires comprise a transition metal tungstate and are doped with magnesium. In other embodiments, the nanowires comprise a transition metal tungstate and are doped with calcium. In other embodiments, the nanowires comprise a transition metal tungstate and are doped with strontium. In other embodiments, the nanowires comprises a transition metal tungstate and are doped with barium.

In some embodiments the nanowires comprise Mn/Mg/O and are doped with a dopant from group 1, group 2, group 7, group 8, group 9 or group 10 or combinations thereof. For example, in some embodiments, the nanowires comprise Mn/Mg/O and are doped with lithium. In other embodiments, the nanowires comprise Mn/Mg/O and are doped with sodium. In other embodiments, the nanowires comprise Mn/Mg/O and are doped with potassium. In other embodiments, the nanowires comprise Mn/Mg/O and are doped with rubidium. In other embodiments, the nanowires comprise Mn/Mg/O and are doped with caesium. In other embodiments, the nanowires comprise Mn/Mg/O and are doped with beryllium. In other embodiments, the nanowires comprise Mn/Mg/O and are doped with magnesium. In other embodiments, the nanowires comprise Mn/Mg/O and are doped with calcium. In other embodiments, the nanowires comprise Mn/Mg/O and are doped with strontium. In other embodiments, the nanowires comprise Mn/Mg/O and are doped with barium.

In yet some other embodiments, the nanowires comprise Mn/Mg/O and are doped with manganese. In other embodiments, the nanowires comprise Mn/Mg/O and are doped with technetium. In other embodiments, the nanowires comprise Mn/Mg/O and are doped with rhenium. In other embodiments, the nanowires comprise Mn/Mg/O and are doped with bohrium. In other embodiments, the nanowires comprise Mn/Mg/O and are doped with iron. In other embodiments, the nanowires comprise Mn/Mg/O and are doped with ruthenium. In other embodiments, the nanowires comprise Mn/Mg/O and are doped with osmium. In other embodiments, the nanowires comprise Mn/Mg/O and are doped with hassium. In other embodiments, the nanowires comprise Mn/Mg/O and are doped with cobalt. In other embodiments, the nanowires comprise Mn/Mg/O and are doped with rhodium. In other embodiments, the nanowires comprise Mn/Mg/O and are doped with iridium. In other embodiments, the nanowires comprise Mn/Mg/O and are doped with meitnerium. In other embodiments, the nanowires comprise Mn/Mg/O and are doped with nickel. In other embodiments, the nanowires comprise Mn/Mg/O and are doped with palladium. In other embodiments, the nanowires comprise Mn/Mg/O and are doped with platinum. In other embodiments, the nanowires comprise Mn/Mg/O and are doped with darmistadtium.

It is contemplated that any one or more of the dopants disclosed herein can be combined with any one of the nanowires disclosed herein to form a doped nanowire comprising one, two, three or more dopants. Tables 1-8 below show exemplary doped nanowires. In some embodiments, the doped nanowires shown in tables 1-8 are doped with one, two, three or more additional dopants.

**TABLE 1 NANOWIRES (NW) DOPED WITH SPECIFIC DOPANTS (DOP)**

| **NW\Dop** | **Li** | **Na** | **K** | **Rb** | **Cs** | **Be** | **Mg** | **Ca** |
|---|---|---|---|---|---|---|---|---|
| **Li₂O** | Li/ Li₂O | Na/ Li₂O | K/ Li₂O | Rb/ Li₂O | Cs/ Li₂O | Be/ Li₂O | Mg/ Li₂O | Ca/ Li₂O |
| **Na₂O** | Li/ Na₂O | Na/ Na₂O | K/ Na₂O | Rb/ Na₂O | Cs/ Na₂O | Be/ Na₂O | Mg/ Na₂O | Ca/ Na₂O |
| **K₂O** | Li/ K₂O | Na/ K₂O | K/ K₂O | Rb/ K₂O | Cs/ K₂O | Be/ K₂O | Mg/ K₂O | Ca/ K₂O |
| **Rb₂O** | Li/ Rb₂O | Na/ Rb₂O | K/ Rb₂O | Rb/ Rb₂O | Cs/ Rb₂O | Be/ Rb₂O | Mg/ Rb₂O | Ca/ Rb₂O |
| **Cs₂O** | Li/ Cs₂O | Na/ Cs₂O | K/ Cs₂O | Rb/ Cs₂O | Cs/ Cs₂O | Be/ Cs₂O | Mg/ Cs₂O | Ca/ Cs₂O |
| **BeO** | Li/ BeO | Na/ BeO | K/ BeO | Rb/ BeO | Cs/ BeO | Be/ BeO | Mg/ BeO | Ca/ BeO |
| **MgO** | Li/ MgO | Na/ MgO | K/ MgO | Rb/ MgO | Cs/ MgO | Be/ MgO | Mg/ MgO | Ca/ MgO |
| **CaO** | Li/ CaO | Na/ CaO | K/ CaO | Rb/ CaO | Cs/ CaO | Be/ CaO | Mg/ CaO | Ca/ CaO |
| **SrO** | Li/ SrO | Na/ SrO | K/ SrO | Rb/ SrO | Cs/ SrO | Be/ SrO | Mg/ SrO | Ca/ SrO |
| **BaO** | Li/ BaO | Na/ BaO | K/ BaO | Rb/ BaO | Cs/ BaO | Be/ BaO | Mg/ BaO | Ca/ BaO |
| **Sc₂O₃** | Li/ SC₂O₃ | Na/ Sc₂O₃ | K/ SC₂O₃ | Rb/ SC₂O₃ | Cs/ SC₂O₃ | Be/ SC₂O₃ | Mg/ SC₂O₃ | Ca/ SC₂O₃ |
| **Y₂O₃** | Li/ Y₂O₃ | Na/ Y₂O₃ | K/ Y₂O₃ | Rb/ Y₂O₃ | Cs/ Y₂O₃ | Be/ Y₂O₃ | Mg/ Y₂O₃ | Ca/ Y₂O₃ |
| **La₂O₃** | Li/ La₂O₃ | Na/ La₂O₃ | K/ La₂O₃ | Rb/ La₂O₃ | Cs/ La₂O₃ | Be/ La₂O₃ | Mg/ La₂O₃ | Ca/ La₂O₃ |
| **CeO₂** | Li/ CeO₂ | Na/ CeO₂ | K/ CeO₂ | Rb/ CeO₂ | Cs/ CeO₂ | Be/ CeO₂ | Mg/ CeO₂ | Ca/ CeO₂ |
| **Ce₂O₃** | Li/ Ce₂O₃ | Na/ Ce₂O₃ | K/ Ce₂O₃ | Rb/ Ce₂O₃ | Cs/ Ce₂O₃ | Be/ Ce₂O₃ | Mg/ Ce₂O₃ | Ca/ Ce₂O₃ |
| **Pr₂O₃** | Li/ Pr₂O₃ | Na/ Pr₂O₃ | K/ Pᵣ₂O₃ | Rb/ Pr₂O₃ | Cs/ Pr₂O₃ | Be/ Pr₂O₃ | Mg/ Pr₂O₃ | Ca/ Pr₂O₃ |
| **Nd₂O₃** | Li/ Nd₂O₃ | Na/ Nd₂O₃ | K/ Nd₂O₃ | Rb/ Nd₂O₃ | Cs/ Nd₂O₃ | Be/ Nd₂O₃ | Mg/ Nd₂O₃ | Ca/ Nd₂O₃ |
| **Sm₂O₃** | Li/ Sm₂O₃ | Na/ Sm₂O₃ | K/ Sm₂O₃ | Rb/ Sm₂O₃ | Cs/ Sm₂O₃ | Be/ Sm₂O₃ | Mg/ Sm₂O₃ | Ca/ Sm₂O₃ |
| **EU₂O₃** | Li/ EU₂O₃ | Na/ Eu₂O₃ | K/ EU₂O₃ | Rb/ EU₂O₃ | Cs/ EU₂O₃ | Be/ EU₂O₃ | Mg/ EU₂O₃ | Ca/ EU₂O₃ |
| **Gd₂O₃** | Li/ Gd₂O₃ | Na/ Gd₂O₃ | K/ Gd₂O₃ | Rb/ Gd₂O₃ | Cs/ Gd₂O₃ | Be/ Gd₂O₃ | Mg/ Gd₂O₃ | Ca/ Gd₂O₃ |
| **Tb₂O₃** | Li/ Tb₂O₃ | Na/ Tb₂O₃ | K/ Tb₂O₃ | Rb/ Tb₂O₃ | Cs/ Tb₂O₃ | Be/ Tb₂O₃ | Mg/ Tb₂O₃ | Ca/ Tb₂O₃ |
| **TbO₂** | Li/ TbO₂ | Na/ TbO₂ | K/ TbO₂ | Rb/ TbO₂ | Cs/ TbO₂ | Be/ TbO₂ | Mg/ TbO₂ | Ca/ TbO₂ |
| **Tb₆O₁₁** | Li/ Tb₆O₁₁ | Na/ Tb₆O₁₁ | K/ Tb₆O₁₁ | Rb/ Tb₆O₁₁ | Cs/ Tb₆O₁₁ | Be/ Tb₆O₁₁ | Mg/ Tb₆O₁₁ | Ca/ Tb₆O₁₁ |
| **Dy₂O₃** | Li/ Dy₂O₃ | Na/ Dy₂O₃ | K/ Dy₂O₃ | Rb/ Dy₂O₃ | Cs/ Dy₂O₃ | Be/ Dy₂O₃ | Mg/ Dy₂O₃ | Ca/ Dy₂O₃ |
| **Ho₂O₃** | Li/ HO₂O₃ | Na/ Ho₂O₃ | K/ Ho₂O₃ | Rb/ Ho₂O₃ | Cs/ Ho₂O₃ | Be/ Ho₂O₃ | Mg/ Ho₂O₃ | Ca/ Ho₂O₃ |
| **Er₂O₃** | Li/ Er₂O₃ | Na/ Er₂O₃ | K/ Er₂O₃ | Rb/ Er₂O₃ | Cs/ Er₂O₃ | Be/ Er₂O₃ | Mg/ Er₂O₃ | Ca/ Er₂O₃ |
| **Tm₂O₃** | Li/ Tm₂O₃ | Na/ Tm₂O₃ | K/ Tm₂O₃ | Rb/ Tm₂O₃ | Cs/ Tm₂O₃ | Be/ Tm₂O₃ | Mg/ Tm₂O₃ | Ca/ Tm₂O₃ |
| **Yb₂O₃** | Li/ Yb₂O₃ | Na/ Yb₂O₃ | K/ Yb₂O₃ | Rb/ Yb₂O₃ | Cs/ Yb₂O₃ | Be/ Yb₂O₃ | Mg/ Yb₂O₃ | Ca/ Yb₂O₃ |
| **Lu₂O₃** | Li/ Lu₂O₃ | Na/ Lu₂O₃ | K/ Lu₂O₃ | Rb/ Lu₂O₃ | Cs/ Lu₂O₃ | Be/ Lu₂O₃ | Mg/ Lu₂O₃ | Ca/ Lu₂O₃ |
| **AC₂O₃** | Li/ AC₂O₃ | Na/ Ac₂O₃ | K/ Ac₂O₃ | Rb/ AC₂O₃ | Cs/ Ac₂O₃ | Be/ AC₂O₃ | Mg/ AC₂O₃ | Ca/ AC₂O₃ |
| **Th₂O₃** | Li/ Th₂O₃ | Na/ Th₂O₃ | K/ Th₂O₃ | Rb/ Th₂O₃ | Cs/ Th₂O₃ | Be/ Th₂O₃ | Mg/ Th₂O₃ | Ca/ Th₂O₃ |
| **ThO₂** | Li/ ThO₂ | Na/ ThO₂ | K/ ThO₂ | Rb/ ThO₂ | Cs/ ThO₂ | Be/ ThO₂ | Mg/ ThO₂ | Ca/ ThO₂ |
| **Pa₂O₃** | Li/ Pa₂O₃ | Na/ Pa₂O₃ | K/ Pa₂O₃ | Rb/ Pa₂O₃ | Cs/ Pa₂O₃ | Be/ Pa₂O₃ | Mg/ Pa₂O₃ | Ca/ Pa₂O₃ |
| **PaO₂** | Li/ PaO₂ | Na/ PaO₂ | K/ PaO₂ | Rb/ PaO₂ | Cs/ PaO₂ | Be/ PaO₂ | Mg/ PaO₂ | Ca/ PaO₂ |
| **TiO₂** | Li/ TiO₂ | Na/ TiO₂ | K/ TiO₂ | Rb/ TiO₂ | Cs/ TiO₂ | Be/ TiO₂ | Mg/ TiO₂ | Ca/ TiO₂ |
| **TiO** | Li/ TiO | Na/ TiO | K/ TiO | Rb/ TiO | Cs/ TiO | Be/ TiO | Mg/ TiO | Ca/ TiO |
| **Ti₂O₃** | Li/ Ti₂O₃ | Na/ Ti₂O₃ | K/ Ti₂O₃ | Rb/ Ti₂O₃ | Cs/ Ti₂O₃ | Be/ Ti₂O₃ | Mg/ Ti₂O₃ | Ca/ Ti₂O₃ |
| **Ti₃O** | Li/ Ti₃O | Na/ Ti₃O | K/ Ti₃O | Rb/ Ti₃O | Cs/ Ti₃O | Be/ Ti₃O | Mg/ Ti₃O | Ca/ Ti₃O |
| **Ti₂O** | Li/ Ti₂O | Na/ Ti₂O | K/ Ti₂O | Rb/ Ti₂O | Cs/ Ti₂O | Be/ Ti₂O | Mg/ Ti₂O | Ca/ Ti₂O |
| **Ti₃O₅** | Li/ Ti₃O₅ | Na/ Ti₃O₅ | K/ Ti₃O₅ | Rb/ Ti₃O₅ | Cs/ Ti₃O₅ | Be/ Ti₃O₅ | Mg/ Ti₃O₅ | Ca/ Ti₃O₅ |
| **Ti₄O₇** | Li/ Ti₄O₇ | Na/ Ti₄O₇ | K/ Ti₄O₇ | Rb/ Ti₄O₇ | Cs/ Ti₄O₇ | Be/ Ti₄O₇ | Mg/ Ti₄O₇ | Ca/ Ti₄O₇ |
| **ZrO₂** | Li/ ZrO₂ | Na/ ZrO₂ | K/ ZrO₂ | Rb/ ZrO₂ | Cs/ ZrO₂ | Be/ ZrO₂ | Mg/ ZrO₂ | Ca/ ZrO₂ |
| **HfO₂** | Li/ HfO₂ | Na/ HfO₂ | K/ HfO₂ | Rb/ HfO₂ | Cs/ HfO₂ | Be/ HfO₂ | Mg/ HfO₂ | Ca/ HfO₂ |
| **VO** | Li/ VO | Na/ VO | K/ VO | Rb/ VO | Cs/ VO | Be/ VO | Mg/ VO | Ca/ VO |
| **V₂O₃** | Li/ V₂O₃ | Na/ V₂O₃ | K/ V₂O₃ | Rb/ V₂O₃ | Cs/ V₂O₃ | Be/ V₂O₃ | Mg/ V₂O₃ | Ca/ V₂O₃ |
| **VO₂** | Li/ VO₂ | Na/ VO₂ | K/ VO₂ | Rb/ VO₂ | Cs/ VO₂ | Be/ VO₂ | Mg/ VO₂ | Ca/ VO₂ |
| **V₂O₅** | Li/ V₂O₅ | Na/ V₂O₅ | K/ V₂O₅ | Rb/ V₂O₅ | Cs/ V₂O₅ | Be/ V₂O₅ | Mg/ V₂O₅ | Ca/ V₂O₅ |
| **V₃O₇** | Li/ V₃O₇ | Na/ V₃O₇ | K/ V₃O₇ | Rb/ V₃O₇ | Cs/ V₃O₇ | Be/ V₃O₇ | Mg/ V₃O₇ | Ca/ V₃O₇ |
| **V₄O₉** | Li/ V₄O₉ | Na/ V₄O₉ | K/ V₄O₉ | Rb/ V₄O₉ | Cs V₄O₉ | Be/ V₄O₉ | Mg/ V₄O₉ | Ca/ V₄O₉ |
| **V₆O₁₃** | Li/ V₆O₁₃ | Na/ V₆O₁₃ | K/ V₆O₁₃ | Rb/ V₆O₁₃ | Cs/ V₆O₁₃ | Be/ V₆O₁₃ | Mg/ V₆O₁₃ | Ca/ V₆O₁₃ |
| **NbO** | Li/ NbO | Na/ NbO | K/ NbO | Rb/ NbO | Cs/ NbO | Be/ NbO | Mg/ NbO | Ca/ NbO |
| **NbO₂** | Li/ NbO₂ | Na/ NbO₂ | K/ NbO₂ | Rb/ NbO₂ | Cs/ NbO₂ | Be/ NbO₂ | Mg/ NbO₂ | Ca/ NbO₂ |
| **Nb₂O₅** | Li/ Nb₂O₅ | Na/ Nb₂O₅ | K/ Nb₂O₅ | Rb/ Nb₂O₅ | Cs/ Nb₂O₅ | Be/ Nb₂O₅ | Mg/ Nb₂O₅ | Ca/ Nb₂O₅ |
| **Nb₈O₁₉** | Li/ Nb₈O₁₉ | Na/ Nb₈O₁₉ | K/ Nb₈O₁₉ | Rb/ Nb₈O₁₉ | Cs/ Nb₈O₁₉ | Be/ Nb₈O₁₉ | Mg/ Nb₈O₁₉ | Ca/ Nb₈O₁₉ |
| **Nb₁₆O₃₈** | Li/ Nb₁₆O₃₈ | Na/ Nb₁₆O₃₈ | K/ Nb₁₆O₃₈ | Rb/ Nb₁₆O_{3S} | Cs/ Nb₁₆O₃₈ | Be/ Nb₁₆O₃₈ | Mg/ Nb₁₆O₃₈ | Ca/ Nb₁₆O₃₆ |
| **Nb₁₂O₂₉** | Li/ Nb₁₂O₂₉ | Na/ Nb₁₂O₂₉ | K/ Nb₁₂O₂₉ | Rb/ Nb₁₂O₂₉ | Cs/ Nb₁₂O₂₉ | Be/ Nb₁₂O₂₉ | Mg/ Nb₁₂O₂₉ | Ca/ Nb₁₂O₂₉ |
| **Nb₄₇O₁₁₆** | Li/ Nb₄₇O₁₁₆ | Na/ Nb₄₇O₁₁₆ | K/ Nb₄₇O₁₁₆ | Rb/ Nb₄₇O₁₁₆ | Cs/ Nb₄₇O₁₁₆ | Be/ Nb₄₇O₁₁₆ | Mg/ Nb₄₇O₁₁₆ | Ca/ Nb₄₇O₁₁₆ |
| **Ta₂O₅** | Li/ Ta₂O₅ | Na/ Ta₂O₅ | K/ Ta₂O₅ | Rb/ Ta₂O₅ | Cs/ Ta₂O₅ | Be/ Ta₂O₅ | Mg/ Ta₂O₅ | Ca/ Ta₂O₅ |
| **CrO** | Li/ CrO | Na/ CrO | K/ CrO | Rb/ CrO | Cs/ CrO | Be/ CrO | Mg/ CrO | Ca/ CrO |
| **Cr₂O₃** | Li/ Cr₂O₃ | Na/ Cr₂O₃ | K/ Cr₂O₃ | Rb/ Cr₂O₃ | Cs/ Cr₂O₃ | Be/ Cr₂O₃ | Mg/ Cr₂O₃ | Ca/ Cr₂O₃ |
| **CrO₂** | Li/ CrO₂ | Na/ CrO₂ | K/ CrO₂ | Rb/ CrO₂ | Cs/ CrO₂ | Be/ CrO₂ | Mg/ CrO₂ | Ca/ CrO₂ |
| **CrO₃** | Li/ CrO₃ | Na/ CrO₃ | K/ CrO₃ | Rb/ CrO₃ | Cs/ CrO₃ | Be/ CrO₃ | Mg/ CrO₃ | Ca/ CrO₃ |
| **Cr₈O₂₁** | Li/ Cr₈O₂₁ | Na/ Cr₈O₂₁ | K/ Cr₈O₂₁ | Rb/ Cr₈O₂₁ | Cs/ Cr₈O₂₁ | Be/ Cr₈O₂₁ | Mg/ Cr₈O₂₁ | Ca/ Cr₈O₂₁ |
| **MoO₂** | Li/ MoO₂ | Na/ MoO₂ | K/ MoO₂ | Rb/ MoO₂ | Cs/ MoO₂ | Be/ MoO₂ | Mg/ MoO₂ | Ca/ MoO₂ |
| **MoO₃** | Li/ MoO₃ | Na/ MoO₃ | K/ MoO₃ | Rb/ MoO₃ | Cs/ MoO₃ | Be/ MoO₃ | Mg/ MoO₃ | Ca/ MoO₃ |
| **W₂O₃** | Li/ W₂O₃ | Na/ W₂O₃ | K/ W₂O₃ | Rb/ W₂O₃ | Cs/ W₂O₃ | Be/ W₂O₃ | Mg/ W₂O₃ | Ca/ W₂O₃ |
| **WoO₂** | Li/ WoO₂ | Na/ WoO₂ | K/ WoO₂ | Rb/ WoO₂ | Cs/ WoO₂ | Be/ WoO₂ | Mg/ WoO₂ | Ca/ WoO₂ |
| **WoO₃** | Li/ WoO₃ | Na/ WoO₃ | K/ WoO₃ | Rb/ WoO₃ | Cs/ WoO₃ | Be/ WoO₃ | Mg/ WoO₃ | Ca/ WoO₃ |
| **MnO** | Li/ MnO | Na/ MnO | K/ MnO | Rb/ MnO | Cs/ MnO | Be/ MnO | Mg/ MnO | Ca/ MnO |
| **Mn/Mg/O** | Li/ Mn/Mg/O | Na/ Mn/Mg/O | K/ Mn/Mg/O | Rb/ Mn/Mg/O | Cs/ Mn/Mg/O | Be/ Mn/Mg/O | Mg/ Mn/Mg/O | Ca/ Mn/Mg/O |
| **Mn₃O₄** | Li/ Mn₃O₄ | Na/ Mn₃O₄ | K/ Mn₃O₄ | Rb/ Mn₃O₄ | Cs/ Mn₃O₄ | Be/ Mn₃O₄ | Mg/ Mn₃O₄ | Ca/ Mn₃O₄ |
| **Mn₂O₃** | Li/ Mn₂O₃ | Na/ Mn₂O₃ | K/ Mn₂O₃ | Rb/ Mn₂O₃ | Cs/ Mn₂O₃ | Be/ Mn₂O₃ | Mg/ Mn₂O₃ | Ca/ Mn₂O₃ |
| **MnO₂** | Li/ MnO₂ | Na/ MnO₂ | K/ MnO₂ | Rb/ MnO₂ | Cs/ MnO₂ | Be/ MnO₂ | Mg/ MnO₂ | Ca/ MnO₂ |
| **Mn₂O₇** | Li/ Mn₂O₇ | Na/ Mn₂O₇ | K/ Mn₂O₇ | Rb/ Mn₂O₇ | Cs/ Mn₂O₇ | Be/ Mn₂O₇ | Mg/ Mn₂O₇ | Ca/ Mn₂O₇ |
| **ReO₂** | Li/ ReO₂ | Na/ ReO₂ | K/ ReO₂ | Rb/ ReO₂ | Cs/ ReO₂ | Be/ ReO₂ | Mg/ ReO₂ | Ca/ ReO₂ |
| **ReO₃** | Li/ ReO₃ | Na/ ReO₃ | K/ ReO₃ | Rb/ ReO₃ | Cs/ ReO₃ | Be/ ReO₃ | Mg/ ReO₃ | Ca/ ReO₃ |
| **Re₂O₇** | Li/ Re₂O₇ | Na/ Re₂O₇ | K/ Re₂O₇ | Rb/ Re₂O₇ | Cs/ Re₂O₇ | Be/ Re₂O₇ | Mg/ Re₂O₇ | Ca/ Re₂O₇ |
| **Mg₃Mn₃**-**B₂O₁₀** | Li/ Mg₃Mn₃-B₂O₁₀ | Na/ Mg₃Mn₃-B₂O₁₀ | K/ Mg₃Mn₃-B₂O₁₀ | Rb/ Mg₃Mn₃-B₂O₁₀ | Cs/ Mg₃Mn₃-B₂O₁₀ | Be/ Mg₃Mn₃-B₂O₁₀ | Mg/ Mg₃Mn₃-B₂O₁₀ | Ca/ Mg₃Mn₃-B₂O₁₀ |
| **Mg₃(BOs)₂** | Li/ Mg₃(BO₃)₂ | Na/ Mg₃(BO₃)₂ | K/ Mg₃(BO₃)₂ | Rb/ Mg₃(BO₃)₂ | Cs/ Mg₃(BO₃)₂ | Be/ Mg₃(BO₃)₂ | Mg/ Mg₃(BO₃)₂ | Ca/ Mg₃(BO₃)₂ |
| **Na₂WO₄** | Li/ Na₂WO₄ | Na/ Na₂WO₄ | K/ Na₂WO₄ | Rb/ Na₂WO₄ | Cs/ Na₂WO₄ | Be/ Na₂WO₄ | Mg/ Na₂WO₄ | Ca/ Na₂WO₄ |
| **Mg₆MnO₈** | Li/ Mg₆MnO₈ | Na/ Mg₆MnO₈ | K/ Mg₆MnO₈ | Rb/ Mg₆MnO₈ | Cs/ Mg₆MnO₈ | Be/ Mg₆MnO₈ | Mg/ Mg₆MnO₈ | Ca/ Mg₆MnO₈ |
| **(Li,Mg)₆**-**MnO₈** | Li/ (Li,Mg)₆-MnO₈ | Na/ (Li,Mg)₆-MnO₈ | K/ (Li,Mg)₆-MnO₈ | Rb/ (Li,Mg)₆-MnO₈ | Cs/ (Li,Mg)₆-MnO₈ | Be/ (Li,Mg)₆-MnO₈ | Mg/ (Li,Mg)₆-MnO₈ | Ca/ (Li,Mg)₆-MnO₈ |
| **Mn₂O₄** | Li/ Mn₂O₄ | Na/ Mn₂O₄ | K/ Mn₂O₄ | Rb/ Mn₂O₄ | Cs/ Mn₂O₄ | Be/ Mn₂O₄ | Mg/ Mn₂O₄ | Ca/ Mn₂O₄ |
| **Na₄P₂O₇** | Li/ Na₄P₂O₇ | Na/ Na₄P₂O₇ | K/ Na₄P₂O₇ | Rb/ Na₄P₂O₇ | Cs/ Na₄P₂O₇ | Be/ Na₄P₂O₇ | Mg/ Na₄P₂O₇ | Ca/ Na₄P₂O₇ |
| **Mo₂O₈** | Li/ Mo₂O₈ | Na/ Mo₂O₈ | K/ Mo₂O₈ | Rb/ Mo₂O₈ | Cs/ Mo₂O₈ | Be/ Mo₂O₈ | Mg/ Mo₂O₈ | Ca/ Mo₂O₈ |
| **Mn₃O₄/ WO₄** | Li/ Mn₃O₄/ WO₄ | Na/ Mn₃O₄/ WO₄ | K/ Mn₃O₄/ WO₄ | Rb/ Mn₃O₄/ WO₄ | Cs/ Mn₃O₄/ WO₄ | Be/ Mn₃O₄/ WO₄ | Mg/ Mn₃O₄/ WO₄ | Ca/ Mn₃O₄/ WO₄ |
| **Na₂WO₄** | Li/ Na₂WO₄ | Na/ Na₂WO₄ | K/ Na₂WO₄ | Rb/ Na₂WO₄ | Cs/ Na₂WO₄ | Be/ Na₂WO₄ | Mg/ Na₂WO₄ | Ca/ Na₂WO₄ |
| **Zr₂Mo₂O₈** | Li/ Zr₂Mo₂O₈ | Na/ Zr₂Mo₂O₈ | K/ Zr₂Mo₂O₈ | Rb/ Zr₂Mo₂O₈ | Cs/ Zr₂Mo₂O₈ | Be/ Zr₂Mo₂O₈ | Mg/ Zr₂Mo₂O₈ | Ca/ Zr₂Mo₂O₈ |
| **NaMnO₄**-**/MgO** | Li/ NaMnO₄-/MgO | Na/ NaMnO₄-/MgO | K/ NaMnO₄-/MgO | Rb/ NaMnO₄-/MgO | Cs/ NaMnO₄-/MgO | Be/ NaMnO₄-/MgO | Mg/ NaMnO₄-/MgO | Ca/ NaMnO₄-/MgO |
| **Na₁₀Mn-W₅O₁₇** | Li/ Na₁₀Mn-W₅O₁₇ | Na/ Na₁₀Mn-W₅O₁₇ | K/ Na₁₀Mn-W₅O₁₇ | Rb/ Na₁₀Mn-W₅O₁₇ | Cs/ Na₁₀Mn-W₅O₁₇ | Be/ Na₁₀Mn-W₅O₁₇ | Mg/ Na₁₀Mn-W₅O₁₇ | Ca/ Na₁₀Mn-W₅O₁₇ |

**TABLE 2 NANOWIRES (NW) DOPED WITH SPECIFIC DOPANTS (DOP)**

| **NW\Dop** | **Sr** | **Ba** | **B** | **P** | **S** | **F** | **Cl** |
|---|---|---|---|---|---|---|---|
| **Li₂O** | Sr/ Li₂O | Ba/ Li₂O | B/ Li₂O | P/ Li₂O | S/ Li₂O | F/ Li₂O | Cl/ Li₂O |
| **Na₂O** | Sr/ Na₂O | Ba/ Na₂O | B/ Na₂O | P/ Na₂O | S/ Na₂O | F/ Na₂O | Cl/ Na₂O |
| **K₂O** | Sr/ K₂O | Ba/ K₂O | B/ K₂O | P/ K₂O | S/ K₂O | F/ K₂O | Cl/ K₂O |
| **Rb₂O** | Sr/ Rb₂O | Ba/ Rb₂O | B/ Rb₂O | P/ Rb₂O | S/ Rb₂O | F/ Rb₂O | Cl/ Rb₂O |
| **Cs₂O** | Sr/ Cs₂O | Ba/ Cs₂O | B/ Cs₂O | P/ Cs₂O | S/ Cs₂O | F/ Cs₂O | Cl/ Cs₂O |
| **BeO** | Sr/ BeO | Ba/ BeO | B/ BeO | P/ BeO | S/ BeO | F/ BeO | Cl/ BeO |
| **MgO** | Sr/ MgO | Ba/ MgO | B/ MgO | P/ MgO | S/ MgO | F/ MgO | Cl/ MgO |
| **CaO** | Sr/ CaO | Ba/ CaO | B/ CaO | P/ CaO | S/ CaO | F/ CaO | Cl/ CaO |
| **SrO** | Sr/ SrO | Ba/ SrO | B/ SrO | P/ SrO | S/ SrO | F/ SrO | Cl/ SrO |
| **BaO** | Sr/ BaO | Ba/ BaO | B/ BaO | P/ BaO | S/ BaO | F/ BaO | Cl/ BaO |
| **Sc₂O₃** | Sr/ Sc₂O₃ | Ba/ Sc₂O₃ | B/ Sc₂O₃ | P/ Sc₂O₃ | S/ Sc₂O₃ | F/ Sc₂O₃ | Cl/ Sc₂O₃ |
| **Y₂O₃** | Sr/ Y₂O₃ | Ba/ Y₂O₃ | B/ Y₂O₃ | P/ Y₂O₃ | S/ Y₂O₃ | F/ Y₂O₃ | Cl/ Y₂O₃ |
| **La₂O₃** | Sr/ La₂O₃ | Ba/ La₂O₃ | B/ La₂O₃ | P/ La₂O₃ | S/ La₂O₃ | F/ La₂O₃ | Cl/ La₂O₃ |
| **CeO₂** | Sr/ CeO₂ | Ba/ CeO₂ | B/ CeO₂ | P/ CeO₂ | S/ CeO₂ | F/ CeO₂ | Cl/ CeO₂ |
| **Ce₂O₃** | Sr/ Ce₂O₃ | Ba/ Ce₂O₃ | B/ Ce₂O₃ | P/ Ce₂O₃ | S/ Ce₂O₃ | F/ Ce₂O₃ | Cl/ Ce₂O₃ |
| **Pr₂O₃** | Sr/ Pr₂O₃ | Ba/ Pr₂O₃ | B/ Pr₂O₃ | P/ Pr₂O₃ | S/ Pr₂O₃ | F/ Pr₂O₃ | Cl/ Pr₂O₃ |
| **Nd₂O₃** | Sr/ Nd₂O₃ | Ba/ Nd₂O₃ | B/ Nd₂O₃ | P/ Nd₂O₃ | S/ Nd₂O₃ | F/ Nd₂O₃ | Cl/ Nd₂O₃ |
| **Sm₂O₃** | Sr/ Sm₂O₃ | Ba/ Sm₂O₃ | B/ Sm₂O₃ | P/ Sm₂O₃ | S/ Sm₂O₃ | F/ Sm₂O₃ | Cl/ Sm₂O₃ |
| **Eu₂O₃** | Sr/ Eu₂O₃ | Ba/ Eu₂O₃ | B/ Eu₂O₃ | P/ Eu₂O₃ | S/ Eu₂O₃ | F/ Eu₂O₃ | Cl/ Eu₂O₃ |
| **Gd₂O₃** | Sr/ Gd₂O₃ | Ba/ Gd₂O₃ | B/ Gd₂O₃ | P/ Gd₂O₃ | S/ Gd₂O₃ | F/ Gd₂O₃ | Cl/ Gd₂O₃ |
| **Tb₂O₃** | Sr/ Tb₂O₃ | Ba/ Tb₂O₃ | B/ Tb₂O₃ | P/ Tb₂O₃ | S/ Tb₂O₃ | F/ Tb₂O₃ | Cl/ Tb₂O₃ |
| **TbO₂** | Sr/ TbO₂ | Ba/ TbO₂ | B/ TbO₂ | P/ TbO₂ | S/ TbO₂ | F/ TbO₂ | Cl/ TbO₂ |
| **Tb₆O₁₁** | Sr/ Tb₆O₁₁ | Ba/ Tb₆O₁₁ | B/ Tb₆O₁₁ | P/ Tb₆O₁₁ | S/ Tb₆O₁₁ | F/ Tb₆O₁₁ | Cl/ Tb₆O₁₁ |
| **Dy₂O₃** | Sr/ Dy₂O₃ | Ba/ Dy₂O₃ | B/ Dy₂O₃ | P/ Dy₂O₃ | S/ Dy₂O₃ | F/ Dy₂O₃ | Cl/ Dy₂O₃ |
| **Ho₂O₃** | Sr/ Ho₂O₃ | Ba/ Ho₂O₃ | B/ Ho₂O₃ | P/ Ho₂O₃ | S/ Ho₂O₃ | F/ Ho₂O₃ | Cl/ Ho₂O₃ |
| **Er₂O₃** | Sr/ Er₂O₃ | Ba/ Er₂O₃ | B/ Er₂O₃ | P/ Er₂O₃ | S/ Er₂O₃ | F/ Er₂O₃ | Cl/ Er₂O₃ |
| **Tm₂O₃** | Sr/ Tm₂O₃ | Ba/ Tm₂O₃ | B/ Tm₂O₃ | P/ Tm₂O₃ | S/ Tm₂O₃ | F/ Tm₂O₃ | Cl/ Tm₂O₃ |
| **Yb₂O₃** | Sr/ Yb₂O₃ | Ba/ Yb₂O₃ | B/ Yb₂O₃ | P/ Yb₂O₃ | S/ Yb₂O₃ | F/ Yb₂O₃ | Cl/ Yb₂O₃ |
| **Lu₂O₃** | Sr/ Lu₂O₃ | Ba/ Lu₂O₃ | B/ Lu₂O₃ | P/ Lu₂O₃ | S/ Lu₂O₃ | F/ Lu₂O₃ | Cl/ Lu₂O₃ |
| **Ac₂O₃** | Sr/ Ac₂O₃ | Ba/ Ac₂O₃ | B/ Ac₂O₃ | P/ Ac₂O₃ | S/ Ac₂O₃ | F/ Ac₂O₃ | Cl/ Ac₂O₃ |
| **Th₂O₃** | Sr/ Th₂O₃ | Ba/ Th₂O₃ | B/ Th₂O₃ | P/ Th₂O₃ | S/ Th₂O₃ | F/ Th₂O₃ | Cl/ Th₂O₃ |
| **ThO₂** | Sr/ ThO₂ | Ba/ ThO₂ | B/ ThO₂ | P/ ThO₂ | S/ ThO₂ | F/ ThO₂ | Cl/ ThO₂ |
| **Pa₂O₃** | Sr/ Pa₂O₃ | Ba/ Pa₂O₃ | B/ Pa₂O₃ | P/ Pa₂O₃ | S/ Pa₂O₃ | F/ Pa₂O₃ | Cl/ Pa₂O₃ |
| **PaO₂** | Sr/ PaO₂ | Ba/ PaO₂ | B/ PaO₂ | P/ PaO₂ | S/ PaO₂ | F/ PaO₂ | Cl/ PaO₂ |
| **TiO₂** | Sr/ TiO₂ | Ba/ TiO₂ | B/ TiO₂ | P/ TiO₂ | S/ TiO₂ | F/ TiO₂ | Cl/ TiO₂ |
| **TiO** | Sr/ TiO | Ba/ TiO | B/ TiO | P/ TiO | S/ TiO | F/ TiO | Cl/ TiO |
| **Ti₂O₃** | Sr/ Ti₂O₃ | Ba/ Ti₂O₃ | B/ Ti₂O₃ | P/ Ti₂O₃ | S/ Ti₂O₃ | F/ Ti₂O₃ | Cl/ Ti₂O₃ |
| **Ti₃O** | Sr/ Ti₃O | Ba/ Ti₃O | B/ Ti₃O | P/ Ti₃O | S/ Ti₃O | F/ Ti₃O | Cl/ Ti₃O |
| **Ti₂O** | Sr/ Ti₂O | Ba/ Ti₂O | B/ Ti₂O | P/ Ti₂O | S/ Ti₂O | F/ Ti₂O | Cl/ Ti₂O |
| **Ti₃O₅** | Sr/ Ti₃O₅ | Ba/ Ti₃O₅ | B/ Ti₃O₅ | P/ Ti₃O₅ | S/ Ti₃O₅ | F/ Ti₃O₅ | Cl/ Ti₃O₅ |
| **Ti₄O₇** | Sr/ Ti₄O₇ | Ba/ Ti₄O₇ | B/ Ti₄O₇ | P/ Ti₄O₇ | S/ Ti₄O₇ | F/ Ti₄O₇ | Cl/ Ti₄O₇ |
| **ZrO₂** | Sr/ ZrO₂ | Ba/ ZrO₂ | B/ ZrO₂ | P/ ZrO₂ | S/ ZrO₂ | F/ ZrO₂ | Cl/ ZrO₂ |
| **HfO₂** | Sr/ HfO₂ | Ba/ HfO₂ | B/ HfO₂ | P/ HfO₂ | S/ HfO₂ | F/ HfO₂ | Cl/ HfO₂ |
| **VO** | Sr/ VO | Ba/ VO | B/ VO | P/ VO | S/ VO | F/ VO | Cl/ VO |
| **V₂O₃** | Sr/ V₂O₃ | Ba/ V₂O₃ | B/ V₂O₃ | P/ V₂O₃ | S/ V₂O₃ | F/ V₂O₃ | Cl/ V₂O₃ |
| **VO₂** | Sr/ VO₂ | Ba/ VO₂ | B/ VO₂ | P/ VO₂ | S/ VO₂ | F/ VO₂ | Cl/ VO₂ |
| **V₂O₅** | Sr/ V₂O₅ | Ba/ V₂O₅ | B/ V₂O₅ | P/ V₂O₅ | S/ V₂O₅ | F/ V₂O₅ | Cl/ V₂O₅ |
| **V₃O₇** | Sr/ V₃O₇ | Ba/ V₃O₇ | B/ V₃O₇ | P/ V₃O₇ | S/ V₃O₇ | F/ V₃O₇ | Cl/ V₃O₇ |
| **V₄O₉** | Sr/ V₄O₉ | Ba/ V₄O₉ | B/ V₄O₉ | P/ V₄O₉ | S/ V₄O₉ | F/ V₄O₉ | Cl/ V₄O₉ |
| **V₆O₁₃** | Sr/ V₆O₁₃ | Ba/ V₆O₁₃ | B/ V₆O₁₃ | P/ V₆O₁₃ | S/ V₆O₁₃ | F/ V₆O₁₃ | Cl/ V₆O₁₃ |
| **NbO** | Sr/ NbO | Ba/ NbO | B/ NbO | P/ NbO | S/ NbO | F/ NbO | Cl/ NbO |
| **NbO₂** | Sr/ NbO₂ | Ba/ NbO₂ | B/ NbO₂ | P/ NbO₂ | S/ NbO₂ | F/ NbO₂ | Cl/ NbO₂ |
| **Nb₂O₅** | Sr/ Nb₂O₅ | Ba/ Nb₂O₅ | B/ Nb₂O₅ | P/ Nb₂O₅ | S/ Nb₂O₅ | F/ Nb₂O₅ | Cl/ Nb₂O₅ |
| **Nb₈O₁₉** | Sr/ Nb₈O₁₉ | Ba/ Nb₈O₁₉ | B/ Nb₈O₁₉ | P/ Nb₈O₁₉ | S/ Nb₈O₁₉ | F/ Nb₈O₁₉ | Cl/ Nb₈O₁₉ |
| **Nb₁₆O₃₈** | Sr/ Nb₁₆O₃₈ | Ba/ Nb₁₆O₃₈ | B/ Nb₁₆O₃₈ | P/ Nb₁₆O₃₈ | S/ Nb₁₆O₃₈ | F/ Nb₁₆O₃₈ | Cl/ Nb₁₆O₃₈ |
| **Nb₁₂O₂₉** | Sr/ Nb₁₂O₂₉ | Ba/ Nb₁₂O₂₉ | B/ Nb₁₂O₂₉ | P/ Nb₁₂O₂₉ | S/ Nb₁₂O₂₉ | F/ Nb₁₂O₂₉ | Cl/ Nb₁₂O₂₉ |
| **Nb₄₇O₁₁₆** | Sr/ Nb₄₇O₁₁₆ | Ba/ Nb₄₇O₁₁₆ | B/ Nb₄₇O₁₁₆ | P/ Nb₄₇O₁₁₆ | S/ Nb₄₇O₁₁₆ | F/ Nb₄₇O₁₁₆ | Cl/ Nb₄₇O₁₁₆ |
| **Ta₂O₅** | Sr/ Ta₂O₅ | Ba/ Ta₂O₅ | B/ Ta₂O₅ | P/ Ta₂O₅ | S/ Ta₂O₅ | F/ Ta₂O₅ | Cl/ Ta₂O₅ |
| **CrO** | Sr/ CrO | Ba/ CrO | B/ CrO | P/ CrO | S/ CrO | F/ CrO | Cl/ CrO |
| **Cr₂O₃** | Sr/ Cr₂O₃ | Ba/ Cr₂O₃ | B/ Cr₂O₃ | P/ Cr₂O₃ | S/ Cr₂O₃ | F/ Cr₂O₃ | Cl/ Cr₂O₃ |
| **CrO₂** | Sr/ CrO₂ | Ba/ CrO₂ | B/ CrO₂ | P/ CrO₂ | S/ CrO₂ | F/ CrO₂ | Cl/ CrO₂ |
| **CrO₃** | Sr/ CrO₃ | Ba/ CrO₃ | B/ CrO₃ | P/ CrO₃ | S/ CrO₃ | F/ CrO₃ | Cl/ CrO₃ |
| **Cr₈O₂₁** | Sr/ Cr₈O₂₁ | Ba/ Cr₈O₂₁ | B/ Cr₈O₂₁ | P/ Cr₈O₂₁ | S/ Cr₈O₂₁ | F/ Cr₈O₂₁ | Cl/ Cr₈O₂₁ |
| **MoO₂** | Sr/ MoO₂ | Ba/ MoO₂ | B/ MoO₂ | P/ MoO₂ | S/ MoO₂ | F/ MoO₂ | Cl/ MoO₂ |
| **MoO₃** | Sr/ MoO₃ | Ba/ MoO₃ | B/ MoO₃ | P/ MoO₃ | S/ MoO₃ | F/ MoO₃ | Cl/ MoO₃ |
| **W₂O₃** | Sr/ W₂O₃ | Ba/ W₂O₃ | B/ W₂O₃ | P/ W₂O₃ | S/ W₂O₃ | F/ W₂O₃ | Cl/ W₂O₃ |
| **WoO₂** | Sr/ WoO₂ | Ba/ WoO₂ | B/ WoO₂ | P/ WoO₂ | S/ WoO₂ | F/ WoO₂ | Cl/ WoO₂ |
| **WoO₃** | Sr/ WoO₃ | Ba/ WoO₃ | B/ WoO₃ | P/ WoO₃ | S/ WoO₃ | F/ WoO₃ | Cl/ WoO₃ |
| **MnO** | Sr/ MnO | Ba/ MnO | B/ MnO | P/ MnO | S/ MnO | F/ MnO | Cl/ MnO |
| **Mn/Mg/O** | Sr/ Mn/Mg/O | Ba/ Mn/Mg/O | B/ Mn/Mg/O | P/ Mn/Mg/O | S/ Mn/Mg/O | F/ Mn/Mg/O | Cl/ Mn/Mg/O |
| **Mn₃O₄** | Sr/ Mn₃O₄ | Ba/ Mn₃O₄ | B/ Mn₃O₄ | P/ Mn₃O₄ | S/ MnO₃O4 | F/ Mn₃O₄ | Cl/ Mn₃O₄ |
| **Mn₂O₃** | Sr/ Mn₂O₃ | Ba/ Mn₂O₃ | B/ Mn₂O₃ | P/ Mn₂O₃ | S/ Mn₂O₃ | F/ Mn₂O₃ | Cl/ Mn₂O₃ |
| **MnO₂** | Sr/ MnO₂ | Ba/ MnO₂ | B/ MnO₂ | P/ MnO₂ | S/ MnO₂ | F/ MnO₂ | Cl/ MnO₂ |
| **Mn₂O₇** | Sr/ Mn₂O₇ | Ba/ Mn₂O₇ | B/ Mn₂O₇ | P/ Mn₂O₇ | S/ Mn₂O₇ | F/ Mn₂O₇ | Cl/ Mn₂O₇ |
| **ReO₂** | Sr/ ReO₂ | Ba/ ReO₂ | B/ ReO₂ | P/ ReO₂ | S/ ReO₂ | F/ ReO₂ | Cl/ ReO₂ |
| **ReO₃** | Sr/ ReO₃ | Ba/ ReO₃ | B/ ReO₃ | P/ ReO₃ | S/ ReO₃ | F/ ReO₃ | Cl/ ReO₃ |
| **Re₂O₇** | Sr/ Re₂O₇ | Ba/ Re₂O₇ | B/ Re₂O₇ | P/ Re₂O₇ | S/ Re₂O₇ | F/ Re₂O₇ | Cl/ Re₂O₇ |
| **Mg₃Mn₃**-**B₂O₁₀** | Sr/ Mg₃Mn₃-B₂O₁₀ | Ba/ Mg₃Mn₃-B₂O₁₀ | B/ Mg₃Mn₃-B₂O₁₀ | P/ Mg₃Mn₃-B₂O₁₀ | S/ Mg₃Mn₃-B₂O₁₀ | F/ Mg₃Mn₃-B₂O₁₀ | Cl/ Mg₃Mn₃-B₂O₁₀ |
| **Mg₃(BO₃)₂** | Sr/ Mg₃(BO₃)₂ | Ba/ Mg₃(BO₃)₂ | B/ Mg₃(BO₃)₂ | P/ Mg₃(BO₃)₂ | S/ Mg₃(BO₃)₂ | F/ Mg₃(BO₃)₂ | Cl/ Mg₃(BO₃)₂ |
| **NaWO₄** | Sr/ NaWO₄ | Ba/ NaWO₄ | B/ NaWO₄ | P/ NaWO₄ | S/ NaWO₄ | F/ NaWO₄ | Cl/ NaWO₄ |
| **Mg₆MnO₈** | Sr/ Mg₆MnO₈ | Ba/ Mg₆MnO₈ | B/ Mg₆MnO₈ | P/ Mg₆MnO₈ | S/ Mg₆MnO₈ | F/ Mg₆MnO₈ | Cl/ Mg₆MnO₈ |
| **(Li,Mg)₆Mn O₈** | Sr/ (Li,M_{g})₆MnO₈ | Ba/ (Li,Mg)₆MnO₈ | B/ (Li,Mg)₆MnO₈ | P/ (Li,Mg)₆MnO₈ | S/ (Li,Mg)₆MnO₈ | F/ (Li,Mg)₆MnO₈ | Cl/ (Li,Mg)₆MnO₈ |
| **Mn₂O₄** | Sr/ Mn₂O₄ | Ba/ Mn₂O₄ | B/ Mn₂O₄ | P/ Mn₂O₄ | S/ Mn₂O₄ | F/ Mn₂O₄ | Cl/ Mn₂O₄ |
| **Na₄P₂O₇** | Sr/ Na₄P₂O₇ | Ba/ Na₄P₂O₇ | B/ Na₄P₂O₇ | P/ Na₄P₂O₇ | S/ Na₄P₂O₇ | F/ Na₄P₂O₇ | Cl/ Na₄P₂O₇ |
| **Mo₂O₈** | Sr/ Mo₂O₈ | Ba/ Mo₂O₈ | B/ Mo₂O₈ | P/ Mo₂O₈ | S/ Mo₂O₈ | F/ Mo₂O₈ | Cl/ Mo₂O₈ |
| **Mn₃O₄/WO₄** | Sr/ Mn₃O₄/WO₄ | Ba/ Mn₃O₄/WO₄ | B/ Mn₃O₄/WO₄ | P/ Mn₃O₄W/O₄ | S/ Mn₃O₄/WO₄ | F/ Mn₃O₄/WO₄ | Cl/ Mn₃O₄/WO₄ |
| **Na₂WO₄** | Sr/ Na₂WO₄ | Ba/ Na₂WO₄ | B/ Na₂WO₄ | P/ Na₂WO₄ | S/ Na₂WO₄ | F/ Na₂WO₄ | Cl/ Na₂WO₄ |
| **Zr₂Mo₂O₈** | Sr/ Zr₂Mo₂O₈ | Ba/ Zr₂Mo₂O₈ | B/ Zr₂Mo₂O₈ | P/ Zr₂Mo₂O₈ | S/ Zr₂Mo₂O₈ | F/ Zr₂Mo₂O₈ | Cl/ Zr₂Mo₂O₈ |
| **NaMnO₄/Mg O** | Sr/ NaMnO₄/Mg O | Ba/ NaMnO₄/Mg O | B/ NaMnO₄/Mg O | P/ NaMnO₄/Mg O | S/ NaMnO₄/Mg O | F/ NaMnO₄/Mg O | Cl/ NaMnO₄/Mg O |
| **Na₁₀Mn-W₅O₁₇** | Sr/ Na₁₀Mn-W₅O₁₇ | Ba/ Na₁₀Mn-W₅O₁₇ | B/ Na₁₀Mn-W₅O₁₇ | P/ Na₁₀Mn-W₅O₁₇ | S/ Na₁₀Mn-W₅O₁₇ | F/ Na₁₀Mn-W₅O₁₇ | Cl/ Na₁₀Mn-W₅O₁₇ |

**TABLE 3 NANOWIRES (NW) DOPED WITH SPECIFIC DOPANTS (DOP)**

| **NW\Dop** | **La** | **Ce** | **Pr** | **Nd** | **Pm** | **Sm** | **Eu** | **Gd** |
|---|---|---|---|---|---|---|---|---|
| **Li₂O** | La/ Li₂O | Ce/ Li₂O | Pr/ Li₂O | Nd/ Li₂O | Pm/ Li₂O | Sm/ Li₂O | Eu/ Li₂O | Gd/ Li₂O |
| **Na₂O** | La/ Na₂O | Ce/ Na₂O | Pr/ Na₂O | Nd/ Na₂O | Pm/ Na₂O | Sm/ Na₂O | Eu/ Na₂O | Gd/ Na₂O |
| **K₂O** | La/ K₂O | Ce/ K₂O | Pr/ K₂O | Nd/ K₂O | Pm/ K₂O | Sm/ K₂O | Eu/ K₂O | Gd/ K₂O |
| **Rb₂O** | La/ Rb₂O | Ce/ Rb₂O | Pr/ Rb₂O | Nd/ Rb₂O | Pm/ Rb₂O | Sm/ Rb₂O | Eu/ Rb₂O | Gd/ Rb₂O |
| **Cs₂O** | La/ Cs₂O | Ce/ Cs₂O | Pr/ Cs₂O | Nd/ Cs₂O | Pm/ Cs₂O | Sm/ Cs₂O | Eu/ Cs₂O | Gd/ Cs₂O |
| **BeO** | La/ BeO | Ce/ BeO | Pr/ BeO | Nd/ BeO | Pm/ BeO | Sm/ BeO | Eu/ BeO | Gd/ BeO |
| **MgO** | La/ MgO | Ce/ MgO | Pr/ MgO | Nd/ MgO | Pm/ MgO | Sm/ MgO | Eu/ MgO | Gd/ MgO |
| **CaO** | La/ CaO | Ce/ CaO | Pr/ CaO | Nd/ CaO | Pm/ CaO | Sm/ CaO | Eu/ CaO | Gd/ CaO |
| **SrO** | La/ SrO | Ce/ SrO | Pr/ SrO | Nd/ SrO | Pm/ SrO | Sm/ SrO | Eu/ SrO | Gd/ SrO |
| **BaO** | La/ BaO | Ce/ BaO | Pr/ BaO | Nd/ BaO | Pm/ BaO | Sm/ BaO | Eu/ BaO | Gd/ BaO |
| **SC₂O₃** | La/ Sc₂O₃ | Ce/ Sc₂O₃ | Pr/ Sc₂O₃ | Nd/ Sc₂O₃ | Pm/ Sc₂O₃ | Sm/ SC₂O₃ | Eu/ Sc₂O₃ | Gd/ Sc₂O₃ |
| **Y₂O₃** | La/ Y₂O₃ | Ce/ Y₂O₃ | Pr/ Y₂O₃ | Nd/ Y₂O₃ | Pm/ Y₂O₃ | Sm/ Y₂O₃ | Eu/ Y₂O₃ | Gd/ Y₂O₃ |
| **La₂O₃** | La/ La₂O₃ | Ce/ La₂O₃ | Pr/ La₂O₃ | Nd/ La₂O₃ | Pm/ La₂O₃ | Sm/ La₂O₃ | Eu/ La₂O₃ | Gd/ La₂O₃ |
| **CeO₂** | La/ CeO₂ | Ce/ CeO₂ | Pr/ CeO₂ | Nd/ CeO₂ | Pm/ CeO₂ | Sm/ CeO₂ | Eu/ CeO₂ | Gd/ CeO₂ |
| **Ce₂O₃** | La/ Ce₂O₃ | Ce/ Ce₂O₃ | Pr/ Ce₂O₃ | Nd/ Ce₂O₃ | Pm/ Ce₂O₃ | Sm/ Ce₂O₃ | Eu/ Ce₂O₃ | Gd/ Ce₂O₃ |
| **Pr₂O₃** | La/ Pr₂O₃ | Ce/ Pr₂O₃ | Pr/ Pr₂O₃ | Nd/ Pr₂O₃ | Pm/ Pr₂O₃ | Sm/ Pr₂O₃ | Eu/ Pr₂O₃ | Gd/ Pr₂O₃ |
| **Nd₂O₃** | La/ Nd₂O₃ | Ce/ Nd₂O₃ | Pr/ Nd₂O₃ | Nd/ Nd₂O₃ | Pm/ Nd₂O₃ | Sm/ Nd₂O₃ | Eu/ Nd₂O₃ | Gd/ Nd₂O₃ |
| **Sm₂O₃** | La/ Sm₂O₃ | Ce/ Sm₂O₃ | Pr/ Sm₂O₃ | Nd/ Sm₂O₃ | Pm/ Sm₂O₃ | Sm/ Sm₂O₃ | Eu/ Sm₂O₃ | Gd/ Sm₂O₃ |
| **Eu₂O₃** | La/ Eu₂O₃ | Ce/ Eu₂O₃ | Pr/ Eu₂O₃ | Nd/ Eu₂O₃ | Pm/ Eu₂O₃ | Sm/ Eu₂O₃ | Eu/ Eu₂O₃ | Gd/ Eu₂O₃ |
| **Gd₂O₃** | La/ Gd₂O₃ | Ce/ Gd₂O₃ | Pr/ Gd₂O₃ | Nd/ Gd₂O₃ | Pm/ Gd₂O₃ | Sm/ Gd₂O₃ | Eu/ Gd₂O₃ | Gd/ Gd₂O₃ |
| **Tb₂O₃** | La/ Tb₂O₃ | Ce/ Tb₂O₃ | Pr/ Tb₂O₃ | Nd/ Tb₂O₃ | Pm/ Tb₂O₃ | Sm/ Tb₂O₃ | Eu/ Tb₂O₃ | Gd/ Tb₂O₃ |
| **TbO₂** | La/ TbO₂ | Ce/ TbO₂ | Pr/ TbO₂ | Nd/ TbO₂ | Pm/ TbO₂ | Sm/ TbO₂ | Eu/ TbO₂ | Gd/ TbO₂ |
| **Tu₆O₁₁** | La/ Tb₆O₁₁ | Ce/ Tb₆O₁₁ | Pr/ Tb₆O₁₁ | Nd/ Tb₆O₁₁ | Pm/ Tb₆O₁₁ | Sm/ Tb₆O₁₁ | Eu/ Tb₆O₁₁ | Gd/ Tb₆O₁₁ |
| **Dy₂O₃** | La/ Dy₂O₃ | Ce/ Dy₂O₃ | Pr/ Dy₂O₃ | Nd/ Dy₂O₃ | Pm/ Dy₂O₃ | Sm/ Dy₂O₃ | Eu/ Dy₂O₃ | Gd/ Dy₂O₃ |
| **Ho₂O₃** | La/ Ho₂O₃ | Ce/ Ho₂O₃ | Pr/ Ho₂O₃ | Nd/ Ho₂O₃ | Pm/ Ho₂O₃ | Sm/ Ho₂O₃ | Eu/ Ho₂O₃ | Gd/ Ho₂O₃ |
| **Er₂O₃** | La/ Er₂O₃ | Ce/ Er₂O₃ | Pr/ Er₂O₃ | Nd/ Er₂O₃ | Pm/ Er₂O₃ | Sm/ Er₂O₃ | Eu/ Er₂O₃ | Gd/ Er₂O₃ |
| **Tm₂O₃** | La/ Tm₂O₃ | Ce/ Tm₂O₃ | Pr/ Tm₂O₃ | Nd/ Tm₂O₃ | Pm/ Tm₂O₃ | Sm/ Tm₂O₃ | Eu/ Tm₂O₃ | Gd/ Tm₂O₃ |
| **Yb₂O₃** | La/ Yb₂O₃ | Ce/ Yb₂O₃ | Pr/ Yb₂O₃ | Nd/ Yb₂O₃ | Pm/ Yb₂O₃ | Sm/ Yb₂O₃ | Eu/ Yb₂O₃ | Gd/ Yb₂O₃ |
| **Lu₂O₃** | La/ Lu_{z}O₃ | Ce/ Lu₂O₃ | Pr/ Lu₂O₃ | Nd/ Lu₂O₃ | Pm/ Lu₂O₃ | Sm/ Lu₂O₃ | Eu/ Lu₂O₃ | Gd/ Lu₂O₃ |
| **Ac₂O₃** | La/ Ac₂O₃ | Ce/ Ac₂O₃ | Pr/ AC₂O₃ | Nd/ AC₂O₃ | Pm/ Ac₂O₃ | Sm/ Ac₂O₃ | Eu/ Ac₂O₃ | Gd/ Ac₂O₃ |
| **Th₂O₃** | La/ Th₂O₃ | Ce/ Th₂O₃ | Pr/ Th₂O₃ | Nd/ Th₂O₃ | Pm/ Th₂O₃ | Sm/ Th₂O₃ | Eu/ Th₂O₃ | Gd/ Th₂O₃ |
| **ThO₂** | La/ ThO₂ | Ce/ ThO₂ | Pr/ ThO₂ | Nd/ ThO₂ | Pm/ ThO₂ | Sm/ ThO₂ | Eu/ ThO₂ | Gd/ ThO₂ |
| **Pa₂O₃** | La/ Pa₂O₃ | Ce/ Pa₂O₃ | Pr/ Pa₂O₃ | Nd/ Pa₂O₃ | Pm/ Pa₂O₃ | Sm/ Pa₂O₃ | Eu/ Pa₂O₃ | Gd/ Pa₂O₃ |
| **PaO₂** | La/ PaO₂ | Ce/ PaO₂ | Pr/ PaO₂ | Nd/ PaO₂ | Pm/ PaO₂ | Sm/ PaO₂ | Eu/ PaO₂ | Gd/ PaO₂ |
| **TiO₂** | La/ TiO₂ | Ce/ TiO₂ | Pr/ TiO₂ | Nd/ TiO₂ | Pm/ TiO₂ | Sm/ TiO₂ | Eu/ TiO₂ | Gd/ TiO₂ |
| **TiO** | La/ TiO | Ce/ TiO | Pr/ TiO | Nd/ TiO | Pm/ TiO | Sm/ TiO | Eu/ TiO | Gd/ TiO |
| **Ti₂O₃** | La/ Ti₂O₃ | Ce/ Ti₂O₃ | Pr/ Ti₂O₃ | Nd/ Ti₂O₃ | Pm/ Ti₂O₃ | Sm/ Ti₂O₃ | Eu/ Ti₂O₃ | Gd/ Ti₂O₃ |
| **Ti₃O** | La/ Ti₃O | Ce/ Ti₃O | Pr/ Ti₃O | Nd/ Ti₃O | Pm/ Ti₃O | Sm/ Ti₃O | Eu/ Ti₃O | Gd/ Ti₃O |
| **Ti₂O** | La/ Ti₂O | Ce/ Ti₂O | Pr/ Ti₂O | Nd/ Ti₂O | Pm/ Ti₂O | Sm/ Ti₂O | Eu/ Ti₂O | Gd/ Ti₂O |
| **Ti₃O₅** | La/ Ti₃O₅ | Ce/ Ti₃O₅ | Pr/ Ti₃O₅ | Nd/ Ti₃O₅ | Pm/ Ti₃O₅ | Sm/ Ti₃O₅ | Eu/ Ti₃O₅ | Gd/ Ti₃O₅ |
| **Ti₄O₇** | La/ Ti₄O₇ | Ce/ Ti₄O₇ | Pr/ Ti₄O₇ | Nd/ Ti₄O₇ | Pm/ Ti₄O₇ | Sm/ Ti₄O₇ | Eu/ Ti₄O₇ | Gd/ Ti₄O₇ |
| **ZrO₂** | La/ ZrO₂ | Ce/ ZrO₂ | Pr/ ZrO₂ | Nd/ ZrO₂ | Pm/ ZrO₂ | Sm/ ZrO₂ | Eu/ ZrO₂ | Gd/ ZrO₂ |
| **HfO₂** | La/ HfO₂ | Ce/ HfO₂ | Pr/ HfO₂ | Nd/ HfO₂ | Pm/ HfO₂ | Sm/ HfO₂ | Eu/ HfO₂ | Gd/ HfO₂ |
| **VO** | La/ VO | Ce/ VO | Pr/ VO | Nd/ VO | Pm/ VO | Sm/ VO | Eu/ VO | Gd/ VO |
| **V₂O₃** | La/ V₂O₃ | Ce/ V₂O₃ | Pr/ V₂O₃ | Nd/ V₂O₃ | Pm/ V₂O₃ | Sm/ V₂O₃ | Eu/ V₂O₃ | Gd/ V₂O₃ |
| **VO₂** | La/ VO₂ | Ce/ VO₂ | Pr/ VO₂ | Nd/ VO₂ | Pm/ VO₂ | Sm/ VO₂ | Eu/ VO₂ | Gd/ VO₂ |
| **V₂O₅** | La/ V₂O₅ | Ce/ V₂O₅ | Pr/ V₂O₅ | Nd/ V₂O₅ | Pm/ V₂O₅ | Sm/ V₂O₅ | Eu/ V₂O₅ | Gd/ V₂O₅ |
| **V₃O₇** | La/ V₃O₇ | Ce/ V₃O₇ | Pr/ V₃O₇ | Nd/ V₃O₇ | Pm/ V₃O₇ | Sm/ V₃O₇ | Eu/ V₃O₇ | Gd/ V₃O₇ |
| **V₄O₉** | La/ V₄O₉ | Ce/ V₄O₉ | Pr/ V₄O₉ | Nd/ V₄O₉ | Pm/ V₄O₉ | Sm/ V₄O₉ | Eu/ V₄O₉ | Gd/ V₄O₉ |
| **V₆O₁₃** | La/ V₆O₁₃ | Ce/ V₆O₁₃ | Pr/ V₆O₁₃ | Nd/ V₆O₁₃ | Pm/ V₆O₁₃ | Sm/ V₆O₁₃ | Eu/ V₆O₁₃ | Gd/ V₆O₁₃ |
| **NbO** | La/ NbO | Ce/ NbO | Pr/ NbO | Nd/ NbO | Pm/ NbO | Sm/ NbO | Eu/ NbO | Gd/ NbO |
| **NbO₂** | La/ NbO₂ | Ce/ NbO₂ | Pr/ NbO₂ | Nd/ NbO₂ | Pm/ NbO₂ | Sm/ NbO₂ | Eu/ NbO₂ | Gd/ NbO₂ |
| **Nb₂O₅** | La/ Nb₂O₅ | Ce/ Nb₂O₅ | Pr/ Nb₂O₅ | Nd/ Nb₂O₅ | Pm/ Nb₂O₅ | Sm/ Nb₂O₅ | Eu/ Nb₂O₅ | Gd/ Nb₂O₅ |
| **Nb₈O₁₉** | La/ Nb₈O₁₉ | Ce/ Nb₈O₁₉ | Pr/ Nb₈O₁₉ | Nd/ Nb₈O₁₉ | Pm/ Nb₈O₁₉ | Sm/ Nb₈O₁₉ | Eu/ Nb₈O₁₉ | Gd/ Nb₈O₁₉ |
| **Nb₁₆O₃₈** | La/ Nb₁₆O₃₈ | Ce/ Nb₁₆O₃₈ | Pr/ Nb₁₆O₃₈ | Nd/ Nb₁₆O₃₈ | Pm/ Nb₁₆O₃₈ | Sm/ Nb₁₆O₃₈ | Eu/ Nb₁₆O₃₈ | Gd/ Nb₁₆O₃₈ |
| **Nb₁₂O₂₉** | La/ Nb₁₂O₂₉ | Ce/ Nb₁₂O₂₉ | Pr/ Nb₁₂O₂₉ | Nd/ Nb₁₂O₂₉ | Pm/ Nb₁₂O₂₉ | Sm/ Nb₁₂O₂₉ | Eu/ Nb₁₂O₂₉ | Gd/ Nb₁₂O₂₉ |
| **Nb₄₇O₁₁₆** | La/ Nb₄₇O₁₁₆ | Ce/ Nb₄₇O₁₁₆ | Pr/ N_{b47}O₁₁₆ | Nd/ Nb₄₇O₁₁₆ | Pm/ Nb₄₇O₁₁₆ | Sm/ Nb₄₇O₁₁₆ | Eu/ Nb₄₇O₁₁₆ | Gd/ Nb₄₇O₁₁₆ |
| **Ta₂O₅** | La/ Ta₂O₅ | Ce/ Ta₂O₅ | Pr/ Ta₂O₅ | Nd/ Ta₂O₅ | Pm/ Ta₂O₅ | Sm/ Ta₂O₅ | Eu/ Te₂O₅ | Gd/ Ta₂O₅ |
| **CrO** | La/ CrO | Ce/ CrO | Pr/ CrO | Nd/ CrO | Pm/ CrO | Sm/ CrO | Eu/ CrO | Gd/ CrO |
| **Cr₂O₃** | La/ Cr₂O₃ | Ce/ Cr₂O₃ | Pr/ Cr₂O₃ | Nd/ Cr₂O₃ | Pm/ Cr₂O₃ | Sm/ Cr₂O₃ | Eu/ Cr₂O₃ | Gd/ Cr₂O₃ |
| **CrO₂** | La/ CrO₂ | Ce/ CrO₂ | Pr/ CrO₂ | Nd/ CrO₂ | Pm/ CrO₂ | Sm/ CrO₂ | Eu/ CrO₂ | Gd/ CrO₂ |
| **CrO₃** | La/ CrO₃ | Ce/ CrO₃ | Pr/ CrO₃ | Nd/ CrO₃ | Pm/ CrO₃ | Sm/ CrO₃ | Eu/ CrO₃ | Gd/ CrO₃ |
| **Cr₈O₂₁** | La/ Cr₈O₂₁ | Ce/ Cr₈O₂₁ | Pr/ Cr₈O₂₁ | Nd/ Cr₈O₂₁ | Pm/ Cr₈O₂₁ | Sm/ Cr₈O₂₁ | Eu/ Cr₈O₂₁ | Gd/ Cr₈O₂₁ |
| **MoO₂** | La/ MoO₂ | Ce/ MoO₂ | Pr/ MoO₂ | Nd/ MoO₂ | Pm/ MoO₂ | Sm/ MoO₂ | Eu/ MoO₂ | Gd/ MoO₂ |
| **MoO₃** | La/ MoO₃ | Ce/ MoO₃ | Pr/ MoO₃ | Nd/ MoO₃ | Pm/ MoO₃ | Sm/ MoO₃ | Eu/ MoO₃ | Gd/ MoO₃ |
| **W₂O₃** | La/ W₂O₃ | Ce/ W₂O₃ | Pr/ W₂O₃ | Nd/ W₂O₃ | Pm/ W₂O₃ | Sm/ W₂O₃ | Eu/ W₂O₃ | Gd/ W₂O₃ |
| **WoO₂** | La/ WoO₂ | Ce/ WoO₂ | Pr/ WoO₂ | Nd/ WoO₂ | Pm/ WoO₂ | Sm/ WoO₂ | Eu/ WoO₂ | Gd/ woO₂ |
| **WoO₃** | La/ WoO₃ | Ce/ WoO₃ | Pr/ WoO₃ | Nd/ WoO₃ | Pm/ WoO₃ | Sm/ WoO₃ | Eu/ WoO₃ | Gd/ WoO₃ |
| **MnO** | La/ MnO | Ce/ MnO | Pr/ MnO | Nd/ MnO | Pm/ MnO | Sm/ MnO | Eu/ MnO | Gd/ MnO |
| **Mn/Mg/O** | La/ Mn/Mp/O | Ce/ Mn/Mg/O | Pr/ Mn/Mg/O | Nd/ Mn/Mg/O | Pm/ Mn/Mg/O | Sm/ Mn/Mg/O | Eu/ Mn/Mg/O | Gd/ Mn/Mg/O |
| **Mn₃O₄** | La/ Mn₃O₄ | Ce/ Mn₃O₄ | Pr/ Mn₃O₄ | Nd/ Mn₃O₄ | Pm/ Mn₃O₄ | Sm/ Mn₃O₄ | Eu/ Mn₃O₄ | Gd/ Mn₃O₄ |
| **Mn₂O₃** | La/ Mn₂O₃ | Ce/ Mn₂O₃ | Pr/ Mn₂O₃ | Nd/ Mn₂O₃ | Pm/ Mn₂O₃ | Sm/ Mn₂O₃ | Eu/ Mn₂O₃ | Gd/ Mn₂O₃ |
| **MnO₂** | La/ MnO₂ | Ce/ MnO₂ | Pr/ MnO₂ | Nd/ MnO₂ | Pm/ MnO₂ | Sm/ MnO₂ | Eu/ MnO₂ | Gd/ MnO₂ |
| **Mn₂O₇** | La/ Mn₂O₇ | Ce/ Mn₂O₇ | Pr/ Mn₂O₇ | Nd/ Mn₂O₇ | Pm/ Mn₂O₇ | Sm/ Mn₂O₇ | Eu/ Mn₂O₇ | Gd/ Mn₂O₇ |
| **ReO₂** | La/ ReO₂ | Ce/ ReO₂ | Pr/ ReO₂ | Nd/ ReO₂ | Pm/ ReO₂ | Sm/ ReO₂ | Eu/ ReO₂ | Gd/ ReO₂ |
| **ReO₃** | La/ ReO₃ | Ce/ ReO₃ | Pr/ ReO₃ | Nd/ ReO₃ | Pm/ ReO₃ | Sm/ ReO₃ | Eu/ ReO₃ | Gd/ ReO₃ |
| **Re₂O₇** | La/ Re₂O₇ | Ce/ Re₂O₇ | Pr/ Re₂O₇ | Nd/ Re₂O₇ | Pm/ Re₂O₇ | Sm/ Re₂O₇ | Eu/ Re₂O₇ | Gd/ Re₂O₇ |
| **Mg₃Mn₃**-**B₂O₁₀** | La/ Mg₃Mn₃-B₂O₁₀ | Ce/ Mg₃Mn₃-B₂O₁₀ | Pr/ Mg₃Mn₃-B₂O₁₀ | Nd/ Mg₃Mn₃-B₂O₁₀ | Pm/ Mg₃Mn₃-B₂O₁₀ | Sm/ Mg₃Mn₃-B₂O₁₀ | Eu/ Mg₃Mn₃-B₂O₁₀ | Gd/ Mg₃Mn₃-B₂O₁₀ |
| **Mg₃(BO₃)₂** | La/ Mg₃(BO₃)₂ | Ce/ Mg₃(BO₃)₂ | Pr/ Mg₃(BO₃)₂ | Nd/ Mg₃(BO₃)₂ | Pm/ Mg₃(BO₃)₂ | Sm/ Mg₃(BO₃)₂ | Eu/ Mg₃(BO₃)₂ | Gd/ Mg₃(BO₃)₂ |
| **NaWO₄** | La/ NaWO₄ | Ce/ NaWO₄ | Pr/ NaWO₄ | Nd/ NaWO₄ | Pm/ NaWO₄ | Sm/ NaWO₄ | Eu/ NaWO₄ | Gd/ NaWO₄ |
| **Mg₆MnO₈** | La/ Mg₆MnO₈ | Ce/ M₉₆MnO₈ | Pr/ Mg₆MnO₈ | Nd/ Mg₆MnO₈ | Pm/ Mg₆MnO₈ | Sm/ Mg₆MnO₈ | Eu/ Mg₆MnO₈ | Gd/ Mg₆MnO₈ |
| **(Li,Mg)₆MnO₈** | La/ (Li,Mg)₆MnO₈ | Ce/ (Li,Mg)₆MnO₈ | Pr/ (Li,Mg)₆MnO₈ | Nd/ (Li,Mg)₆MnO₈ | Pm/ (Li,Mg)₆MnO8 | Sm/ (Li,Mg)₆MnO₈ | Eu/ (Li,Mg)₆MnO₈ | Gd/ (Li,Mg)₆MnO₈ |
| **Mn₂O₄** | La/ Mn₂O₄ | Ce/ Mn₂O₄ | Pr/ Mn₂O₄ | Nd/ Mn₂O₄ | Pm/ Mn₂O₄ | Sm/ Mn₂O₄ | Eu/ Mn₂O₄ | Gd/ Mn₂O₄ |
| **Na₄P₂O₇** | La/ Na₄P₂O₇ | Ce/ Na₄P₂O₇ | Pr/ Na₄P₂O₇ | Nd/ Na₄P₂O₇ | Pm/ Na₄P₂O₇ | Sm/ Na₄P₂O₇ | Eu/ Na₄P₂O₇ | Gd/ Na₄P₂O₇ |
| **Mo₂O₈** | La/ Mo₂O₈ | Ce/ Mo₂O₈ | Pr/ Mo₂O₈ | Nd/ Mo₂O₈ | Pm/ Mo₂O₈ | Sm/ Mo₂O₈ | Eu/ Mo₂O₈ | Gd/ Mo₂O₈ |
| **Mn₃O₄/WO₄** | La/ Mn₃O₄/WO₄ | Ce/ Mn₃O₄/WO₄ | Pr/ Mn₃O₄/WO₄ | Nd/ Mn₃O₄/WO₄ | Pm/ Mn₃O₄/WO₄ | Sm/ Mn₃O₄/WO₄ | Eu/ Mn₃O₄/WO₄ | Gd/ Mn₃O₄/WO₄ |
| **Na₂WO₄** | La/ Na₂WO₄ | Ce/ Na₂WO₄ | Pr/ Na₂WO₄ | Nd/ Na₂WO₄ | Pm/ Na₂WO₄ | Sm/ Na₂WO₄ | Eu/ Na₂WO₄ | Gd/ Na₂WO₄ |
| **Zr₂Mo₂O₈** | La/ Zr₂MO₂O₈ | Ce/ Zr₂Mo₂O₈ | Pr/ Zr₂MO₂0₈ | Nd/ Zr₂Mo₂O₈ | Pm/ Zr₂Mo₂O₈ | Sm/ Zr₂MO₂O₈ | Eu/ Zr₂Mo₂O₈ | Gd/ Zr₂Mo₂O₈ |
| **NaMnO₄**-**/Mgo** | La/ NaMnO₄-/MgO | Ce/ NaMnO₄-/MgO | Pr/ NaMnO₄-/MgO | Nd/ NaMnO₄-/MgO | Pm/ NaMnO₄-/MgO | Sm/ NaMnO₄-/MgO | Eu/ NaMnO₄-/MgO | Gd/ NaMnO₄-/MgO |
| **Na₁₀Mn-W₅O₁₇** | La/ Na₁₀Mn-W₅O₁₇ | Ce/ Na₁₀Mn-W₅O₁₇ | Pr/ Na₁₀Mn-W₅O₁₇ | Nd/ Na₁₀Mn-W₅O₁₇ | Pm/ Na₁₀Mn-W₅O₁₇ | Sm/ Na₁₀Mn-W₅O₁₇ | Eu/ Na₁₀Mn-W₅O₁₇ | Gd/ Na₁₀Mn-W₅O₁₇ |

**TABLE 4 NANOWIRES (NW) DOPED WITH SPECIFIC DOPANTS (DOP)**

| **NW\Dop** | **Tb** | **Dy** | **Ho** | **Er** | **Tm** | **Yb** | **Lu** | **In** |
|---|---|---|---|---|---|---|---|---|
| **Li₂O** | Tb/ Li₂O | Dy/ Li₂O | Ho/ Li₂O | Er/ Li₂O | Tm/ Li₂O | Yb/ Li₂O | Lu/ Li₂O | In/ Li₂O |
| **Na₂O** | Tb/ Na₂O | Dy/ Na₂O | Ho/ Na₂O | Er/ Na₂O | Tm/ Na₂O | Yb/ Na₂O | Lu/ Na₂O | In/ Na₂O |
| **K₂O** | Tb/ K₂O | Dy/ K₂O | Ho/ K₂O | Er/ K₂O | Tm/ K₂O | Yb/ K₂O | Lu/ K₂O | In/ K₂O |
| **Rb₂O** | Tb/ Rb₂O | Dy/ Rb₂O | Ho/ Rb₂O | Er/ Rb₂O | Tm/ Rb₂O | Yb/ Rb₂O | Lu/ Rb₂O | In/ Rb₂O |
| **Cs₂O** | Tb/ Cs₂O | Dy/ CS₂O | Ho/ Cs₂O | Er/ CS₂O | Tm/ Cs₂O | Yb/ Cs₂O | Lu/ Cs₂O | In/ Cs₂O |
| **BeO** | Tb/ BeO | Dy/ BeO | Ho/ BeO | Er/ BeO | Tm/ BeO | Yb/ BeO | Lu/ BeO | In/ BeO |
| **MgO** | Tb/ MgO | Dy/ MgO | Ho/ MgO | Er/ MgO | Tm/ MgO | Yb/ MgO | Lu/ MgO | In/ MgO |
| **CaO** | Tb/ CaO | Dy/ CaO | Ho/ CaO | Er/ CaO | Tm/ CaO | Yb/ CaO | Lu/ CaO | In/ CaO |
| **SrO** | Tb/ SrO | Dy/ SrO | Ho/ SrO | Er/ SrO | Tm/ SrO | Yb/ SrO | Lu/ SrO | In/ SrO |
| **BaO** | Tb/ BaO | Dy/ BaO | Ho/ BaO | Er/ BaO | Tm/ BaO | Yb/ BaO | Lu/ BaO | In/ BaO |
| **Sc₂O₃** | Tb/ Sc₂O₃ | Dy/ Sc₂O₃ | Ho/ Sc₂O₃ | Er/ Sc₂O₃ | Tm/ Sc₂O₃ | Yb/ Sc₂O₃ | Lu/ Sc₂O₃ | In/ Sc₂O₃ |
| **Y₂O₃** | Tb/ Y₂O₃ | Dy/ Y₂O₃ | Ho/ Y₂O₃ | Er/ Y₂O₃ | Tm/ Y₂O₃ | Yb/ Y₂O₃ | Lu/ Y₂O₃ | In/ Y₂O₃ |
| **La₂O₃** | Tb/ La₂O₃ | Dy/ La₂O₃ | Ho/ La₂O₃ | Er/ La₂O₃ | Tm/ La₂O₃ | Yb/ La₂O₃ | Lu/ La₂O₃ | In/ La₂O₃ |
| **CeO₂** | Tb/ CeO₂ | Dy/ CeO₂ | Ho/ CeO₂ | Er/ CeO₂ | Tm/ CeO₂ | Yb/ CeO₂ | Lu/ CeO₂ | In/ CeO₂ |
| **Ce₂O₃** | Tb/ Ce₂O₃ | Dy/ Ce₂O₃ | Ho/ Ce₂O₃ | Er/ Ce₂O₃ | Tm/ Ce₂O₃ | Yb/ Ce₂O₃ | Lu/ Ce₂O₃ | In/ Ce₂O₃ |
| **Pr₂O₃** | Tb/ Pr₂O₃ | Dy/ Pr₂O₃ | Ho/ Pr₂O₃ | Er/ Pr₂O₃ | Tm/ Pr₂O₃ | Yb/ Pr₂O₃ | Lu/ Pr₂O₃ | In/ Pr₂O₃ |
| **Nd₂O₃** | Tb/ Nd₂O₃ | Dy/ Nd₂O₃ | Ho/ Nd₂O₃ | Er/ Nd₂O₃ | Tm/ Nd₂O₃ | Yb/ Nd₂O₃ | Lu/ Nd₂O₃ | In/ Nd₂O₃ |
| **Sm₂O₃** | Tb/ Sm₂O₃ | Dy/ Sm₂O₃ | Ho/ Sm₂O₃ | Er/ Sm₂O₃ | Tm/ Sm₂O₃ | Yb/ Sm₂O₃ | Lu/ Sm₂O₃ | In/ Sm₂O₃ |
| **Eu₂O₃** | Tb/ Eu₂O₃ | Dy/ Eu₂O₃ | Ho/ Eu₂O₃ | Er/ Eu₂O₃ | Tm/ Eu₂O₃ | Yb/ Eu₂O₃ | Lu/ Eu₂O₃ | In/ Eu₂O₃ |
| **Gd₂O₃** | Tb/ Gd₂O₃ | Dy/ Gd₂O₃ | Ho/ Gd₂O₃ | Er/ Gd₂O₃ | Tm/ Gd₂O₃ | Yb/ Gd₂O₃ | Lu/ Gd₂O₃ | In/ Gd₂O₃ |
| **Tb₂O₃** | Tb/ Tb₂O₃ | Dy/ Tb₂O₃ | Ho/ Tb₂O₃ | Er/ Tb₂O₃ | Tm/ Tb₂O₃ | Yb/ Tb₂O₃ | Lu/ Tb₂O₃ | In/ Tb₂O₃ |
| **TbO₂** | Tb/ TbO₂ | Dy/ TbO₂ | Ho/ TbO₂ | Er/ TbO₂ | Tm/ TbO₂ | Yb/ TbO₂ | Lu/ TbO₂ | In/ TbO₂ |
| **Tb₆O₁₁** | Tb/ Tb₆O₁₁ | Dy/ Tb₆O₁₁ | Ho/ Tb₆O₁₁ | Er/ Tb₆O₁₁ | Tm/ Tb₆O₁₁ | Yb/ Tb₆O₁₁ | Lu/ Tb₆O₁₁ | In/ Tb₆O₁₁ |
| **Dy₂O₃** | Tb/ Dy₂O₃ | Dy/ Dy₂O₃ | Ho/ Dy₂O₃ | Er/ Dy₂O₃ | Tm/ Dy₂O₃ | Yb/ Dy₂O₃ | Lu/ Dy₂O₃ | In/ Dy₂O₃ |
| **Ho₂O₃** | Tb/ Ho₂O₃ | Dy/ Ho₂O₃ | Ho/ Ho₂O₃ | Er/ Ho₂O₃ | Tm/ Ho₂O₃ | Yb/ Ho₂O₃ | Lu/ Ho₂O₃ | In/ Ho₂O₃ |
| **Er₂O₃** | Tb/ Er₂O₃ | Dy/ Er₂O₃ | Ho/ Er₂O₃ | Er/ Er₂O₃ | Tm/ Er₂O₃ | Yb/ Er₂O₃ | Lu/ Er₂O₃ | In/ Er₂O₃ |
| **Tm₂O₃** | Tb/ Tm₂O₃ | Dy/ Tm₂O₃ | Ho/ Tm₂O₃ | Er/ Tm₂O₃ | Tm/ Tm₂O₃ | Yb/ Tm₂O₃ | Lu/ Tm₂O₃ | In/ Tm₂O₃ |
| **Yb₂O₃** | Tb/ Yb₂O₃ | Dy/ Yb₂O₃ | Ho/ Yb₂O₃ | Er/ Yb₂O₃ | Tm/ Yb₂O₃ | Yb/ Yb₂O₃ | Lu/ Yb₂O₃ | In/ Yb₂O₃ |
| **Lu₂O₃** | Tb/ Lu₂O₃ | Dy/ Lu₂O₃ | Ho/ Lu₂O₃ | Er/ Lu₂O₃ | Tm/ Lu₂O₃ | Yb/ Lu₂O₃ | Lu/ Lu₂O₃ | In/ Lu₂O₃ |
| **Ac₂O₃** | Tb/ Ac₂O₃ | Dy/ Ac₂O₃ | Ho/ Ac₂O₃ | Er/ Ac₂O₃ | Tm/ Ac₂O₃ | Yb/ Ac₂O₃ | Lu/ Ac₂O₃ | In/ Ac₂O₃ |
| **Th₂O₃** | Tb/ Th₂O₃ | Dy/ Th₂O₃ | Ho/ Th₂O₃ | Er/ Th₂O₃ | Tm/ Th₂O₃ | Yb/ Th₂O₃ | Lu/ Th₂O₃ | In/ Th₂O₃ |
| **ThO₂** | Tb/ ThO₂ | Dy/ ThO₂ | Ho/ ThO₂ | Er/ ThO₂ | Tm/ ThO₂ | Yb/ ThO₂ | Lu/ ThO₂ | In/ ThO₂ |
| **Pa₂O₃** | Tb/ Pa₂O₃ | Dy/ Pa₂O₃ | Ho/ Pa₂O₃ | Er/ Pa₂O₃ | Tm/ Pa₂O₃ | Yb/ Pa₂O₃ | Lu/ Pa₂O₃ | In/ Pa₂O₃ |
| **PaO₂** | Tb/ PaO₂ | Dy/ PaO₂ | Ho/ PaO₂ | Er/ PaO₂ | Tm/ PaO₂ | Yb/ PaO₂ | Lu/ PaO₂ | In/ PaO₂ |
| **TiO₂** | Tb/ TiO₂ | Dy/ TiO₂ | Ho/ TiO₂ | Er/ TiO₂ | Tm/ TiO₂ | Yb/ TiO₂ | Lu/ TiO₂ | In/ TiO₂ |
| **TiO** | Tb/ TiO | Dy/ TiO | Ho/ TiO | Er/ TiO | Tm/ TiO | Yb/ TiO | Lu/ TiO | In/ TiO |
| **Ti₂O₃** | Tb/ Ti₂O₃ | Dy/ Ti₂O₃ | Ho/ Ti₂O₃ | Er/ Ti₂O₃ | Tm/ Ti₂O₃ | Yb/ Ti₂O₃ | Lu/ Ti₂O₃ | In/ Ti₂O₃ |
| **Ti₃O** | Tb/ Ti₃O | Dy/ Ti₃O | Ho/ Ti₃O | Er/ Ti₃O | Tm/ Ti₃O | Yb/ Ti₃O | Lu/ Ti₃O | In/ Ti₃O |
| **Ti₂O** | Tb/ Ti₂O | Dy/ Ti₂O | Ho/ Ti₂O | Er/ Ti₂O | Tm/ Ti₂O | Yb/ Ti₂O | Lu/ Ti₂O | In/ Ti₂O |
| **Ti₃O₅** | Tb/ Ti₃O₅ | Dy/ Ti₃O₅ | Ho/ Ti₃O₅ | Er/ Ti₃O₅ | Tm/ Ti₃O₅ | Yb/ Ti₃O₅ | Lu/ Ti₃O₅ | In/ Ti₃O₅ |
| **Ti₄O₇** | Tb/ Ti₄O₇ | Dy/ Ti₄O₇ | Ho/ Ti₄O₇ | Er/ Ti₄O₇ | Tm/ Ti₄O₇ | Yb/ Ti₄O₇ | Lu/ Ti₄O₇ | In/ Ti₄O₇ |
| **ZrO₂** | Tb/ ZrO₂ | Dy/ ZrO₂ | Ho/ ZrO₂ | Er/ ZrO₂ | Tm/ ZrO₂ | Yb/ ZrO₂ | Lu/ ZrO₂ | In/ ZrO₂ |
| **HfO₂** | Tb/ HfO₂ | Dy/ HfO₂ | Ho/ HfO₂ | Er/ HfO₂ | Tm/ HfO₂ | Yb/ HfO₂ | Lu/ HfO₂ | In/ HfO₂ |
| **VO** | Tb/ VO | Dy/ VO | Ho/ VO | Er/ VO | Tm/ VO | Yb/ VO | Lu/ VO | In/ VO |
| **V₂O₃** | Tb/ V₂O₃ | Dy/ V₂O₃ | Ho/ V₂O₃ | Er/ V₂O₃ | Tm/ V₂O₃ | Yb/ V₂O₃ | Lu/ V₂O₃ | In/ V₂O₃ |
| **VO₂** | Tb/ VO₂ | Dy/ VO₂ | Ho/ VO₂ | Er/ VO₂ | Tm/ VO₂ | Yb/ VO₂ | Lu/ VO₂ | In/ VO₂ |
| **V₂O₅** | Tb/ V₂O₅ | Dy/ V₂O₅ | Ho/ V₂O₅ | Er/ V₂O₅ | Tm/ V₂O₅ | Yb/ V₂O₅ | Lu/ V₂O₅ | In/ V₂O₅ |
| **V₃O₇** | Tb/ V₃O₇ | Dy/ V₃O₇ | Ho/ V₃O₇ | Er/ V₃O₇ | Tm/ V₃O₇ | Yb/ V₃O₇ | Lu/ V₃O₇ | In/ V₃O₇ |
| **V₄O₉** | Tb/ V₄O₉ | Dy/ V₄O₉ | Ho/ V₄O₉ | Er/ V₄O₉ | Tm/ V₄O₉ | Yb/ V₄O₉ | Lu/ V₄O₉ | In/ V₄O₉ |
| **V₆O₁₃** | Tb/ V₆O₁₃ | Dy/ V₆O₁₃ | Ho/ V₆O₁₃ | Er/ V₆O₁₃ | Tm/ V₆O₁₃ | Yb/ V₆O₁₃ | Lu/ V₆O₁₃ | In/ V₆O₁₃ |
| **NbO** | Tb/ NbO | Dy/ NbO | Ho/ NbO | Er/ NbO | Tm/ NbO | Yb/ NbO | Lu/ NbO | In/ NbO |
| **NbO₂** | Tb/ NbO₂ | Dy/ NbO₂ | Ho/ NbO₂ | Er/ NbO₂ | Tm/ NbO₂ | Yb/ NbO₂ | Lu/ NbO₂ | In/ NbO₂ |
| **Nb₂O₅** | Tb/ Nb₂O₅ | Dy/ Nb₂O₅ | Ho/ Nb₂O₅ | Er/ Nb₂O₅ | Tm/ Nb₂O₅ | Yb/ Nb₂O₅ | Lu/ Nb₂O₅ | In/ Nb₂O₅ |
| **Nb₈O₁₉** | Tb/ Nb₈O₁₉ | Dy/ Nb₈O₁₉ | Ho/ Nb₈O₁₉ | Er/ Nb₈O₁₉ | Tm/ Nb₈O₁₉ | Yb/ Nb₈O₁₉ | Lu/ Nb₈O₁₉ | In/ Nb₈O₁₉ |
| **Nb₁₆O₃₈** | Tb/ Nb₁₆O₃₈ | Dy/ Nb₁₆O₃₈ | Ho/ Nb₁₆O₃₈ | Er/ Nb₁₆O₃₈ | Tm/ Nb₁₆O₃₈ | Yb/ Nb₁₆O₃₈ | Lu/ Nb₁₆O₃₈ | In/ Nb₁₆O₃₈ |
| **Nb₁₂O₂₉** | Tb/ Nb₁₂O₂₉ | Dy/ Nb₁₂O₂₉ | Ho/ Nb₁₂O₂₉ | Er/ Nb₁₂O₂₉ | Tm/ Nb₁₂O₂₉ | Yb/ Nb₁₂O₂₉ | Lu/ Nb₁₂O₂₉ | In/ Nb₁₂O₂₉ |
| **Nb₄₇O₁₁₆** | Tb/ Nb₄₇O₁₁₆ | Dy/ Nb₄₇O₁₁₆ | Ho/ Nib₄₇O₁₁₆ | Er/ Nb₄₇O₁₁₆ | Tm/ Nb₄₇O₁₁₆ | Yb/ Nb₄₇O₁₁₆ | Lu/ Nb₄₇O₁₁₆ | In/ Nb₄₇O₁₁₆ |
| **Ta₂O₅** | Tb/ Ta₂O₅ | Dy/ Ta₂O₅ | Ho/ Ta₂O₅ | Er/ Ta₂O₅ | Tm/ Ta₂O₅ | Yb/ Ta₂O₅ | Lu/ Ta₂O₅ | In/ Ta₂O₅ |
| **CrO** | Tb/ CrO | Dy/ CrO | Ho/ CrO | Er/ CrO | Tm/ CrO | Yb/ CrO | Lu/ CrO | In/ CrO |
| **Cr₂O₃** | Tb/ Cr₂O₃ | Dy/ Cr₂O₃ | Ho/ Cr₂O₃ | Er/ Cr₂O₃ | Tm/ Cr₂O₃ | Yb/ Cr₂O₃ | Lu/ Cr₂O₃ | In/ Cr₂O₃ |
| **CrO₂** | Tb/ CrO₂ | Dy/ CrO₂ | Ho/ CrO₂ | Er/ CrO₂ | Tm/ CrO₂ | Yb/ CrO₂ | Lu/ CrO₂ | In/ CrO₂ |
| **CrO₃** | Tb/ CrO₃ | Dy/ CrO₃ | Ho/ CrO₃ | Er/ CrO₃ | Tm/ CrO₃ | Yb/ CrO₃ | Lu/ CrO₃ | In/ CrO₃ |
| **Cr₈O₂₁** | Tb/ Cr₈O₂₁ | Dy/ Cr₈O₂₁ | Ho/ Cr₈O₂₁ | Er/ Cr₈O₂₁ | Tm/ Cr₈O₂₁ | Yb/ Cr₈O₂₁ | Lu/ Cr₈O₂₁ | In/ Cr₈O₂₁ |
| **MoO₂** | Tb/ MoO₂ | Dy/ MoO₂ | Ho/ MoO₂ | Er/ MoO₂ | Tm/ MoO₂ | Yb/ MoO₂ | Lu/ MoO₂ | In/ MoO₂ |
| **MoO₃** | Tb/ MoO₃ | Dy/ MoO₃ | Ho/ MoO₃ | Er/ MoO₃ | Tm/ MoO₃ | Yb/ MoO₃ | Lu/ MoO₃ | In/ MoO₃ |
| **W₂O₃** | Tb/ W₂O₃ | Dy/ W₂O₃ | Ho/ W₂O₃ | Er/ W₂O₃ | Tm/ W₂O₃ | Yb/ W₂O₃ | Lu/ W₂O₃ | In/ W₂O₃ |
| **WoO₂** | Tb/ WoO₂ | Dy/ WoO₂ | Ho/ WoO₂ | Er/ WoO₂ | Tm/ WoO₂ | Yb/ WoO₂ | Lu/ WoO₂ | In/ WoO₂ |
| **WoO₃** | Tb/ WoO₃ | Dy/ WoO₃ | Ho/ WoO₃ | Er/ WoO₃ | Tm/ WoO₃ | Yb/ WoO₃ | Lu/ WoO₃ | In/ WoO₃ |
| **MnO** | Tb/ MnO | Dy/ MnO | Ho/ MnO | Er/ MnO | Tm/ MnO | Yb/ MnO | Lu/ MnO | In/ MnO |
| **Mn/Mg/O** | Tb/ Mn/Mg/O | Dy/ Mn/Mg/O | Ho/ Mn/Mg/O | Er/ Mn/Mg/O | Tm/ Mn/Mg/O | Yb/ Mn/Mg/O | Lu/ Mn/Mg/O | In/ Mn/Mg/O |
| **Mn₃O₄** | Tb/ Mn₃O4 | Dy/ Mn₃O₄ | Ho/ Mn₃O₄ | Er/ Mn₃O₄ | Tm/ Mn₃O₄ | Yb/ Mn₃O₄ | Lu/ Mn₃O₄ | In/ Mn₃O₄ |
| **Mn₂O₃** | Tb/ Mn₂O₃ | Dy/ Mn₂O₃ | Ho/ Mn₂O₃ | Er/ Mn₂O₃ | Tm/ Mn₂O₃ | Yb/ Mn₂O₃ | Lu/ Mn₂O₃ | In/ Mn₂O₃ |
| **MnO₂** | Tb/ MnO₂ | Dy/ MnO₂ | Ho/ MnO₂ | Er/ MnO₂ | Tm/ MnO₂ | Yb/ MnO₂ | Lu/ MnO₂ | In/ MnO₂ |
| **Mn₂O₇** | Tb/ Mn₂O₇ | Dy/ Mn₂O₇ | Ho/ Mn₂O₇ | Er/ Mn₂O₇ | Tm/ Mn₂O₇ | Yb/ Mn₂O₇ | Lu/ Mn₂O₇ | In/ Mn₂O₇ |
| **ReO₂** | Tb/ ReO₂ | Dy/ ReO₂ | Ho/ ReO₂ | Er/ ReO₂ | Tm/ ReO₂ | Yb/ ReO₂ | Lu/ ReO₂ | In/ ReO₂ |
| **ReO₃** | Tb/ ReO₃ | Dy/ ReO₃ | Ho/ ReO₃ | Er/ ReO₃ | Tm/ ReO₃ | Yb/ ReO₃ | Lu/ ReO₃ | In/ ReO₃ |
| **Re₂O₇** | Tb/ Re₂O₇ | Dy/ Re₂O₇ | Ho/ Re₂O₇ | Er/ Re₂O₇ | Tm/ Re₂O₇ | Yb/ Re₂O₇ | Lu/ Re₂O₇ | In/ Re₂O₇ |
| **Mg₃Mn₃**-**B₂O₁₀** | Tb/ Mg₃Mn₃-B₂O₁₀ | Dy/ Mg₃Mn₃-B₂O₁₀ | Ho/ Mg₃Mn₃-B₂O₁₀ | Er/ Mg₃Mn₃-B₂O₁₀ | Tm/ Mg₃Mn₃-B₂O₁₀ | Yb/ Mg₃Mn₃-B₂O₁₀ | Lu/ Mg₃Mn3-B₂0₁₀ | In/ Mg₃Mn₃-B₂O₁₀ |
| **Mg₃(BO₃)₂** | Tb/ Mg₃(BO₃)₂ | Dy/ Mg₃(BO₃)₂ | Ho/ Mg₃(BO₃)₂ | Er/ Mg₃(BO₃)₂ | Tm/ Mg₃(BO₃)₂ | Yb/ Mg₃(BO₃)₂ | Lu/ Mg₃(BO₃)₂ | In/ Mg₃(BO₃)₂ |
| **NaWO₄** | Tb/ NaWO₄ | Dy/ NaWO₄ | Ho/ NaWO₄ | Er/ NaWO₄ | Tm/ NaWO₄ | Yb/ NaWO₄ | Lu/ NaWO₄ | In/ NaWO₄ |
| **Mg₆MnO₈** | Tb/ Mg₆MnO₈ | Dy/ Mg₆MnO₈ | Ho/ Mg₆MnO₈ | Er/ Mg₆MnO₈ | Tm/ Mg₆MnO₈ | Yb/ Mg₆MnO₈ | Lu/ Mg₆MnO₈ | In/ Mg₆MnO₈ |
| **Mn₂O₄** | Tb/ Mn₂O₄ | Dy/ Mn₂O₄ | Ho/ Mn₂O₄ | Er/ Mn₂O₄ | Tm/ Mn₂O₄ | Yb/ Mn₂O₄ | Lu/ Mn₂O₄ | In/ Mn₂O₄ |
| **(Li,Mg)₆MnO₈** | Tb/ (Li,Mg)₆MnO₈ | Dy/ (Li,Mg)₆MnO₈ | Ho/ (Li,Mg)₆MnO₈ | Er/ (Li,Mg)₆MnO₈ | Tm/ (Li,Mg)₆MnO₈ | Yb/ (Li,Mg)₆MnO₈ | Lu/ (Li,Mg)₆MnO₈ | In/ (Li,Mg)₆MnO₈ |
| **Na₄P₂O₇** | Tb/ Na₄P₂O₇ | Dy/ Na₄P₂O₇ | Ho/ Na₄P₂O₇ | Er/ Na₄P₂O₇ | Tm/ Na₄P₂O₇ | Yb/ Na₄P₂O₇ | Lu/ Na₄P₂O₇ | In/ Na₄P₂O₇ |
| **Mo₂O₈** | Tb/ Mo₂O₈ | Dy/ Mo₂O₈ | Ho/ Mo₂O₈ | Er/ Mo₂O₈ | Tm/ Mo₂O₈ | Yb/ Mo₂O₈ | Lu/ Mo₂O₈ | In/ Mo₂O₈ |
| **Mn₃O₄/WO₄** | Tb/ Mn₃O₄/WO₄ | Dy/ Mn₃O₄/WO₄ | Ho/ Mn₃O₄/WO₄ | Er/ Mn₃O₄/WO₄ | Tm/ Mn₃O₄/WO₄ | Yb/ Mn₃O₄/WO₄ | Lu/ Mn₃O₄/WO₄ | In/ Mn₃O₄/WO₄ |
| **Na₂WO₄** | Tb/ Na₂WO₄ | Dy/ Na₂WO₄ | Ho/ Na₂WO₄ | Er/ Na₂WO₄ | Tm/ Na₂WO₄ | Yb/ Na₂WO₄ | Lu/ Na₂WO₄ | In/ Na₂WO₄ |
| **Zr₂Mo₂O₈** | Tb/ Zr₂Mo₂O₈ | Dy/ Zr₂Mo₂O₈ | Ho/ Zr₂Mo₂O₈ | Er/ Zr₂Mo₂O₈ | Tm/ Zr₂Mo₂O₈ | Yb/ Zr₂Mo₂O₈ | Lu/ Zr₂MO₂O₈ | In/ Zr₂Mo₂O₈ |
| **NaMnO₄**-**/Mgo** | Tb/ NaMnO₄-/MgO | Dy/ NaMnO₄-/MgO | Ho/ NaMnO₄-/MgO | Er/ NaMnO₄-/MgO | Tm/ NaMnO₄-/MgO | Yb/ NaMnO₄-/MgO | Lu/ NaMnO₄-/MgO | In/ NaMnO₄-/MgO |
| **Na₁₀Mn-W₅O₁₇** | Tb/ Na₁₀Mn-W₅O₁₇ | Dy/ Na₁₀Mn-W₅O₁₇ | Ho/ Na₁₀Mn-W₅O₁₇ | Er/ Na₁₀Mn-W₅O₁₇ | Tm/ Na₁₀Mn-W₅O₁₇ | Yb/ Na₁₀Mn-W₅O₁₇ | Lu/ Na₁₀Mn-W₅O₁₇ | In/ Na₁₀Mn-W₅O₁₇ |

**TABLE 5 NANOWIRES (NW) DOPED WITH SPECIFIC DOPANTS (DOP)**

| **NW\Dop** | **Y** | **Sc** | **Al** | **Cu** | **Ga** | **Hf** | **Fe** | **Cr** |
|---|---|---|---|---|---|---|---|---|
| **Li₂O** | Y/ Li₂O | Sc/ Li₂O | Al/ Li₂O | Cu/ Li₂O | Ga/ Li₂O | Hf/ Li₂O | Fe/ Li₂O | Cr/ Li₂O |
| **Na₂O** | Y/ Na₂O | Sc/ Na₂O | Al/ Na₂O | Cu/ Na₂O | Ga/ Na₂O | Hf/ Na₂O | Fe/ Na₂O | Cr/ Na₂O |
| **K₂O** | Y/ K₂O | Sc/ K₂O | Al/ K₂O | Cu/ K₂O | Ga/ K₂O | Hf/ K₂O | Fe/ K₂O | Cr/ K₂O |
| **Rb₂O** | Y/ Rb₂O | Sc/ Rb₂O | Al/ Rb₂O | Cu/ Rb₂O | Ga/ Rb₂O | Hf/ Rb₂O | Fe/ Rb₂O | Cr/ Rb₂O |
| **Cs₂O** | Y/ Cs₂O | Sc/ Cs₂O | Al/ Cs₂O | Cu/ Cs₂O | Ga/ Cs₂O | Hf/ Cs₂O | Fe/ Cs₂O | Cr/ CS₂O |
| **BeO** | Y/ BeO | Sc/ BeO | Al/ BeO | Cu/ BeO | Ga/ BeO | Hf/ BeO | Fe/ BeO | Cr/ BeO |
| **MgO** | Y/ MgO | Sc/ MgO | Al/ MgO | Cu/ MgO | Ga/ MgO | Hf/ MgO | Fe/ MgO | Cr/ MgO |
| **CaO** | Y/ CaO | Sc/ CaO | Al/ CaO | Cu/ CaO | Ga/ CaO | Hf/ CaO | Fe/ CaO | Cr/ CaO |
| **SrO** | Y/ SrO | Sc/ SrO | Al/ SrO | Cu/ SrO | Ga/ SrO | Hf/ SrO | Fe/ SrO | Cr/ SrO |
| **BaO** | Y/ BaO | Sc/ BaO | Al/ BaO | Cu/ BaO | Ga/ BaO | Hf/ BaO | Fe/ BaO | Cr/ BaO |
| **SC₂O₃** | Y/ SC₂O₃ | Sc/ SC₂O₃ | Al/ SC₂O₃ | Cu/ SC₂O₃ | Ga/ Sc₂O₃ | Hf/ SC₂O₃ | Fe/ SC₂O₃ | Cr/ SC₂O₃ |
| **Y₂O₃** | Y/ Y₂O₃ | Sc/ Y₂O₃ | Al/ Y₂O₃ | Cu/ Y₂O₃ | Ga/ Y₂O₃ | Hf/ Y₂O₃ | Fe/ Y₂O₃ | Cr/ Y₂O₃ |
| **La₂O₃** | Y/ La₂O₃ | Sc/ La₂O₃ | Al/ La₂O₃ | Cu/ La₂O₃ | Ga/ La₂O₃ | Hf/ La₂O₃ | Fe/ La₂O₃ | Cr/ La₂O₃ |
| **CeO₂** | Y/ CeO₂ | Sc/ CeO₂ | Al/ CeO₂ | Cu/ CeO₂ | Ga/ CeO₂ | Hf/ CeO₂ | Fe/ CeO₂ | Cr/ CeO₂ |
| **Ce₂O₃** | Y/ Ce₂O₃ | Sc/ Ce₂O₃ | Al/ Ce₂O₃ | Cu/ Ce₂O₃ | Ga/ Ce₂O₃ | Hf/ Ce₂O₃ | Fe/ Ce₂O₃ | Cr/ Ce₂O₃ |
| **Pr₂O₃** | Y/ Pr₂O₃ | Sc/ Pr₂O₃ | Al/ Pr₂O₃ | Cu/ Pr₂O₃ | Ga/ Pr₂O₃ | Hf/ Pr₂O₃ | Fe/ Pr₂O₃ | Cr/ Pr₂O₃ |
| **Nd₂O₃** | Y/ Nd₂O₃ | Sc/ Nd₂O₃ | Al/ Nd₂O₃ | Cu/ Nd₂O₃ | Ga/ Nd₂O₃ | Hf/ Nd₂O₃ | Fe/ Nd₂O₃ | Cr/ Nd₂O₃ |
| **Sm₂O₃** | Y/ Sm₂O₃ | Sc/ Sm₂O₃ | Al/ Sm₂O₃ | Cu/ Sm₂O₃ | Ga/ Sm₂O₃ | Hf/ Sm₂O₃ | Fe/ Sm₂O₃ | Cr/ Sm₂O₃ |
| **Eu₂O₃** | Y/ EU₂O₃ | Sc/ EU₂O₃ | Al/ Eu₂O₃ | Cu/ Eu₂O₃ | Ga/ Eu₂O₃ | Hf/ Eu₂O₃ | Fe/ EU₂O₃ | Cr/ EU₂O₃ |
| **Gd₂O₃** | Y/ Gd₂O₃ | Sc/ Gd₂O₃ | Al/ Gd₂O₃ | Cu/ Gd₂O₃ | Ga/ Gd₂O₃ | Hf/ Gd₂O₃ | Fe/ Gd₂O₃ | Cr/ Gd₂O₃ |
| **Tb₂O₃** | Y/ Tb₂O₃ | Sc/ Tb₂O₃ | Al/ Tb₂O₃ | Cu/ Tb₂O₃ | Ga/ Tb₂O₃ | Hf/ Tb₂O₃ | Fe/ Tb₂O₃ | Cr/ Tb₂O₃ |
| **TbO₂** | Y/ TbO₂ | Sc/ TbO₂ | Al/ TbO₂ | Cu/ TbO₂ | Ga/ TbO₂ | Hf/ TbO₂ | Fe/ TbO₂ | Cr/ TbO₂ |
| **Tb₆O₁₁** | Y/ Tb₆O₁₁ | Sc/ Tb₆O₁₁ | Al/ Tb₆O₁₁ | Cu/ Tb₆O₁₁ | Ga/ Tb₆O₁₁ | Hf/ Tb₆O₁₁ | Fe/ Tb₆O₁₁ | Cr/ Tb₆O₁₁ |
| **Dy₂O₃** | Y/ Dy₂O₃ | Sc/ Dy₂O₃ | Al/ Dy₂O₃ | Cu/ Dy₂O₃ | Ga/ Dy₂O₃ | Hf/ Dy₂O₃ | Fe/ Dy₂O₃ | Cr/ Dy₂O₃ |
| **Ho₂O₃** | Y/ Ho₂O₃ | Sc/ Ho₂O₃ | Al/ Ho₂O₃ | Cu/ Ho₂O₃ | Ga/ Ho₂O₃ | Hf/ Ho₂O₃ | Fe/ Ho₂O₃ | Cr/ Ho₂O₃ |
| **Er₂O₃** | Y/ Er₂O₃ | Sc/ Er₂O₃ | Al/ Er₂O₃ | Cu/ Er₂O₃ | Ga/ Er₂O₃ | Hf/ Er₂O₃ | Fe/ Er₂O₃ | Cr/ Er₂O₃ |
| **Tm₂O₃** | Y/ Tm₂O₃ | Sc/ Tm₂O₃ | Al/ Tm₂O₃ | Cu/ Tm₂O₃ | Ga/ Tm₂O₃ | Hf/ Tm₂O₃ | Fe/ Tm₂O₃ | Cr/ Tm₂O₃ |
| **Yb₂O₃** | Y/ Yb₂O₃ | Sc/ Yb₂O₃ | Al/ Yb₂O₃ | Cu/ Yb₂O₃ | Ga/ Yb₂O₃ | Hf/ Yb₂O₃ | Fe/ Yb₂O₃ | Cr/ Yb₂O₃ |
| **Lu₂O₃** | Y/ Lu₂O₃ | Sc/ Lu₂O₃ | Al/ Lu₂O₃ | Cu/ Lu₂O₃ | Ga/ Lu₂O₃ | Hf/ Lu₂O₃ | Fe/ Lu₂O₃ | Cr/ Lu₂O₃ |
| **Ac₂O₃** | Y/ Ac₂O₃ | Sc/ Ac₂O₃ | Al/ Ac₂O₃ | Cu/ Ac₂O₃ | Ga/ Ac₂O₃ | Hf/ Ac₂O₃ | Fe/ Ac₂O₃ | Cr/ Ac₂O₃ |
| **Th₂O₃** | Y/ Th₂O₃ | Sc/ Th₂O₃ | Al/ Th₂O₃ | Cu/ Th₂O₃ | Ga/ Th₂O₃ | Hf/ Th₂O₃ | Fe/ Th₂O₃ | Cr/ Th₂O₃ |
| **ThO₂** | Y/ ThO₂ | Sc/ ThO₂ | Al/ ThO₂ | Cu/ ThO₂ | Ga/ ThO₂ | Hf/ ThO₂ | Fe/ ThO₂ | Cr/ ThO₂ |
| **Pa₂O₃** | Y/ Pa₂O₃ | Sc/ Pa₂O₃ | Al/ Pa₂O₃ | Cu/ Pa₂O₃ | Ga/ Pa₂O₃ | Hf/ Pa₂O₃ | Fe/ Pa₂O₃ | Cr/ Pa₂O₃ |
| **PaO₂** | Y/ PaO₂ | Sc/ PaO₂ | Al/ PaO₂ | Cu/ PaO₂ | Ga/ PaO₂ | Hf/ PaO₂ | Fe/ PaO₂ | Cr/ PaO₂ |
| **TiO₂** | Y/ TiO₂ | Sc/ TiO₂ | Al/ TiO₂ | Cu/ TiO₂ | Ga/ TiO₂ | Hf/ TiO₂ | Fe/ TiO₂ | Cr/ TiO₂ |
| **TiO** | Y/ TiO | Sc/ TiO | Al/ TiO | Cu/ TiO | Ga/ TiO | Hf/ TiO | Fe/ TiO | Cr/ TiO |
| **Ti₂O₃** | Y/ Ti₂O₃ | Sc/ Ti₂O₃ | Al/ Ti₂O₃ | Cu/ Ti₂O₃ | Ga/ Ti₂O₃ | Hf/ Ti₂O₃ | Fe/ Ti₂O₃ | Cr/ Ti₂O₃ |
| **Ti₃O** | Y/ Ti₃O | Sc/ Ti₃O | Al/ Ti₃O | Cu/ Ti₃O | Ga/ Ti₃O | Hf/ Ti₃O | Fe/ Ti₃O | Cr/ Ti₃O |
| **Ti₂O** | Y/ Ti₂O | Sc/ Ti₂O | Al/ Ti₂O | Cu/ Ti₂O | Ga/ Ti₂O | Hf/ Ti₂O | Fe/ Ti₂O | Cr/ Ti₂O |
| **Ti₃O₅** | Y/ Ti₃O₅ | Sc/ Ti₃O₅ | Al/ Ti₃O₅ | Cu/ Ti₃O₅ | Ga/ Ti₃O₅ | Hf/ Ti₃O₅ | Fe/ Ti₃O₅ | Cr/ Ti₃O₅ |
| **Ti₄O₇** | Y/ Ti₄O₇ | Sc/ Ti₄O₇ | Al/ Ti₄O₇ | Cu/ Ti₄O₇ | Ga/ Ti₄O₇ | Hf/ Ti₄O₇ | Fe/ Ti₄O₇ | Cr/ Ti₄O₇ |
| **ZrO₂** | Y/ ZrO₂ | Sc/ ZrO₂ | Al/ ZrO₂ | Cu/ ZrO₂ | Ga/ ZrO₂ | Hf/ ZrO₂ | Fe/ ZrO₂ | Cr/ ZrO₂ |
| **HfO₂** | Y/ HfO₂ | Sc/ HfO₂ | Al/ HfO₂ | Cu/ HfO₂ | Ga/ HfO₂ | Hf/ HfO₂ | Fe/ HfO₂ | Cr/ HfO₂ |
| **VO** | Y/ VO | Sc/ VO | Al/ VO | Cu/ VO | Ga/ VO | Hf/ VO | Fe/ VO | Cr/ VO |
| **V₂O₃** | Y/ V₂O₃ | Sc/ V₂O₃ | Al/ V₂O₃ | Cu/ V₂O₃ | Ga/ V₂O₃ | Hf/ V₂O₃ | Fe/ V₂O₃ | Cr/ V₂O₃ |
| **VO₂** | Y/ VO₂ | Sc/ VO₂ | Al/ VO₂ | Cu/ VO₂ | Ga/ VO₂ | Hf/ VO₂ | Fe/ VO₂ | Cr/ VO₂ |
| **V₂O₅** | Y/ V₂O₅ | Sc/ V₂O₅ | Al/ V₂O₅ | Cu/ V₂O₅ | Ga/ V₂O₅ | Hf/ V₂O₅ | Fe/ V₂O₅ | Cr/ V₂O₅ |
| **V₃O₇** | Y/ V₃O₇ | Sc/ V₃O₇ | Al/ V₃O₇ | Cu/ V₃O₇ | Ga/ V₃O₇ | Hf/ V₃O₇ | Fe/ V₃O₇ | Cr/ V₃O₇ |
| **V₄O₉** | Y/ V₄O₉ | Sc/ V₄O₉ | Al/ V₄O₉ | Cu/ V₄O₉ | Ga/ V₄O₉ | Hf/ V₄O₉ | Fe/ V₄O₉ | Cr/ V₄O₉ |
| **V₆O₁₃** | Y/ V₆O₁₃ | Sc/ V₆O₁₃ | Al/ V₆O₁₃ | Cu/ V₆O₁₃ | Ga/ V₆O₁₃ | Hf/ V₆O₁₃ | Fe/ V₆O₁₃ | Cr/ V₆O₁₃ |
| **NbO** | Y/ NbO | Sc/ NbO | Al/ NbO | Cu/ NbO | Ga/ NbO | Hf/ NbO | Fe/ NbO | Cr/ NbO |
| **NbO₂** | Y/ NbO₂ | Sc/ NbO₂ | Al/ NbO₂ | Cu/ NbO₂ | Ga/ NbO₂ | Hf/ NbO₂ | Fe/ NbO₂ | Cr/ NbO₂ |
| **Nb₂O₅** | Y/ Nb₂O₅ | Sc/ Nb₂O₅ | Al/ Nb₂O₅ | Cu/ Nb₂O₅ | Ga/ Nb₂O₅ | Hf/ Nb₂O₅ | Fe/ Nb₂O₅ | Cr/ Nb₂O₅ |
| **Nb₈O₁₉** | Y/ Nb₈O₁₉ | Sc/ Nb₈O₁₉ | Al/ Nb₈O₁₉ | Cu/ Nb₈O₁₉ | Ga/ Nb₈O₁₉ | Hf/ Nb₈O₁₉ | Fe/ Nb₈O₁₉ | Cr/ Nb₈O₁₉ |
| **Nb₁₆O₃₈** | Y/ Nb₁₆O₃₈ | Sc/ Nb₁₆O₃₈ | Al/ Nb₁₆O₃₈ | Cu/ Nb₁₆O₃₈ | Ga/ Nb₁₆O₃₈ | Hf/ Nb₁₆O₃₈ | Fe/ Nb₁₆O₃₈ | Cr/ Nb₁₆O₃₈ |
| **Nb₁₂O₂₉** | Y/ Nb₁₂O₂₉ | Sc/ Nb₁₂O₂₉ | Al/ Nb₁₂O₂₉ | Cu/ Nb₁₂O₂₉ | Ga/ Nb₁₂O₂₉ | Hf/ Nb₁₂O₂₉ | Fe/ Nb₁₂O₂₉ | Cr/ Nb₁₂O₂₉ |
| **Nb₄₇O₁₁₆** | Y/ Nb₄₇O₁₁₆ | Sc/ Nb₄₇O₁₁₆ | Al/ Nb₄₇O₁₁₆ | Cu/ Nb₄₇O₁₁₆ | Ga/ Nb₄₇O₁₁₆ | Hf/ Nb₄₇O₁₁₆ | Fe/ Nb₄₇O₁₁₆ | Cr/ Nb₄₇O₁₁₆ |
| **Ta₂O₅** | Y/ Ta₂O₅ | Sc/ Ta₂O₅ | Al/ Ta₂O₅ | Cu/ Ta₂O₅ | Ga/ Ta₂O₅ | Hf/ Ta₂O₅ | Fe/ Ta₂O₅ | Cr/ Ta₂O₅ |
| **CrO** | Y/ CrO | Sc/ CrO | Al/ CrO | Cu/ CrO | Ga/ CrO | Hf/ CrO | Fe/ CrO | Cr/ CrO |
| **Cr₂O₃** | Y/ Cr₂O₃ | Sc/ Cr₂O₃ | Al/ Cr₂O₃ | Cu/ Cr₂O₃ | Ga/ Cr₂O₃ | Hf/ Cr₂O₃ | Fe/ Cr₂O₃ | Cr/ Cr₂O₃ |
| **CrO₂** | Y/ CrO₂ | Sc/ CrO₂ | Al/ CrO₂ | Cu/ CrO₂ | Ga/ CrO₂ | Hf/ CrO₂ | Fe/ CrO₂ | Cr/ CrO₂ |
| **CrO₂** | Y/ CrO₃ | Sc/ CrO₃ | Al/ CrO₃ | Cu/ CrO₃ | Ga/ CrO₃ | Hf/ CrO₃ | Fe/ CrO₃ | Cr/ CrO₃ |
| **Cr₈O₂₁** | Y/ Cr₈O₂₁ | Sc/ Cr₈O₂₁ | Al/ Cr₈O₂₁ | Cu/ Cr₈O₂₁ | Ga/ Cr₈O₂₁ | Hf/ Cr₈O₂₁ | Fe/ Cr₈O₂₁ | Cr/ Cr₈O₂₁ |
| **MoO₂** | Y/ MoO₂ | Sc/ MoO₂ | Al/ MoO₂ | Cu/ MoO₂ | Ga/ MoO₂ | Hf/ MoO₂ | Fe/ MoO₂ | Cr/ MoO₂ |
| **MoO₃** | Y/ MoO₃ | Sc/ MoO₃ | Al/ MoO₃ | Cu/ MoO₃ | Ga/ MoO₃ | Hf/ MoO₃ | Fe/ MoO₃ | Cr/ MoO₃ |
| **W₂O₃** | Y/ W₂O₃ | Sc/ W₂O₃ | Al/ W₂O₃ | Cu/ W₂O₃ | Ga/ W₂O₃ | Hf/ W₂O₃ | Fe/ W₂O₃ | Cr/ W₂O₃ |
| **WoO₂** | Y/ WoO₂ | Sc/ WoO₂ | Al/ WoO₂ | Cu/ WoO₂ | Ga/ WoO₂ | Hf/ WoO₂ | Fe/ WoO₂ | Cr/ WoO₂ |
| **WoO₃** | Y/ WoO₃ | Sc/ WoO₃ | Al/ WoO₃ | Cu/ WoO₃ | Ga/ WoO₃ | Hf/ WoO₃ | Fe/ WoO₃ | Cr/ WoO₃ |
| **MnO** | Y/ MnO | Sc/ MnO | Al/ MnO | Cu/ MnO | Ga/ MnO | Hf/ MnO | Fe/ MnO | Cr/ MnO |
| **Mn/Mg/O** | Y/ Mn/Mg/O | Sc/ Mn/Mg/O | Al/ Mn/Mg/O | Cu/ Mn/Mg/O | Ga/ Mn/Mg/O | Hf/ Mn/Mg/O | Fe/ Mn/Mg/O | Cr/ Mn/Mg/O |
| **Mn₃O₄** | Y/ Mn₃O₄ | Sc/ Mn₃O₄ | Al/ Mn₃O₄ | Cu/ Mn₃O₄ | Ga/ Mn₃O₄ | Hf/ Mn₃O₄ | Fe/ Mn₃O₄ | Cr/ Mn₃O₄ |
| **Mn₂O₃** | Y/ Mn₂O₃ | Sc/ Mn₂O₃ | Al/ Mn₂O₃ | Cu/ Mn₂O₃ | Ga/ Mn₂O₃ | Hf/ Mn₂O₃ | Fe/ Mn₂O₃ | Cr/ Mn₂O₃ |
| **MnO₂** | Y/ MnO₂ | Sc/ MnO₂ | Al/ MnO₂ | Cu/ MnO₂ | Ga/ MnO₂ | Hf/ MnO₂ | Fe/ MnO₂ | Cr/ MnO₂ |
| **Mn₂O₇** | Y/ Mn₂O₇ | Sc/ Mn₂O₇ | Al/ Mn₂O₇ | Cu/ Mn₂O₇ | Ga/ Mn₂O₇ | Hf/ Mn₂O₇ | Fe/ Mn₂O₇ | Cr/ Mn₂O₇ |
| **ReO₂** | Y/ ReO₂ | Sc/ ReO₂ | Al/ ReO₂ | Cu/ ReO₂ | Ga/ ReO₂ | Hf/ ReO₂ | Fe/ ReO₂ | Cr/ ReO₂ |
| **ReO₃** | Y/ ReO₃ | Sc/ ReO₃ | Al/ ReO₃ | Cu/ ReO₃ | Ga/ ReO₃ | Hf/ ReO₃ | Fe/ ReO₃ | Cr/ ReO₃ |
| **Re₂O₇** | Y/ Re₂O₇ | Sc/ Re₂O₇ | Al/ Re₂O₇ | Cu/ Re₂O₇ | Ga/ Re₂O₇ | Hf/ Re₂O₇ | Fe/ Re₂O₇ | Cr/ Re₂O₇ |
| **Mg₃Mn₃**-**B₂O₁₀** | Y/ Mg₃Mn₃-B₂O₁₀ | Sc/ Mg₃Mn₃-B₂O₁₀ | Al/ Mg₃Mn₃-B₂O₁₀ | Cu/ Mg₃Mn₃-B₂O₁₀ | Ga/ Mg₃Mn₃-B₂O₁₀ | Hf/ Mg₃Mn₃-B₂O₁₀ | Fe/ Mg₃Mn₃-B₂O₁₀ | Cr/ Mg₃Mn₃-B₂O₁₀ |
| **Mg₃(BO₃)₂** | Y/ Mg₃(BO₃)₂ | Sc/ Mg₃(BO₃)₂ | Al/ Mg₃(BO₃)₂ | Cu/ Mg₃(BO₃)₂ | Ga/ Mg₃(BO₃)₂ | Hf/ Mg₃(BO₃)₂ | Fe/ Mg₃(BO₃)₂ | Cr/ Mg₃(BO₃)₂ |
| **NaWO₄** | Y/ NaWO₄ | Sc/ NaWO₄ | Al/ NaWO₄ | Cu/ NaWO₄ | Ga/ NaWO₄ | Hf/ NaWO₄ | Fe/ NaWO₄ | Cr/ NaWO₄ |
| **Mg₆MnO₈** | Y/ Mg₆MnO₈ | Sc/ Mg₆MnO₈ | Al/ Mg₆MnO₈ | Cu/ Mg₆MnO₈ | Ga/ Mg₆MnO₈ | Hf/ Mg₆MnO₈ | Fe/ Mg₆MnO₈ | Cr/ Mg₆MnO₈ |
| **Mn₂O₄** | Y/ Mn₂O₄ | Sc/ Mn₂O₄ | Al/ Mn₂O₄ | Cu/ Mn₂O₄ | Ga/ Mn₂O₄ | Hf/ Mn₂O₄ | Fe/ Mn₂O₄ | Cr/ Mn₂O₄ |
| **(Li,Mg)₆MnO⁸** | Y/ (Li,Mg)₆MnO₈ | Sc/ (Li,Mg)₆MnO₈ | Al/ (Li,Mg)₆MnO₈ | Cu/ (Li,Mg)₆MnO₈ | Ga/ (Li,Mg)₆MnO₈ | Hf/ (Li,Mg)₆MnO₈ | Fe/ (Li,Mg)₆MnO₈ | Cr/ (Li,Mg)₆MnO₈ |
| **Na₄P₂O₇** | Y/ Na₄P₂O₇ | Sc/ Na₄P₂O₇ | Al/ Na₄P₂O₇ | Cu/ Na₄P₂O₇ | Ga/ Na₄P₂O₇ | Hf/ Na₄P₂O₇ | Fe/ Na₄P₂O₇ | Cr/ Na₄P₂O₇ |
| **Mo₂O₈** | Y/ Mo₂O₈ | Sc/ Mo₂O₈ | Al/ Mo₂O₈ | Cu/ Mo₂O₈ | Ga/ Mo₂O₈ | Hf/ Mo₂O₈ | Fe/ Mo₂O₈ | Cr/ Mo₂O₈ |
| **Mn₃O₄/WO₄** | Y/ Mn₃O₄/WO₄ | Sc/ Mn₃O₄/WO₄ | Al/ Mn₃O₄/WO₄ | Cu/ Mn₃O₄/WO₄ | Ga/ Mn₃O₄/WO₄ | Hf/ Mn₃O₄/W O₄ | Fe/ Mn₃O₄/WO₄ | Cr/ Mn₃O₄/WO₄ |
| **Na₂WO₄** | Y/ Na₂WO₄ | Sc/ Na₂WO₄ | Al/ Na₂WO₄ | Cu/ Na₂WO₄ | Ga/ Na₂WO₄ | Hf/ Na₂WO₄ | Fe/ Na₂WO₄ | Cr/ Na₂WO₄ |
| **Zr₂Mo₂O₈** | Y/ Zr₂Mo₂O₈ | Sc/ Zr₂Mo₂O₈ | Al/ Zr₂Mo₂O₈ | Cu/ Zr₂Mo₂O₈ | Ga/ Zr₂Mo₂O₈ | Hf/ Zr₂Mo₂O₈ | Fe/ Zr₂Mo₂O₈ | Cr/ Zr₂Mo₂O₈ |
| **NaMnO₄**-**/MgO** | Y/ NaMnO₄-/MgO | Sc/ NaMnO₄-/MgO | Al/ NaMnO₄-/MgO | Cu/ NaMnO₄-/MgO | Ga/ NaMnO₄-/MgO | Hf/ NaMnO₄-/MgO | Fe/ NaMnO₄-/MgO | Cr/ NaMnO₄-/MgO |
| **Na₁₀Mn-W₅O₁₇** | Y/ Na₁₀Mn-W₅O₁₇ | Sc/ Na₁₀Mn-W₅O₁₇ | Al/ Na₁₀Mn-W₅O₁₇ | Cu/ Na₁₀Mn-W₅O₁₇ | Ga/ Na₁₀Mn-W₅O₁₇ | Hf/ Na₁₀Mn-W₅O₁₇ | Fe/ Na₁₀Mn-W₅O₁₇ | Cr/ Na₁₀Mn-W₅O₁₇ |

**TABLE 6 NANOWIRES (NW) DOPED WITH SPECIFIC DOPANTS (DOP)**

| **NW**\**Dop** | **Ru** | **Sr** | **Zr** | **Ta** | **Rh** | **Au** | **Mo** | **Ni** |
|---|---|---|---|---|---|---|---|---|
| **Li₂O** | Ru/ Li₂O | Sr/ Li₂O | Zr/ Li₂O | Ta/ Li₂O | Rh/ Li₂O | Au/ Li₂O | Mo/ Li₂O | Ni/ Li₂O |
| **Na₂O** | Ru/ Na₂O | Sr/ Na₂O | Zr/ Na₂O | Ta/ Na₂O | Rh/ Na₂O | Au/ Na₂O | Mo/ Na₂O | Ni/ Na₂O |
| **K₂O** | Ru/ K₂O | Sr/ K₂O | Zr/ K₂O | Ta/ K₂O | Rh/ K₂O | Au/ K₂O | Mo/ K₂O | Ni/ K₂O |
| **Rb₂O** | Ru/ Rb₂O | Sr/ Rb₂O | Zr/ Rb₂O | Ta/ Rb₂O | Rh/ Rb₂O | Au/ Rb₂O | Mo/ Rb₂O | Ni/ Rb₂O |
| **Cs₂O** | Ru/ Cs₂O | Sr/ Cs₂O | Zr/ Cs₂O | Ta/ Cs₂O | Rh/ Cs₂O | Au/ Cs₂O | Mo/ Cs₂O | Ni/ Cs₂O |
| **BeO** | Ru/ BeO | Sr/ BeO | Zr/ BeO | Ta/ BeO | Rh/ BeO | Au/ BeO | Mo/ BeO | Ni/ BeO |
| **MgO** | Ru/ MgO | Sr/ MgO | Zr/ MgO | Ta/ MgO | Rh/ MgO | Au/ MgO | Mo/ MgO | Ni/ MgO |
| **CaO** | Ru/ CaO | Sr/ CaO | Zr/ CaO | Ta/ CaO | Rh/ CaO | Au/ CaO | Mo/ CaO | Ni/ CaO |
| **SrO** | Ru/ SrO | Sr/ SrO | Zr/ SrO | Ta/ SrO | Rh/ SrO | Au/ SrO | Mo/ SrO | Ni/ SrO |
| **BaO** | Ru/ BaO | Sr/ BaO | Zr/ BaO | Ta/ BaO | Rh/ BaO | Au/ BaO | Mo/ BaO | Ni/ BaO |
| **Sc₂O₃** | Ru/ Sc₂O₃ | Sr/ Sc₂O₃ | Zr/ Sc₂O₃ | Ta/ Sc₂O₃ | Rh/ Sc₂O₃ | Au/ Sc₂O₃ | Mo/ Sc₂O₃ | Ni/ Sc₂O₃ |
| **Y₂O₃** | Ru/ Y₂O₃ | Sr/ Y₂O₃ | Zr/ Y₂O₃ | Ta/ Y₂O₃ | Rh/ Y₂O₃ | Au/ Y₂O₃ | Mo/ Y₂O₃ | Ni/ Y₂O₃ |
| **La₂O₃** | Ru/ La₂O₃ | Sr/ La₂O₃ | Zr/ La₂O₃ | Ta/ La₂O₃ | Rh/ La₂O₃ | Au/ La₂O₃ | Mo/ La₂O₃ | Ni/ La₂O₃ |
| **CeO₂** | Ru/ CeO₂ | Sr/ CeO₂ | Zr/ CeO₂ | Ta/ CeO₂ | Rh/ CeO₂ | Au/ CeO₂ | Mo/ CeO₂ | Ni/ CeO₂ |
| **Ce₂O₃** | Ru/ Ce₂O₃ | Sr/ Ce₂O₃ | Zr/ Ce₂O₃ | Ta/ Ce₂O₃ | Rh/ Ce₂O₃ | Au/ Ce₂O₃ | Mo/ Ce₂O₃ | Ni/ Ce₂O₃ |
| **Pr₂O₃** | Ru/ Pr₂O₃ | Sr/ Pr₂O₃ | Zr/ Pr₂O₃ | Ta/ Pr₂O₃ | Rh/ Pr₂O₃ | Au/ Pr₂O₃ | Mo/ Pr₂O₃ | Ni/ Pr₂O₃ |
| **Nd₂O₃** | Ru/ Nd₂O₃ | Sr/ Nd₂O₃ | Zr/ Nd₂O₃ | Ta/ Nd₂O₃ | Rh/ Nd₂O₃ | Au/ Nd₂O₃ | Mo/ Nd₂O₃ | Ni/ Nd₂O₃ |
| **Sm₂O₃** | Ru/ Sm₂O₃ | Sr/ Sm₂O₃ | Zr/ Sm₂O₃ | Ta/ Sm₂O₃ | Rh/ Sm₂O₃ | Au/ Sm₂O₃ | Mo/ Sm₂O₃ | Ni/ Sm₂O₃ |
| **Eu₂O₃** | Ru/ Eu₂O₃ | Sr/ Eu₂O₃ | Zr/ Eu₂O₃ | Ta/ Eu₂O₃ | Rh/ Eu₂O₃ | Au/ Eu₂O₃ | Mo/ Eu₂O₃ | Ni/ Eu₂O₃ |
| **Gd₂O₃** | Ru/ Gd₂O₃ | Sr/ Gd₂O₃ | Zr/ Gd₂O₃ | Ta/ Gd₂O₃ | Rh/ Gd₂O₃ | Au/ Gd₂O₃ | Mo/ Gd₂O₃ | Ni/ Gd₂O₃ |
| **Tb₂O₃** | Ru/ Tb₂O₃ | Sr/ Tb₂O₃ | Zr/ Tb₂O₃ | Ta/ Tb₂O₃ | Rh/ Tb₂O₃ | Au/ Tb₂O₃ | Mo/ Tb₂O₃ | Ni/ Tb₂O₃ |
| **TbO₂** | Ru/ TbO₂ | Sr/ TbO₂ | Zr/ TbO₂ | Ta/ TbO₂ | Rh/ TbO₂ | Au/ TbO₂ | Mo/ TbO₂ | Ni/ TbO₂ |
| **Tub₆O₁₁** | Ru/ Tb₆O₁₁ | Sr/ Tb₆O₁₁ | Zr/ Tb₆O₁₁ | Ta/ Tb₆O₁₁ | Rh/ Tb₆O₁₁ | Au/ Tb₆O₁₁ | Mo/ Tb₆O₁₁ | Ni/ Tb₆O₁₁ |
| **Dy₂O₃** | Ru/ Dy₂O₃ | Sr/ Dy₂O₃ | Zr/ Dy₂O₃ | Ta/ Dy₂O₃ | Rh/ Dy₂O₃ | Au/ Dy₂O₃ | Mo/ Dy₂O₃ | Ni/ Dy₂O₃ |
| **Ho₂O₃** | Ru/ Ho₂O₃ | Sr/ Ho₂O₃ | Zr/ Ho₂O₃ | Ta/ Ho₂O₃ | Rh/ Ho₂O₃ | Au/ Ho₂O₃ | Mo/ Ho₂O₃ | Ni/ Ho₂O₃ |
| **Er₂O₃** | Ru/ Er₂O₃ | Sr/ Er₂O₃ | Zr/ Er₂O₃ | Ta/ Er₂O₃ | Rh/ Er₂O₃ | Au/ Er₂O₃ | Mo/ Er₂O₃ | Ni/ Er₂O₃ |
| **Tm₂O₃** | Ru/ Tm₂O₃ | Sr/ Tm₂O₃ | Zr/ Tm₂O₃ | Ta/ Tm₂O₃ | Rh/ Tm₂O₃ | Au/ Tm₂O₃ | Mo/ Tm₂O₃ | Ni/ Tm₂O₃ |
| **Yb₂O₃** | Ru/ Yb₂O₃ | Sr/ Yb₂O₃ | Zr/ Yb₂O₃ | Ta/ Yb₂O₃ | Rh/ Yb₂O₃ | Au/ Yb₂O₃ | Mo/ Yb₂O₃ | Ni/ Yb₂O₃ |
| **Lu₂O₃** | Ru/ Lu₂O₃ | Sr/ Lu₂O₃ | Zr/ Lu₂O₃ | Ta/ Lu₂O₃ | Rh/ Lu₂O₃ | Au/ Lu₂O₃ | Mo/ Lu₂O₃ | Ni/ Lu₂O₃ |
| **Ac₂O₃** | Ru/ Ac₂O₃ | Sr/ Ac₂O₃ | Zr/ Ac₂O₃ | Ta/ Ac₂O₃ | Rh/ Ac₂O₃ | Au/ Ac₂O₃ | Mo/ Ac₂O₃ | Ni/ Ac₂O₃ |
| **Th₂O₃** | Ru/ Th₂O₃ | Sr/ Th₂O₃ | Zr/ Th₂O₃ | Ta/ Th₂O₃ | Rh/ Th₂O₃ | Au/ Th₂O₃ | Mo/ Th₂O₃ | Ni/ Th₂O₃ |
| **ThO₂** | Ru/ ThO₂ | Sr/ ThO₂ | Zr/ ThO₂ | Ta/ ThO₂ | Rh/ ThO₂ | Au/ ThO₂ | Mo/ ThO₂ | Ni/ ThO₂ |
| **Pa₂O₃** | Ru/ Pa₂O₃ | Sr/ Pa₂O₃ | Zr/ Pa₂O₃ | Ta/ Pa₂O₃ | Rh/ Pa₂O₃ | Au/ Pa₂O₃ | Mo/ Pa₂O₃ | Ni/ Pa₂O₃ |
| **PaO₂** | Ru/ PaO₂ | Sr/ PaO₂ | Zr/ PaO₂ | Ta/ PaO₂ | Rh/ PaO₂ | Au/ PaO₂ | Mo/ PaO₂ | Ni/ PaO₂ |
| **TiO₂** | Ru/ TiO₂ | Sr/ TiO₂ | Zr/ TiO₂ | Ta/ TiO₂ | Rh/ TiO₂ | Au/ TiO₂ | Mo/ TiO₂ | Ni/ TiO₂ |
| **TiO** | Ru/ TiO | Sr/ TiO | Zr/ TiO | Ta/ TiO | Rh/ TiO | Au/ TiO | Mo/ TiO | Ni/ TiO |
| **Ti₂O₃** | Ru/ Ti₂O₃ | Sr/ Ti₂O₃ | Zr/ Ti₂O₃ | Ta/ Ti₂O₃ | Rh/ Ti₂O₃ | Au/ Ti₂O₃ | Mo/ Ti₂O₃ | Ni/ Ti₂O₃ |
| **Ti₃O** | Ru/ Ti₃O | Sr/ Ti₃O | Zr/ Ti₃O | Ta/ Ti₃O | Rh/ Ti₃O | Au/ Ti₃O | Mo/ Ti₃O | Ni/ Ti₃O |
| **Ti₂O** | Ru/ Ti₂O | Sr/ Ti₂O | Zr/ Ti₂O | Ta/ Ti₂O | Rh/ Ti₂O | Au/ Ti₂O | Mo/ Ti₂O | Ni/ Ti₂O |
| **Ti₃O₅** | Ru/ Ti₃O₅ | Sr/ Ti₃O₅ | Zr/ Ti₃O₅ | Ta/ Ti₃O₅ | Rh/ Ti₃O₅ | Au/ Ti₃O₅ | Mo/ Ti₃O₅ | Ni/ Ti₃O₅ |
| **Ti₄O₇** | Ru/ Ti₄O₇ | Sr/ Ti₄O₇ | Zr/ Ti₄O₇ | Ta/ Ti₄O₇ | Rh/ Ti₄O₇ | Au/ Ti₄O₇ | Mo/ Ti₄O₇ | Ni/ Ti₄O₇ |
| **ZrO₂** | Ru/ ZrO₂ | Sr/ ZrO₂ | Zr/ ZrO₂ | Ta/ ZrO₂ | Rh/ ZrO₂ | Au/ ZrO₂ | Mo/ ZrO₂ | Ni/ ZrO₂ |
| **HfO₂** | Ru/ HfO₂ | Sr/ HfO₂ | Zr/ HfO₂ | Ta/ HfO₂ | Rh/ HfO₂ | Au/ HfO₂ | Mo/ HfO₂ | Ni/ HfO₂ |
| **VO** | Ru/ VO | Sr/ VO | Zr/ VO | Ta/ VO | Rh/ VO | Au/ VO | Mo/ VO | Ni/ VO |
| **V₂O₃** | Ru/ V₂O₃ | Sr/ V₂O₃ | Zr/ V₂O₃ | Ta/ V₂O₃ | Rh/ V₂O₃ | Au/ V₂O₃ | Mo/ V₂O₃ | Ni/ V₂O₃ |
| **VO₂** | Ru/ VO₂ | Sr/ VO₂ | Zr/ VO₂ | Ta/ VO₂ | Rh/ VO₂ | Au/ VO₂ | Mo/ VO₂ | Ni/ VO₂ |
| **V₂O₅** | Ru/ V₂O₅ | Sr/ V₂O₅ | Zr/ V₂O₅ | Ta/ V₂O₅ | Rh/ V₂O₅ | Au/ V₂O₅ | Mo/ V₂O₅ | Ni/ V₂O₅ |
| **V₃O₇** | Ru/ V₃O₇ | Sr/ V₃O₇ | Zr/ V₃O₇ | Ta/ V₃O₇ | Rh/ V₃O₇ | Au/ V₃O₇ | Mo/ V₃O₇ | Ni/ V₃O₇ |
| **V₄O₉** | Ru/ V₄O₉ | Sr/ V₄O₉ | Zr/ V₄O₉ | Ta/ V₄O₉ | Rh/ V₄O₉ | Au/ V₄O₉ | Mo/ V₄O₉ | Ni/ V₄O₉ |
| **V₆O₁₃** | Ru/ V₆O₁₃ | Sr/ V₆O₁₃ | Zr/ V₆O₁₃ | Ta/ V₆O₁₃ | Rh/ V₆O₁₃ | Au/ V₆O₁₃ | Mol V₆O₁₃ | Ni/ V₆O₁₃ |
| **NbO** | Ru/ NbO | Sr/ NbO | Zr/ NbO | Ta/ NbO | Rh/ NbO | Au/ NbO | Mol NbO | Ni/ NbO |
| **NbO₂** | Ru/ NbO₂ | Sr/ NbO₂ | Zr/ NbO₂ | Ta/ NbO₂ | Rh/ NbO₂ | Au/ NbO₂ | Mo/ NbO₂ | Ni/ NbO₂ |
| **Nb₂O₅** | Ru/ Nb₂O₅ | Sr/ Nb₂O₅ | Zrl Nb₂O₅ | Ta/ Nb₂O₅ | Rh/ Nb₂O₅ | Au/ Nb₂O₅ | Mo/ Nb₂O₅ | Ni/ Nb₂O₅ |
| **Nb₈O₁₉** | Ru/ Nb₈O₁₉ | Sr/ Nb₈O₁₉ | Zr/ Nb₈O₁₉ | Ta/ Nb₈O₁₉ | Rh/ Nb₈O₁₉ | Au/ Nb₈O₁₉ | Mo/ Nb₈O₁₉ | Ni/ Nb₈O₁₉ |
| **Nbi₁₆O₃₈** | Ru/ Nb₁₆O₃₈ | Sr/ Nb₁₆O₃₈ | Zr/ Nb₁₆O₃₈ | Ta/ Nb₁₆O₃₈ | Rh/ Nb₁₆O₃₈ | Au/ Nb₁₆O₃₈ | Mo/ Nb₁₆O₃₈ | Ni/ Nb₁₆O₃₈ |
| **Nb₁₂O₂₉** | Ru/ Nb₁₂O₂₉ | Sr/ Nb₁₂O₂₉ | Zr/ Nb₁₂O₂₉ | Ta/ Nb₁₂O₂₉ | Rh/ Nb₁₂O₂₉ | Au/ Nb₁₂O₂₉ | Mo/ Nb₁₂O₂₉ | Ni/ Nb₁₂O₂₉ |
| **Nb₄₇O₁₁₆** | Ru/ Nb₄₇O₁₁₆ | Sr/ Nb₄₇O₁₁₆ | Zr/ Nb₄₇O₁₁₆ | Ta/ Nb₄₇o₁₁₆ | Rh/ Nb₄₇O₁₁₆ | Au/ Nb₄₇O₁₁₆ | Mo/ Nb₄₇O₁₁₆ | Ni/ Nb₄₇O₁₁₆ |
| **Ta₂O₅** | Ru/ Ta₂O₅ | Sr/ Ta₂O₅ | Zr/ Ta₂O₅ | Ta/ Ta₂O₅ | Rh/ Ta₂O₅ | Au/ Ta₂O₅ | Mo/ Ta₂O₅ | Ni/ Ta₂O₅ |
| **CrO** | Ru/ CrO | Sr/ CrO | Zr/ CrO | Ta/ CrO | Rh/ CrO | Au/ CrO | Mo/ CrO | Ni/ CrO |
| **Cr₂O₃** | Ru/ Cr₂O₃ | Sr/ Cr₂O₃ | Zr/ Cr₂O₃ | Ta/ Cr₂O₃ | Rh/ Cr₂O₃ | Au/ Cr₂O₃ | Mo/ Cr₂O₃ | Ni/ Cr₂O₃ |
| **CrO₂** | Ru/ CrO₂ | Sr/ CrO₂ | Zr/ CrO₂ | Ta/ CrO₂ | Rh/ CrO₂ | Au/ CrO₂ | Mo/ CrO₂ | Ni/ CrO₂ |
| **CrO₃** | Ru/ CrO₃ | Sr/ CrO₃ | Zr/ CrO₃ | Ta/ CrO₃ | Rh/ CrO₃ | Au/ CrO₃ | Mo/ CrO₃ | Ni/ CrO₃ |
| **Cr₈O₂₁** | Ru/ Cr₈O₂₁ | Sr/ Cr₈O₂₁ | Zr/ Cr₈O₂₁ | Ta/ Cr₈O₂₁ | Rh/ Cr₈O₂₁ | Au/ Cr₈O₂₁ | Mo/ Cr₈O₂₁ | Ni/ Cr₈O₂₁ |
| **MoO₂** | Ru/ MoO₂ | Sr/ MoO₂ | Zr/ MoO₂ | Ta/ MoO₂ | Rh/ MoO₂ | Au/ MoO₂ | Mo/ MoO₂ | Ni/ MoO₂ |
| **MoO₃** | Ru/ MoO₃ | Sr/ MoO₃ | Zr/ MoO₃ | Ta/ MoOs | Rh/ MoO₃ | Au/ MoO₃ | Mo/ MoO₃ | Ni/ MoO₃ |
| **W₂O₃** | Ru/ W₂O₃ | Sr/ W₂O₃ | Zr/ W₂O₃ | Ta/ W₂O₃ | Rh/ W₂O₃ | Au/ W₂O₃ | Mo/ W₂O₃ | Ni/ W₂O₃ |
| **WoO₂** | Ru/ WoO₂ | Sr/ WoO₂ | Zr/ WoO₂ | Ta/ WoO₂ | Rh/ WoO₂ | Au/ WoO₂ | Mo/ WoO₂ | Ni/ WoO₂ |
| **WoO₃** | Ru/ WoO₃ | Sr/ WoO₃ | Zr/ WoO₃ | Ta/ WoO₃ | Rh/ WoO₃ | Au/ WoO₃ | Mo/ WoO₃ | Ni/ WoO₃ |
| **MnO** | Ru/ MnO | Sr/ MnO | Zr/ MnO | Ta/ MnO | Rh/ MnO | Au/ MnO | Mo/ MnO | Ni/ MnO |
| **Mn/Mg/O** | Ru/ Mn/Mg/O | Sr/ Mn/Mg/O | Zr/ Mn/Mg/O | Ta/ Mn/Mg/O | Rh/ Mn/Mg/O | Au/ Mn/Mg/O | Mo/ Mn/Mg/O | Ni/ Mn/Mg/O |
| **Mn₃O₄** | Ru/ Mn₃O₄ | Sr/ Mn₃O₄ | Zr/ Mn₃O₄ | Ta/ Mn₃O₄ | Rh/ Mn₃O₄ | Au/ Mn₃O₄ | Mo/ Mn₃O₄ | Ni/ Mn₃O₄ |
| **Mn₂O₃** | Ru/ Mn₂O₃ | Sr/ Mn₂O₃ | Zr/ Mn₂O₃ | Ta/ Mn₂O₃ | Rh/ Mn₂O₃ | Au/ Mn₂O₃ | Mo/ Mn₂O₃ | Ni/ Mn₂O₃ |
| **MnO₂** | Ru/ MnO₂ | Sr/ MnO₂ | Zr/ MnO₂ | Ta/ MnO₂ | Rh/ MnO₂ | Au/ MnO₂ | Mo/ MnO₂ | Ni/ MnO₂ |
| **Mn₂O₇** | Ru/ Mn₂O₇ | Sr/ Mn₂O₇ | Zr/ Mn₂O₇ | Ta/ Mn₂O₇ | Rh/ Mn₂O₇ | Au/ Mn₂O₇ | Mo/ Mn₂O₇ | Ni/ Mn₂O₇ |
| **ReO₂** | Ru/ ReO₂ | Sr/ ReO₂ | Zr/ ReO₂ | Ta/ ReO₂ | Rh/ ReO₂ | Au/ ReO₂ | Mo/ ReO₂ | Ni/ ReO₂ |
| **ReO₃** | Ru/ ReO₃ | Sr/ ReO₃ | Zr/ ReO₃ | Ta/ ReO₃ | Rh/ ReO₃ | Au/ ReO₃ | Mo/ ReO₃ | Ni/ ReO₃ |
| **Re₂O₇** | Ru/ Re₂O₇ | Sr/ Re₂O₇ | Zr/ Re₂O₇ | Ta/ Re₂O₇ | Rh/ Re₂O₇ | Au/ Re₂O₇ | Mo/ Re₂O₇ | Ni/ Re₂O₇ |
| **Mg₃Mn₃**-**B₂O₁₀** | Ru/ Mg₃Mn₃-B₂O₁₀ | Sr/ Mg₃Mn₃-B₂O₁₀ | Zr/ Mg₃Mn₃-B₂O₁₀ | Ta/ Mg₃Mn₃-B₂O₁₀ | Rh/ Mg₃Mn₃-B₂O₁₀ | Au/ Mg₃Mn₃-B₂O₁₀ | Mo/ Mg₃Mn₃-B₂O₁₀ | Ni/ Mg₃Mn₃-B₂O₁₀ |
| **Mg₃(BO₃)₂** | Ru/ Mg₃(BO₃)₂ | Sr/ Mg₃(BO₃)₂ | Zr/ Mg₃(BO₃)₂ | Ta/ Mg₃(BO₃)₂ | Rh/ Mg₃(BO₃)₂ | Au/ Mg₃(BO₃)₂ | Mo/ Mg₃(BO₃)₂ | Ni/ Mg₃(BO₃)₂ |
| **NaWO₄** | Ru/ NaWO₄ | Sr/ NaWO₄ | Zr/ NaWO₄ | Ta/ NaWO₄ | Rh/ NaWO₄ | Au/ NaWO₄ | Mo/ NaWO₄ | Ni/ NaWO₄ |
| **Mg₆MnO₈** | Ru/ Mg₆MnO₈ | Sr/ Mg₆MnO₈ | Zr/ Mg₆MnO₈ | Ta/ Mg₆MnO₈ | Rh/ Mg₆MnO₈ | Au/ Mg₆MnO₈ | Mo/ Mg₆MnO₈ | Ni/ Mg₆MnO₈ |
| **Mn₂O₄** | Ru/ Mn₂O₄ | Sr/ Mn₂O₄ | Zr/ Mn₂O₄ | Ta/ Mn₂O₄ | Rh/ Mn₂O₄ | Au/ Mn₂O₄ | Mo/ Mn₂O₄ | Ni/ Mn₂O₄ |
| **(Li,Mg)₆**-**MnO₈** | Ru/ (Li,Mg)₆-MnO₈ | Sr/ (Li,Mg)₆-MnO₈ | Zr/ (Li,Mg)₆-MnO₈ | Ta/ (Li,Mg)₆-MnO₈ | Rh/ (Li,Mg)₆-MnO₈ | Au/ (Li,Mg)₆-MnO₈ | Mo/ (Li,Mg)₆-MnO₈ | Ni/ (Li,Mg)₆-MnO₈ |
| **Na₄P₂O₇** | Ru/ Na₄P₂O₇ | Sr/ Na₄P₂O₇ | Zr/ Na₄P₂O₇ | Ta/ Na₄P₂O₇ | Rh/ Na₄P₂O₇ | Au/ Na₄P₂O₇ | Mo/ Na₄P₂O₇ | Ni/ Na₄P₂O₇ |
| **Mo₂O₈** | Ru/ Mo₂O₈ | Sr/ Mo₂O₈ | Zr/ Mo₂O₈ | Ta/ Mo₂O₈ | Rh/ Mo₂O₈ | Au/ Mo₂O₈ | Mo/ Mo₂O₈ | Ni/ Mo₂O₈ |
| **Mn₃O₄/WO₄** | Ru/ Mn₃O₄/WO₄ | Sr/ Mn₃O₄/WO₄ | Zr/ Mn₃O₄/WO₄ | Ta/ Mn₃O₄/WO₄ | Rh/ Mn₃O₄/WO₄ | Au/ Mn₃O₄/WO₄ | Mo/ Mn₃O₄/WO₄ | Ni/ Mn₃O₄/WO₄ |
| **Na₂WO₄** | Ru/ Na₂WO₄ | Sr/ Na₂WO₄ | Zr/ Na₂WO₄ | Ta/ Na₂WO₄ | Rh/ Na₂WO₄ | Au/ Na₂WO₄ | Mo/ Na₂WO₄ | Ni/ Na₂WO₄ |
| **Zr₂Mo₂O₈** | Ru/ Zr₂Mo₂O₈ | Sr/ Zr₂Mo₂O₈ | Zr/ Zr₂Mo₂O₈ | Ta/ Zr₂Mo₂O₈ | Rh/ Zr₂Mo₂O₈ | Au/ Zr₂Mo₂O₈ | Mo/ Zr₂Mo₂O₈ | Ni/ Zr₂Mo₂O₈ |
| **NaMnO₄**-**/MgO** | Ru/ NaMnO₄-/MgO | Sr/ NaMnO₄-/MgO | Zr/ NaMnO₄-/MgO | Ta/ NaMnO₄-/MgO | Rh/ NaMnO₄-/MgO | Au/ NaMnO₄-/MgO | Mol NaMnO₄-/MgO | Ni/ NaMnO₄-/MgO |
| **Na₁₀Mn-W₅O₁₇** | Ru/ Na₁₀Mn-W₅O₁₇ | Sr/ Na₁₀Mn-W₅O₁₇ | Zr/ Na₁₀Mn-W₅O₁₇ | Ta/ Na₁₀Mn-W₅O₁₇ | Rh/ Na₁₀Mn-W₅O₁₇ | Au/ Na₁₀Mn-W₅O₁₇ | Mo/ Na₁₀Mn-W₅O₁₇ | Ni/ Na₁₀Mn-W₅O₁₇ |

**TABLE 7 NANOWIRES (NW) DOPED WITH SPECIFIC DOPANTS (DOP)**

| **NW\Dop** | **Co** | **Sb** | **W** | **V** | **Ag** | **Te** | **Pd** | **Ir** |
|---|---|---|---|---|---|---|---|---|
| **Li₂O** | Co/ Li₂O | Sb/ Li₂O | W/ Li₂O | V/ Li₂O | Ag/ Li₂O | Te/ Li₂O | Pd/ Li₂O | Ir/ Li₂O |
| **Na₂O** | Co/ Na₂O | Sb/ Na₂O | W/ Na₂O | V/ Na₂O | Ag/ Na₂O | Te/ Na₂O | Pd/ Na₂O | Ir/ Na₂O |
| **K₂O** | Co/ K₂O | Sb/ K₂O | W/ K₂O | V/ K₂O | Ag/ K₂O | Te/ K₂O | Pd/ K₂O | Ir/ K₂O |
| **Rb₂O** | Co/ Rb₂O | Sb/ Rb₂O | W/ Rb₂O | V/ Rb₂O | Ag/ Rb₂O | Te/ Rb₂O | Pd/ Rb₂O | Ir/ Rb₂O |
| **CS₂O** | Co/ Cs₂O | Sb/ Cs₂O | W/ CS₂O | V/ CS₂O | Ag/ Cs₂O | Te/ Cs₂O | Pd/ Cs₂O | Ir/ Cs₂O |
| **BeO** | Co/ BeO | Sb/ BeO | W/ BeO | V/ BeO | Ag/ BeO | Te/ BeO | Pd/ BeO | Ir/ BeO |
| **MgO** | Co/ MgO | Sb/ MgO | W/ MgO | V/ MgO | Ag/ MgO | Te/ MgO | Pd/ MgO | Ir/ MgO |
| **CaO** | Co/ CaO | Sb/ CaO | W/ CaO | V/ CaO | Ag/ CaO | Te/ CaO | Pd/ CaO | Ir/ CaO |
| **SrO** | Co/ SrO | Sb/ SrO | W/ SrO | V/ SrO | Ag/ SrO | Te/ SrO | Pd/ SrO | Ir/ SrO |
| **BaO** | Co/ BaO | Sb/ BaO | W/ BaO | V/ BaO | Ag/ BaO | Te/ BaO | Pd/ BaO | Ir/ BaO |
| **Sc₂O₃** | Co/ Sc₂O₃ | Sb/ Sc₂O₃ | W/ Sc₂O₃ | V/ Sc₂O₃ | Ag/ Sc₂O₃ | Te/ Sc₂O₃ | Pd/ Sc₂O₃ | Ir/ Sc₂O₃ |
| **Y₂O₃** | Co/ Y₂O₃ | Sb/ Y₂O₃ | W/ Y₂O₃ | V/ Y₂O₃ | Ag/ Y₂O₃ | Te/ Y₂O₃ | Pd/ Y₂O₃ | Ir/ Y₂O₃ |
| **La₂O₃** | Co/ La₂O₃ | Sb/ La₂O₃ | W/ La₂O₃ | V/ La₂O₃ | Ag/ La₂O₃ | Te/ La₂O₃ | Pd/ La₂O₃ | Ir/ La₂O₃ |
| **CeO₂** | Co/ CeO₂ | Sb/ CeO₂ | W/ CeO₂ | V/ CeO₂ | Ag/ CeO₂ | Te/ CeO₂ | Pd/ CeO₂ | Ir/ CeO₂ |
| **Ce₂O₃** | Co/ Ce₂O₃ | Sb/ Ce₂O₃ | W/ Ce₂O₃ | V/ Ce₂O₃ | Ag/ Ce₂O₃ | Te/ Ce₂O₃ | Pd/ Ce₂O₃ | Ir/ Ce₂O₃ |
| **Pr₂O₃** | Co/ Pr₂O₃ | Sb/ Pr₂O₃ | W/ Pr₂O₃ | V/ Pr₂O₃ | Ag/ Pr₂O₃ | Te/ Pr₂O₃ | Pd/ Pr₂O₃ | I r/ Pr₂O₃ |
| **Nd₂O₃** | Co/ Nd₂O₃ | Sb/ Nd₂O₃ | W/ Nd₂O₃ | V/ Nd₂O₃ | Ag/ Nd₂O₃ | Te/ Nd₂O₃ | Pd/ Nd₂O₃ | Ir/ Nd₂O₃ |
| **Sm₂O₃** | Co/ Sm₂O₃ | Sb/ Sm₂O₃ | W/ Sm₂O₃ | V/ Sm₂O₃ | Ag/ Sm₂O₃ | Te/ Sm₂O₃ | Pd/ Sm₂O₃ | Ir/ Sm₂O₃ |
| **EU₂O_{3O}** | Co/ Eu₂O₃ | Sb/ Eu₂O₃ | W/ Eu₂O₃ | V/ Eu₂O₃ | Ag/ Eu₂O₃ | Te/ Eu₂O₃ | Pd/ Eu₂O₃ | Ir/ Eu₂O₃ |
| **Gd₂O₃** | Co/ Gd₂O₃ | Sb/ Gd₂O₃ | W/ Gd₂O₃ | V/ Gd₂O₃ | Ag/ Gd₂O₃ | Te/ Gd₂O₃ | Pd/ Gd₂O₃ | Ir/ Gd₂O₃ |
| **Tb₂O₃** | Co/ Tb₂O₃ | Sb/ Tb₂O₃ | W/ Tb₂O₃ | V/ Tb₂O₃ | Ag/ Tb₂O₃ | Te/ Tb₂O₃ | Pd/ Tb₂O₃ | Ir/ Tb₂O₃ |
| **TbO₂** | Co/ TbO₂ | Sb/ TbO₂ | W/ TbO₂ | V/ TbO₂ | Ag/ TbO₂ | Te/ TbO₂ | Pd/ TbO₂ | Ir/ TbO₂ |
| **Tb₆O₁₁** | Co/ Tb₆O₁₁ | Sb/ Tb₆O₁₁ | W/ Tb₆O₁₁ | V/ Tb₆O₁₁ | Ag/ Tb₆O₁₁ | Te/ Tb₆O₁₁ | Pd/ Tb₆O₁₁ | Ir/ Tb₆O₁₁ |
| **Dy₂O₃** | Co/ Dy₂O₃ | Sb/ Dy₂O₃ | W/ Dy₂O₃ | V/ Dy₂O₃ | Ag/ Dy₂O₃ | Te/ Dy₂O₃ | Pd/ Dy₂O₃ | Ir/ Dy₂O₃ |
| **Ho₂O₃** | Co/ Ho₂O₃ | Sb/ Ho₂O₃ | W/ Ho₂O₃ | V/ Ho₂O₃ | Ag/ Ho₂O₃ | Te/ Ho₂O₃ | Pd/ Ho₂O₃ | Ir/ Ho₂O₃ |
| **Er₂O₃** | Co/ Er₂O₃ | Sb/ Er₂O₃ | W/ Er₂O₃ | V/ Er₂O₃ | Ag/ Er₂O₃ | Te/ Er₂O₃ | Pd/ Er₂O₃ | Ir/ Er₂O₃ |
| **Tm₂O₃** | Co/ Tm₂O₃ | Sb/ Tm₂O₃ | W/ Tm₂O₃ | V/ Tm₂O₃ | Ag/ Tm₂O₃ | Te/ Tm₂O₃ | Pd/ Tm₂O₃ | Ir/ Tm₂O₃ |
| **Yb₂O₃** | Co/ Yb₂O₃ | Sb/ Yb₂O₃ | W/ Yb₂O₃ | V/ Yb₂O₃ | Ag/ Yb₂O₃ | Te/ Yb₂O₃ | Pd/ Yb₂O₃ | Ir/ Yb₂O₃ |
| **Lu₂O₃** | Co/ Lu₂O₃ | Sb/ Lu₂O₃ | W/ Lu₂O₃ | V/ Lu₂O₃ | Ag/ Lu₂O₃ | Te/ Lu₂O₃ | Pd/ Lu₂O₃ | Ir/ Lu₂O₃ |
| **AC₂O₃** | Co/ Ac₂O₃ | Sb/ Ac₂O₃ | W/ Ac₂O₃ | V/ Ac₂O₃ | Ag/ Ac₂O₃ | Te/ Ac₂O₃ | Pd/ Ac₂O₃ | Ir/ Ac₂O₃ |
| **Th₂O₃** | Co/ Th₂O₃ | Sb/ Th₂O₃ | W/ Th₂O₃ | V/ Th₂O₃ | Ag/ Th₂O₃ | Te/ Th₂O₃ | Pd/ Th₂O₃ | Ir/ Th₂O₃ |
| **ThO₂** | Co/ ThO₂ | Sb/ ThO₂ | W/ ThO₂ | V/ ThO₂ | Ag/ ThO₂ | Te/ ThO₂ | Pd/ ThO₂ | Ir/ ThO₂ |
| **Pa₂O₃** | Co/ Pa₂O₃ | Sb/ Pa₂O₃ | W/ Pa₂O₃ | V/ Pa₂O₃ | Ag/ Pa₂O₃ | Te/ Pa₂O₃ | Pd/ Pa₂O₃ | Ir/ Pa₂O₃ |
| **PaO₂** | Co/ PaO₂ | Sb/ PaO₂ | W/ PaO₂ | V/ PaO₂ | Ag/ PaO₂ | Te/ PaO₂ | Pd/ PaO₂ | Ir/ PaO₂ |
| **TiO₂** | Co/ TiO₂ | Sb/ TiO₂ | W/ TiO₂ | V/ TiO₂ | Ag/ TiO₂ | Te/ TiO₂ | Pd/ TiO₂ | Ir/ TiO₂ |
| **TiO** | Co/ TiO | Sb/ TiO | W/ TiO | V/ TiO | Ag/ TiO | Te/ TiO | Pd/ TiO | Ir/ TiO |
| **Ti₂O₃** | Co/ Ti₂O₃ | Sb/ Ti₂O₃ | W/ Ti₂O₃ | V/ Ti₂O₃ | Ag/ Ti₂O₃ | Te/ Ti₂O₃ | Pd/ Ti₂O₃ | Ir/ Ti₂O₃ |
| **Ti₃O** | Co/ Ti₃O | Sb/ Ti₃O | W/ Ti₃O | V/ Ti₃O | Ag/ Ti₃O | Te/ Ti₃O | Pd/ Ti₃O | Ir/ Ti₃O |
| **Ti₂O** | Co/ Ti₂O | Sb/ Ti₂O | W/ Ti₂O | V/ Ti₂O | Ag/ Ti₂O | Te/ Ti₂O | Pd/ Ti₂O | Ir/ Ti₂O |
| **Ti₃O₅** | Co/ Ti₃O₅ | Sb/ Ti₃O₅ | W/ Ti₃O₅ | V/ Ti₃O₅ | Ag/ Ti₃O₅ | Te/ Ti₃O₅ | Pd/ Ti₃O₅ | Ir/ Ti₃O₅ |
| **Ti₄O₇** | Co/ Ti₄O₇ | Sb/ Ti₄O₇ | W/ Ti₄O₇ | V/ Ti₄O₇ | Ag/ Ti₄O₇ | Te/ Ti₄O₇ | Pd/ Ti₄O₇ | Ir/ Ti₄O₇ |
| **ZrO₂** | Co/ ZrO₂ | Sb/ ZrO₂ | W/ ZrO₂ | V/ ZrO₂ | Ag/ ZrO₂ | Te/ ZrO₂ | Pd/ ZrO₂ | Ir/ ZrO₂ |
| **HfO₂** | Co/ HfO₂ | Sb/ HfO₂ | W/ HfO₂ | V/ HfO₂ | Ag/ HfO₂ | Te/ HfO₂ | Pd/ HfO₂ | Ir/ HfO₂ |
| **VO** | Co/ VO | Sb/ VO | W/ VO | V/ VO | Ag/ VO | Te/ VO | Pd/ VO | Ir/ VO |
| **V₂O₃** | Co/ V₂O₃ | Sb/ V₂O₃ | W/ V₂O₃ | V/ V₂O₃ | Ag/ V₂O₃ | Te/ V₂O₃ | Pd/ V₂O₃ | Ir/ V₂O₃ |
| **VO₂** | Co/ VO₂ | Sb/ VO₂ | W/ VO₂ | V/ VO₂ | Ag/ VO₂ | Te/ VO₂ | Pd/ VO₂ | Ir/ VO₂ |
| **V₂O₅** | Co/ V₂O₅ | Sb/ V₂O₅ | W/ V₂O₅ | V/ V₂O₅ | Ag/ V₂O₅ | Te/ V₂O₅ | Pd/ V₂O₅ | Ir/ V₂O₅ |
| **V₃O₇** | Co/ V₃O₇ | Sb/ V₃O₇ | W/ V₃O₇ | V/ V₃O₇ | Ag/ V₃O₇ | Te/ V₃O₇ | Pd/ V₃O₇ | Ir/ V₃O₇ |
| **V₄O₉** | Co/ V₄O₉ | Sb/ V₄O₉ | W/ V₄O₉ | V/ V₄O₉ | Ag/ V₄O₉ | Te/ V₄0₉ | Pd/ V₄O₉ | Ir/ V₄O₉ |
| **V₆O₁₃** | Co/ V₆O₁₃ | Sb/ V₆O₁₃ | W/ V₆O₁₃ | V/ V₆O₁₃ | Ag/ V₆O₁₃ | Te/ V₆O₁₃ | Pd/ V₆O₁₃ | Ir/ V₆O₁₃ |
| **NbO** | Co/ NbO | Sb/ NbO | W/ NbO | V/ NbO | Ag/ NbO | Te/ NbO | Pd/ NbO | Ir/ NbO |
| **NbO₂** | Co/ NbO₂ | Sb/ NbO₂ | W/ NbO₂ | V/ NbO₂ | Ag/ NbO₂ | Te/ NbO₂ | Pd/ NbO₂ | Ir/ NbO₂ |
| **Nb₂O₅** | Co/ Nb₂O₅ | Sb/ Nb₂O₅ | W/ Nb₂O₅ | V/ Nb₂O₅ | Ag/ Nb₂O₅ | Te/ Nb₂O₅ | Pd/ Nb₂O₅ | Ir/ Nb₂O₅ |
| **Nb₈O₁₉** | Co/ Nb₈O₁₉ | Sb/ Nb₈O₁₉ | W/ Nb₈O₁₉ | V/ Nb₈O₁₉ | Ag/ Nb₈O₁₉ | Te/ Nb₈O₁₉ | Pd/ Nb₈O₁₉ | Ir/ Nb₈O₁₉ |
| **Nb₁₆O₃₈** | Co/ Nb₁₆O₃₈ | Sb/ Nb₁₆O₃₈ | W/ Nb₁₆O₃₈ | V/ Nb₁₆O₃₈ | Ag/ Nb₁₆O₃₈ | Te/ Nb₁₆O₃₈ | Pd/ Nb₁₆O₃₈ | Ir/ Nb₁₆O₃₈ |
| **Nb₁₂O₂₉** | Co/ Nb₁₂O₂₉ | Sb/ Nb₁₂O₂₉ | W/ Nb₁₂O₂₉ | V/ Nb₁₂O₂₉ | Ag/ Nb₁₂O₂₉ | Te/ Nb₁₂O₂₉ | Pd/ Nb₁₂O₂₉ | Ir/ Nb₁₂O₂₉ |
| **Nb₄₇O₁₁₆** | Co/ Nb₄₇O₁₁₆ | Sb/ Nb₄₇O₁₁₆ | W/ Nb₄₇O₁₁₆ | V/ Nb₄₇O₁₁₆ | Ag/ Nb₄₇O₁₁₆ | Te/ Nb₄₇O₁₁₆ | Pd/ Nb₄₇O₁₁₆ | Ir/ Nb₄₇O₁₁₆ |
| **Ta₂O₅** | Co/ Ta₂O₅ | Sb/ Ta₂O₅ | W/ Ta₂O₅ | V/ Ta₂O₅ | Ag/ Ta₂O₅ | Te/ Ta₂O₅ | Pd/ Ta₂O₅ | Ir/ Ta₂O₅ |
| **CrO** | Co/ CrO | Sb/ CrO | W/ CrO | V/ CrO | Ag/ CrO | Te/ CrO | Pd/ CrO | Ir/ CrO |
| **Cr₂O₃** | Co/ Cr₂O₃ | Sb/ Cr₂O₃ | W/ Cr₂O₃ | V/ Cr₂O₃ | Ag/ Cr₂O₃ | Te/ Cr₂O₃ | Pd/ Cr₂O₃ | Ir/ Cr₂O₃ |
| **CrO₂** | Co/ CrO₂ | Sb/ CrO₂ | W/ CrO₂ | V/ CrO₂ | Ag/ CrO₂ | Te/ CrO₂ | Pd/ CrO₂ | Ir/ CrO₂ |
| **CrO₃** | Co/ CrO₃ | Sb/ CrO₃ | W/ CrO₃ | V/ CrO₃ | Ag/ CrO₃ | Te/ CrO₃ | Pd/ CrO₃ | Ir/ CrO₃ |
| **Cr₈O₂₁** | Co/ Cr₈O₂₁ | Sb/ Cr₈O₂₁ | W/ Cr₈O₂₁ | V/ Cr₈O₂₁ | Ag/ Cr₈O₂₁ | Te/ Cr₈O₂₁ | Pd/ Cr₈O₂₁ | Ir/ Cr₈O₂₁ |
| **MoO₂** | **Co/** MoO₂ | Sb/ MoO₂ | W/ MoO₂ | V/ MoO₂ | Ag/ MoO₂ | Te/ MoO₂ | Pd/ MoO₂ | Ir/ MoO₂ |
| **MoO₃** | Co/ MoO₃ | Sb/ MoO₃ | W/ MoO₃ | V/ MoO₃ | Ag/ MoO₃ | Te/ MoO₃ | Pd/ MoO₃ | Ir/ MoO₃ |
| **W₂O₃** | Co/ W₂O₃ | Sb/ W₂O₃ | W/ W₂O₃ | V/ W₂O₃ | Ag/ W₂O₃ | Te/ W₂O₃ | Pd/ W₂O₃ | Ir/ W₂O₃ |
| **WoO₂** | Co/ WoO₂ | Sb/ WoO₂ | W/ WoO₂ | V/ WoO₂ | Ag/ WoO₂ | Te/ WoO₂ | Pd/ WoO₂ | Ir/ WoO₂ |
| **WoO₃** | Co/ WoO₃ | Sb/ WoO₃ | W/ WoO₃ | V/ WoO₃ | Ag/ WoO₃ | Te/ WoO₃ | Pd/ WoO₃ | Ir/ WoO₃ |
| **MnO** | Co/ MnO | Sb/ MnO | W/ MnO | V/ MnO | Ag/ MnO | Te/ MnO | Pd/ MnO | Ir/ MnO |
| **Mn/Mg/O** | Co/ Mn/Mg/O | Sb/ Mn/Mg/O | W/ Mn/Mg/O | V/ Mn/Mg/O | Ag/ Mn/Mg/O | Te/ Mn/Mg/O | Pd/ Mn/Mg/O | Ir/ Mn/Mg/O |
| **Mn₃O₄** | Co/ Mn₃O₄ | Sb/ Mn₃O₄ | W/ Mn₃O₄ | V/ Mn₃O₄ | Ag/ Mn₃O₄ | Te/ Mn₃O₄ | Pd/ Mn₃O₄ | Ir/ Mn₃O₄ |
| **Mn₂O₃** | Co/ Mn₂O₃ | Sb/ Mn₂O₃ | W/ Mn₂O₃ | V/ Mn₂O₃ | Ag/ Mn₂O₃ | Te/ Mn₂O₃ | Pd/ Mn₂O₃ | Ir/ Mn₂O₃ |
| **MnO₂** | Co/ MnO₂ | Sb/ MnO₂ | W/ MnO₂ | V/ MnO₂ | Ag/ MnO₂ | Te/ MnO₂ | Pd/ MnO₂ | Ir/ MnO₂ |
| **Mn₂O₇** | Co/ Mn₂O₇ | Sb/ Mn₂O₇ | W/ Mn₂O₇ | V/ Mn₂O₇ | Ag/ Mn₂O₇ | Te/ Mn₂O₇ | Pd/ Mn₂O₇ | Ir/ Mn₂O₇ |
| **ReO₂** | Co/ ReO₂ | Sb/ ReO₂ | W/ ReO₂ | V/ ReO₂ | Ag/ ReO₂ | Te/ ReO₂ | Pd/ ReO₂ | Ir/ ReO₂ |
| **ReO₃** | Co/ ReO₃ | Sb/ ReO₃ | W/ ReO₃ | V/ ReO₃ | Ag/ ReO₃ | Te/ ReO₃ | Pd/ ReO₃ | Ir/ ReO₃ |
| **Re₂O₇** | Co/ Re₂O₇ | Sb/ Re₂O₇ | W/ Re₂O₇ | V/ Re₂O₇ | Ag/ Re₂O₇ | Te/ Re₂O₇ | Pd/ Re₂O₇ | Ir/ Re₂O₇ |
| **Mg₃Mn₃**-**B₂O₁₀** | Co/ Mg₃Mn₃-B₂O₁₀ | Sb/ Mg₃Mn₃-B₂O₁₀ | W/ Mg₃Mn₃-B₂O₁₀ | V/ Mg₃Mn₃-B₂O₁₀ | Ag/ Mg₃Mn₃-B₂O₁₀ | Te/ Mg₃Mn₃-B₂O₁₀ | Pd/ Mg₃Mn₃-B₂O₁₀ | Ir/ Mg₃Mn₃-B₂O₁₀ |
| **Mg₃(BO₃)₂** | Co/ Mg₃(BO₃)₂ | Sb/ MC)₃(BO₃)₂ | W/ M9₃(BO₃)₂ | V/ Mg₃(BO₃)₂ | Ag/ Mg₃(BO₃)₂ | Te/ Mg₃(BO₃)₂ | Pd/ Mg₃(BO₃)₂ | Ir/ Mg₃(BO₃)₂ |
| **NaWO₄** | Co/ NaWO₄ | Sb/ NaWO₄ | W/ NaWO₄ | V/ NaWO₄ | Ag/ NaWO₄ | Te/ NaWO₄ | Pd/ NaWO₄ | Ir/ NaWO₄ |
| **Mg₆MnO₈** | Co/ Mg₆MnO₈ | Sb/ Mg₆MnO₈ | W/ Mg₆MnO₈ | V/ Mg₆MnO₈ | Ag/ Mg₆MnO₈ | Te/ Mg₆MnO₈ | Pd/ Mg₆MnO₈ | Ir/ Mg₆MnO₈ |
| **Mn₂O₄** | Co/ Mn₂O₄ | Sb/ Mn₂O₄ | W/ Mn₂O₄ | V/ Mn₂O₄ | Ag/ Mn₂O₄ | Te/ Mn₂O₄ | Pd/ Man₂O₄ | Ir/ Mn₂O₄ |
| **(Li,Mg)₆**-**MnO₈** | Co/ (Li,Mg)₆-MnO₈ | Sb/ (Li,Mg)₆-MnO₈ | W/ (Li,Mg)₆-MnO₈ | V/ (Li,Mg)₆-MnO₈ | Ag/ (Li,Mg)₆-MnO₈ | Te/ (Li,Mg)₆-MnO₈ | Pd/ (Li,Mg)₆-MnO₈ | Ir/ (Li,Mg)₆-MnO₈ |
| **Na₄P₂O₇** | Co/ Na₄P₂O₇ | Sb/ Na₄P₂O₇ | W/ Na₄P₂O₇ | V/ Na₄P₂O₇ | Ag/ Na₄P₂O₇ | Te/ Na₄P₂O₇ | Pd/ Na₄P₂O₇ | Ir/ Na₄P₂O₇ |
| **Mo₂O₈** | Co/ Mo₂O₈ | Sb/ Mo₂O₈ | W/ Mo₂O₈ | V/ Mo₂O₈ | Ag/ Mo₂O₈ | Te/ Mo₂O₈ | Pd/ Mo₂O₈ | Ir/ Mo₂O₈ |
| **Mn₃O₄/WO₄** | Co/ Mn₃O₄/WO₄ | Sb/ Mn₃O₄/WO₄ | W/ Mn₃O₄/WO4 | V/ Mn₃O₄/WO₄ | Ag/ Mn₃O₄/WO₄ | Te/ Mn₃O₄/WO₄ | Pd/ Mn₃O₄/WO₄ | Ir/ Mn₃O₄/WO₄ |
| **Na₂WO₄** | Co/ Na₂WO₄ | Sb/ Na₂WO₄ | W/ Na₂WO₄ | V/ Na₂WO₄ | Ag/ Na₂WO₄ | Te/ Na₂WO₄ | Pd/ Na₂WO₄ | Ir/ Na₂WO₄ |
| **Zr₂Mo₂O₈** | Co/ Zr₂Mo₂O₈ | Sb/ Zr₂Mo₂O₈ | W/ Zr₂Mo₂O₈ | V/ Zr₂Mo₂O₈ | Ag/ Zr₂Mo₂O₈ | Te/ Zr₂Mo₂O₈ | Pd/ Zr₂Mo₂O₈ | Ir/ Zr₂Mo₂O₈ |
| **NaMnO₄**-**/MgO** | Co/ NaMnO₄-/MgO | Sb/ NaMnO₄-/MgO | W/ NaMnO₄-/MgO | V/ NaMnO₄-/MgO | Ag/ NaMnO₄-/MgO | Te/ NaMnO₄-/MgO | Pd/ NaMnO₄-/MnO | Ir/ NaMnO₄-/MgO |
| **Na₁₀Mn-W₅O₁₇** | Co/ Na₁₀Mn-W₅O₁₇ | Sb/ Na₁₀Mn-W₅O₁₇ | W/ Na₁₀Mn-W₅O₁₇ | V/ Na₁₀Mn-W₅O₁₇ | Ag/ Na₁₀Mn-W₅O₁₇ | Te/ Na₁₀Mn-W₅O₁₇ | Pd/ Na₁₀Mn-W₅O₁₇ | Ir/ Na₁₀Mn-W₅O₁₇ |

**TABLE 8 NANOWIRES (NW) DOPED WITH SPECIFIC DOPANTS (DOP)**

| **NW\Dop** | **Mn** | **Ti** |
|---|---|---|
| **Li₂O** | Mn/ Li₂O | Ti/ Li₂O |
| **Na₂O** | Mn/ Na₂O | Ti/ Na₂O |
| **K₂O** | Mn/ K₂O | Ti/ K₂O |
| **Rb₂O** | Mn/ Rb₂O | Ti/ Rb₂O |
| **Cs₂O** | Mn/ Cs₂O | Ti/ Cs₂O |
| **BeO** | Mn/ BeO | Ti/ BeO |
| **MgO** | Mn/ MgO | Ti/ MgO |
| **CaO** | Mn/ CaO | Ti/ CaO |
| **SrO** | Mn/ SrO | Ti/ SrO |
| **BaO** | Mn/ BaO | Ti/ BaO |
| **Sc₂O₃** | Mn/ Sc₂O₃ | Ti/ Sc₂O₃ |
| **Y₂O₃** | Mn/ Y₂O₃ | Ti/ Y₂O₃ |
| **La₂O₃** | Mn/ La₂O₃ | Ti/ La₂O₃ |
| **CeO₂** | Mn/ CeO₂ | Ti/ CeO₂ |
| **Ce₂O₃** | Mn/ Ce₂O₃ | Ti/ Ce₂O₃ |
| **Pr₂O₃** | Mn/ Pr₂O₃ | Ti/ Pr₂O₃ |
| **Nd₂O₃** | Mn/ Nd₂O₃ | Ti/ Nd₂O₃ |
| **Sm₂O₃** | Mn/ Sm₂O₃ | Ti/ Sm₂O₃ |
| **Eu₂O₃** | Mn/ Eu₂O₃ | Ti/ Eu₂O₃ |
| **Gd₂O₃** | Mn/ Gd₂O₃ | Ti/ Gd₂O₃ |
| **Tb₂O₃** | Mn/ Tb₂O₃ | Ti/ Tb₂O₃ |
| **TbO₂** | Mn/ TbO₂ | Ti/ TbO₂ |
| **Tb₆O₁₁** | Mn/ Tb₆O₁₁ | Ti/ Tb₆O₁₁ |
| **DY₂O₃** | Mn/ Dy₂O₃ | Ti/ Dy₂O₃ |
| **HO₂O₃** | Mn/ HO₂O₃ | Ti/ HO₂O₃ |
| **Er₂O₃** | Mn/ Er₂O₃ | Ti/ Er₂O₃ |
| **Tm₂O₃** | Mn/ Tm₂O₃ | Ti/ TM₂O₃ |
| **Yb₂O₃** | Mn/ Yb₂O₃ | Ti/ Yb₂O₃ |
| **LU₂O₃** | Mn/ Lu₂O₃ | Ti/ Lu₂O₃ |
| **AC₂O₃** | Mn/ Ac₂O₃ | Ti/ Ac₂O₃ |
| **Th₂O₃** | Mn/ Th₂O₃ | Ti/ Th₂O₃ |
| **ThO₂** | Mn/ ThO₂ | Ti/ ThO₂ |
| **Pa₂O₃** | Mn/ Pa₂O₃ | Ti/ Pa₂O₃ |
| **PaO₂** | Mn/ PaO₂ | Ti/ PaO₂ |
| **TiO₂** | Mn/ TiO₂ | Ti/ TiO₂ |
| **TiO** | Mn/ TiO | Ti/ TiO |
| **Ti₂O₃** | Mn/ Ti₂O₃ | Ti/ Ti₂O₃ |
| **Ti₃O** | Mn/ Ti₃O | Ti/ Ti₃O |
| **Ti₂O** | Mn/ Ti₂O | Ti/ Ti₂O |
| **Ti₃O5** | Mn/ Ti₃O₅ | Ti/ Ti₃O₅ |
| **Ti₄O₇** | Mn/ Ti₄O₇ | Ti/ Ti₄O₇ |
| **ZrO₂** | Mn/ ZrO₂ | Ti/ ZrO₂ |
| **HfO₂** | Mn/ HfO₂ | Ti/ HfO₂ |
| **VO** | Mn/ VO | Ti/ VO |
| **V₂O₃** | Mn/ V₂O₃ | Ti/ V₂O₃ |
| **VO₂** | Mn/ VO₂ | Ti/ VO₂ |
| **V₂O₅** | Mn/ V₂O₅ | Ti/ V₂O₅ |
| **V₃O₇** | Mn/ V₃O₇ | Ti/ V₃O₇ |
| **V₄O₉** | Mn/ V₄O₉ | Ti/ V₄O₉ |
| **V₆O₁₃** | Mn/ V₆O₁₃ | Ti/ V₆O₁₃ |
| **NbO** | Mn/ NbO | Ti/ NbO |
| **NbO₂** | Mn/ NbO₂ | Ti/ NbO₂ |
| **Nb₂O₅** | Mn/ Nb₂O₅ | Ti/ Nb₂O₅ |
| **Nb₈O₁₉** | Mn/ Nb₈O₁₉ | Ti/ Nb₈O₁₉ |
| **Nb₁₆O₃₈** | Mn/ Nb₁₆O₃₈ | Ti/ Nb₁₆O₃₈ |
| **Nb₁₂O₂₉** | Mn/ Nb₁₂O₂₉ | Ti/ Nb₁₂O₂₉ |
| **Nb₄₇O₁₆** | Mn/ Nb₄₇O₁₁₆ | Ti/ Nb₄₇O₁₁₆ |
| **Ta₂O₅** | Mn/ Ta₂O₅ | Ti/ Ta₂O₅ |
| **CrO** | Mn/ CrO | Ti/ CrO |
| **Cr₂O₃** | Mn/ Cr₂O₃ | Ti/ Cr₂O₃ |
| **CrO₂** | Mn/ CrO₂ | Ti/ CrO₂ |
| **CrO₃** | Mn/ CrO₃ | Ti/ CrO₃ |
| **Cr₈O₂ₗ** | Mn/ Cr₈O₂₁ | Ti/ Cr₈O₂₁ |
| **MoO₂** | Mn/ MoO₂ | Ti/ MoO₂ |
| **MoO₃** | Mn/ MoO₃ | Ti/ MoO₃ |
| **W₂O₃** | Mn/ W₂O₃ | Ti/ W₂O₃ |
| **WoO₂** | Mn/ WoO₂ | Ti/ WoO₂ |
| **WoO₃** | Mn/ WoO₃ | Ti/ WoO₃ |
| **MnO** | Mn/ MnO | Ti/ MnO |
| **Mn/Mg/O** | Mn/ Mn/Mg/O | Ti/ Mn/Mg/O |
| **Mn₃O₄** | Mn/ Mn₃O₄ | Ti/ Mn₃O₄ |
| **Mn₂O₃** | Mn/ Mn₂O₃ | Ti/ Mn₂O₃ |
| **MnO₂** | Mn/ MnO₂ | Ti/ MnO₂ |
| **Mn₂O₇** | Mn/ Mn₂O₇ | Ti/ Mn₂O₇ |
| **ReO₂** | Mn/ ReO₂ | Tri/ ReO₂ |
| **ReO₃** | Mn/ ReO₃ | Ti/ ReO₃ |
| **Re₂O₇** | Mn/ Re₂O₇ | Ti/ Re₂O₇ |
| **Mg₃Mn₃**-**B₂O₁₀** | Mn/ Mg₃Mn₃-B₂O₁₀ | Ti/ Mg₃Mn₃-B₂O₁₀ |
| **Mg₃(BO₃)₂** | Mn/ Mg₃(BO₃)₂ | Ti/ Mg₃(BO₃)₂ |
| **NaWO₄** | Mn/ NaWO₄ | Ti/ NaWO₄ |
| **Mg₆MnO₈** | Mn/ Mg₆MnO₈ | Ti/ Mg₆MnO₈ |
| **Mn₂O₄** | Mn/ Mn₂O₄ | Ti/ Mn₂O₄ |
| **(Li,Mg)₆**-**MnO₈** | Mn/ (Li,Mg)₆-MnO₈ | Mn/ (Li,Mg)₆-MnO₈ |
| **Na₄P₂O₇** | Mn/ Na₄P₂O₇ | Ti/ Na₄P₂O₇ |
| **Mo₂O₈** | Mn/ Mo₂O₈ | Ti/ Mo₂O₈ |
| **Mn₃O₄/WO₄** | Mn/ Mn₃O₄/WO₄ | Ti/ Mn₃O₄/WO₄ |
| **Na₂WO₄** | Mn/ Na₂WO₄ | Ti/ Na₂WO₄ |
| **Zr₂Mo₂O₈** | Mn/ Zr₂Mo₂O₈ | Ti/ Zr₂MO₂O₈ |
| **NaMnO₄**-**/MgO** | Mn/ NaMnO₄-/MgO | Ti/ NaMnO₄-/MgO |
| **Na₁₀Mn-W₅O₁₇** | Mn/ Na₁₀Mn-W₅O₁₇ | Ti/ Na₁₀Mn-W₅O₁₇ |

As used in Tables 1-8 and throughout the specification, a nanowire composition represented by E¹/E²/E³, wherein E¹, E² and E³ are each independently an element or a compound comprising one or more elements, refers to a nanowire composition comprised of a mixture of E¹, E² and E³. E¹/E²/E³, are not necessarily present in equal amounts and need not form a bond with one another. For example, a nanowire comprising Li/MgO refers to a nanowire comprising Li and MgO, for example, Li/MgO may refer to a MgO nanowire doped with Li. By way of another example, a nanowire comprising NaMnO₄/MgO refers to a nanowire comprised of a mixture of NaMnO₄ and MgO. Dopants may be added in suitable form. For example in a lithium doped magnesium oxide nanowire (Li/MgO), the Li dopant can be incorporated in the form of Li₂O, Li₂CO₃, LiOH, or other suitable forms. Li may be fully incorporated in the MgO crystal lattice → (e.g., (Li,Mg)O) as well. Dopants for other nanowires may be incorporated analogously.

In some more specific embodiments, the dopant is selected from Li, Ba and Sr. In other specific embodiments, the nanowires comprise Li/MgO, Ba/MgO, Sr/La₂O₃, Ba/La₂O₃, Mn/Na₂WO₄, Mn₂O₃/Na₂WO₄, Mn₃O₄/Na₂WO₄, Mg₆MnO₈, Li/B/Mg₆MnO₈, Na/B/Mg₆MnO₈, Zr₂Mo₂O₈ or NaMnO₄/MgO.

In some other specific embodiments, the nanowire comprises a mixed oxide of Mn and Mg with or without B and with or without Li. Additional dopants for such nanowires may comprise doping elements selected from Group 1 and 2 and groups 7-13. The dopants may be present as single dopants or in combination with other dopants. In certain specific embodiments of nanowires comprising a mixed oxide of Mn and Mg with or without B and with or without Li., the dopant comprises a combination of elements from group 1 and group 8-11.

Nanowires comprising mixed oxides of Mn and Mg are well suited for incorporation of dopants because magnesium atoms can be easily substituted by other atoms as long as their size is comparable with magnesium. A family of "doped" Mg₆MnO₈ compounds with the composition M₍ₓ₎Mg₍₆₋ₓ₎MnO₈, wherein each M is independently a dopant as defined herein and x is 0 to 6, can thus be created. The oxidation state of Mn can be tuned by selecting different amounts (*i.e.,* different values of x) of M with different oxidation states, for example Li₍ₓ₎Mg₍₆₋ₓ)MnO₈ would contain a mixture of Mn(IV) and Mn(V) with x < 1 and a mixture that may include Mn(V), Mn(VI), Mn(VII) with x > 1. The maximum value of x depends on the ability of a particular atom M to be incorporated in the Mg₆MnO₈ crystal structure and therefore varies depending on M. It is believed that the ability to tune the manganese oxidation state as described above could have advantageous effect on the catalytic activity of the disclosed nanowires.

Examples of nanowires comprising Li/Mn/Mg/B and an additional dopant include; Li/Mn/Mg/B doped with Co; Li/Mn/Mg/B doped with Na, Li/Mn/Mg/B doped with Be; Li/Mn/Mg/B doped with Al; Li/Mn/Mg/B doped with Hf; Li/Mn/Mg/B doped with Zr; Li/Mn/Mg/B doped with Zn; Li/Mn/Mg/B doped with Rh and Li/Mn/Mg/B doped with Ga. Nanowires comprising Li/Mn/Mg/B doped with different combinations of these dopants are also provided. For example, in some embodiments the Li/Mn/Mg/B nanowires are doped with Na and Co. In other embodiments, the Li/Mn/Mg/B nanowires are doped with Ga and Na.

In other embodiments, nanowires comprising Mn/W with or without dopants are provided. For example, the present inventors have found through high throughput testing that nanowires comprising Mn/W and various dopants are good catalysts in the OCM reaction. Accordingly, in some embodiments, the Mn/W nanowires are doped with Ba. In other embodiments, the Mn/W nanowires are doped with Be. In yet other embodiments, the Mn/W nanowires are doped with Te.

In any of the above embodiments, the Mn/W nanowires may comprise a SiO₂ support. Alternatively, the use of different supports such as ZrO₂, HfO₂ and In₂O₃ in any of the above embodiments has been shown to promote OCM activity at reduced temperature compared to the same catalyst supported on silica with limited reduction in selectivity.

Nanowires comprising rare earth oxides or Yttria doped with various elements are also effective catalysts in the OCM reaction. In certain specific embodiments, the rare earth oxide or oxy-hydroxide can be any rare earth, preferably La, Nd, Eu, Sm, Yb, Gd. In certain embodiments of the nanowires comprising rare earth elements or yttria, the dopant comprises alkali earth (group 2) elements. The degree of effectiveness of a particular dopant is a function of the rare earth used and the concentration of the alkali earth dopant. In addition to Alkali earth elements, further embodiments of the rare earth or yttria nanowires include embodiments wherein the nanowires comprise alkali elements as dopants which further promote the selectivity of the OCM catalytic activity of the doped material. In yet other embodiments of the foregoing, the nanowires comprise both an alkali element and alkali earth element as dopant. In still further embodiments, an additional dopant can be selected from an additional rare earth and groups 3, 4, 8, 9, 10, 13, 14.

The foregoing rare earth or yttria catalyst may be doped prior to, or after formation of the rare earth or yttria oxide. In one, the rare earth or yttria salt is mixed with the precursor salt to form a solution or a slurry which is dried and then calcined in a range of 400 °C to 900 °C, or between 500 °C and 700 °C. In another embodiment, the rare earth or yttria oxide is formed first through calcination of a rare earth or yttria salt and then contacted with a solution comprising the doping element prior to drying and calcination between 300 °C and 800 °C, or between 400 °C and 700 °C.

In other embodiments, the nanowires comprise La₂O₃ or LaO_{y}(OH)ₓ, wherein y ranges from 0 to 1.5, x ranges from 0 to 3 and 2y + x =3, doped with Na, Mg, Ca, Sr, Ga, Sc, Y, Zr, In, Nd, Eu, Sm, Ce, Gd or combinations thereof. In yet further embodiments, the La₂O₃ or LaO_{y}(OH)ₓ nanowires are doped with binary dopant combinations, for example Eu/Na; Eu/Gd; Ca/Na; Eu/Sm; Eu/Sr; Mg/Sr; Ce/Mg; Gd/Sm, Mg/Na, Mg/Y, Ga/Sr, Nd/Mg, Gd/Na or Sm/Na. In some other embodiments, the La₂O₃ or LaO_{y}(OH)ₓ nanowires are doped with a binary dopant combination, for example Ca-Mg-Na.

In other embodiments, the nanowires comprise Nd₂O₃ or NdO_{y}(OH)ₓ, wherein y ranges from 0 to 1.5, x ranges from 0 to 3 and 2y + x =3, doped with Sr, Ca, Rb, Li, Na or combinations thereof. In certain other embodiments, the Nd₂O₃ or NdOy(OH)ₓ nanowires are doped with binary dopant combinations, for example Ca/Sr or Rb/Sr, Ta/Sr or Al/Sr.

In still other examples of doped nanowires, the nanowires comprise Yb₂O₃ or YbO_{y}(OH)ₓ, wherein y ranges from 0 to 1.5, x ranges from 0 to 3 and 2y + x =3, doped with Sr, Ca, Ba, Nd or combinations thereof. In certain other embodiments, the Yb₂Oₛ or YbO_{y}(OH)ₓ OCM nanowires are doped with a binary combination, for example of Sr/Nd.

Still other examples of doped nanowires Eu₂O₃ or EuO_{y}(OH)ₓ nanowires, wherein y ranges from 0 to 1.5, x ranges from 0 to 3 and 2y + x =3, doped with Sr, Ba, Sm, Gd, Na or combinations thereof or a binary dopant combination, for example Sr/Na or Sm/Na.

Example of dopants for Sm₂O₃ or SmO_{y}(OH)ₓ nanowires, wherein x and y are each independently an integer from 1 to 10, include Sr, and examples of dopants for Y₂O₃ or YO_{y}(OH)ₓ nanowires, wherein y ranges from 0 to 1.5, x ranges from 0 to 3 and 2y + x =3, comprise Ga, La, Nd or combinations thereof. In certain other embodiments, the Y₂O₃ or YO_{y}(OH)ₓ nanowires comprise a binary dopant combination, for example Sr/Nd, Eu/Y or Mg/Nd or a tertiary dopant combination, for example Mg/Nd/Fe.

Rare earth nanowires which without doping have low OCM selectivity can be greatly improved by doping to reduce their combustion activity. In particular, nanowires comprising CeO₂ and Pr₂O₃ tend to have strong total oxidation activity for methane, however doping with additional rare earth elements can significantly moderate the combustion activity and improve the overall utility of the catalyst. Example of dopants which improving the selectivity for Pr₂O₃ or PrO_{y}(OH)ₓ nanowires, , wherein y ranges from 0 to 1.5, x ranges from 0 to 3 and 2y + x =3, comprise binary dopants, for example Nd/Mg, La/Mg or Yb/Sr.

In some embodiments, dopants are present in the nanowires in, for example, less than 50 at%, less than 25 at%, less than 10 at%, less than 5 at% or less than 1 at%.

In other embodiments of the nanowires, the atomic ratio (w/w) of the one or more metal elements selected from Groups I-7 and lanthanides and actinides in the form of an oxide and the dopant ranges from 1:1 to 10,000:1, 1:1 to 1,000:1 or 1:1 to 500:1.

In further embodiments, the nanowires comprise one or more metal elements from Group2 in the form of an oxide and a dopant from Group I. In further embodiments, the nanowires comprise magnesium and lithium. In other embodiments, the nanowires comprise one or more metal elements from Group2 and a dopant from Group 2, for example, in some embodiments, the nanowires comprise magnesium oxide and barium. In another embodiment, the nanowires comprise an element from the lanthanides in the form of an oxide and a dopant from Group 1 or Group 2. In further embodiments, the nanowires comprise lanthanum and strontium.

Various methods for preparing doped nanowires are described. The doped nanowires can be prepared by co-precipitating a nanowire metal oxide precursor and a dopant precursor. In these embodiments, the doping element may be directly incorporated into the nanowire.

### Template Directed Synthesis of Nanowires

The nanowires can be prepared in a solution phase using an appropriate template. In this context, an appropriate template can be any synthetic or natural material, or combination thereof, that provides nucleation sites for binding ions (*e.g*. metal element ions and/or hydroxide or other anions) and causing the growth of a nanowire. The templates can be selected such that certain control of the nucleation sites, in terms of their composition, quantity and location can be achieved in a statistically significant manner. The templates are typically linear or anisotropic in shape, thus directing the growth of a nanowire.

In contrast to other template directed preparation of nanowires, the present nanowires are generally not prepared from nanoparticles deposited on a template in a reduced state which are then heat treated and fused into a nanowire. Such methods are not generally applicable to nanowires comprising one or more elements from any of Groups 1 through 7, lanthanides, actinides or combinations thereof. Instead, the nanowires are prepared by nucleation of an oxidized metal element (e.g., in the form of a metal salt) and subsequent growth of nanowire. The nanowires are then generally calcined to produce the desired oxide, but annealing of nanoparticles is not necessary to form the nanowires.

### 1. Biological Template

Because peptide sequences have been shown to have specific and selective binding affinity for many different types of metal element ions, biological templates incorporating peptide sequences as nucleation sites are preferred. Moreover, biological templates can be engineered to comprise pre-determined nucleation sites in pre-determined spatial relationships (*e.g.,* separated by a few to tens of nanometers).

Both wild type and genetically engineered biological templates can be used. As discussed herein, biological templates such as proteins and bacteriophage can be engineered based on genetics to ensure control over the type of nucleation sites (*e.g.*, by controlling the peptide sequences), their locations on the templates and their respective density and/or ratio to other nucleation sites. See, *e.g.,* Mao, C.B. et al., (2004) Science, 303, 213-217; Belcher, A. et al., (2002) Science 296, 892-895; Belcher, A. et al., (2000) Nature 405 (6787) 665-668; Reiss et al., (2004) Nanoletters, 4 (6), 1127-1132, Flynn, C. et al., (2003) J. Mater. Sci., 13, 2414-2421; Mao, C.B. et al., (2003) PNAS, 100 (12), 6946-6951. This allows for the ability to control the composition and distribution of the nucleation sites on the biological template.

Thus, biological templates may be particularly advantageous for a controlled growth of nanowires. Biological templates can be biomolecules (*e.g*., proteins) as well as multi-molecular structures of a biological origin, including, for example, bacteriophage, virus, amyloid fiber, and capsid.

### (a) Biomolecules

Preferably, the biological templates are biomolecules. More preferably, the biological templates are anisotropic biomolecules. Typically, a biomolecule comprises a plurality of subunits (building blocks) joined together in a sequence via chemical bonds. Each subunit comprises at least two reactive groups such as hydroxyl, carboxylic acid and amino groups, which enable the bond formations that interconnect the subunits. Examples of the subunits include, but are not limited to: amino acids (both natural and synthetic) and nucleotides. Accordingly, the biomolecule template may be a peptide, protein, nucleic acid, polynucleotide, amino acid, antibody, enzyme, or single-stranded or double-stranded nucleic acid or any modified and/or degraded forms thereof.

Because protein synthesis can be genetically directed, proteins can be readily manipulated and functionalized to contain desired peptide sequences (*i.e.,* nucleation sites) at desired locations within the primary structure of the protein. The protein can then be assembled to provide a template.

Thus, in various embodiments, the templates are biomolecules are native proteins or proteins that can be engineered to have nucleation sites for specific ions.

### (b) Baceteriophage

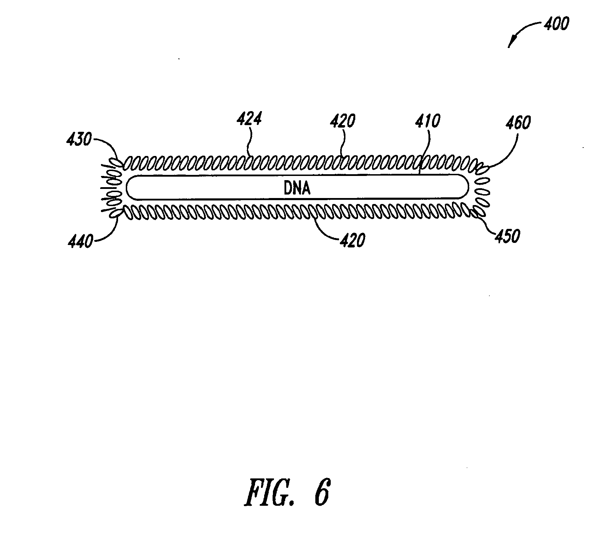

Preferably, the biological template comprises a M13 bacteriophage which has or can be engineered to have one or more particular peptide sequences bound onto the coat proteins. Figure 6 schematically shows a filamentous bacteriophage 400, in which a single-stranded DNA core 410 is surrounded by a proteinaceous coat 420. The coat is composed mainly of pVIII proteins 424 that cover the length of the bacteriophage. The ends of the bacteriophage are capped by minor coat proteins 430 (pIII), 440 (pVI), 450 (pVII) and 460 (pIX).

Using genetic engineering, a library of diverse, novel peptide sequences (up to 10¹² unique peptides) can be expressed on the surface of the phage, so that each individual phage displays at least one unique peptide sequence. These externally facing peptide sequences can be tested, through the iterative steps of screening, amplification and optimization, for the ability to control nucleation and growth of specific catalytic nanowires.

For example, peptide sequences having one or more particular nucleation sites specific for various ions are bound onto the coat proteins. For example, the coat protein is pVIII with peptide sequences having one or more particular nucleation sites specific for various ions bound thereto. Alternatively, the peptide sequences bound to the coat protein comprise 2 or more amino acids, 5 or more amino acids, 10 or more amino acids, 20 or more amino acids, or 40 or more amino acids. Or, the peptide sequences bound to the coat protein comprise between 2 and 40 amino acids, between 5 and 20 amino acids, or between 7 and 12 amino acids.

One of the approaches to obtain different types of M13 bacteriophage is to modify the viral genetic code in order to change the amino acid sequence of the phage coating protein pVIII. The changes in sequence only affect the last amino acids of the pVIII protein, which are the ones that make the surface of the M13 phage, while the first 45 amino acids are left unchanged so that the packing of the pVIII proteins around the phage is not compromised. By changing the outer amino acids on the pVIII protein, the surface characteristics of the phage can be tailored to higher affinities to specific metal ions and thus promoting selective growth of specific inorganic materials on the phage surface.

### (c) Amyloid Fibers

Also, amyloid fibers can be used as the biological template on which metal ions can nucleate and assemble into a catalytic nanowire. Under certain conditions, one or more normally soluble proteins (*i.e*., a precursor protein) may fold and assemble into a filamentous structure and become insoluble. Amyloid fibers are typically composed of aggregated β-strands, regardless of the structure origin of the precursor protein. As used herein, the precursor protein may contain natural or unnatural amino acids. The precursor protein may be further modified with a fatty acid tail.

### (d) Virus and Capsid

Alternatively, a virus or a capsid can be used as a biological template. Similar to a bacteriophage, a virus also comprises a protein coat and a nucleic acid core. In particular, viruses of anisotropic shapes, such as viral fibers, are suitable for nucleating and growing the catalytic nanowires described herein. Further, a virus can be genetically manipulated to express specific peptides on its coat for desirable binding to the ions. Viruses that have elongated or filamentous structures include those that are described in, for example, Christopher Ring, Genetically Engineered Viruses, (Ed) Bios Scientific (2001).

The virus may have its genetic materials removed and only the exterior protein coat (capsid) remains as the biological template.

### 2. Nucleation

Nucleation is the process of forming an inorganic nanowire *in situ* by converting soluble precursors (*e.g.,* metal salts and anions) into nanocrystals in the presence of a template (*e.g.,* a biological template). Typically, the nucleation and growth takes place from multiple binding sites along the length of the biological template in parallel. The growth continues until a structure encasing the biological template is formed. The structure is polycrystalline. If desired, upon completion of the synthesis the organic biological template (*e.g.,* bacteriophage) can be removed by thermal treatment (∼300 °C) in air or oxygen, without significantly affecting either the structure or shape of the inorganic material. In addition, dopants can be either simultaneously incorporated during the growth process or, dopants can be incorporated via impregnation techniques.

### (a) Nanowire Growth Methods

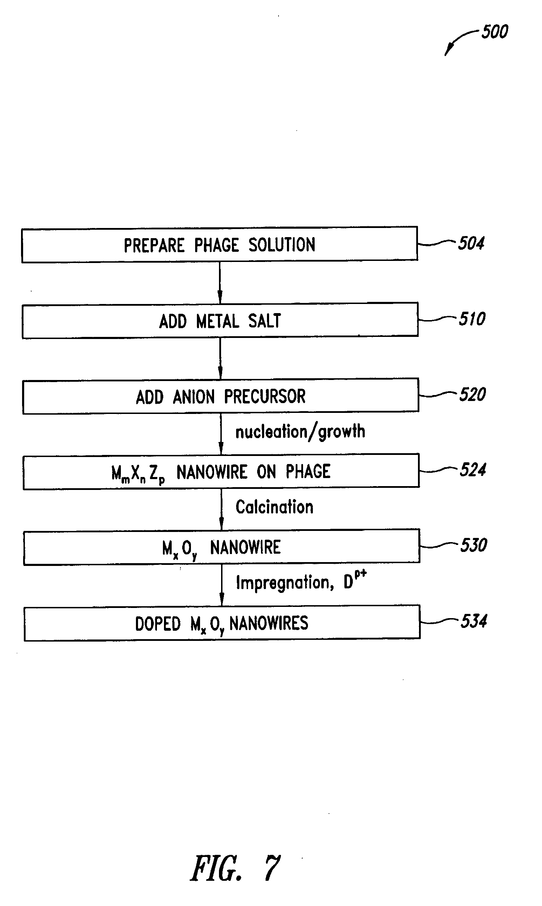

Figure 7 shows a flow chart of a nucleation process for forming a nanowire comprising a metal oxide. A phage solution is first prepared (block 504), to which metal salt precursor comprising metal ions is added (block 510). Thereafter, an anion precursor is added (block 520). It is noted that, the additions of the metal ions and anion precursor can be simultaneous or sequentially in any order. Under appropriate conditions (*e.g.,* pH, molar ratio of the phage and metal salt, molar ratio of the metal ions and anions, addition rate), the metal ions and anions become bound to the phage, nucleate and grow into a nanowire of MₘXₙZₚ composition (block 524). Following calcinations, nanowires comprising MₘXₙ are transformed to nanowires comprising metal oxide (MₓO_{y}) (block 530). An optional step of doping (block 534) incorporates a dopant (D^{P+})in the nanowires comprising metal oxide (MₓO_{y}, wherein x and y are each independently a number from 1 to 100.

Thus, a metal oxide nanowire comprising a plurality of metal oxides (MₓO_{y}), can be prepared by:
a) providing a solution comprising a plurality of biological templates;
(b) introducing at least one metal ion and at least one anion to the solution under conditions and for a time sufficient to allow for nucleation and growth of a nanowire comprising a plurality of metal salts (MₘXₙZₚ) on the template; and
(c) converting the nanowire (MₘXₙZₚ) to a metal oxide nanowire comprising a plurality of metal oxides (MₓO_{y}),
wherein:
M is, at each occurrence, independently a metal element from any of Groups 1 through 7, lanthanides or actinides;
X is, at each occurrence, independently hydroxides, carbonates, bicarbonates, phosphates, hydrogenphosphates, dihydrogenphasphates, sulfates, nitrates or oxalates;
Z is O;
n, m, x and y are each independently a number from 1 to 100; and
p is a number from 0 to 100.

In certain variations of the foregoing, two or more different metal ions may be used. This produces nanowires comprising a mixture of two or more metal oxides. Such nanowires may be advantageous in certain catalytic reactions. For example, a nanowire may comprise two or more different metal oxides where at least one of the metal oxides has good OCM activity and at least one metal oxide has good ODH activity.

Applicants have found that it may be advantageous to perform multiple sequential additions of the metal ion, This addition technique may be particularily applicable wherein two or more different metal ions eare employed to form a mixed nanowire (M1M2XₓY_{y}, wherein M1 and M2 are different metal elements), which can be converted to M1M2O**_{z}**, for example by calcination. The slow addition may be performed over any period of time, for example from 1 day to 1 week. In this regard, use of a syringe pump may be advantageous. Slow addition of the components help ensure that they will nucleate on the biological template instead of non-selectively precipitate.

The biological templates can be phages, as defined herein. The metal ion may be provided by adding one or more metal salt (as described herein) to the solution. Or, the anion is provided by adding one or more anion precursor to the solution. Alternatively, the metal ion and the anion can be introduced to the solution simultaneously or sequentially in any order. Preferably, the nanowire (MₘXₙZₚ) is converted to a metal oxide nanowire by calcination, which is a thermal treatment that transforms or decomposes the MₘXₙZₚ nanowire to a metal oxide. The method can comprise doping the metal oxide nanowire with a dopant. Converting the nanowire to a metal oxide generally comprises calcining.

In a variation of the above method, mixed metal oxides can be prepared (as opposed to a mixture of metal oxides). Mixed metal oxides can be represented by the following formula M1_{w}M2ₓM3_{y}O_{z}, wherein M1, M2 and M3 are each independently absent or a metal element, and w, x, y and z are integers such that the overall charge is balanced. Mixed metal oxides comprising more than three metals are also contemplated and can be prepared via an analogous method. Such mixed metal oxides find utility in a variety of the catalytic reactions disclosed herein. One exemplary mixed metal oxide is Na₁₀MnW₅O₁₇ (Example 18).

Thus, a mixed metal oxide nanowire comprising a plurality of mixed metal oxides (M1_{w}M2ₓM3_{y}O_{z}), can be prepared by:
a) providing a solution comprising a plurality of biological templates;
(b) introducing metal salts comprising M1, M2 and M3 to the solution under conditions and for a time sufficient to allow for nucleation and growth of a nanowire comprising a plurality of the metal salts on the template; and
(c) converting the nanowire to a mixed metal oxide nanowire comprising a plurality of mixed metal oxides (M1_{W}M2ₓM3_{y}O_{z}),
wherein:
M1, M2 and M3 are, at each occurrence, independently a metal element from any of Groups 1 through 7, lanthanides or actinides;
n, m, x and y are each independently a number from 1 to 100; and
p is a number from 0 to 100.

The method for preparing metal oxide nanowires may not require a calcination step. Thus, the method for preparing metal oxide nanowires comprises:
(a) providing a solution that includes a plurality of biological templates; and
(b) introducing a compound comprising a metal to the solution under conditions and for a time sufficient to allow for nucleation and growth of a nanowire (MₘYₙ) on the template;
wherein:
M is a metal element from any of Groups 1 through 7, lanthanides or actinides;
Y is O;
n and m are each independently a number from 1 to 100.

M can be an early transition metal, for example V, Nb, Ta, Ti, Zr, Hf, W, Mo or Cr. Alternatively, the metal oxide is WO₃. The method may further comprises doping the metal oxide nanowire with a dopant. Also, a reagent can be added which converts the compound comprising a metal into a metal oxide.

The nanowires can be prepared by using metal salts sensitive to water hydrolysis, for example NbCl₅, WCl₆, TiCl₄, ZrCl₄. A template can be placed in ethanol along with the metal salt. Water is then slowly added to the reaction in order to convert the metals salts to metal oxide coated template.

By varying the nucleation conditions, including (without limitation): incubation time of phage and metal salt; incubation time of phage and anion; concentration of phage; metal ion concentration, anion concentration, sequence of adding anion and metal ions; pH; phage sequences; solution temperature in the incubation step and/or growth step; types of metal precursor salt; types of anion precursor; addition rate; number of additions; amount of metal salt and/or anion precursor per addition, the time that lapses between the additions of the metal salt and anion precursor, including, *e.g.,* simultaneous (zero lapse) or sequential additions followed by respective incubation times for the metal salt and the anion precursor, stable nanowires of diverse compositions and surface properties can be prepared. For example, the pH of the nucleation conditions can be at least 7.0, at least 8.0, at least 9.0, at least 10.0, at least 11.0, at least 12.0 or at least 13.0.

As noted above, the rate of addition of reactants (*e.g.,* metal salt, metal oxide, anion precursor) is one parameter that can be controlled and varied to produce nanowires having different properties. During the addition of reactants to a solution containing an existing nanowire and/or a templating material (*e.g.,* phage), a critical concentration is reached for which the speed of deposition of solids on the existing nanowire and/or templating material matches the rate of addition of reactants to the reaction mixture. At this point, the concentration of soluble cation stabilizes and stops rising. Thus, nanowire growth can be controlled and maximized by maintaining the speed of addition of reactants such that near super-saturation concentration of the cation is maintained. This helps ensure that no undesirable nucleation occurs. If super-saturation of the anion (*e.g.,* hydroxide) is exceeded, a new solid phase can start nucleating which allows for non-selective solid precipitation, rather than nanowire growth. Thus, in order to selectively deposit an inorganic layer on an existing nanowire and/or a templating material, the addition rate of reactants should be controlled to avoid reaching super-saturation of the solution containing the suspended solids.

Accordingly, the reactant can be repeatedly added in small doses to slowly build up the concentration of the reactant in the solution containing the template. Preferably, the speed of addition of reactant is such that the reactant concentration in the solution containing the template is near (but less than) the saturation point of the reactant. Further preferably, the reactant is added portion wise (*i.e.,* step addition) rather than continuously. In these case, the amount of reactant in each portion, and the time between addition of each portion, is controlled such that the reactant concentration in the solution containing the template is near (but less than) the saturation point of the reactant. In the foregoing, the reactant is a metal cation , or the reactant is an anion.

Initial formation of nuclei on a template can be obtained by the same method described above, wherein the concentration of reactant is increased until near, but not above, the supersaturation point of the reactant. Such an addition method facilitates nucleation of the solid phase on the template, rather than homogeneous non-seeding nucleation. It is desirable to use a slower reactant addition speed during the initial nucleation phase as the super-saturation depression due to the template might be quite small at this point. Once the first layer of solid (*i.e.,* nanowire) is formed on the template, the addition speed can be increased.

Preferably, the addition rate of reactant is controlled such that the precipitation rate matches the addition rate of the reactant. In these cases, nanowires comprising two or more different metals can be prepared by controlling the addition rates of two or more different metal cation solutions such that the concentration of each cation in the templating solution is maintained at or near (but does not exceed) the saturation point for each cation.

Preferably, the optimal speed of addition (and step size if using step additions) is controlled as a function of temperature. For example, the nanowire growth rate is accelerated at higher temperatures. Thus, the addition rate of reactants is adjusted according to the temperature of the templating solution.

Alternatively, modeling (iterative numeric rather than algebraic) of the nanowire growth process is used to determine optimal solution concentrations and supernatant re-cycling strategies.

As noted above, the addition rate of reactants can be controlled and modified to change the properties of the nanowires. Alternatively, the addition rate of a hydroxide source must be controlled such that the pH of the templating solution is maintained at the desired level. This method may require specialized equipment, and depending on the addition rate, the potential for localized spikes in pH upon addition of the hydroxide source is possible. Thus, a method wherein the template solution comprises a weak base that slowly generates hydroxide in-situ, obviating the need for an automated addition sequence is preferred.

In the above, organic epoxides, such as but not limited to propylene oxide and epichlorohydrin, are used to slowly increase the template solution pH without the need for automated pH control. The epoxides are proton scavengers and undergo an irreversible ring-opening reaction with a nucleophilic anion of the metal oxide precursor (such as but not limited to Cl⁻ or NO₃⁻). The net effect is a slow homogenous raise in pH to form metal hydroxy species in solution that deposit onto the template surface. Preferably, the organic epoxide is propylene oxide.

An attractive feature of this method is that the organic epoxide can be added all at once, there is no requirement for subsequent additions of organic epoxide to grow metal oxide coatings over the course of the reaction. Due to the flexibility of the "epoxide-assisted" coatings, it is anticipated that many various embodiments can be employed to make new templated materials (*e.g.,* nanowires). For example, mixed metal oxide nanowires can be prepared by starting with appropriate ratios of metal oxide precursors and propylene oxide in the presence of bacteriophage. In other cases, metal oxide deposition on bacteriophage can be done sequentially to prepare core/shell materials (described in more detail below).

### (b) Metal Salt

As noted above, the nanowires are prepared by nucleation of metal ions in the presence of an appropriate template, for example, a bacteriophage. In this respect, any soluble metal salt may be used as the precursor of metal ions that nucleate on the template. Soluble metal salts of the metals from Groups 1 through 7, lanthanides and actinides are particularly useful and all such salts are contemplated.

The soluble metal salt may comprises chlorides, bromides, iodides, nitrates, sulfates, acetates, oxides, oxyhalides, oxynitrates, phosphates (including hydrogenphosphate and dihydrogenphosphate) or oxalates of metal elements from Groups 1 through 7, lanthanides, actinides or combinations thereof. Preferably, the soluble metal salt comprises chlorides, nitrates or sulfates of metal elements from Groups 1 through 7, lanthanides, actinides or combinations thereof. The present disclosure contemplates all possible chloride, bromide, iodide, nitrate, sulfate, acetate, oxide, oxyhalides, oxynitrates, phosphates (including hydrogenphosphate and dihydrogenphosphate) and oxalate salts of metal elements from Groups 1 through 7, lanthanides, actinides or combinations thereof.

Preferably, the metal salt comprises LiCl, LiBr, Lil, LiNO₃, Li₂SO₄, LiCO₂CH₃, Li₂C₂O₄, NaCl, NaBr, Nal, NaNO₃, Na₂SO₄, NaCO₂CH₃, Na₂C₂O₄, KCl, KBr, Kl, KNO₃, K₂SO₄, KCO₂CH₃, K₂C₂O₄, RbCl, RbBr, Rbl, RbNO₃, Rb₂SO₄, RbCO₂CH₃, Rb₂C₂O₄, CsCl, CsBr, Csl, CsNO₃, Cs₂SO₄, CsCO₂CH₃, Cs₂C₂O₄, BeCl₂, BeBr₂, Bel₂, Be(NO₃)₂, BeSO₄, Be(CO₂CH₃)₂, BeC₂O₄, MgCl₂, MgBr₂, Mgl₂, Mg(NO₃)₂, MgSO₄, Mg(CO₂CH₃)₂, MgC₂O₄, CaCl₂, CaBr₂, Cal₂, Ca(NO₃)₂, CaSO₄, Ca(CO₂CH₃)₂, CaC₂O₄, SrCl₂, SrBr₂, Srl₂, Sr(NO₃)₂, SrSO₄, Sr(CO₂CH₃)₂, SrC₂O₄, BaCl₂, BaBr₂, Bal₂, Ba(NO₃)₂, BaSO₄, Ba(CO₂CH₃)₂, BaC₂O₄, ScCl₃, ScBr₃, Scl₃, Sc(NO₃)₃, Sc₂(SO₄)₃, Sc(CO₂CH₃)₃, Sc₂(C₂O₄)₃, YCl₃, YBr₃, YI₃, Y(NO₃)₃, Y₂(SO₄)₃, Y(CO₂CH₃)₃, Y₂(C₂O₄)₃, TiCl₄, TiBr₄, TiI₄. Ti(NO₃)₄, Ti(SO₄)₂, Ti(CO₂CH₃)₄, Ti(C₂O₄)₂, ZrCl₄, ZrOCl₂, ZrBr₄, ZrI₄, Zr(NO₃)₄, ZrO(NO₃)₂, Zr(SO₄)₂, Zr(CO₂CH₃)₄, Zr(C₂O₄)₂, HfCl₄, HfBr₄, Hfl₄, Hf(NO₃)₄, Hf(SO₄)₂, Hf(CO₂CH₃)₄, Hf(C₂O₄)₂,LaCl₃, LaBr₃, Lal₃, La(NO₃)₃, La₂(SO₄)₃, La(CO₂CH₃)₃, La₂(C₂O₄)₃, WCl₂, WCl₃, WCl₄, WCl₅, WCl₆, WBr₂, WBr₃, WBr₄, WBr₅, WBr₆, WI₂, WI₃, WI₄, WI₅, WI₆, W(NO₃)₂, W(NO₃)₃, W(NO₃)₄, W(NO₃)₅, W(NO₃)₆, W(CO₂CH₃)₂, W(CO₂CH₃)₃, W(CO₂CH₃)₄, W(CO₂CH₃)₅, W(CO₂CH₃)₆, WC₂O₄, W₂(C₂O₄)₃, W(C₂O₄)₂, W₂(C₂O₄)₅, W(C₂O₄)₆, MoCl₄, MnCl₂ MnCl₃, MnBr₂ MnBr₃, MnI₂ MnI₃, Mn(NO₃)₂, Mn(NO₃)₃, MnSO₄, Mn₂(SO₄)₃, Mn(CO₂CH₃)₂, Mn(CO₂CH₃)₃, MnC₂O₄, Mn₂(C₂O₄)₃, MoCl₂, MₒCl₃, MoCl₄, MoCl₅, MoBr₂, MoBr₃, MoBr₄, MoBr₅, Mol₂, Mol₃, Mol₄, Mol₅, Mo(NO₃)₂, Mo(NO₃)₃, Mo(NO₃)₄, Mo(NO₃)₅, MoSO₄, Mo₂(SO₄)₃, Mo(SO₄)₂, Mo₂(SO₄)₅, Mo(CO₂CH₃)₂, Mo(CO₂CH₃)₃, Mo(CO₂CH₃)₄, Mo(CO₂CH₃)₅, MoC₂O₄, Mo₂(C₂O₄)₃, MO(C₂O₄)₂, Mo₂(C₂O₄)₅, VCI, VCl₂ VCl₃, VCl₄, VBr, VBr₂, VBr₃, VBr₄, VI, VI₂, VI₃, VI₄, VNO₃, V(NO₃)₂, V(NO₃)₃, V(NO₃)₄, V₂SO₄, VSO₄, V₂(SO₄)₃, V(SO₄)₄, VCO₂CH₃, V(CO₂CH₃)₂, V(CO₂CH₃)₃, V(CO₂CH₃)₄, V₂C₂O₄, VC₂O₄, V₂(C₂O₄)₃, V(C₂O₄)₄, NdCl₃, NdBr₃, NdI₃, Nd(NO₃)₃, Ndy(SO₄)₃, Nd(CO₂CH₃)₃, Nd₂(C₂O₄)₃, EuCl₃, EuBr₃, Eul₃, Eu(NO₃)₃, Eu₂(SO₄)₃, Eu(CO₂CH₃)₃, Eu₂(C₂O₄)₃, PrCl₃, PrBr₃, PrI₃, Pr(NO₃)₃, Pr₂(SO₄)₃, Pr(CO₂CH₃)₃, Pr₂(C₂O₄)₃, SmCl₃, SmBr₃, SmI₃, Sm(NO₃)₃, Sm₂(SO₄)₃, Sm(CO₂CH₃)₃, Sm₂(C₂O₄)₃, CeCl₃, CeBr₃, Cel₃, Ce(NO₃)₃, Ce₂(SO₄)₃, Ce(CO₂CH₃)₃, Ce₂(C₂O₄)₃ or combinations thereof.

More preferably, the metal salt comprises MgCl₂, LaCl₃, ZrCl₄, WCl₄, MoCl₄, MnCl₂ MnCl₃, Mg(NO₃)₂, La(NO₃)₃, ZrOCl₂, Mn(NO₃)2, Mn(NO₃)₃, ZrO(NO₃)₂, Zr(NO₃)₄, or combinations thereof.

Also preferably, the metal salt comprises NdCl₃, NdBr₃, NdI₃, Nd(NO₃)₃, Nd₂(SO₄)₃, Nd(CO₂CH₃)₃, Nd₂(C₂O₄)₃, EuCl₃, EuBr₃, Eul₃, Eu(NO₃)₃, Eu₂(SO₄)₃, EU(CO₂CH₃)₃, Eu₂(C₂O₄)₃, PrCl₃, PrBr₃, Prl₃, Pr(NO₃)₃, Pr₂(SO₄)₃, Pr(CO₂CH₃)₃, Pr₂(C₂O₄)₃ or combinations thereof.

Further preferably, the metal salt comprises Mg, Ca, Mg, W, La, Nd, Sm, Eu, W, Mn, Zr or mixtures thereof. The salt may be in the form of (oxy)chlorides, (oxy)nitrates or tungstates.

### (c) Anion Precursor

The anions, or counter ions of the metal ions that nucleate on the template, are provided in the form of an anion precursor. The anion precursor dissociates in the solution phase and releases an anion. Thus, the anion precursor can be any stable soluble salts having the desired anion. For instance, bases such as alkali metal hydroxides (*e.g.,* sodium hydroxide, lithium hydroxide, potassium hydroxides) and ammonium hydroxide are anion precursors that provide hydroxide ions for nucleation. Alkali metal carbonates (*e.g.,* sodium carbonate, potassium carbonates) and ammonium carbonate are anion precursors that provide carbonates ions for nucleation.

The anion precursor may comprises one or more metal hydroxide, metal carbonate, metal bicarbonate, or metal oxalate. Preferably, the metal is an alkali or an alkaline earth metal. Thus, the anion precursor may comprise any one of alkali metal hydroxides, carbonates, bicarbonates, or oxalate; or any one of alkaline earth metal hydroxide, carbonates, bicarbonates, or oxalate.

Preferably, the one or more anion precursors comprises LiOH, NaOH, KOH, Sr(OH)₂, Ba(OH)₂, Na₂CO₃, K₂CO₃, NaHCO₃, KHCO₃, and NR₄OH, wherein R is selected from H, and C₁-C₆ alkyl. Ammonium salts may provide certain advantages in that there is less possibility of introducing unwanted metal impurities. Accordingly, more preferably, the anion precursor comprises ammonium hydroxide.

The dimensions of the nanowires are comparable to those of the biological templates (*e.g.,* phage), although they can have different aspect ratios as longer growth can be used to increase the diameter while the length will increase in size at a much slower rate. The spacing of peptides on the phage surface controls the nucleation location and the catalytic nanowire size based on steric hindrance. The specific peptide sequence information can (or may) dictate the identity, size, shape and crystalline face of the catalytic nanowire being nucleated. To achieve the desired stochiometry between metal elements, support and dopants, multiple peptides specific for these discrete materials can be co-expressed within the same phage. Alternatively, precursor salts for the materials can be combined in the reaction at the desired stochiometry. The techniques for phage propagation and purification are also well established, robust and scalable. Multi-kilogram amounts of phage can be easily produced, thus assuring straightforward scale up to large, industrial quantities.

Typical functional groups in amino acids that can be used to tailor the phage surface affinity to metal ions include: carboxylic acid (-COOH), amino (-NH₃⁺ or -NH₂), hydroxyl (-OH), and/or thiol (-SH) functional groups. Table 9 summarizes a number of exemplary phages used in the present invention for preparing nanowires of inorganic metal oxides. Sequences within Table 9 refer to the amino acid sequence of the pVIII protein (single-letter amino acid code). Underlined portions indicate the terminal sequence which was varied to tailor the phage surface affinity to metal ions. SEQ ID NO 14 represents wild type pVIII protein while SEQ ID NO 15 represents wild type pVIII protein including the signaling peptide portion (bold).

**TABLE 9**

| **SEQ ID NO** | **Sequence** |
|---|---|
| 1 | AEEGSEDPAKAAFNSLQASATEYIGYAWAMVVVIVGATIGIKLFKKFTSKAS |
| 2 | EEGSDDPAKAAFNSLQASATEYIGYAWAMVVVIVGATIGIKLFKKFTSKAS |
| 3 | AEEEDPAKAAFNSLQASATEYIGYAWAMVVVIVGATIGIKLFKKFTSKAS |
| 4 | EEEDPAKAAFNSLQASATEYIGYAWAMVWIVGATIGIKLFKKFTSKAS |
| 5 | AEEEEDPAKAAFNSLQASATEYIGYAWAMVVVIVGATIGIKLFKKFTSKAS |
| 6 | AEEAEDPAKAAFNSLQASATEYIGYAWAMVVVIVGATIGIKLFKKFTSKAS |
| 7 | EEXEDPAKAAFNSLQASATEYIGYAWAMVVVIVGATIGIKLFKKFTSKAS |
| | X = E or G |
| 8 | AEDDDPAKAAFNSLQASATEYIGYAWAMVWIVGATIGIKLFKKFTSKAS |
| 9 | AVSGSSPGDDPAKAAFNSLQASATEYIGYAWAMVVVIVGATIGIKLFKKFTSKAS |
| 10 | AVSGSSPDSDPAKAAFNSLQASATEYIGYAWAMVWIVGATIGIKLFKKFTSKAS |
| 11 | AGETQQAMEDPAKAAFNSLQASATEYIGYAWAMVVVIVGATIGIKLFKKFTSKAS |
| 12 | AAGETQQAMDPAKAAFNSLQASA TEYIGY AWAMVWIVGA TIGIKLFKKFTSKAS |
| 13 | AEPGHDAVPEDPAKAAFNSLQASATEYIGYAWAMVVVIVGATIGIKLFKKFTSKAS |
| 14 | AEGDDDPAKAAFNSLQASATEYIGYAWAMVWIVGATIGIKLFKKFTSKAS |
| 15 | **MKKSLVLKASVAVATLVPMLSFA** |
| | AEGDDDPAKAAFNSLQASA TEYIGY AWAMWVIVGA TIGIKLFKKFTSKAS |

### 3. Core/Shell Structures

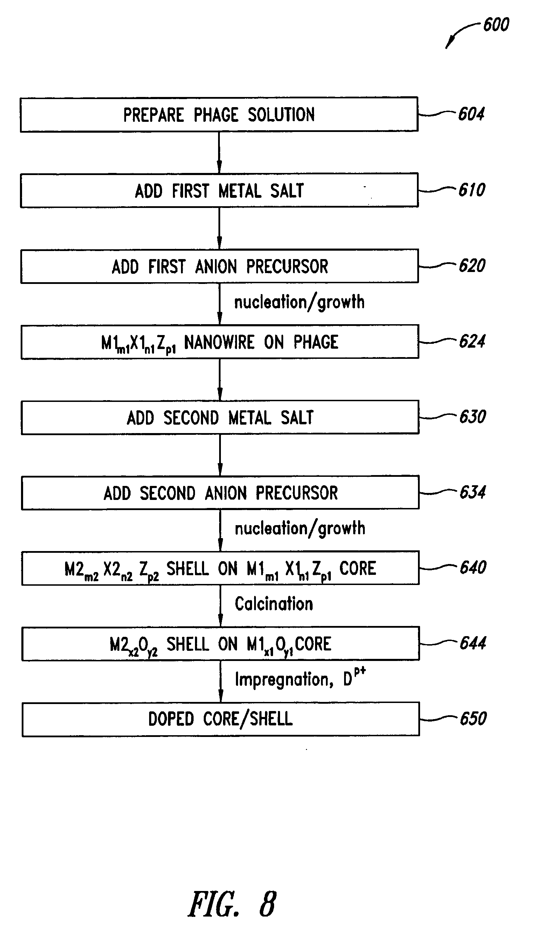

The nanowires can be grown on a support nanowire that has no or a different catalytic property. Figure 8 shows an exemplary process 600 for growing a core/shell structure. Similar to Figure 7, a phage solution is prepared (block 604), to which a first metal salt and a first anion precursor are sequentially added (blocks 610 and 620) in appropriate conditions to allow for the nucleation and growth of a nanowire (M1ₘ₄X1ₙ₁Zₚ₁) on the phage (block 624). Thereafter, a second metal salt and a second anion precursor are sequentially added (blocks 630 and 634), under conditions to cause the nucleation and growth of a coating of M2ₘ₂X2ₙ₂ Zₚ₂ on the nanowire M1ₘ₁X1ₙ₁ Zp₁ (block 640). Following calcinations, nanowires of a core/shell structure M1ₓ₁O_{y1}/M2ₓ₂O_{y2} are formed, wherein x1, y1, x2 and y2 are each independently a number from 1 to 100, and p1 and p2 are each independently a number from 0 to 100 (block 644). A further step of impregnation (block 650) produces a nanowire comprising a dopant and comprising a core of M1ₓ₁O_{y1} coated with a shell of M2ₓ₂O_{y2}. Preferably, M1 is Mg, Al, Ga, Ca or Zr, or M1 is Mn and M2 is Mg. More preferably, M1 is Mg and M2 is Mn, or M1 is La and M2 is Mg, Ca, Sr, Ba, Zr, Nd, Y, Yb, Eu, Sm or Ce. Alternatively, M1 is Mg and M2 is La or Nd.

Preferably, M1ₓ₁O_{y1} comprises La₂O₃, or M2ₓ₂O_{y2} comprises La₂O₃. Preferably, M1ₓ₁O_{y1} or M2ₓ₂O_{y2} further comprises a dopant, wherein the dopant comprises Nd, Mn, Fe, Zr, Sr, Ba, Y or combinations thereof.

Thus, metal oxide nanowires in a core/shell structure can be prepared by:
(a) providing a solution that includes a plurality of biological templates;
(b) introducing a first metal ion and a first anion to the solution under conditions and for a time sufficient to allow for nucleation and growth of a first nanowire (M1ₘ₁X1ₙ₁ Zₚ₁) on the template; and
(c) introducing a second metal ion and optionally a second anion to the solution under conditions and for a time sufficient to allow for nucleation and growth of a second nanowire (M2ₘ₂X2ₙ₂ Zp₂) on the first nanowire (M1ₘ₁X1ₙ₁ Zₚ₁);
(d) converting the first nanowire (M1ₘ₁X1ₙ₁ Zₚ₁) and the second nanowire (M2ₘ₂X2ₙ₂ Zₚ₂) to respective metal oxide nanowires (M1ₓ₁O_{y1}) and (M2ₓ₂O_{y2}),
wherein:
M1 and M2 are the same or different and independently selected from a metal element;
X1 and X2 are the same or different and independently hydroxides, carbonates, bicarbonates, phosphates, hydrogenphosphates, dihydrogenphosphates, sulfates, nitrates or oxalates;
Z is O;
n1, m1" m1, m2, x1, y1, x2 and y2 are each independently a number from 1 to 100; and
p1 and p2 are independently a number from 0 to 100.

M1 and M2 are the same or different and independently selected from a metal element from any of Groups 2 through 7, lanthanides or actinides.

The biological templates can be phages, as defined herein.

The method may comprises doping the metal oxide nanowire in a core/shell structure with a dopant.

By varying the nucleation conditions, including the pH of the solution, relative ratio of metal salt precursors and the anion precursors, relative ratios of the precursors and the phage of the synthetic mixture, stable nanowires of diverse compositions and surface properties can be prepared.

The core nanowire (the first nanowire) may be catalytically inactive or less so than the shell nanowire (the second nanowire), and the core nanowire serve as an intrinsic catalytic support for the more active shell nanowire. For example, ZrO₂ may nt have high catalytic activity in an OCM reaction, whereas Sr²⁺ doped La₂O₃ does. A ZrO₂ core thus may serve as a support for the catalytic Sr²⁺ doped La₂O₃ shell.

### 4. Diversity

As noted above, the disclosed template-directed synthesis provides nanowires having diverse compositions and/or morphologies. This method combines two extremely powerful approaches, evolutionary selection and inorganic synthesis, to produce a library of nanowire catalysts with a new level of control over materials composition, materials surface and crystal structure. These nanowires prepared by biologically-templated methods take advantage of genetic engineering techniques to enable combinatorial synthesis of robust, active and selective inorganic catalytic polycrystalline nanowires. With selection, evolution and a combinatorial library with over a hundred billion sequence possibilities, nanowires having high specificity and product conversion yields in catalytic reactions are generated. This permits simultaneous optimization the nanowires' catalytic properties in a high-dimensional space.

The synthetic parameters for nucleating and growing nanowires can be manipulated to create nanowires of diverse compositions and morphologies. Typical synthetic parameters include, without limitation, concentration ratios of metal ions and active functional groups on the phage; concentration ratios of metal and anions (*e.g*., hydroxide); incubation time of phage and metal salt; incubation time of phage and anion; concentration of phage; sequence of adding anion and metal ions; pH; phage sequences; solution temperature in the incubation step and/or growth step; types of metal precursor salt; types of anion precursor; addition rate, number of additions; the time that lapses between the additions of the metal salt and anion precursor, including, *e.g*., simultaneous (zero lapse) or sequential additions followed by respective incubation times for the metal salt and the anion precursor.

Additional variable synthetic parameters include, growth time once both metal and anion are present in the solution; choice of solvents (although water is typically used, certain amounts of alcohol, such as methanol, ethanol and propanol, can be mixed with water); choice and the number of metal salts used (*e.g.,* both LaCl₃ and La(NO₃)₃ can be used to provide La³⁺ ions); choice and the number of anion precursors used (*e.g*., both NaOH then LiOH can be used to provide the hydroxide); choice or the number of different phage sequences used; the presence or absence of a buffer solution; the different stages of the growing (*e.g.,* nanowires may be precipitated and cleaned and resuspended in a second solution and perform a second growth of the same material (thicker core) or different material to form a core/shell structure.

Thus, libraries of nanowires can be generated with diverse physical properties and characteristics such as: composition, *e.g.,* basic metal oxides (MₓO_{y}), size, shape, surface morphology, exposed crystal faces/edge density, crystallinity, dispersion, and stoichiometry and nanowire template physical characteristics including length, width, porosity and pore density. High throughput, combinatorial screening methods are then applied to evaluate the catalytic performance characteristics of the nanowires (*see, e.g.,* Figure 2). Based on these results, lead target candidates are identified. From these lead targets, further rational modifications to the synthetic designs can be made to create nanowires that satisfy certain catalytic performance criteria. This results in further refinement of the nanowire design and material structure.

### Direct Synthesis of Nanowires

### 1. Oxidative Coupling of Methane (OCM)

As noted above, described are nanowires having catalytic activity and related approaches to nanowire design and preparation for improving the yield, selectivity and/or conversion of any number of catalyzed reactions, including the OCM reaction. As mentioned above, there exists a tremendous need for catalyst technology capable of addressing the conversion of methane into high value chemicals (*e.g.,* ethylene and products prepared therefrom) using a direct route that does not go through syngas. Accomplishing this task will dramatically impact and redefine a non-petroleum based pathway for feedstock manufacturing and liquid fuel production yielding reductions in GHG emissions, as well as providing new fuel sources.

Ethylene has the largest carbon footprint compared to all industrial chemical products in part due to the large total volume consumed into a wide range of downstream important industrial products including plastics, surfactants and pharmaceuticals. In 2008, worldwide ethylene production exceeded 120 M metric tons while growing at a robust rate of 4% per year. The United States represents the largest single producer at 28% of the world capacity. Ethylene is primarily manufactured from high temperature cracking of naphtha (*e.g.,* oil) or ethane that is separated from natural gas. The true measurement of the carbon footprint can be difficult as it depends on factors such as the feedstock and the allocation as several products are made and separated during the same process. However, some general estimates can be made based on published data.

Cracking consumes a significant portion (about 65%) of the total energy used in ethylene production and the remainder is for separations using low temperature distillation and compression. The total tons of CO₂ emission per ton of ethylene are estimated at between 0.9 to 1.2 from ethane cracking and 1 to 2 from naphtha cracking. Roughly, 60% of ethylene produced is from naphtha, 35% from ethane and 5% from others sources (Ren, T.; Patel, M. Res. Consetv. Recycl. 53:513, 2009). Therefore, based on median averages, an estimated amount of CO₂ emissions from the cracking process is 114M tons per year (based on 120M tons produced). Separations would then account for an additional 61 M tons CO₂ per year.

Nanowires provide an alternative to the need for the energy intensive cracking step. Additionally, because of the high selectivity of the nanowires, downstream separations are dramatically simplified, as compared to cracking which yields a wide range of hydrocarbon products. The reaction is also exothermic so it can proceed via an autothermal process mechanism. Overall, it is estimated that up to a potential 75% reduction in CO₂ emission compared to conventional methods could be achieved. This would equate to a reduction of one billion tons of CO₂ over a ten-year period and would save over 1 M barrels of oil per day.

The nanowires also permit converting ethylene into liquid fuels such as gasoline or diesel, given ethylene's high reactivity and numerous publications demonstrating high yield reactions, in the lab setting, from ethylene to gasoline and diesel. On a life cycle basis from well to wheel, recent analysis of methane to liquid (MTL) using F-T process derived gasoline and diesel fuels has shown an emission profile approximately 20% greater to that of petroleum based production (based on a worst case scenario) (Jaramillo, P., Griffin, M., Matthews, S., Env. Sci. Tech 42:7559, 2008). In the model, the CO₂ contribution from plant energy was a dominating factor at 60%. Thus, replacement of the cracking and F-T process would be expected to provide a notable reduction in net emissions, and could be produced at lower CO₂ emissions than petroleum based production.

Furthermore, a considerable portion of natural gas is found in regions that are remote from markets or pipelines. Most of this gas is flared, re-circulated back into oil reservoirs, or vented given its low economic value. The World Bank estimates flaring adds 400M metric tons of CO₂ to the atmosphere each year as well as contributing to methane emissions. The nanowires of this disclosure also provide economic and environmental incentive to stop flaring. Also, the conversion of methane to fuel has several environmental advantages over petroleum-derived fuel. Natural gas is the cleanest of all fossil fuels, and it does not contain a number of impurities such as mercury and other heavy metals found in oil. Additionally, contaminants including sulfur are also easily separated from the initial natural gas stream. The resulting fuels burn much cleaner with no measurable toxic pollutants and provide lower emissions than conventional diesel and gasoline in use today.

The selective, catalytic oxidative coupling of methane to ethylene (i.e. the OCM reaction) is shown by the following reaction (1):

2CH₄ + O₂ 〉̶ CH₂CH₂ + 2 H₂O (1)

This reaction is exothermic (Heat of Reaction -67kcals/mole) and usually occurs at very high temperatures (> 700°C). During this reaction, it is believed that the methane (CH₄) is first oxidatively coupled into ethane (C₂H₆), and subsequently the ethane (C₂H₆) is oxidatively dehydrogenated into ethylene (C₂H₄). Because of the high temperatures used in the reaction, it has been suggested that the ethane is produced mainly by the coupling in the gas phase of the surface-generated methyl (CH₃) radicals. Reactive metal oxides (oxygen type ions) are apparently required for the activation of CH₄ to produce the CH₃ radicals. The yield of C₂H₄ and C₂H₆ is limited by further reactions in the gas phase and to some extent on the catalyst surface. A few of the possible reactions that occur during the oxidation of methane are shown below as reactions (2) through (8):

CH₄ → CH₃ radical (2)

CH₃ radical → C₂H₆ (3)

CH₃ radical + 2.5 O₂ → CO₂ + 1.5 H₂O (4)

C₂H₆ → C₂H₄ + H₂ (5)

C₂H₆ + 0.5 O₂ → C₂H₄ + H₂O (6)

C₂H₄ +3 O₂ → 2CO₂ + 2H₂O (7)

CH₃ radical + CₓH_{y} +O₂ → Higher HC's -Oxidation/ CO₂ +H₂O (8)

With conventional heterogeneous catalysts and reactor systems, the reported performance is generally limited to < 25% CH₄ conversion at < 80% combined C₂ selectivity, with the performance characteristics of high selectivity at low conversion, or the low selectivity at high conversion. In contrast, the nanowires of this disclosure are highly active and can optionally operate at a much lower temperature. The nanowires disclosed herein enable efficient conversion of methane to ethylene in the OCM reaction at temperatures less than when the corresponding bulk material is used as a catalyst. For example, the nanowires disclosed herein enable efficient conversion (*i.e.,* high yield, conversion, and/or selectivity) of methane to ethylene at temperatures of less than 900 °C, less than 800 °C, less than 700 °C, less than 600 °C, or less than 500 °C. In other embodiments, the use of staged oxygen addition, designed heat management, rapid quench and/or advanced separations may also be employed.

Typically, the OCM reaction is run in a mixture of oxygen and nitrogen or other inert gas. Such gasses are expensive and increase the overall production costs associated with preparation of ethylene or ethane from methane. However, the present inventors have now discovered that such expensive gases are not required and high yield, conversion, selectivity, etc. can be obtained when air is used as the gas mixture instead of pre-packaged and purified sources of oxygen and other gases. Accordingly, in one embodiment the disclosure provides a method for performing the OCM reaction in air.

Accordingly, in one embodiment a stable, very active, high surface area, multifunctional nanowire catalyst is disclosed having active sites that are isolated and precisely engineered with the catalytically active metal centers/sites in the desired proximity (see, *e.g.,* Figure 1).

The exothermic heats of reaction (free energy) follows the order of reactions depicted above and, because of the proximity of the active sites, will mechanistically favor ethylene formation while minimizing complete oxidation reactions that form CO and CO₂. Representative nanowire compositions useful for the OCM reaction include, but are not limited to: highly basic oxides selected from the early members of the Lanthanide oxide series; Group 1 or 2 ions supported on basic oxides, such as Li/MgO, Ba/MgO and Sr/La₂O₃; and single or mixed transition metal oxides, such as VOₓ and Re/Ru that may also contain Group 1 ions. Other nanowire compositions useful for the OCM reaction comprise any of the compositions disclosed herein, for example MgO, La₂O₃, Na₂WO₄, Mn₂O₃, Mn₃O₄, Mg₆MnO₈, Zr₂Mo₂O₈, NaMnO₄, Mn₂O₃/Na₂WO₄, Mn₃O₄/Na₂WO₄ or Na/MnO₄/MgO, Mn/WO4, Nd₂O₃, Sm₂O₃, Eu₂O₃ or combinations thereof. Activating promoters (*i.e.,* dopants), such as chlorides, nitrates and sulfates, or any of the dopants described above may also be employed.

As noted above, the OCM reaction employing known bulk catalysts suffers from poor yield, selectivity, or conversion. In contrast to a corresponding bulk catalyst, Applicants have found that the nanowires disclosed herein, posses a catalytic activity in the OCM reaction such that the yield, selectivity, and/or conversion is better than when the OCM reaction is catalyzed by a corresponding bulk catalyst.

### 2. Downstream Products

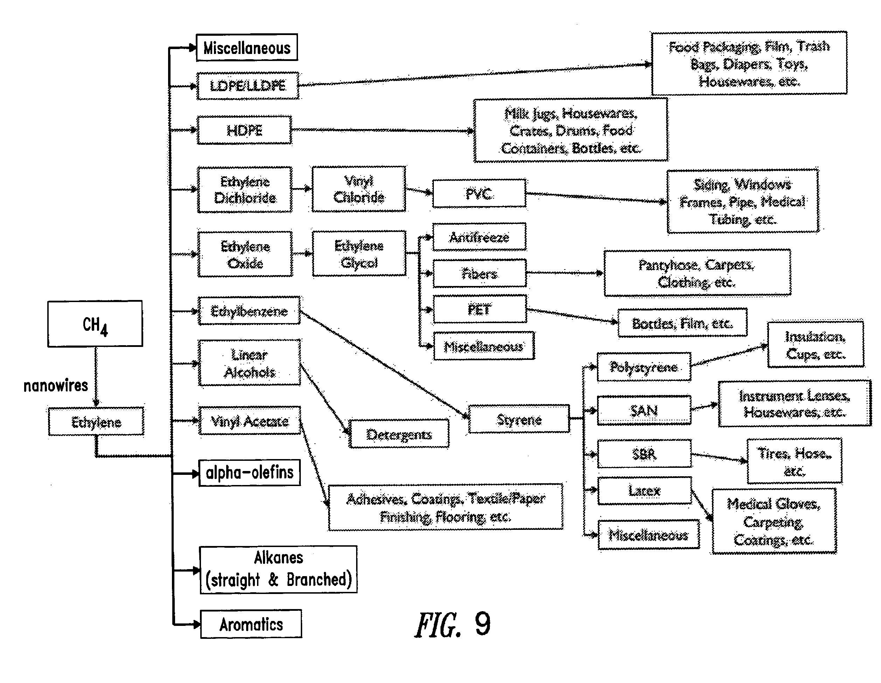

As noted above, the present disclosure is directed to nanowires useful as catalysts in reactions for the preparation of a number of valuable hydrocarbon compounds. For example, in one embodiment the nanowires are useful as catalysts for the preparation of ethylene from methane via the OCM reaction. Ethylene and propylene are valuable compounds which can be converted into a variety of consumer products. For example, as shown in Figure 9 ethylene can be converted into many various compounds including low density polyethylene, high density polyethylene, ethylene dichloride, ethylene oxide, ethylbenzene, linear alcohols, vinyl acetate, alkanes, alpha olefins, various hydrocarbon-based fuels, ethanol. These compounds can then be further processed using methods well known to one of ordinary skill in the art to obtain other valuable chemicals and consumer products (*e.g.* the downstream products shown in Figure 9). Propylene can be analogously converted into various compounds and consumer goods including polypropylenes, propylene oxides, propanol.

Accordingly, in one embodiment the disclosure provides a method of preparing the downstream products of ethylene noted in Figure 9 . The method comprises converting ethylene into a downstream product of ethylene, wherein the ethylene has been prepared via a catalytic reaction employing the nanowires disclosed herein. In another embodiment the disclosure provides a method of preparing low density polyethylene, high density polyethylene, ethylene dichloride, ethylene oxide, ethylbenzene, ethanol or vinyl acetate from ethylene, wherein the ethylene has been prepared as described above.

In another embodiment, the disclosure provides a method of preparing a product comprising low density polyethylene, high density polyethylene, ethylene dichloride, ethylene oxide, ethylbenzene, ethanol or vinyl acetate, alkenes, alkanes, aromatics, alcohols, or mixtures thereof. The method comprises converting ethylene into low density polyethylene, high density polyethylene, ethylene dichloride, ethylene oxide, ethylbenzene, ethanol or vinyl acetate, wherein the ethylene has been prepared via a catalytic reaction employing a nanowires, for example any of the exemplary nanowires disclosed herein.

In one particular embodiment, the disclosure provides a method of preparing a downstream product of ethylene, wherein the downstream product is a hydrocarbon fuel. For example, the downstream product of ethylene may be a C₄-C₁₄ hydrocarbon, including alkanes, alkenes and aromatics. Some specific examples include 1-butene, 1-hexene, 1-octene, xylenes. The method comprises converting methane into ethylene by use of the catalytic nanowires disclosed herein, and further oligomerizing the ethylene and to prepare a downstream product of ethylene. For example, the methane may be converted to ethylene thereof via the OCM reaction as discussed above. The catalytic nanowire is an inorganic catalytic polycrystalline nanowire, the nanowire having a ratio of effective length to actual length of less than one and an aspect ratio of greater than ten as measured by TEM in bright field mode at 5 keV, wherein the nanowire comprises one or more elements from any of Groups 1 through 7, lanthanides, actinides or combinations thereof. The nanowires may additionally comprise any number of doping elements as discussed above.

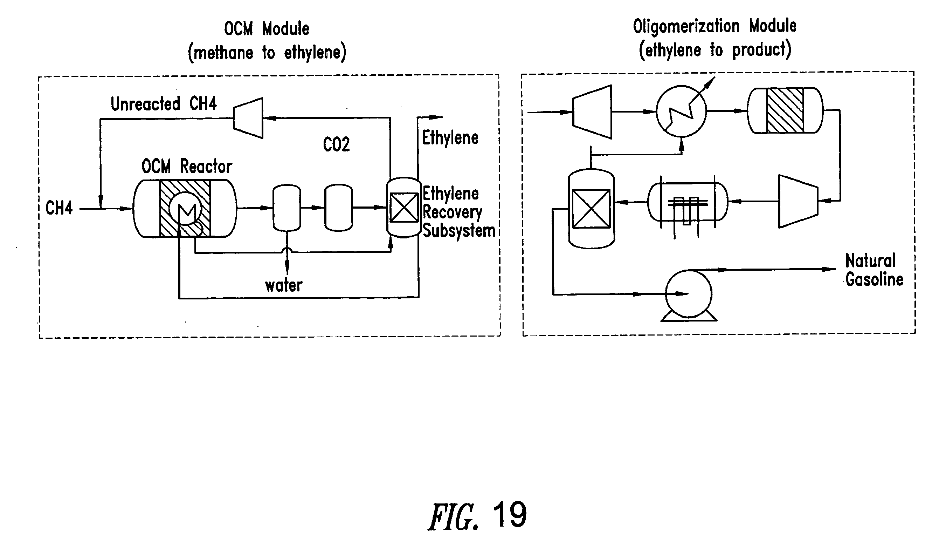

As depicted in Figure 19 the method begins with charging methane (e.g., as a component in natural gas) into an OCM reactor. The OCM reaction may then be performed utilizing a nanowire under any variety of conditions. Water and CO₂ are optionally removed from the effluent and unreacted methane is recirculated to the OCM reactor.

Ethylene is recovered and charged to an oligomerization reactor. Optionally the ethylene stream may contain CO₂, H₂O, N₂, ethane, C3's and/or higher hydrocarbons. Oligomerization to higher hydrocarbons (e.g., C₄-C₁₄) then proceeds under any number of conditions known to those of skill in the art. For example oligomerization may be effected by use of any number of catalysts known to those skilled in the art. Examples of such catalysts include catalytic zeolites, crystalline borosilicate molecular sieves, homogeneous metal halide catalysts, Cr catalysts with pyrrole ligands or other catalysts. Exemplary methods for the conversion of ethylene into higher hydrocarbon products are disclosed in the following references: Catalysis Science & Technology (2011), 1(1), 69-75; Coordination Chemistry Reviews (2011), 255(7-8), 861-880; Eur. Pat. Appl. (2011), EP 2287142 A1 20110223; Organometallics (2011), 30(5), 935-941; Designed Monomers and Polymers (2011), 14(1), 1-23; Journal of Organometallic Chemistry 689 (2004) 3641-3668; Chemistry--A European Journal (2010), 16(26), 7670-7676; Acc. Chem. Res. 2005, 38, 784-793; Journal of Organometallic Chemistry, 695 (10-11): 1541-1549 May 15 2010; Catalysis Today Volume 6, Issue 3, January 1990, Pages 329-349; U.S. Patent No. 5,968,866; U.S. Patent No. 6,800,702; U.S. Patent No. 6,521,806; U.S. Patent No. 7,829,749; U.S. Patent No. 7,867,938; U.S. Patent No. 7,910,670; U.S. Patent No. 7,414,006 and Chem. Commun., 2002, 858-859.

In certain embodiments, the exemplary OCM and oligomerization modules depicted in Figure 19 may be adapted to be at the site of natural gas production, for example a natural gas field. Thus the natural gas can be efficiently converted to more valuable and readily transportable hydrocarbon commodities without the need for transport of the natural gas to a processing facility.

Referring to Figure 19 "natural gasoline" refers to a mixture of oligomerized ethylene products. The mixture may comprise 1-hexene, 1-octene, linear, branched or cyclic alkanes of 6 or more hydrocarbons, linear, branched, or cyclic alkenes of 6 or more hydrocarbons, aromatics, such as benzene, toluene, dimethyl benzene, xylenes, napthalene, or other oligomerized ethylene products and combinations thereof. This mixture finds particular utility in any number of industrial applications, for example natural gasoline is used as feedstock in oil refineries, as fuel blend stock by operators of fuel terminals, as diluents for heavy oils in oil pipelines and other applications. Other uses for natural gasoline are well-known to those of skill in the art.

### EXAMPLES

### EXAMPLE 1 (Reference)

### GENETIC ENGINEERING/PREPARATION OF PHAGE

Phage were amplified in DH5 derivative E. coli (New England Biolabs, NEB5-alpha F' Iq; genotype: F' *proA*+B+ *laclq* Δ(*lacZ*)M15 *zzf::Tn10* (TetR) *lfhuA2*Δ*(argF-lacZ)U969 phoA glnV44* Φ*80*Δ*(lacZ)*M15 *gyrA96 recA1 endA1 thi-1 hsdR17)* and purified using standard polyethylene glycol and sodium chloride precipitation protocols as described in the following references: Kay, B. K.; Winter, J.; McCafferty, J. Phage Display of Peptides and Proteins: A Laboratory Manual; Academic Press: San Diego (1996); C.F. Barbas, et al., ed., Phage Display: A Laboratory Manual; Cold Spring Harbor Laboratory Press, Cold Spring Harbor, NY, USA (2001); and Joseph Sambrook and David W. Russell, Molecular Cloning, 3rd edition, Cold Spring Harbor Laboratory Press, Cold Spring Harbor, NY, USA, 2001.

### EXAMPLE 2 (Reference)

### PREPARATION OF PHAGE SOLUTIONS

The phage solutions were additionally purified by centrifuging at an acceleration of 10000 g at least once (until no precipitated material was observed), decanting the supernatant and splitting it in 50ml containers, which were then stored frozen at -20°C. The frozen phage solutions were thawed only shortly before being used.

The concentration of the phage solutions was measured using a UV-VIS spectrometer. The concentration of each of the frozen phage aliquots was measured prior to use. This spectroscopic method relies on the absorption of the nucleotides in the DNA of the phage and is described in more detail in "Phage Display: A Laboratory Manual" by Babas, Burton, Scott and Silverman (Cold Spring Harbor Laboratory Press, 2001). The concentration of phage solutions is expressed in pfu/ml (plague forming units per milliliter).

### EXAMPLE 3 (Reference)

### PREPARATION MG(OH)₂ NANOWIRES

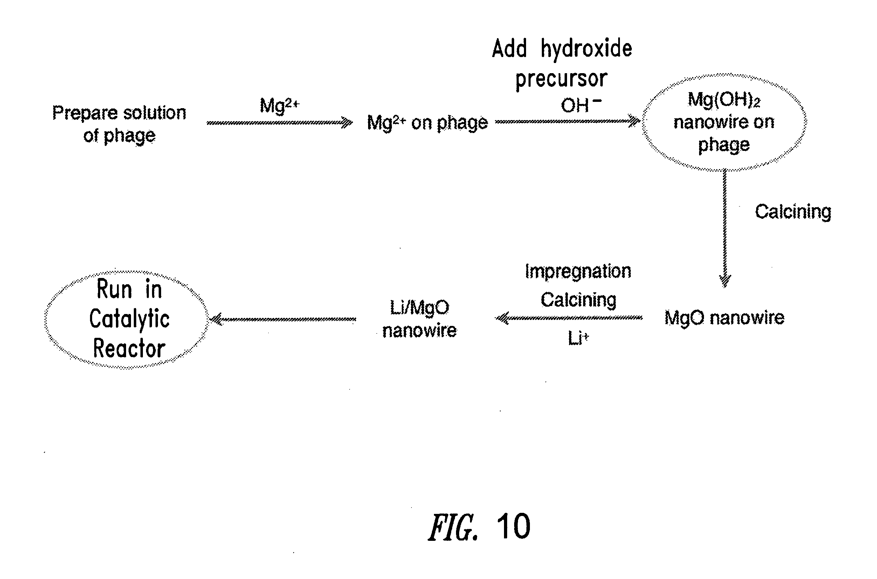

Figure 10 shows a generic reaction scheme for preparing MgO nanowires (with dopant). First, the phage solution is thawed and its concentration determined according to the method described above. The phage solution is diluted with water to adjust its concentration in the reaction mixture (*i.e.* with all the ingredients added) to the desired value, typically 5e12 pfu/ml or higher. The reaction container can be anything from a small vial (for milliliter scale reactions) up to large bottles (for liter reaction scale reactions).

A magnesium solution and a base solution are added to the phage solution in order to precipitate Mg(OH)₂. The magnesium solution can be of any soluble magnesium salt, *e.g.* MgX2•6H2O (X = Cl, Br, I), Mg(NO₃)₂, MgSO₄, magnesium acetate, etc. The range of the magnesium concentration in the reaction mixture is quite narrow, typically at 0.01 M. The combination of the phage concentration and the magnesium concentration (*i.e.* the ratio between the pVIII proteins and magnesium ions) is very important in determining both the nanowires formation process window and their morphology.

The base can be any alkali metal hydroxide (*e.g.* LiOH, NaOH, KOH), soluble alkaline earth metal hydroxide (*e.g.* Sr(OH)₂, Ba(OH)₂) or any ammonium hydroxide (*e.g.,* NR₄OH, R = H, CH₃, C₂H₅, etc.). Certain selection criteria for the base include: adequate solubility (at least several orders of magnitude higher than Mg(OH)₂ for Mg(OH)₂ nanowires), high enough strength (pH of the reaction mixture should be at least 11) and an inability to coordinate magnesium (for Mg(OH)₂ nanowires) to form soluble products. LiOH is a preferred choice for Mg(OH)₂ nanowires formation because lithium may additionally be incorporated in the Mg(OH)₂ as a dopant, providing a Li/MgO doped catalyst for OCM.

Another factor concerning the base is the amount of base used or the concentration ratio of OH⁻/Mg²⁺, *i.e.* the ratio between the number of OH equivalents added and the number of moles of Mg added. In order to fully convert the Mg ions in solution to Mg(OH)₂, the OH/Mg ratio needed is 2. The OH⁻/Mg²⁺ used in the formation of Mg(OH)₂ nanowires ranges from 0.5 to 2 and, depending on this ratio, the morphology of the reaction product changes from thin nanowires to agglomerations of nanoparticles. The OH⁻/Mg²⁺ ratio is determined by the pH of the reaction mixture, which needs to be at least 11. If the pH is below 11, no precipitation is observed, i.e. no Mg(OH)₂ is formed. If the pH is above 12, the morphology of the nanowires begins to change and more nanoparticles are obtained, i.e. non-selective precipitation.

Considering the narrow window of magnesium concentration in which Mg(OH)₂ nanowires can be obtained, the other key synthetic parameters that determine the nanowires formation and morphology include but are not limited to: phage sequence and concentration thereof, the concentration ratio of Mg²⁺/pVIII protein, the concentration ratio of OH⁻/Mg²⁺, the incubation time of phage and Mg²⁺ ; incubation time of phage and the OH⁻; the sequence of adding anion and metal ions; pH; the solution temperature in the incubation step and/or growth step; the types of metal precursor salt (*e.g.,* MgCl₂ or Mg(NO₃)₂); the types of anion precursor (*e.g.,* NaOH or LiOH); the number of additions; the time that lapses between the additions of the metal salt and anion precursor, including, *e.g.,* simultaneous (zero lapse) or sequential additions.

The Mg salt solution and the base were added sequentially, separated by an incubation time (*i.e.,* the first incubation time). The sequence of addition has an effect on the morphology of the nanowires. The first incubation time can be at least 1 h and it should be longer in the case the magnesium salt solution is added first. The Mg salt solution and the base can be added in a single "shot" or in a continuous slow flow using a syringe pump or in multiple small shots using a liquid dispenser robot. The reaction is then carried either unstirred or with only mild to moderate stirring for a specific time (*i.e.,* the second incubation time). The second incubation time is not as strong a factor in the synthesis of Mg(OH)₂ nanowires, but it should be long enough for the nanowires to precipitate out of the reaction solution (*e.g.,* several minutes). For practical reasons, the second incubation time can be as long as several hours. The reaction temperature can be anything from just above freezing temperature (*e.g.,* 4°C) up to 80°C. The temperature affects the nanowires morphology.

The precipitated Mg(OH)₂ nanowires are isolated by centrifuging the reaction mixture and decanting the supernatant. The precipitated material is then washed at least once with a water solution with pH > 10 to avoid redissolution of the Mg(OH)₂ nanowires. Typically, the washing solution used can be ammonium hydroxide water solution or an alkali metal hydroxide solution (*e.g.,* LiOH, NaOH, KOH). This mixture is centrifuged and the supernatant decanted. Finally, the product can be either dried (*see,* Example 5) or resuspended in ethanol for TEM analysis.

The decanted supernatant of the reaction mixture can be analyzed by UV-VIS to determine the phage concentration (*see,* Example 2) and thus give an estimate of the amount of phage incorporated in the precipitated Mg(OH)₂, *i.e*. the amount of "mineralized" phage.

Figure 10 depicts one embodiment for preparing Mg(OH)₂ nanowires. In a different embodiment, the order of addition may be reversed, for example in an exemplary 4 ml scale synthesis of Mg(OH)₂ nanowires, 3.94 ml of concentrated solution of phages (*e.g.,* SEQ ID NO: 3 at a concentration of ~5E12 pfu/ml) were mixed in a 8 ml vial with 0.02 ml of 1 M LiOH aqueous solution and left incubating overnight (~15h). 0.04ml of 1 M MgCl₂ aqueous solution were then added using a pipette and the mixture was mixed by gentle shaking. The reaction mixture was left incubating unstirred for 24 h. After the incubation time, the mixture was centrifuged, and the supernatant was decanted and saved for phage concentration measurement by UV-VIS. The precipitated material was resuspended in 2 ml of 0.001 M LiOH aqueous solution (pH = 11), the mixture was centrifuged and the supernatant decanted. The obtained Mg(OH)₂ nanowires were characterized by TEM as described in Example 4.

### EXAMPLE 4 (Reference)

### CHARACTERIZATION OF MG(OH)₂ NANOWIRES

Mg(OH)₂ nanowires prepared according to Example 3 were characterized by TEM in order to determine their morphology. First, a few microliters (~500) of ethanol was used to suspend the isolated Mg(OH)₂. The nanowires were then deposited on a TEM grid (copper grid with a very thin carbon layer) placed on filter paper to help wick out any extra liquid. After allowing the ethanol to dry, the TEM grid was loaded in a TEM and characterized. TEM was carried out at 5KeV in bright field mode in a DeLong LVEM5.

The nanowires were additionally characterized by XRD (for phase identification) and TGA (for calcination optimization).

### EXAMPLE 5 (Reference)

### CALCINATION OF MG(OH)₂ NANOWIRES

The isolated nanowires as prepared in Example 3 were dried in an oven at relatively low temperature (60-120 °C) prior to calcination.

The dried material was placed in a ceramic boat and calcined in air at 450 C° in order to convert the Mg(OH)₂ nanowires into MgO nanowires. The calcination recipe can be varied considerably. For example, the calcination can be done relatively quickly like in these two examples:
- load in a muffle oven preheated at 450°C, calcination time = 120 min
- load in a muffle oven (or tube furnace) at room temperature and ramp to 450°C with 5°C/min rate, calcination time = 60min

Alternatively, the calcination can be done in steps that are chosen according to the TGA signals like in the following example:
- load in a muffle oven (or tube furnace) at room temperature, ramp to 100°C with 2°C/min rate, dwell for 60min, ramp to 280°C with 2°C/min rate, dwell for 60min, ramp to 350°C with 2°C/min rate, dwell for 60min and finally ramp to 450°C with 2°C/min rate, dwell for 60min.

Generally, a step recipe is preferable since it should allow for a better, smoother and more complete conversion of Mg(OH)₂ into MgO. Optionally, the calcined product is ground into a fine powder.

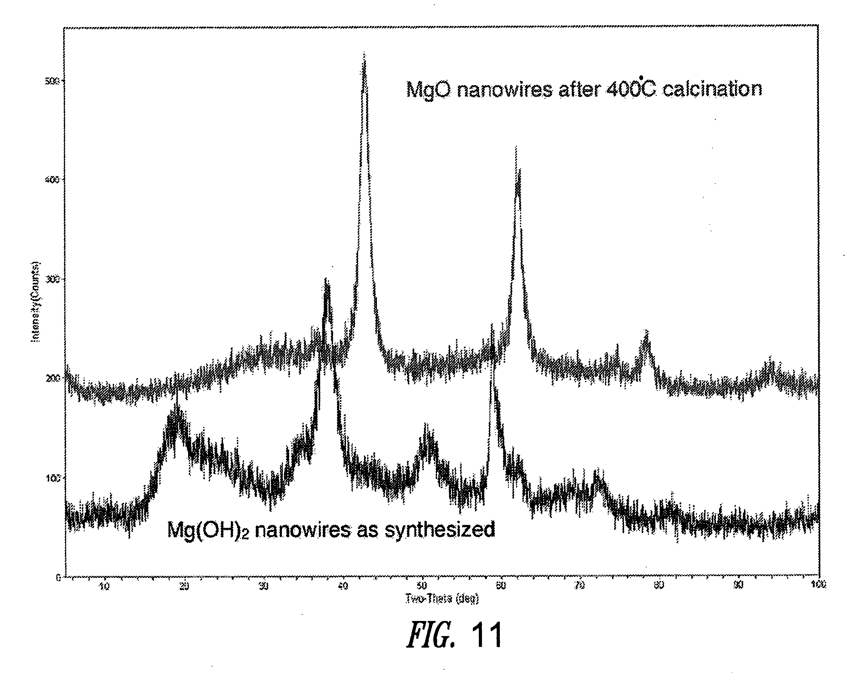

Figure 11 shows the X-ray diffraction patterns of the Mg(OH₂) nanowires and the MgO nanowires following calcinations. Crystalline structures of both types of nanowires were confirmed.

### EXAMPLE 6 (Reference)

### PREPARATION OF LI DOPED MGO NANOWIRES

Doping of nanowires is achieved by using the incipient wetness impregnation method. Before impregnating the MgO nanowires with the doping solution, the maximum wettability (*i.e.* the ability of the nanowires to absorb the doping solution before becoming a suspension or before "free" liquid is observed) of the nanowires was determined. This is a very important step for an accurate absorption of the doping metal on the MgO surface. If too much dopant solution is added and a suspension is formed, a significant amount of dopant will crystallize unabsorbed upon drying and if not enough dopant solution is added, significant portions of the MgO surface will not be doped.

In order to determine the maximum wettability of the MgO nanowires, small portions of water were dropped on the calcined MgO powder until a suspension was formed, *i.e.* until "free" liquid is observed. The maximum wettability was determined to be the total amount of water added before the suspension formed. The concentration of the doping solution was then calculated so that the desired amount of dopant was contained in the volume of doping solution corresponding to the maximum wettability of the MgO nanowires. In another way to describe the incipient wetness impregnation method, the volume of the doping solution is set to be equal to the pore volume of the nanowires, which can be determined by BET (Brunauer, Emmett, Teller) measurements. The doping solution is then drawn into the pores by capillary action.

In one embodiment, the doping metal for MgO based catalysts for OCM is lithium (*see*, *also*, Figure 10). Thus, in one embodiment the dopant source can be any soluble lithium salt as long as it does not introduce undesired contaminants. Typically, the lithium salts used were LiNO₃, LiOH or Li₂CO₃. LiNO₃ and LiOH are preferred because of their higher solubility. In one embodiment, the lithium content in MgO catalysts for OCM ranges from 0 to 10 wt% (*i.e.* about 0 to 56 at%).

The calculated amount of dopant solution of the desired concentration was dropped onto the calcined MgO nanowires. The obtained wet powder was dried in an oven at relatively low temperature (60-120 °C) and calcined using one of the recipes described above. It is noted that, during this step, no phase transition occurs (MgO has already been formed in the previous calcination step) and thus a step recipe (see previous paragraph) may not be necessary.

The dopant impregnation step can also be done prior to the calcination, after drying the Mg(OH)₂ nanowires isolated from the reaction mixture. In this case, the catalyst can be calcined immediately after the dopant impregnation, i.e. no drying and second calcination steps would be required since its goals are accomplished during the calcination step.

Three identical synthesis were made in parallel. In each synthesis, 80 ml of concentrated solution of phages (SEQ ID NO: 3 at a concentration of ≥5E12 pfu/ml) were mixed in a 100 ml glass bottle with 0.4 ml of 1 M LiOH aqueous solution and left incubating for 1 h. 0.8ml of 1 M MgCl₂ aqueous solution were added using a pipette and the mixture was mixed by gently shaking it. The reaction mixture was left incubating unstirred for 72h at 60°C in an oven. After the incubation time, the mixture was centrifuged. The precipitated material was resuspended in 20 ml of 0.06 M NH₄OH aqueous solution (pH = 11), the mixture was centrifuged and the supernatant decanted. The obtained Mg(OH)₂ nanowires were resuspended in ethanol. The ethanol suspensions of the three identical syntheses were combined and a few microliters of the ethanol suspension were used for TEM analysis. The ethanol suspension was centrifuged and the supernatant decanted. The gel-like product was transferred in a ceramic boat and dried for 1 h at 120 °C in a vacuum oven.

The dried product was calcined in a tube furnace using a step recipe (load in the furnace at room temperature, ramp to 100 °C with 2 °C/min rate, dwell for 60 min, ramp to 280 °C with 2 °C/min rate, dwell for 60 min, ramp to 350 °C with 2 °C/min rate, dwell for 60 min, ramp to 450 °C with 2 °C/min rate, dwell for 60min and finally cool to room temperature). The yield was 24 mg. The calcined product was ground to a fine powder.

10 mg of the calcined product were impregnated with a LiOH aqueous solution. First, the maximum wettability was determined by adding water to the calcined product in a ceramic boat until the powder was saturated but no "free" liquid was observed. The maximum wettability was 12 µl. Since the target doping level was 1 wt% lithium, the necessary concentration of the LiOH aqueous solution was calculated to be 1.2 M. The calcined product was dried again for 1 h at 120 °C to remove the water used to determine the wettability of the powder. 12 µl of the 1.2 M LiOH solution were dropped on the MgO nanowires powder. The wet powder was dried for 1 h at 120 °C in a vacuum oven and finally calcined in a muffle oven (load at room temperature, ramp to 460 °C with 2 °C/min ramp, dwell for 120 min).

### EXAMPLE 7 (Reference)

### CREATING DIVERSITY BY VARYING THE REACTION PARAMETERS

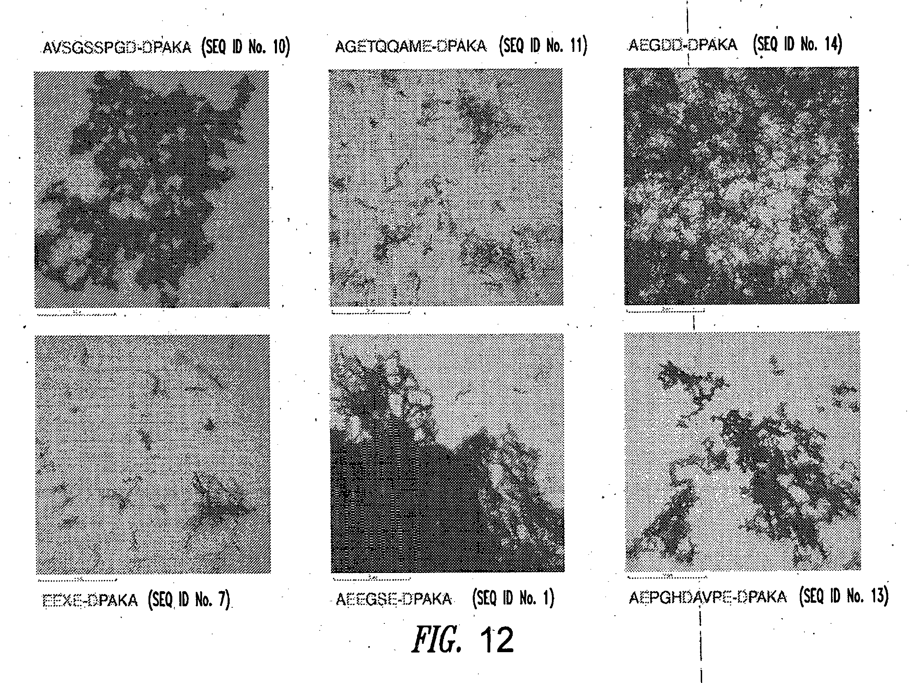

Certain synthetic parameters strongly influence the nanowire formation on phage, including selective binding of metal and/or anions, as well as surface morphologies. Figure 12 shows a number of MgO nanowires synthesized in the presence of different phage sequence (*e.g.,* different pVIII) while keeping the other reaction conditions constant. Phages of SEQ ID NOs. 1, 7, 10, 11, 13 and 14 were the respective phage of choice in six reactions carried out in otherwise identical conditions. The constant reaction conditions may include: concentration ratios of Mg²⁺ and active functional groups on the phage; concentration ratios of OH⁻/Mg²⁺; incubation time of phage and Mg²⁺; incubation time of phage and OH⁻; concentration of phage; sequence of adding anion and metal ions; solution temperature in the incubation step and/or growth step; etc. As shown, the morphologies of MgO nanowires are significantly influenced by the phage sequences.

Thus, varying these and other reaction conditions may produce a diverse class of nanowire catalysts. In addition, certain correlation between the reaction conditions and the surface morphologies of the nanowires can be empirically established, thus enabling rational designs of catalytic nanowires.

### EXAMPLE 8 (Reference)

### PREPARATION OF SR-DOPED LA₂O₃ NANOWIRES

23 ml of 2.5 e12 pfu solution of phages (SEQ ID NO: 3) was mixed in a 40 ml glass bottle with 0.046 ml of 0.1 M LaCl₃ aqueous solution and left incubating for 16h. After this incubation period, a slow multistep addition is conducted with 1.15 ml of 0.05 M LaCl₃ solution and 1.84 ml of 0.3 M NH₄OH. This addition is conducted in six hours and twenty steps. The reaction mixture was left stirred another 2 h at room temperature. After that time the suspension was centrifuged in order to separate the solid phase from the liquid phase. The precipitated material was then resuspended in 5ml of water and centrifuged in order to further remove un-reacted species. A final wash was conducted with 2 ml ethanol. The gel-like product remaining is then dried for 30 minutes at 110°C in a vacuum oven.

The dried product was then calcined in a muffle furnace using a step recipe (load in the furnace at room temperature, ramp to 200°C with 3°C/min rate, dwell for 120min, ramp to 400°C with 3°C/min rate, dwell for 120min, cool to room temperature). The calcined product was then ground to a fine powder.

5mg of the calcined product were impregnated with 0.015ml Sr(NO₃)₂ 0.1 M aqueous solution. Powder and solution is mixed on hot plate at 90C until forming a paste. The paste was then dried for 1h at 120°C in a vacuum oven and finally calcined in a muffle oven in air. (load in the furnace at room temperature, ramp to 200°C with 3°C/min rate, dwell for 120min, ramp to 400°C with 3°C/min rate, dwell for 120min, ramp to 500°C with 3°C/min rate, dwell for 120min, cool to room temperature).

### EXAMPLE 9 (Reference)

### PREPARATION OF ZRO₂/LA₂O₃ CORE/SHELL NANOWIRES

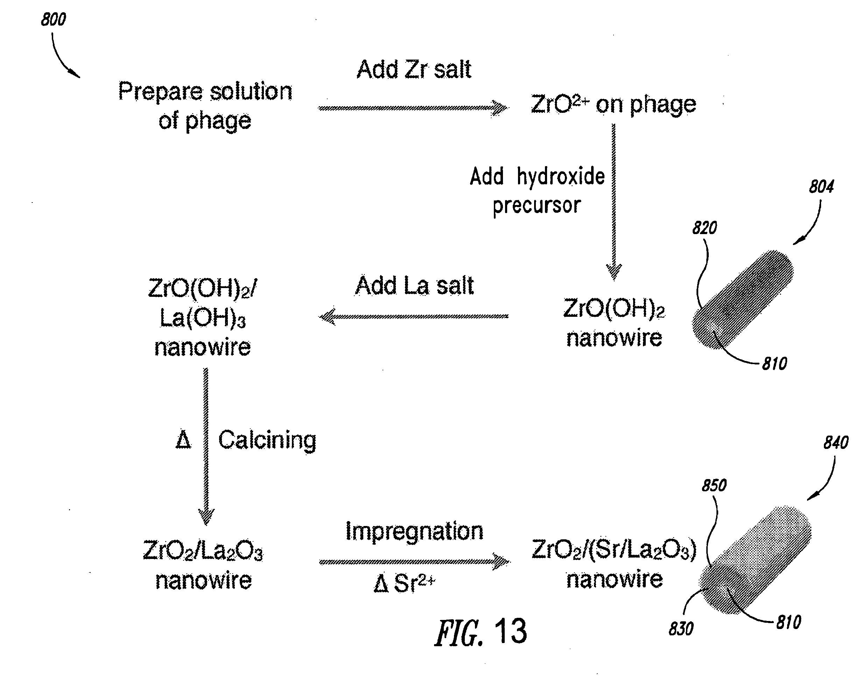

As an example, Figure 13 shows schematically an integrated process 800 for growing a core/shell structure of ZrO₂/La₂O₃ nanowire. A phase solution is prepared, to which a zirconium salt precursor (*e.g.,* ZrCl₂) is added to allow for the nucleation of ZrO²⁺ on the phage. Subsequently, a hydroxide precursor (*e.g.,* LiOH) is added to cause the nucleation of hydroxide ions on the phage. Nanowires 804 is thus formed in which the phage 810 is coated with a continuous and crystalline layer 820 of ZrO(OH)₂. To this reaction mixture, a lanthanum salt precursor (*e.g.,* LaCl₃) is added under a condition to cause the nucleation of La(OH)₃ over the ZrO(OH)₂ nanowire 804. Following calcinations, nanowires of a core/shell structure of ZrO₂/La₂O₃ are formed. A further step of impregnation produces nanowires of ZrO₂/La₂O₃ doped with strontium ions (Sr²⁺) 840, in which the phage 810 is coated with a layer of ZrO₂ 830, which is in turn coated with a shell of La₂O₃ 850.

ZrO₂/La₂O₃ nanowires were thus prepared by mixing 20 ml of 2.5e12 pfu E3 Phage solution to 0.1ml of 0.5M ZrO(NO₃)₂ aqueous solution. The solution was incubated under stirring for 16 hours. Any solids formed following incubation were removed by centrifugation at 4000rpm for 5 minutes and redispersed in 0.5 ml ethanol. A small aliquot was retrieved for TEM characterization.

Thereafter, the ethanol solution was mixed with 10 ml water and 2 ml of 0.05M ZrO(NO₃)₂ with 2ml of 0.1 M NH₄OH were added during a period of 200 minutes using syringe pumps. Wash solids with water and resuspend in ethanol for TEM observation.

To about 18 mg of ZrO(OH)₂ nanowires in suspension, 10 ml of water was added, followed by the addition of 0.5 ml of LaCl₃ 0.083 M with 0.5 ml of NH₄OH 0.3 M solution during a period of 50 minutes using syringe pumps. The solids thus formed were separated by centrifugation to obtain a powder, which was dried in a vacuum oven at 110 °C for one hour. A small aliquot of the dried powder is then suspended in ethanol for TEM observation.

### EXAMPLE 10 (Reference)

### PREPARATION OF LA(OH)₃/ZRO₂ CORE/SHELL NANOWIRES

Similar to Example 9, La(OH)₃ nanowires were coated with ZrO₂ shell according to the following process. To 6.8 mg of La(OH)₃ nanowires (prepared by LaCl₃ and NH₄OH in a process similar to that of Example 9), which had been dried at 110 °C, was added 4 ml of water to suspend the solids. 0.5ml of 0.05M ZrO(NO₃)₂ and 0.5ml of 0.1 M NH₄OH were slowly added in 50 minutes. The solids were retrieved by centrifugation and calcined at 500 °C for one hour. TEM observation showed nanowires as the major morphology.

### EXAMPLE 11 (Reference)

### PREPARATION OF HOLLOW-CORED ZRO₂ NANOWIRES

To the La(OH)₃/ZrO₂ core/shell nanowires prepared Example 10, additional processing can be used to create hollow ZrO₂ shell nanowires. The La(OH)₃ core can be etched using 1 M citric acid solution. Controlled experiments on calcined and un-calcined La(OH)₃ nanowires shows that the entire nanowires are fully etched in about one hour at room temperature. Etching of La(OH)₃/ZrO₂ core/shell nanowires was conducted overnight (about 16 hours).

The remaining solid was then separated by centrifugation and TEM observation is conducted on the washed solids (water wash). Low contrast zirconia nanowires were observed after etching, which indicates that hollow zirconia "straws" can be formed using La(OH)₃ nanowire as template.

### EXAMPLE 12

### OCM CATALYZED BY LA₂O₃ NANOWIRES

A 20mg sample of a phage-based Sr (5%) doped La₂O₃ catalyst was diluted with 80mg of quartz sand and placed into a reactor (run WPS21). The gas flows were held constant at 9 seem methane, 3 seem oxygen and 6 seem of argon. The upstream temperature (just above the bed) was varied from 500 °C to 800 °C in 100 °C increments and then decreased back down to 600 °C in 50 °C increments. The vent gas analysis was gathered at each temperature level.

As a point of comparison, 20 mg of bulk 5% Sr on La₂O₃ catalyst was diluted in the same manner and run through the exact flow and temperature protocol.

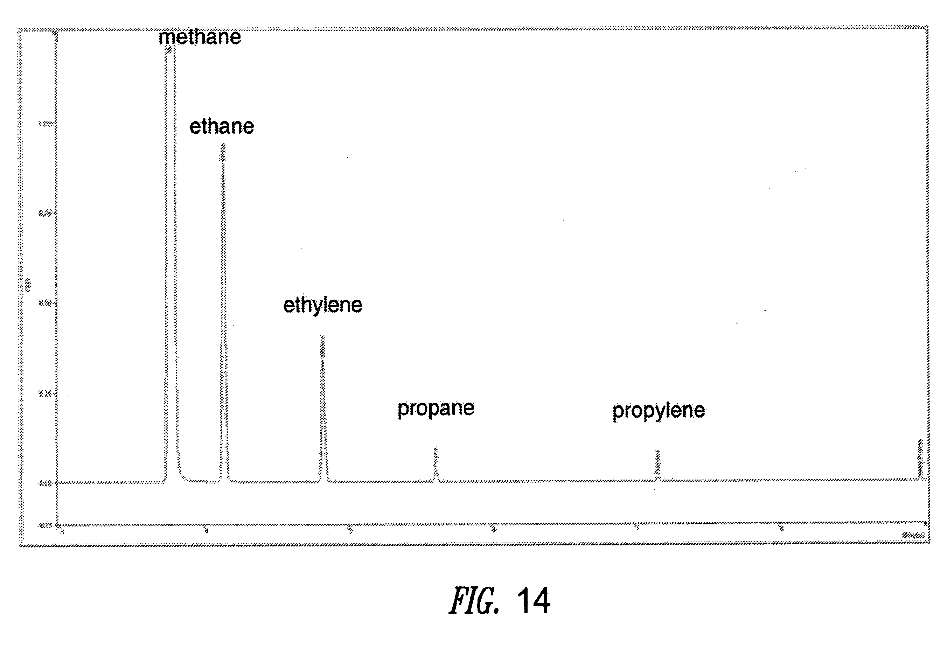

Figure 14 shows the formation of OCM products at 700 °C, including C2 (ethane and ethylene) as well as further coupling products (propane and propylene).

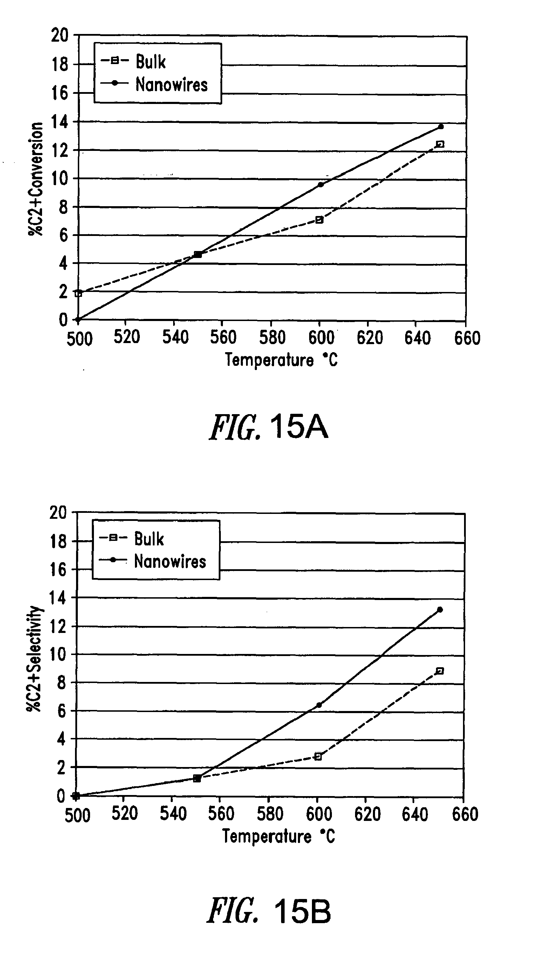

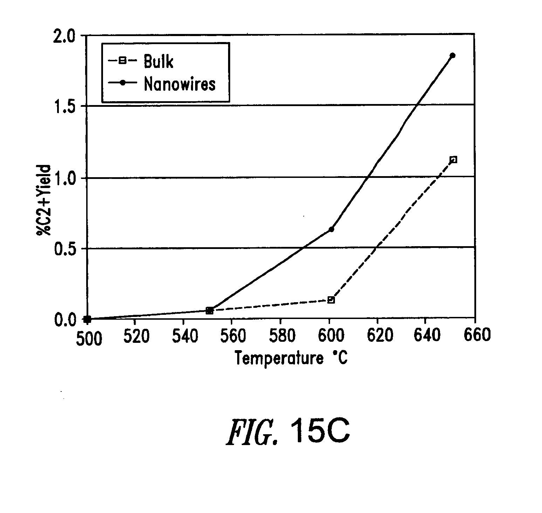

Figure 15A, 15B and 15C show the comparative results in catalytic performance parameters for a nanowire catalyst (Sr²⁺/La₂O₃) vs. its corresponding bulk material (Sr²⁺/La₂O₃ bulk). Methane conversion rates, C2 selectivities and C2 yields are among the important parameters by which the catalytic properties were measured. More specifically, Figure 15A shows the methane conversion rates are higher for the nanowire catalyst compared to the bulk material across a wide temperature range (*e.g.,* 550 to 650 °C). Likewise, Figure 15B and 15C show that the C2 selectivities and C2 yields are also higher for the nanowire catalyst as compared to the bulk catalyst across a wide temperature range (*e.g.,* 550 to 650 °C). Thus, it is demonstrated that by improving both conversion and selectivity simultaneously that the C2 yield can be improved over traditional bulk catalysts.

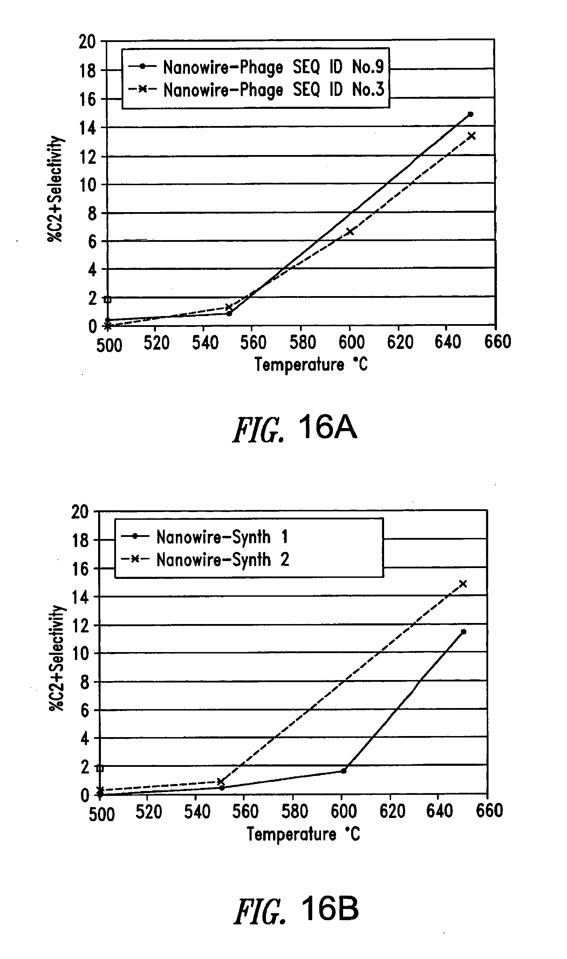

Figures 16A and 16B demonstrate that nanowires prepared under different synthetic conditions afforded different catalytic performances, suggesting that the various synthetic parameters resulted in divergent nanowire morphologies. Figure 16A shows that nanowires prepared using different phage templates (SEQ ID NO: 9 and SEQ ID NO:3) in otherwise identical synthetic conditions created nanowire catalysts that perform differently in terms of the C2 selectivity in an OCM reaction. Figure 16B shows the comparative C2 selectivities of nanowires prepared by an alternative adjustment of the synthetic parameters. In this case, the phage template was the same for both nanowires (SEQ ID NO:3), but the synthetic conditions were different. Specifically, the nanowires of Figure 16A were prepared with shorter incubation and growth times than the nanowires of Figure 16B. Additionally, the nanowires of Figure 16A were calcined in a single step at 400 °C instead of the ramped temperature calcinations performed on the nanowires of Figure 16B.

These results confirm that the nanowire catalysts behave differently from their bulk material counterparts. In particular, the nanowire catalysts allow for adjustments of the surface morphologies through synthetic design and screening to ultimately produce high-performance catalysts.

### EXAMPLE 13 (Reference)

### OXIDATIVE DEHYDROGENATION CATALYZED BY MGO NANOWIRES

A 10 mg sample of phage-based Li doped MgO catalyst was diluted with 90 mg of quartz sand and placed in a reactor. The gas flows were held constant at 8 seem alkane mix, 2 seem oxygen and 10 seem of argon. The upstream temperature (just above the bed) was varied from 500 °C to 750 °C in 50-100 °C increments. The vent gas analysis was gathered at each temperature level.

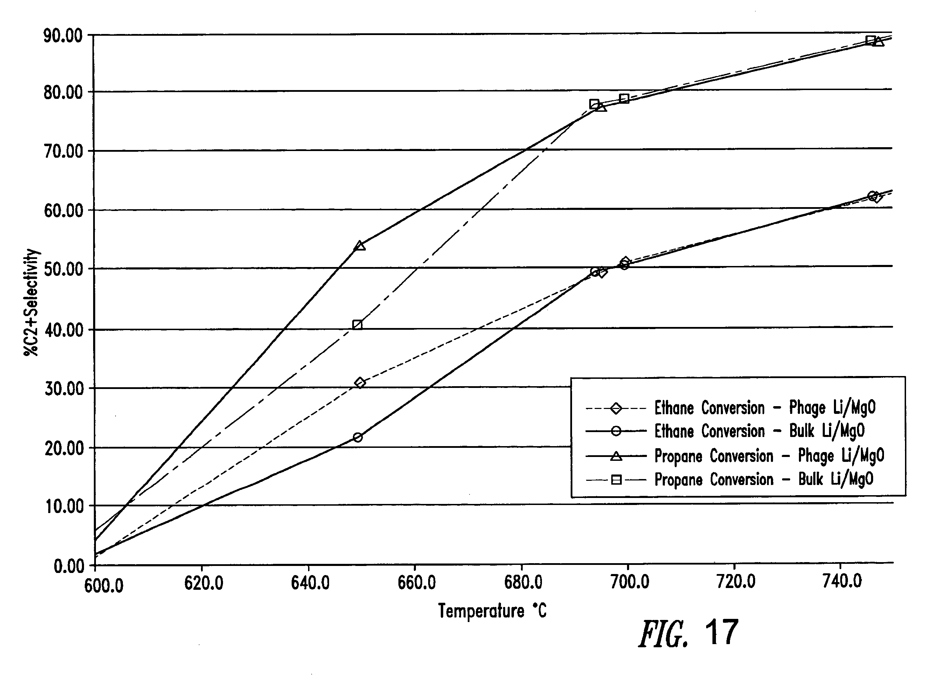

As a point of comparison, 10 mg of bulk 1 wt% Li on MgO catalyst was diluted in the same manner and run through the exact flow and temperature protocol. The results of this experiment are shown in Figure 17 . As can be seen in Figure 17 phage-based nanowires according to the present disclosure comprise better conversion of ethane and propane compared to a corresponding bulk catalyst.

### EXAMPLE 14 (Reference)

### SYNTHESIS OF SR DOPED LA₂O₃ NANOWIRES

Sr doped La₂O₃ nanowires were prepared according to the following non-template directed method.

A La(OH)₃ gel was prepared by adding 0.395 g of NH₄OH (25%) to 19.2 ml of water followed by addition of 2 ml of a 1 M solution of La(NO₃)₃. The solution was then mixed vigorously. The solution first gelled but the viscosity dropped with continuous agitation. The solution was then allowed to stand for a period of between 5 and 10 minutes. The solution was then centrifuged at 10,000 g for 5 minutes. The centrifuged gel was retrieved and washed with 30 ml of water and the centrifugation washing procedure was repeated.

To the washed gel was added 10.8 ml of water to suspend the solid. The suspension was then transferred to a hydrothermal bomb (20 ml volume, not stirred). The hydrothermal bomb was then loaded in a muffle furnace at 160 °C and the solution was allowed to stand under autogenous pressure at 160 °C for 16 hours.

The solids were then isolated by centrifugation at 10,000 g for 5 minutes, and wash with 10 ml of water to yield about 260 mg of solid (after drying). The obtained solids were calcined in a muffle oven according to the following procedure: (1) load in the furnace at room temperature; (2) ramp to 200 °C with 3 °C/min rate; (3) dwell for 120 min; (4) ramp to 400°C with 3 °C/min rate; and (5) dwell for 120 min. About 220 mg of nanowires were retrieved after calcination.

A 57 mg aliquot of nanowires was then mixed with 0.174 ml of a 0.1 M solution of Sr(NO₃)₂. This mixture was then stirred on a hot plate at 90 °C until a paste was formed.

The paste was then dried for 1 h at 120 °C in a vacuum oven and finally calcined in a muffle oven in air according to the following procedure: (1) load in the furnace at room temperature; (2) ramp to 200°C with 3 °C/min rate; (3) dwell for 120 min; (3) ramp to 400 °C with 3 °C/min rate; (4) dwell for 120 min; (5) ramp to 500 °C with 3 °C/min rate; and (6) dwell for 120min.The calcined product was then ground to a fine powder.

5mg of the calcined product were impregnated with 0.015ml Sr(NO₃)₂ 0.1 M aqueous solution. Powder and solution is mixed on hot plate at 90C until forming a paste. The paste was then dried for 1 h at 120°C in a vacuum oven and finally calcined in a muffle oven in air. (load in the furnace at room temperature, ramp to 200°C with 3°C/min rate, dwell for 120min, ramp to 400°C with 3°C/min rate, dwell for 120min, ramp to 500°C with 3°C/min rate, dwell for 120min).

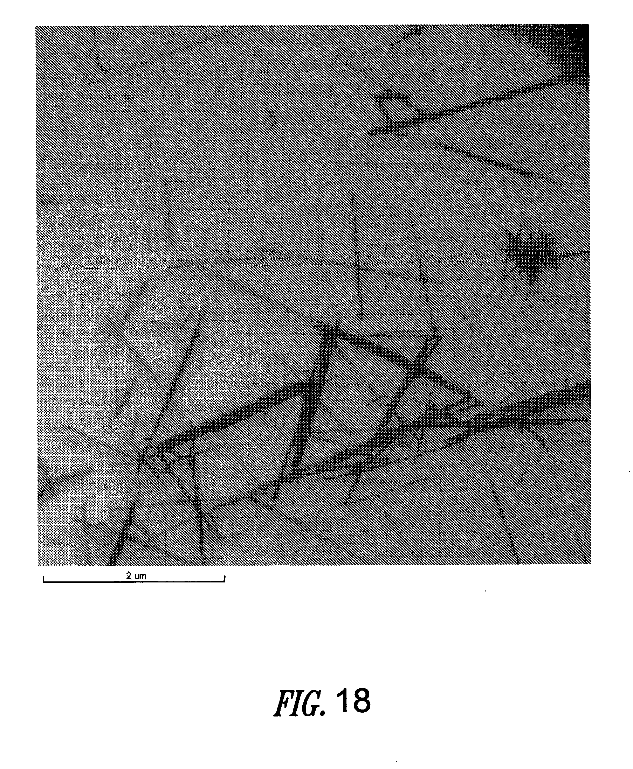

Figure 18 shows a TEM image of the nanowires obtained from this non-template directed method. As shown in Figure 18 the nanowires comprise a ratio of effective length to actual length of about 1 (i.e., the nanowires comprise a "straight" morphology).

### EXAMPLE 15 (Reference)

### SYNTHESIS OF LA₂O₃ NANOWIRES

La(NO₃)₃ 6 H₂O (10.825 g) is added to 250 mL distilled water and stirred until all solids are dissolved. Concentrated ammonium hydroxide (4.885 mL) is added to this mixture and stirred for at least one hour resulting in a white gel. This mixture is transferred equally to 5 centrifuge tubes and centrifuged for at least 15 minutes. The supernatant is discarded and each pellet is rinsed with water, centrifuged for at least 15 minutes and the supernatant is again discarded.

The resulting pellets are all combined, suspended in distilled water (125 mL) and heated at 105 °C for 24 hours. The lanthanum hydroxide is isolated by centrifugation and suspended in ethanol (20 mL). The ethanol supernatant is concentrated and the product is dried at 65 °C until all ethanol is removed.

The lanthanum hydroxide nanowires prepared above are calcined by heating at 100 °C for 30 min., 400 °C for 4 hours and then 550 °C for 4 hours to obtain the La₂O₃ nanowires.

### EXAMPLE 16 (Reference)

### PREPARATION OF NA₁₀MNW₅O₁₇ NANOWIRES

25 ml of concentrated reagent grade NH₄OH are dissolved in 25 ml of distilled water, and 1 ml of 0.001 M aqueous solution of M13 bacteriophage is then added. 0.62 g of Mn(NO₃)₂, 1.01 g of NaCl and 2.00 g of WO₃ are then added to the mixture with stirring. The mixture is heated at a temperature of about 95 °C for 15 minutes. The mixture is then dried overnight at about 110 °C and calcined at about 400 °C for 3 hours.

### EXAMPLE 17 (Reference)

### PREPARATION OF NA₁₀MNW₅O₁₇ NANOWIRES

25 ml of concentrated reagent grade NH₄OH are dissolved in 25 ml of distilled water, and 1 ml of 0.001 M aqueous solution of M13 bacteriophage is then added. 1.01 g of NaCl and 2.00 g of WO₃ are then added to the mixture with stirring. The mixture is heated at a temperature of about 95 °C for 15 minutes. The mixture is then dried overnight at about 110 °C and calcined at about 400 °C for 3 hours. The resulting material is then suspended in 10 ml of distilled water and 0.62 g of Mn(NO₃)₂ is added to the mixture with stirring. The mixture is heated at a temperature of about 115°C for 15 minutes. The mixture is then dried overnight at about 110 °C and calcined at about 400 °C for 3 hours.

### EXAMPLE 18 (Reference)

### PREPARATION OF NA₁₀MNW₅O₁₇/SIO₂ NANOWIRES

Nanowire material Na₁₀MnW₅O₁₇ (2.00 g), prepared as described in Example 16 above, is suspended in water, and about 221.20 g of a 40% by weight colloidal dispersion of SiO₂ (silica) is added while stirring. The mixture is heated at about 100 °C until near dryness. The mixture is then dried overnight at about 110 °C and heated under a stream of oxygen gas (*i.e.,* calcined) at about 400 °C for 3 hours. The calcined product is cooled to room temperature and then ground to a 10-30 mesh size.

### EXAMPLE 19 (Reference)

### PREPARATION OF LA₂O₃ NANOWIRES

Two identical syntheses were made in parallel. In each synthesis, 360 ml of 4 e12 pfu/ml solution of phage (SEQ ID NO: 3) were mixed in a 500 ml plastic bottle with 1.6 ml of 0.1 M LaCl3 aqueous solution and left incubating for at least 1 hour. After this incubation period, a slow multistep addition was conducted with 20 ml of 0.1 M LaCl3 solution and 40 ml of 0.3 M NH40H. This addition was conducted in 24 hours and 100 steps. The reaction mixture was left stirred for at least another hour at room temperature. After that time the suspension was centrifuged in order to separate the solid phase from the liquid phase. The precipitated material was then re-suspended in 25 ml of ethanol. The ethanol suspensions from the two identical syntheses were combined and centrifuged in order to remove un-reacted species. The gel-like product remaining was then dried for 15 hours at 65°C in an oven and then calcined in a muffle oven in air (load in the furnace at room temperature, ramp to 100°C with 2°C/min rate, dwell for 30min, ramp to 400°C with 2°C/min rate, dwell for 240min, ramp to 550°C with 2°C/min rate, dwell for 240min, cool to room temperature).

### EXAMPLE 20 (Reference)

### PREPARATION OF MG/NA DOPED LA₂O₃ NANOWIRES

Two identical syntheses were made in parallel. In each synthesis, 360 ml of 4 e12 pfu solution of phage (SEQ ID NO: 3) were mixed in a 500 ml plastic bottle with 1.6 ml of 0.1 M LaCl₃ aqueous solution and left incubating for at least 1 hour. After this incubation period, a slow multistep addition was conducted with 20 ml of 0.1 M LaCl₃ solution and 40 ml of 0.3 M NH₄OH. This addition was conducted in 24 hours and 100 steps. The reaction mixture was left stirred for at least another hour at room temperature. After that time, the suspension was centrifuged in order to separate the solid phase from the liquid phase. The precipitated material was then resuspended in 25 ml of ethanol. The ethanol suspensions from the two identical syntheses were combined and centrifuged in order to remove un-reacted species. The gel-like product remaining was then dried for 15 hours at 65 °C in an oven.

The target doping level was 20 at% Mg and 5 at% Na at% refers to atomic percent). 182 mg of the dried product were suspended in 2.16 ml deionized water, 0.19 ml 1 M Mg(NO₃)₂ aqueous solution and 0.05 ml 1 M NaNO₃ aqueous solution. The resulting slurry was stirred at room temperature for 1 hour, sonicated for 5 min, then dried at 120 °C in and oven until the powder was fully dried and finally calcined in a muffle oven in air (load in the furnace at room temperature, ramp to 100°C with 2°C/min rate, dwell for 30min, ramp to 400°C with 2°C/min rate, dwell for 60min, ramp to 550°C with 2°C/min rate, dwell for 60min, ramp to 650°C with 2°C/min rate, dwell for 60min, ramp to 750°C with 2°C/min rate, dwell for 240min, cool to room temperature).

### EXAMPLE 21

### OXIDATIVE COUPLING OF METHANE CATALYZED BY MG/NA DOPED LA₂O₃ NANOWIRES

50mg of Mg/Na-doped La₂O₃ nanowires catalyst from example 20 were placed into a reactor tube (4 mm ID diameter quartz tube with a 0.5 mm ID capillary downstream), which was then tested in an Altamira Benchcat 203. The gas flows were held constant at 46 seem methane and 54 seem air, which correspond to a CH₄/O₂ ratio of 4 and a feed gas-hour space velocity (GHSV) of about 130000 h-1. The reactor temperature was varied from 400 °C to 450 °C in a 50 °C increment, from 450 °C to 550 °C in 25 °C increments and from 550 °C to 750 °C in 50 °C increments. The vent gases were analyzed with gas chromatography (GC) at each temperature level.

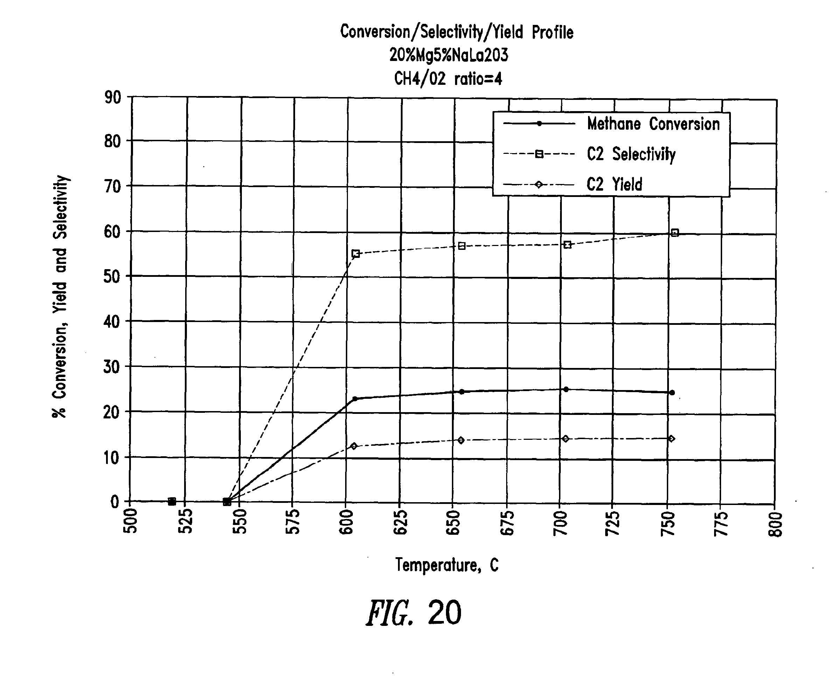

Figure 20 shows the onset of OCM between 550 °C and 600 °C. The C2 selectivity, methane conversion and C2 yield at 650 °C were 57 %, 25 % and 14 %, respectively.

In another example, 50mg of Mg/Na-doped La₂O₃ nanowires catalyst from example 20 were placed into a reactor tube (4 mm ID diameter quartz tube with a 0.5 mm ID capillary downstream), which was then tested in an Altamira Benchcat 203. The gas flows were held constant at 46 seem methane and 54 seem air, which correspond to a feed gas-hour space velocity (GHSV) of about 130000 h⁻¹. The CH4/02 ratio was 5.5. The reactor temperature was varied from 400 °C to 450 °C in a 50 °C increment, from 450 °C to 550 °C in a 25 °C increments and from 550 °C to 750 °C in 50 °C increments. The vent gases were analyzed with gas chromatography (GC) at each temperature level.

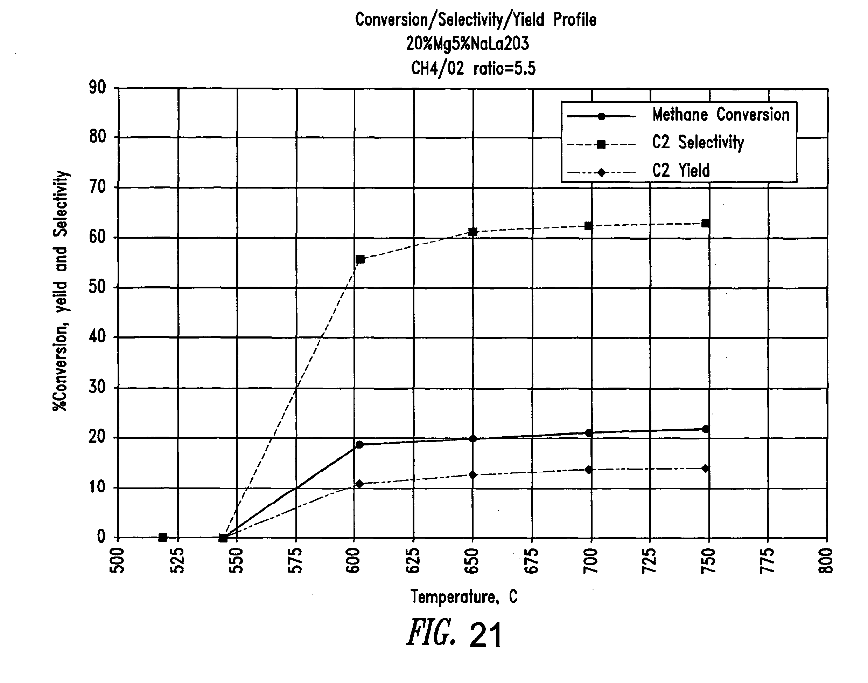

Figure 21 shows the onset of OCM between 550 °C and 600 °C. The C2 selectivity, methane conversion and C2 yield at 650 °C were 62 %, 20 % and 12%, respectively.

### EXAMPLE 22 (Reference)

### NANOWIRE SYNTHESIS

Nanowires may be prepared by hydrothermal synthesis from metal hydroxide gels (made from metal salt + base). In some embodiments, this method is applicable to lanthanides, for example La, Nd, Pr, Sm, Eu, and lanthanide containing mixed oxides.

Alternatively, nanowires can be prepared by synthesis from metal hydroxide gel (made from metal salt + base) under reflux conditions. In some embodiments, this method is applicable to lanthanides, for example La, Nd, Pr, Sm, Eu, and lanthanide containing mixed oxides.

Alternatively, the gel can be aged at room temperature. Certain embodiments of this method are applicable for making magnesium hydroxychloride nanowires, which can be converted to magnesium hydroxide nanowires and eventually to MgO nanowires. In a related method, hydrothermal treatment of the gel instead of aging, is used.

Nanowires may also be prepared by polyethyleneglycol assisted hydrothermal synthesis. For example, Mn containing nanowires may be prepared according to this method using methods known to those skilled in the art. Alternatively, hydrothermal synthesis directly from the oxide can be used.

### EXAMPLE 23 (Reference)

### PREPARATION OF NANOWIRES

Nanostructured catalyst materials can be prepared by a variety of starting materials. In certain embodiments, the rare earth oxides are attractive starting materials since they can be obtained at high purity and are less expensive than the rare earth salt precursors that are typically used in preparative synthesis work. Methods for making rare earth oxide needles/nanowires and derivatives thereof are described below.

Method A: Lanthanide oxide starting material can be hydrothermally treated in the presence of ammonium halide to prepare rare earth oxide nanowires/needles. The preparation is a simple one-pot procedure with high yield. For example, one gram of lanthanum oxide was placed in 10 mL of distilled water. Ammonium chloride (0.98 g) was added to the water, the mixture was placed in an autoclave, and the autoclave was placed in a 160 C oven for 18 h. The autoclave was taken out of the oven, cooled, and the product was isolated by filtration. Micron and submicron needles were observed in the TEM images of the product. This method could also be used to prepare mixed metal oxides, metal oxyhalides, metal oxynitrates, and metal sulfates.

Method B: Mixed metal oxide materials can be prepared using a solid-state reaction of rare earth oxide or bismuth oxide in the presence of ammonium halide. The solid-state reaction is used to prepare the rare earth or bismuth oxyhalide. The metal oxyhalide is then placed in water at room temperature and the oxyhalide is slowly converted to metal oxide with nanowire/needle morphology. For example: lanthanum oxide, bismuth oxide, and ammonium chloride were ground and fired in a ceramic dish to make the mixed metal oxychloride. The metal oxychloride is then placed in water to form the mixed metal oxide needles.

### EXAMPLE 24 (Reference)

### PREPARATION OF MGO/MN₂O₃ CORE/SHELL NANOWIRES

19.7 ml of concentrated solution of phages (e.g., SEQ ID NO: 3 at a concentration of ~5E12 pfu/ml) were mixed in a 20 ml vial with 0.1 ml of 1 M LiOH aqueous solution and left incubating overnight (~15h). 0.2 ml of 1 M MgCl₂ aqueous solution were then added using a pipette, and the mixture was mixed by gentle shaking. The reaction mixture was left incubating unstirred for 72 h. After the incubation time, the mixture was centrifuged and the supernatant decanted. The precipitated material was re-suspended in 5 ml of 0.001 M LiOH aqueous solution (pH = 11), the mixture was centrifuged and the supernatant decanted.

19.8 ml of deionized water were added to the obtained Mg(OH)₂ nanowires. The mixture was left incubating for 1 h. After the incubation time, 0.2 ml of 1 M MnCl₂ aqueous solution were then added using a pipette and the mixture was mixed by gentle shaking. The reaction mixture was left incubating unstirred for 24 h. After the incubation time, the mixture was centrifuged and the supernatant decanted. The precipitated material was re-suspended in 3 ml of 0.001 M LiOH aqueous solution (pH = 11), the mixture was centrifuged and the supernatant decanted. The precipitated material was finally re-suspended in 7 ml ethanol, the mixture was centrifuged and the supernatant decanted.

The obtained MnO(OH) coated Mg(OH)₂ nanowires were dried at 65 °C for 15 h in an oven. Finally, the dried product was calcined in a muffle furnace using a step recipe (load in the furnace at room temperature, ramp to 100 °C with 2 °C/min rate, dwell for 60 min, ramp to 280 °C with 2 °C/min rate, dwell for 60 min, ramp to 350 °C with 2 °C/min rate, dwell for 60 min, ramp to 450 °C with 2 °C/min rate, dwell for 60min, ramp to 550 °C with 2 °C/min rate, dwell for 60min, cool to room temperature) to convert it to MgO/Mn₂O₃ core-shell nanowires.

The surface area of the nanowires was determined by BET (Brunauer, Emmett, Teller) measurement at 111.5 m²/g.

### EXAMPLE 25 (Reference)

### PREPARATION OF MN₂O₃ NANOWIRES

3.96 ml of concentrated solution of phages (e.g., SEQ ID NO: 3 at a concentration of ~5E12 pfu/ml) were mixed in a 8 ml vial with 0.04 ml of 1 M MnCl₂ aqueous solution and left incubating for 20 h. 0.02ml of 1 M LiOH aqueous solution were then added using a pipette and the mixture was mixed by gentle shaking. The reaction mixture was left incubating unstirred for 72 h. After the incubation time, the mixture was centrifuged, and the supernatant was decanted. The precipitated material was re-suspended in 2 ml of 0.001 M LiOH aqueous solution (pH = 11), the mixture was centrifuged and the supernatant decanted. The precipitated material was re-suspended in 2 ml ethanol, the mixture was centrifuged and the supernatant decanted. The obtained MnO(OH) nanowires were dried at 65 °C for 15 h in an oven. Finally, the dried product was calcined in a muffle furnace using a step recipe (load in the furnace at room temperature, ramp to 100 °C with 2 °C/min rate, dwell for 60 min, ramp to 280 °C with 2 °C/min rate, dwell for 60 min, ramp to 350 °C with 2 °C/min rate, dwell for 60 min, ramp to 450 °C with 2 °C/min rate, dwell for 60min, ramp to 550 °C with 2 °C/min rate, dwell for 60min, cool to room temperature) to convert it to Mn₂O₃ nanowires.

### EXAMPLE 26 (Reference)

### PREPARATION OF V₂O₅ NANOWIRES

1.8 mg of V₂O₅ were dissolved in a 10 ml of a 2.5 wt% aqueous solution of HF. 1 ml of the V₂O₅/HF solution was mixed with 1 ml of concentrated solution of phages (e.g., SEQ ID NO: 3 at a concentration of ~5E12 pfu/ml) in a 15 ml plastic centrifugation tube and left incubating for 2 h. 1 ml of a saturated solution of boric acid (supernatant of nominally 1 M boric acid aqueous solution) were then added using a pipette and the mixture was mixed by gentle shaking. The reaction mixture was left incubating unstirred for 170 h. After the incubation time, the mixture was centrifuged, and the supernatant was decanted. The precipitated material was suspended in 2 ml ethanol, the mixture was centrifuged and the supernatant decanted. The obtained V₂O₅ nanowires were characterized by TEM.

### EXAMPLE 27 (Reference)

### SYNTHESIS OF MGO NANOWIRES

12.5 ml of a 4M MgCl₂ aqueous solution were heated to 70 °C on a hotplate. 0.1 g of MgO (from Aldrich) were then slowly added, over a span of at least 5 minutes, to the solution while it was vigorously stirred. The mixture was kept stirring at 70 °C for 3 h and then cooled down overnight (~ 15 h) without stirring.

The obtained gel was transferred in a 25 ml hydrothermal bomb (Parr Bomb No. 4749). The hydrothermal bomb was then loaded in an oven at 120 °C and the solution was allowed to stand under autogenous pressure at 120 °C for 3 hours.

The product was centrifuged and the supernatant decanted. The precipitated product was suspended in about 50 ml ethanol and filtered over a 0.45 m polypropylene hydrophilic filter using a Büchner funnel. Additional 200 ml ethanol were used to wash the product.

The obtained magnesium hydroxide chloride hydrate nanowires were suspended in 12 ml ethanol and 2.4 ml deionized water in a 20 ml vial. 1.6 ml of 5M NaOH aqueous solution were added and the vial was sealed with its cap. The mixture was then heated at 65°C in an oven for 15 h.

The product was filtered over a 0.45 µm polypropylene hydrophilic filter using a Büchner funnel. About 250 ml ethanol were used to wash the product. The obtained Mg(OH)₂ nanowires were dried at 65 °C for 15 h in an oven. Finally, the dried product was calcined in a muffle furnace using a step recipe (load in the furnace at room temperature, ramp to 100 °C with 2 °C/min rate, dwell for 60 min, ramp to 280 °C with 2 °C/min rate, dwell for 60 min, ramp to 350 °C with 2 °C/min rate, dwell for 60 min, ramp to 450 °C with 2 °C/min rate, dwell for 60min, cool to room temperature) to convert it to MgO nanowires.

### EXAMPLE 28 (Reference)

### SYNTHESIS OF MG(OH)₂ NANOWIRES

6.8 g of MgCl₂•6H2O were dissolved in 5 ml deionized water in a 20 ml vial. 0.4 g of MgO (from Aldrich) were then slowly added to the solution while it was vigorously stirred. The mixture was kept stirring at room temperature until it completely jellified (~ 2 h) and then it was left aging for 48 h without stirring.

The gel was transferred in a 50 ml centrifuge tube, which was then filled with deionized water and vigorously shaken until an homogenous suspension was obtained. The suspension was centrifuged and the supernatant decanted. The precipitated product was suspended in about 50 ml ethanol and filtered over a 0.45 m polypropylene hydrophilic filter using a Büchner funnel. Additional 350 ml ethanol were used to wash the product.

The obtained magnesium hydroxide chloride hydrate nanowires were suspended in 24 ml ethanol in a 50 ml media bottle. The mixture was stirred for a few minutes, then 4.8 ml deionized water and 3.2 ml of 5M NaOH aqueous solution were added. The media bottle was sealed with its cap and the mixture was stirred for few more minutes. The mixture was then heated at 65°C in an oven for 15 h.

The product was filtered over a 0.45 µm polypropylene hydrophilic filter using a Büchner funnel. About 400 ml ethanol were used to wash the product. The obtained Mg(OH)₂ nanowires were dried at 65 °C for 72 h in an oven and then additionally dried at 120 °C for 2 h in a vacuum oven. About 0.697 g of Mg(OH)₂ nanowires were obtained and the surface area of the nanowires was determined by BET (Brunauer, Emmett, Teller) measurement at 100.4 m²/g.

### EXAMPLE 29 (Reference)

### SYNTHESIS OF MNO/MN₂O₃ CORE/SHELL NANOWIRES

This example describes a method for coating the Mg(OH)₂ nanowires from example 28 with MnO(OH).

Three almost identical syntheses were conducted in parallel. In each synthesis, the Mg(OH)₂ nanowires, prepared using the method described in example 28 but without the drying steps, were mixed with 250 ml deionized water in a 500 ml plastic bottle and stirred for 20 minutes. 2.4 ml of a 1M MnCl₂ solution were added to the first synthesis, 6 ml of a 1 M MnCl₂ solution were added to the second synthesis and 9.6 ml of a 1 M MnCl₂ solution were added to the third synthesis. The mixtures were stirred for 2 hours at room temperature. After this incubation period, a slow multistep addition was conducted with 1.2 ml, 3 ml and 4.8 ml of 0.1 M LiOH solution for the first, second and third synthesis, respectively. This addition was conducted in 2 hours and 20 steps. The reaction mixture was left stirred overnight (~ 15 h) at room temperature. After that time the suspensions were centrifuged in order to separate the solid phase from the liquid phase. The precipitated materials were then re-suspended in 50 ml of ethanol for each synthesis and filtered over a 0.45 µm polypropylene hydrophilic filter using a Büchner funnel. Additional 350 ml ethanol were used to wash each product of the three synthesis.

The obtained Mg(OH)₂/MnO(OH) core/shell nanowires were characterized by TEM before being dried at 65 °C for 72 h in an oven and then additionally dried at 120 °C for 2 h in a vacuum oven. The yield for the three syntheses was 0.675g, 0.653g and 0.688g, respectively. The surface area of the nanowires was determined by BET (Brunauer, Emmett, Teller) measurement at 94.6 m²/g, 108.8 m²/g and 108.7 m²/g, respectively.

The Mg(OH)₂/MnO(OH) core/shell nanowires can be converted into MgO/Mn₂O₃ nanowires by calcining them in a muffle furnace using a step recipe (load in the furnace at room temperature, ramp to 100 °C with 2 °C/min rate, dwell for 60 min, ramp to 280 °C with 2 °C/min rate, dwell for 60 min, ramp to 350 °C with 2 °C/min rate, dwell for 60 min, ramp to 450 °C with 2 °C/min rate, dwell for 60min, ramp to 550 °C with 2 °C/min rate, dwell for 60 min, cool to room temperature).

### EXAMPLE 30 (Reference)

### PREPARATION OF ND₂O₃, Eu₂O₃ AND PR₂O₃ NANOWIRES

Three syntheses were made in parallel. In each synthesis, 10 ml of a 2.5 e12 pfu/ml solution of phage (SEQ ID NO: 14) were mixed in a 60 ml glass vial with 25 µl of 0.08M NdCl₃, EuCl₃ or PrCl₃ aqueous solutions, respectively and left incubating for at least 1 hour. After this incubation period, a slow multistep addition was conducted with 630 µl of 0.08M LaCl₃, EuCl₃ or PrCl₃ aqueous solutions, respectively and 500 µl of 0.3M NH40H. This addition was conducted in 33 hours and 60 steps. The reaction mixtures were left stirred for at least another 10 hour at room temperature. After that time the suspensions were centrifuged in order to separate the solid phase from the liquid phase. The precipitated material was then re-suspended in 4 ml of ethanol. The ethanol suspensions were centrifuged in order to finish removing un-reacted species. The gel-like product remaining was then dried for 1 hours at 65°C in an oven and then calcined in a muffle oven in air (load in the furnace at room temperature, ramp to 100°C with 2°C/min rate, dwell for 30min, ramp to 500°C with 2°C/min rate, dwell for 240 min, cool to room temperature). The obtained Nd(OH)₃, Eu(OH)₃ and Pr(OH)₃ nanowires were characterized by TEM before being dried.

### EXAMPLE 31 (Reference)

### PREPARATION OF CE₂O₃/LA₂O₃ MIXED OXIDE NANOWIRES

In the synthesis, 15 ml of a 5 e12 pfu/ml solution of phage (SEQ ID NO: 3) were mixed in a 60 ml glass vial with 15 µl of 0.1 M La(NO₃)₃ aqueous solution and left incubating for about 16 hour. After this incubation period, a slow multistep addition was conducted with 550 µl of 0.2M Ce(NO₃)₃ aqueous solution, 950 µl of 0.2M La(NO₃)₃ aqueous solution and 1500 µl of 0.4M NH₄OH. This addition was conducted in 39 hours and 60 steps. The reaction mixtures were left stirred for at least another 10 hours at room temperature. After that time the suspensions were centrifuged in order to separate the solid phase from the liquid phase. The precipitated material was then re-suspended in 4 ml of ethanol. The ethanol suspensions were centrifuged in order to finish removing un-reacted species. The gel-like product remaining was then dried for 1 hours at 65 °C in an oven and then calcined in a muffle oven in air (load in the furnace at room temperature, ramp to 100°C with 2°C/min rate, dwell for 30min, ramp to 500 °C with 2°C/min rate, dwell for 120min, cool to room temperature).

### EXAMPLE 32 (Reference)

### SYNTHESIS OF PR₂O₃/LA₂O₃ MIXED OXIDE NANOWIRES

0.5 ml of 1 M Pr(NO₃)₃ aqueous solution and 4.5ml of 1 M La(NO₃)₃ aqueous solution were mixed with 40ml deionized water. Once well mixed, 5ml of a 3M NH₄OH aqueous solution were quickly injected in the mixture. A precipitate immediately formed. The suspension was kept stirring for another 10 minutes then transferred to centrifuge tubes and centrifuged in order to separate the solid phase from the liquid phase. The precipitated material was then re-suspended in 35 ml of deionized water. The solid fraction was separated again by centrifugation and the washing step was repeated one more time. The gel-like product remaining was then dispersed in deionized water and the suspension volume adjusted to 20 ml. The suspension was then transferred to a hydrothermal bomb and placed in an oven at 120 °C for 2 hours. The solids obtained after hydrothermal treatment were then separated by centrifugation and washed once with 35 ml of deionized water. The washed hydrothermally treated powder was then dried at 120 °C for 16 hours. The surface area, determine by BET, of the dried powder was about 41 m²/g. Transmission electron microscopy was used to characterize the morphology of this sample further. The powder was constituted of large aspect ratio particles with about 30nm wide by 0.5 to 2 µm length. The powder was calcined in three temperature steps at 200, 400 and 500 °C with 3 °C /min ramp and 2 hours of dwell time at each step. The surface area of the Pr₂O₃/La₂O₃ mixed oxide nanowires was about 36 m²/g.

### EXAMPLE 33 (Reference)

### SYNTHESIS OF MGO/EU₂O₃ CORE/SHELL NANOWIRES

In this example, Mg(OH)₂ nanowires are used as a support to grow a shell of Eu(OH)₃. Mg(OH)₂ nanowires, prepared according to the methods described in example 28 (wet product, before being dried) were used to prepare a suspension in deionized water with a concentration of 3g/l of dried Mg(OH)₂. To 30ml of the Mg(OH)₂ suspension, 3 ml of 0.1 M Eu(NO₃)₃ aqueous solution and 3ml of 0.3M NH₄OH aqueous solution were added in a slow multistep addition. This addition was conducted in 48 hours and 360 steps. The solids were then separated using centrifugation. The powder is washed with 30 ml DI water and centrifuged again. An aliquot is retrieved prior to calcination for transmission electron miscopy evaluation of the sample morphology. The sample is mainly constituted of high aspect ratio wires with a rough surface. The general morphology of the support is preserved and no separate phase is observed.

The remaining of the powder was dried at 120 °C for 3 hours and calcined in three steps at 200, 400 and 500 °C with 2 hours at each step and a ramp rate of 3 °C /min. The surface area, determined by BET, of the MgO/Eu₂O₃ core/shell nanowires is 209 m²/g.

### EXAMPLE 34 (Reference)

### SYNTHESIS OF Y₂O₃/LA₂O₃ MIXED OXIDE NANOWIRES

0.5 ml of 1 M Y(NO₃)₃ aqueous solution and 4.5ml of 1 M La(NO₃)₃ aqueous solution were mixed with 40ml deionized water. Once well mixed, 5ml of a 3M NH40H aqueous solution was quickly injected in the mixture. A precipitate immediately forms. The suspension was kept stirring for another 10 minutes then transferred to centrifuge tubes and centrifuged in order to separate the solid phase from the liquid phase. The precipitated material was then re-suspended in 35 ml deionized water. The solid fraction was separated again by centrifugation and the washing step was repeated one more time. The gel-like product remaining was then dispersed in deionized water and the suspension volume adjusted to 20 ml. The suspension was then transferred to a hydrothermal bomb and placed in an oven at 120 °C for 2 hours. The solids obtained after hydrothermal treatment were then separated by centrifugation and washed once with 35 ml of deionized water. The washed hydrothermally treated powder was then dried at 120 °C for 16 hours. The surface area, determined by BET, of the dried powder is about 20 m2/g. Transmission electron microscopy was used to characterize the morphology of this sample further. The powder was constituted of large aspect ratio particles with about 20 to 40nm wide by 0.5 to 2 micron length. The Y₂O₃/La₂O₃ mixed oxide nanowires ware calcined in three temperature steps at 200, 400 and 500 °C with 3 °C /min ramp and 2 hours of dwell time at each step.

### EXAMPLE 35 (Reference)

### SYNTHESIS OF LA₂O₃ NANOWIRES

1g of La₂O₃ (13.1 mmol) and 0.92 g of NH₄Cl (18.6 mmol) were placed in a 25ml stainless steel autoclave with a Teflon liner (Parr Bomb No. 4749). 10ml deionized water were then added to the dry reactants. The autoclave was sealed and placed in a 160 °C oven for 12 h. After 12h, the autoclave was allowed to cool. The nanowires were washed several times with 10 mL of water to remove any excess NH₄Cl. The product was then dried in an oven for 15 hours at 65 °C in an oven and then calcined in a muffle oven in air (load in the furnace at room temperature, ramp to 100°C with 2 °C/min rate, dwell for 30min, ramp to 400°C with 2°C/min rate, dwell for 240min, ramp to 550°C with 2°C/min rate, dwell for 240min, cool to room temperature.)

### EXAMPLE 36 (Reference)

### SYNTHESIS OF LA₂O₃/ND₂O₃ MIXED OXIDE NANOWIRES

0.5g of La₂O₃ (1.5 mmol), 0.52g of Nd₂O₃ (1.5 mmol), and 0.325g of NH₄Cl (6 mmol) were ground together using a mortar and pestle. Once the dry reactants were well mixed, the ground powder was placed in a ceramic crucible and then the crucible was transferred to a tube furnace. The tube furnace atmosphere was flushed with nitrogen for 0.5 h. The reactants were then calcined under nitrogen (25 °C - 450 °C, 2 °C /min ramp, dwell 1 h; 450 °C -900 °C; 2 °C /min ramp, 1 h hold, cool to room temperature.) The product (0.2 g) was placed in 10 mL of deionized water and stirred at room temperature for 24 h. The nanowires were then washed several times with deionized H₂O and dried in an oven for 15 hours at 65 °C in an oven and then calcined in a muffle oven in air (load in the furnace at room temperature, ramp to 100°C with 2°C/min rate, dwell for 30min, ramp to 400°C with 2°C/min rate, dwell for 240min, ramp to 550°C with 2°C/min rate, dwell for 240 min, cool to room temperature.)

### EXAMPLE 37

### OLIGOMERIZATION OF ETHYLENE TO LIQUID HYDROCARBON FUELS WITH HIGH AROMATICS CONTENT

0.1g of the zeolite ZSM-5 is loaded into a fixed bed micro-reactor and heated at 400 °C for 2 h under nitrogen to activate the catalyst. The OCM effluent, containing ethylene and ethane, is reacted over the catalyst at 400 °C at a flow rate of 50 mL/min and GSHV = 3000 - 10000 mL/(g h). The reaction products are separated into liquid and gas components using a cold trap. The gas and liquid components are analyzed by gas chromatography. C5-C10 hydrocarbon liquid fractions, such as xylene and isomers thereof, represent ≥90 % of the liquid product ratio while the C11-C15 hydrocarbon fraction represents the remaining 10% of the product ratio.

### EXAMPLE 38

### OLIGOMERIZATION OF ETHYLENE TO LIQUID HYDROCARBON FUELS WITH HIGH OLEFINS CONTENT

0.1g of the zeolite ZSM-5 doped with nickel is loaded into a fixed bed micro-reactor and heated at 350 °C for 2 h under nitrogen to activate the catalyst. The OCM effluent, containing ethylene and ethane, is reacted over the catalyst at 250 - 400 °C temperature rage with GSHV = 1000 - 10000 mL/(g h). The reaction products are separated into liquid and gas components using a cold trap. The gas and liquid components are analyzed by gas chromatography. C₄-C₁₀ olefin hydrocarbon liquid fractions, such as butene, hexane and octene represent ≥95 % of the liquid product ratio while the C₁₂-C₁₈ hydrocarbon fraction represents the remaining 5% of the product ratio. Some trace amounts of odd numbered olefins are also possible in the product.

### EXAMPLE 39 (Reference)

### SYNTHESIS OF MNWO₄ NANOWIRES

0.379g of Na₂WO₄ (0.001 mol) was dissolved in 5 mL of deionized water. 0.197g of MnCl₂•6H2O (0.001 mol) was dissolved in 2 mL of deionized water. The two solutions were then mixed and a precipitate was observed immediately. The mixture was placed in a stainless steel autoclave with a Teflon liner (Parr Bomb No. 4749). 40ml of deionized water was added to the reaction mixture and the pH was adjusted to 9.4 with NH40H. The autoclave was sealed and placed in a 120 °C oven. The reaction was left to react for 18h and then it was cooled to room temperature. The product was washed with deionized water and then dried in a 65 °C oven. The samples were calcined in a muffle oven in air (load in the furnace at room temperature, ramp to 400 °C with 5°C/min rate, dwell for 2 h, ramp to 850 °C with 5°C/min rate, dwell for 8 h, cool to room temperature).

### EXAMPLE 40 (Reference)

### PREPARATION OF SUPPORTED MNWO₄ NANOWIRE CATALYSTS

Supported MnWO₄ nanowires catalysts are prepared using the following general protocol. MnWO₄ nanowires are prepared using the method described in example 42. Manganese tungstate nanowires, support, and water are slurried for 6 h at room temperature. The manganese tungstate to support ratio is 2-10 wt %. The mixture is dried in a 65 °C oven and then calcined in a muffle oven in air: load in the furnace at room temperature, ramp to 400°C with 5°C/min rate, dwell for 2 h, ramp to 850°C with 5°C/min rate, dwell for 8 h, cool to room temperature. The following is a list of exemplary supports that may be used: SiO₂, Al₂O₃, SiO₂-Al₂O₃, ZrO₂, TiO₂, HfO₂, Silica-Aluminum Phosphate, and Aluminum Phosphate.

### EXAMPLE 41

### OCM CATALYZED BY LA₂O₃ NANOWIRES

50mg of La₂O₃ nanowires catalyst, prepared using the method described in example 19, were placed into a reactor tube (4 mm ID diameter quartz tube with a 0.5 mm ID capillary downstream), which was then tested in an Altamira Benchcat 203. The gas flows were held constant at 46 sccm methane and 54 sccm air, which correspond to a CH4/02 ratio of 4 and a feed gas-hour space velocity (GHSV) of about 130000 h-1. The reactor temperature was varied from 400°C to 500 °C in a 100 °C increment and from 500 °C to 850 °C in 50 °C increments. The vent gases were analyzed with gas chromatography (GC) at each temperature level.

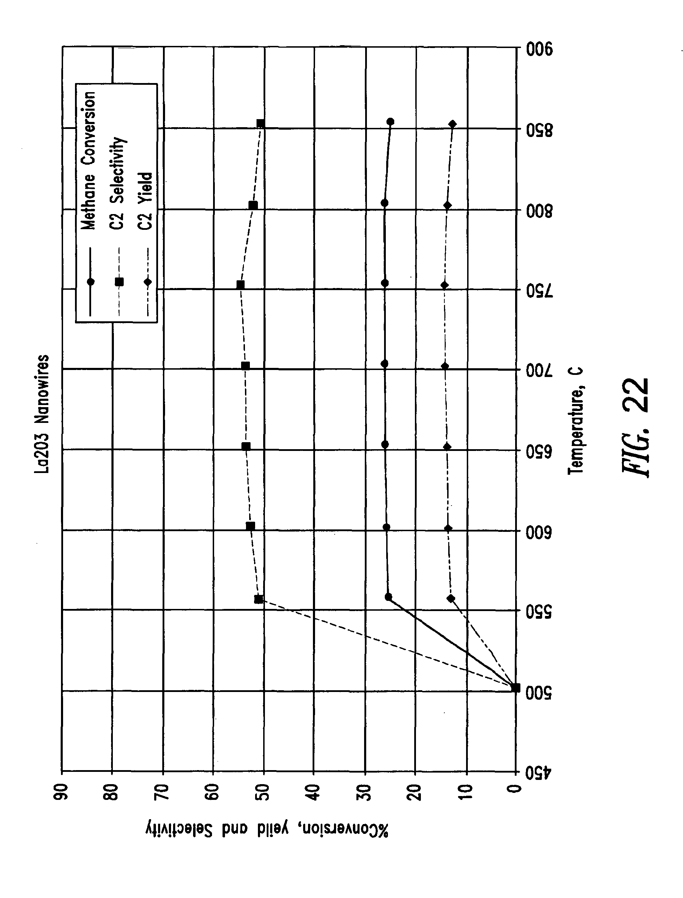

Figure 22 shows the onset of OCM between 500 °C and 550 °C. The C2 selectivity, methane conversion and C2 yield at 650 °C were 54 %, 27 % and 14 %, respectively.

## Claims

1. A method for the preparation of ethylene from methane comprising contacting a mixture comprising oxygen and methane at a temperature below 900 °C with a catalytic nanowire,
wherein the catalytic nanowire is an inorganic catalytic polycrystalline nanowire having a ratio of effective length to actual length of less than one and an aspect ratio of greater than ten as measured by TEM in bright field mode at 5 keV, and comprising one or more elements from any of Groups 1 through 7, lanthanides, actinides or combinations thereof in the form of oxides, hydroxides, oxyhydroxides, sulfates, carbonates, oxide carbonates, oxalates, phosphates, hydrogenphosphates, dihydrogenphosphates, oxyhalides, hydroxihalides, oxyhydroxides, oxysulfates or combinations thereof.

2. A method for the preparation of a downstream product of ethylene, the method comprising converting methane into ethylene in the presence of a catalytic nanowire and further oligomerizing the ethylene to prepare a downstream product of ethylene,
wherein the catalytic nanowire is an inorganic catalytic polycrystalline nanowire having a ratio of effective length to actual length of less than one and an aspect ratio of greater than ten as measured by TEM in bright field mode at 5 keV, and comprising one or more elements from any of Groups 1 through 7, lanthanides, actinides or combinations thereof in the form of oxides, hydroxides, oxyhydroxides, sulfates, carbonates, oxide carbonates, oxalates, phosphates, hydrogenphosphates, dihydrogenphosphates, oxyhalides, hydroxihalides, oxyhydroxides, oxysulfates or combinations thereof.

3. The method of claim 1 or 2, wherein the one or more elements are in the form of oxides.

4. The method of claim 1 or 2, wherein the one or more elements are in the form of hydroxides.

5. The method of any of the preceding claims, wherein the nanowire comprises Mg, Ca, La, W, Mn, Mo, Nd, Sm, Eu, Pr, Zr or combinations thereof.

6. The method of any of the preceding claims, wherein the nanowire comprises MgO, CaO, La₂O₃, Na₂WO₄, Mn₂O₃, Mn₃O₄, Nd₂O₃, Sm₂O₃, Eu₂O₃, Pr₂O₃, M₉₆MnO₈, NaMnO₄, Na/Mn/W/O, MnWO₄ or combinations thereof.

7. The method of any of the preceding claims, wherein the nanowire further comprises one or more dopants comprising metal elements, semi-metal elements, non-metal elements or combinations thereof.

8. The method of claim 7, wherein the dopant comprises Li, Na, K, Mg, Ca, Ba, Sr, Eu, Sm, Co or Mn.

9. The method of claim 8, wherein the nanowire comprises Li/MgO, Ba/MgO, Sr/La₂O₃, Mg/Na/La₂O₃, Sr/Nd₂O₃, or Mn/Na₂WO₄.

10. The method of claim 7, wherein the atomic ratio of the one or more elements from Groups 1 through 7, lanthanides or actinides to the dopant ranges from 1:1 to 10,000:1.

11. The method of any of the preceding claims, wherein the nanowire comprises a combination of two or more compounds comprising the one or more elements.

12. The method of claim 11., wherein the nanowire comprises Mn₂O₃/Na₂WO₄, Mn₃O₄/Na₂WO₄ MnWO₄/Na₂WO₄/Mn₂O₃, MnWO₄/Na₂WO₄/Mn₃O₄ or NaMnO₄/MgO.

13. The method of any of the preceding claims, wherein the nanowire comprises a solid core.

14. The method of any of the preceding claims, wherein the nanowire comprises a hollow core.

15. The method of any of the preceding claims, wherein the nanowire has a diameter of between 7 nm and 200 nm as determined by TEM in bright field mode at 5 keV.

16. The method of any of the preceding claims, wherein the nanowire has an actual length of between 100 nm and 10 µm as determined by TEM in bright field mode at 5 keV.

17. The method of any of the preceding claims, wherein the nanowire has a ratio of effective length to actual length of less than 0.8.

18. The method of any of the preceding claims, wherein the nanowire has a bent morphology as determined by TEM in bright field mode at 5 keV.

19. The method of any of the preceding claims, wherein the powder x-ray diffraction pattern of the nanowire shows an average crystalline domain size of less than 50 nm.

20. The method of any of the preceding claims, wherein the catalytic nanowire further comprises a support material.

21. The method of claim 20, wherein the support material comprises an inorganic oxide, Al₂O₃, SiO₂, TiO₂, MgO, ZrO₂, HfO₂, CaO, ZnO, LiAlO₂, MgAl₂O₄, MnO, MnO₂, Mn₂O₄, Mn₃O₄, La₂O₃, activated carbon, silica gel, zeolites, activated clays, activated Al₂O₃, diatomaceous earth, magnesia, aluminosilicates, calcium aluminate, support nanowires or combinations thereof.

22. The method of claim 21, wherein the support material comprises SiO₂, ZrO₂, CaO, La₂O₃ or MgO.

23. The method of any of claims 1-13 and 15-22, wherein the nanowire comprises an inner core and an outer layer, the inner core and outer layer each independently comprising one or more elements selected from Groups 1 through 7, lanthanides and actinides.

24. The method of any of the preceding claims, wherein the methane is converted to ethylene via the oxidative coupling of methane (OCM) reaction.

25. The method of claim 24, wherein oxidative coupling of methane is performed at a temperature below about 700 °C.

26. The method of any of the preceding claims, wherein the catalytic nanowire further comprises a biomolecule or modified or degraded forms thereof.

27. The method of any of claims 1 or 3-23, wherein the mixture comprising oxygen and methane comprises air.

28. The method of any of claims 2-24, wherein the catalytic nanowire comprises one or more doping elements.

29. The method of any of claims 2-28, wherein the downstream product of ethylene is natural gasoline.

30. The method of any of claims 2-29, wherein the downstream product of ethylene comprises 1-hexene, 1-octene or combinations thereof.

## Patentansprüche

1. Verfahren zur Herstellung von Ethylen aus Methan umfassend das Inkontaktbringen einer Mischung umfassend Sauerstoff und Methan bei einer Temperatur unter 900°C mit einem katalytischen Nanodraht,
wobei der katalytische Nanodraht ein anorganischer katalytischer polykristalliner Nanodraht ist, der ein Verhältnis der effektiven Länge zur tatsächlichen Länge von weniger als eins und ein Aspektverhältnis von mehr als zehn, gemessen durch TEM im Hellfeldmodus bei 5 keV, aufweist und ein oder mehrere Elemente von beliebigen der Gruppen 1 bis 7, Lanthanoiden, Actinoiden oder Kombinationen davon in Form von Oxiden, Hydroxiden, Oxyhydroxiden, Sulfaten, Carbonaten, Oxidcarbonaten, Oxalaten, Phosphaten, Hydrogenphosphaten, Dihydrogenphosphaten, Oxyhalogeniden, Hydroxyhalogeniden, Oxyhydroxiden, Oxysulfaten oder Kombinationen davon umfasst.

2. Verfahren zur Herstellung eines Folgeprodukts (downstream product) von Ethylen, wobei das Verfahren das Umwandeln von Methan in Ethylen in Gegenwart eines katalytischen Nanodrahts und außerdem das Oligomerisieren des Ethylens zum Herstellen eines Folgeprodukts von Ethylen umfasst,
wobei der katalytische Nanodraht ein anorganischer katalytischer polykristalliner Nanodraht ist, der ein Verhältnis der effektiven Länge zur tatsächlichen Länge von weniger als eins und ein Aspektverhältnis von mehr als zehn, gemessen durch TEM im Hellfeldmodus bei 5 keV, aufweist und ein oder mehrere Elemente von beliebigen der Gruppen 1 bis 7, Lanthanoiden, Actinoiden oder Kombinationen davon in Form von Oxiden, Hydroxiden, Oxyhydroxiden, Sulfaten, Carbonaten, Oxidcarbonaten, Oxalaten, Phosphaten, Hydrogenphosphaten, Dihydrogenphosphaten, Oxyhalogeniden, Hydroxyhalogeniden, Oxyhydroxiden, Oxysulfaten oder Kombinationen davon umfasst.

3. Verfahren nach Anspruch 1 oder 2, wobei das eine oder die mehreren Elemente in Form von Oxiden vorliegen.

4. Verfahren nach Anspruch 1 oder 2, wobei das eine oder die mehreren Elemente in Form von Hydroxiden vorliegen.

5. Verfahren nach einem der vorangehenden Ansprüche, wobei der Nanodraht Mg, Ca, La, W, Mn, Mo, Nd, Sm, Eu, Pr, Zr oder Kombinationen davon umfasst.

6. Verfahren nach einem der vorangehenden Ansprüche, wobei der Nanodraht MgO, CaO, La₂O₃, Na₂WO₄, Mn₂O₃, Mn₃O₄, Nd₂O₃, Sm₂O₃, Eu₂O₃, Pr₂O₃, Mg₆MnO₈, NaMnO₄, Na/Mn/W/O, MnWO₄ oder Kombinationen davon umfasst.

7. Verfahren nach einem der vorangehenden Ansprüche, wobei der Nanodraht außerdem ein oder mehrere Dotiermittel, umfassend Metallelemente, Halbmetallelemente, Nichtmetallelemente oder Kombinationen davon, umfasst.

8. Verfahren nach Anspruch 7, wobei das Dotiermittel Li, Na, K, Mg, Ca, Ba, Sr, Eu, Sm, Co oder Mn umfasst.

9. Verfahren nach Anspruch 8, wobei der Nanodraht Li/MgO, Ba/MgO, Sr/La₂O₃, Mg/Na/La₂O₃, Sr/Nd₂O₃, oder Mn/Na₂WO₄ umfasst.

10. Verfahren nach Anspruch 7, wobei das Atomverhältnis von dem einen oder den mehreren Elementen aus den Gruppen 1 bis 7, Lanthanoiden oder Actinoiden zu dem Dotiermittel in dem Bereich von 1:1 bis 10.000:1 liegt.

11. Verfahren nach einem der vorangehenden Ansprüche, wobei der Nanodraht eine Kombination von zwei oder mehr Verbindungen umfasst, welche das eine oder die mehreren Elemente umfassen.

12. Verfahren nach Anspruch 11, wobei der Nanodraht Mn₂O₃/Na₂WO₄, Mn₃O₄/Na₂WO₄ MnWO₄/Na₂WO₄/Mn₂O₃, MnWO₄/Na₂WO₄/Mn₃O₄ oder NaMnO₄/MgO umfasst.

13. Verfahren nach einem der vorangehenden Ansprüche, wobei der Nanodraht einen festen Kern umfasst.

14. Verfahren nach einem der vorangehenden Ansprüche, wobei der Nanodraht einen hohlen Kern umfasst.

15. Verfahren nach einem der vorangehenden Ansprüche, wobei der Nanodraht einen Durchmesser zwischen 7 nm und 200 nm, bestimmt durch TEM im Hellfeldmodus bei 5 keV, aufweist.

16. Verfahren nach einem der vorangehenden Ansprüche, wobei der Nanodraht eine tatsächliche Länge zwischen 100 nm und 10 µm, bestimmt durch TEM im Hellfeldmodus bei 5 keV, aufweist.

17. Verfahren nach einem der vorangehenden Ansprüche, wobei der Nanodraht ein Verhältnis von effektiver Länge zu tatsächlicher Länge von weniger als 0,8 aufweist.

18. Verfahren nach einem der vorangehenden Ansprüche, wobei der Nanodraht eine gebogene Morphologie, bestimmt durch TEM im Hellfeldmodus bei 5 keV, aufweist.

19. Verfahren nach einem der vorangehenden Ansprüche, wobei das Röntgenpulverbeugungsdiagramm des Nanodrahts eine mittlere Kristalldomänengröße von weniger als 50 nm aufweist.

20. Verfahren nach einem der vorangehenden Ansprüche, wobei der katalytische Nanodraht außerdem ein Trägermaterial umfasst.

21. Verfahren nach Anspruch 20, wobei das Trägermaterial ein anorganisches Oxid, Al₂O₃, SiO₂, TiO₂, MgO, ZrO₂, HfO₂, CaO, ZnO, LiAlO₂, MgAl₂O₄, MnO, MnO₂, Mn₂O₄, Mn₃O₄, La₂O₃, Aktivkohle, Silicagel, Zeolithe, aktivierte Tonerden, aktiviertes Al₂O₃, Diatomeenerde, Magnesia, Aluminiumsilicate, Calciumaluminat, Träger-Nanodrähte oder Kombinationen davon umfasst.

22. Verfahren nach Anspruch 21, wobei das Trägermaterial SiO₂, ZrO₂, CaO, La₂O₃ oder MgO umfasst.

23. Verfahren nach einem der Ansprüche 1-13 und 15-22, wobei der Nanodraht einen inneren Kern und eine äußere Schicht umfasst, wobei der innere Kern und die äußere Schicht jeweils unabhängig voneinander ein oder mehrere Elemente, ausgewählt aus den Gruppen 1 bis 7, Lanthanoiden und Actinoiden, umfasst.

24. Verfahren nach einem der vorangehenden Ansprüche, wobei das Methan über die oxidative Kupplung von Methan (oxidative coupling of methane, OCM) -Reaktion in Ethylen umgewandelt wird.

25. Verfahren nach Anspruch 24, wobei die oxidative Kupplung von Methan bei einer Temperatur unter ungefähr 700°C durchgeführt wird.

26. Verfahren nach einem der vorangehenden Ansprüche, wobei der katalytische Nanodraht außerdem ein Biomolekül oder modifizierte oder abgebaute Formen davon umfasst.

27. Verfahren nach einem der Ansprüche 1 oder 3-23, wobei die Mischung, die Sauerstoff und Methan umfasst, Luft umfasst.

28. Verfahren nach einem der Ansprüche 2-24, wobei der katalytische Nanodraht ein oder mehrere dotierende Elemente umfasst.

29. Verfahren nach einem der Ansprüche 2-28, wobei das Folgeprodukt von Ethylen Naturbenzin ist.

30. Verfahren nach einem der Ansprüche 2-29, wobei das Folgeprodukt von Ethylen 1-Hexen, 1-Octen oder Kombinationen davon umfasst.

## Revendications

1. Une méthode pour la préparation d'éthylène à partir de méthane comprenant la mise en contact d'un mélange comprenant de l'oxygène et du méthane à une température inférieur à 900°C avec un nanofil catalytique,
dans laquelle le nanofil catalytique est un nanofil catalytique polycristallin inorganique ayant un rapport de la longueur effective sur longueur réelle inférieure à un et un rapport de forme supérieur à dix tel que mesuré par TEM en mode champ lumineux à 5 keV, et comprenant un ou plusieurs éléments parmi n'importe quel des groupes 1 à 7, des lanthanides, des actinides ou des combinaisons de ces derniers sous forme d'oxydes, d'hydroxydes, d'oxyhydroxydes, de sulfates, de carbonates, de carbonates d'oxyde, d'oxalates, de phosphates, d'hydrogénophosphates, de dihydrogénophosphates, d'oxyhalides, d'hydroxyhalides, d'oxyhydroxydes, d'oxysulfates ou des combinaisons de ces derniers.

2. Une méthode pour la préparation d'un produit en aval de l'éthylène, la méthode comprenant la conversion du méthane en éthylène en présence d'un nanofil catalytique et en outré l'oligomérisation de l'éthylène pour préparer un produit en aval de l'éthylène,
dans laquelle le nanofil catalytique est un nanofil catalytique polycristallin inorganique ayant un rapport de la longueur effective sur longueur réelle inférieure à un et un rapport de forme supérieur à dix tel que mesuré par TEM en mode champ lumineux à 5 keV, et comprenant un ou plusieurs éléments parmi n'importe quel des groupes 1 à 7, des lanthanides, des actinides ou des combinaisons de ces derniers sous forme d'oxydes, d'hydroxydes, d'oxyhydroxydes, de sulfates, de carbonates, de carbonates d'oxyde, d'oxalates, de phosphates, d'hydrogénophosphates, de dihydrogénophosphates, d'oxyhalides, d'hydroxyhalides, d'oxyhydroxydes, d'oxysulfates ou des combinaisons de ces derniers.

3. La méthode selon la revendication 1 ou 2, dans laquelle lesdits un ou plusieurs éléments sont sous forme d'oxydes.

4. La méthode selon la revendication 1 ou 2, dans laquelle lesdits un ou plusieurs éléments sont sous forme d'hydroxydes.

5. La méthode selon une quelconque des revendications précédentes, dans laquelle le nanofil comprend Mg, Ca, La, W, Mn, Mo, Nd, Sm, Eu, Pr, Zr ou des combinaisons de ces derniers.

6. La méthode selon une quelconque des revendications précédentes, dans laquelle le nanofil comprend MgO, CaO, La₂O₃, Na₂WO₄, Mn₂O₃, Mn₃O₄, Nd₂O₃, Sm₂O₃, Eu₂O₃, Pr₂O₃, Mg₆MnO₈, NaMnO₄, Na/Mn/W/O, MnWO₄ ou des combinaisons de ces derniers.

7. La méthode selon une quelconque des revendications précédentes, dans laquelle le nanofil comprend en outre un ou plusieurs dopants comprenant des éléments métalliques, des éléments semi-métallique, des éléments non métalliques ou des combinaisons de ces derniers.

8. La méthode selon la revendication 7, dans laquelle le dopant comprend Li, Na, K, Mg, Ca, Ba, Sr, Eu, Sm, Co ou Mn.

9. La méthode selon la revendication 8, dans laquelle le nanofil comprend Li/MgO, Ba/MgO, Sr/La₂O₃, Mg/Na/La₂O₃, Sr/Nd₂O₃, ou Mn/Na₂WO₄.

10. La méthode selon la revendication 7, dans laquelle le rapport atomique d'un ou plusieurs éléments parmi les groupes 1 à 7, les lanthanides ou les actinides avec le dopant est compris entre 1:1 et 10,000:1.

11. La méthode selon une quelconque des revendications précédentes, dans laquelle le nanofil comprend une combinaison de deux ou plusieurs composés comprenant l'un ou plusieurs éléments.

12. La méthode selon la revendication 11, dans laquelle le nanofil comprend Mn₂O₃/Na₂WO₄, Mn₃O₄/Na₂WO₄ MnWO₄/Na₂WO₄/Mn₂O₃, MnWO₄/Na₂WO₄/Mn₃O₄ ou NaMnO₄/MgO.

13. La méthode selon une quelconque des revendications précédentes, dans laquelle le nanofil comprend un noyau solide.

14. La méthode selon une quelconque des revendications précédentes, dans laquelle le nanofil comprend un noyau creux.

15. La méthode selon une quelconque des revendications précédentes, dans laquelle le nanofil a un diamètre entre 7 nm et 200 nm tel que déterminé par TEM en mode champ lumineux à 5 keV.

16. La méthode selon une quelconque des revendications précédentes, dans laquelle le nanofil a une longueur réelle entre 100 nm et 10 µm telle que déterminée par TEM en mode champ lumineux à 5 keV.

17. La méthode selon une quelconque des revendications précédentes, dans laquelle le nanofil a un rapport de longueur effective sur longueur réelle de moins de 0.8.

18. La méthode selon une quelconque des revendications précédentes, dans laquelle le nanofil a une morphologie pliée telle que déterminée par TEM en mode champ lumineux à 5 keV.

19. La méthode selon une quelconque des revendications précédentes, dans laquelle le motif de diffraction des rayons X sur poudre du nanofil montre une taille moyenne de domaine cristallin de moins de 50 nm.

20. La méthode selon une quelconque des revendications précédentes, dans laquelle le nanofil catalytique comprend en outre un matériau de support.

21. La méthode selon la revendication 20, dans laquelle le matériau de support comprend un oxyde inorganique, Al₂O₃, SiO₂, TiO₂, MgO, ZrO₂, HfO₂, CaO, ZnO, LiAlO₂, MgAl₂O₄, MnO, MnO₂, Mn₂O₄, Mn₃O₄, La₂O₃, du charbon actif, du gel de silice, des zéolites, des argiles activées, Al₂O₃ activé, un Kieselguhr, une magnésie, des aluminosilicates, de l'aluminate de calcium, des nanofils de support ou une combinaison de ces derniers.

22. La méthode selon la revendication 21, dans laquelle le matériau de support comprend SiO₂, ZrO₂, CaO, La₂O₃ ou MgO.

23. La méthode selon une quelconque des revendications 1 à 13 et 15 à 22, dans laquelle le nanofil comprend un noyau interne et une couche externe, le noyau interne et la couche externe comprenant, chacun, indépendamment, un ou plusieurs éléments sélectionnés parmi les groupes 1 à 7, les lanthanides et les actinides.

24. La méthode selon une quelconque des revendications précédentes, dans laquelle le méthane est converti en éthylène via la réaction de couplage oxydant du méthane (OCM).

25. La méthode selon la revendication 24, dans laquelle le couplage oxydant du méthane est effectué à une température inférieure à environ 700 °C.

26. La méthode selon une quelconque des revendications précédentes, dans laquelle le nanofil catalytique comprend en outre une biomolécule ou des formes modifiées ou dégradées de celle-ci.

27. La méthode selon une quelconque des revendications 1 ou 3 à 23, dans laquelle le mélange comprenant de l'oxygène et le méthane comprend de l'air.

28. La méthode selon une quelconque des revendications 2 à 24, dans laquelle le nanofil catalytique comprend un ou plusieurs éléments dopants.

29. La méthode selon une quelconque des revendications 2 à 28, dans laquelle le produit en aval de l'éthylène est de l'essence naturelle.

30. La méthode selon une quelconque des revendications 2 à 29, dans laquelle le produit en aval de l'éthylène comprend de l'1-hexène, 1-octène ou des combinaisons de ces derniers.


### SEQUENCE LISTING

<110> Siluria Technologies, Inc.
   Scher, Erik, C.
   Zurcher, Fabio R.
   Cizeron, Joel M.
   Schammel, Wayne P.
   Tkachenko, Alex
   Gamoras, Joel
   Karshtedt, Dmitry
   Nyce, Greg
<120> NANOWIRE CATALYSTS
<130> 860158.418PC
<140> PCT
   <141> 2011-05-24
<150> US 61/425,631
   <151> 2010-12-21
<150> US 61/347,774
   <151> 2010-05-24
<160> 15
<170> FastSEQ for Windows Version 4.0
<210> 1
   <211> 52
   <212> PRT
   <213> Artificial Sequence
<220>
   <223> M13 bacteriophage pVIII protein tailored for surface affinity to metal ions
<400> 1
   Ala Glu Glu Gly Ser Glu Asp Pro Ala Lys Ala Ala Phe Asn Ser Leu
    1               5                  10                  15
   Gln Ala Ser Ala Thr Glu Tyr Ile Gly Tyr Ala Trp Ala Met Val Val
               20                  25                  30
   Val Ile Val Gly Ala Thr Ile Gly Ile Lys Leu Phe Lys Lys Phe Thr
           35                  40                  45
   Ser Lys Ala Ser
       50
<210> 2
   <211> 51
   <212> PRT
   <213> Artificial Sequence
<220>
   <223> M13 bacteriophage pVIII protein tailored for surface affinity to metal ions
<400> 2
   Glu Glu Gly Ser Asp Asp Pro Ala Lys Ala Ala Phe Asn Ser Leu Gln
    1               5                  10                  15
   Ala Ser Ala Thr Glu Tyr Ile Gly Tyr Ala Trp Ala Met Val Val Val
               20                  25                  30
   Ile Val Gly Ala Thr Ile Gly Ile Lys Leu Phe Lys Lys Phe Thr Ser
           35                  40                  45
   Lys Ala Ser
       50
<210> 3
   <211> 50
   <212> PRT
   <213> Artificial Sequence
<220>
   <223> M13 bacteriophage pVIII protein tailored for surface affinity to metal ions
<400> 3
   Ala Glu Glu Glu Asp Pro Ala Lys Ala Ala Phe Asn Ser Leu Gln Ala
    1               5                  10                  15
   Ser Ala Thr Glu Tyr Ile Gly Tyr Ala Trp Ala Met Val Val Val Ile
               20                  25                  30
   Val Gly Ala Thr Ile Gly Ile Lys Leu Phe Lys Lys Phe Thr Ser Lys
           35                  40                  45
   Ala Ser
       50
<210> 4
   <211> 49
   <212> PRT
   <213> Artificial Sequence
<220>
   <223> M13 bacteriophage pVIII protein tailored for surface affinity to metal ions
<400> 4
   Glu Glu Glu Asp Pro Ala Lys Ala Ala Phe Asn Ser Leu Gln Ala Ser
    1               5                  10                  15
   Ala Thr Glu Tyr Ile Gly Tyr Ala Trp Ala Met Val Val Val Ile Val
               20                  25                  30
   Gly Ala Thr Ile Gly Ile Lys Leu Phe Lys Lys Phe Thr Ser Lys Ala
           35                  40                  45
   Ser
<210> 5
   <211> 51
   <212> PRT
   <213> Artificial Sequence
<220>
   <223> M13 bacteriophage pVIII protein tailored for surface affinity to metal ions
<400> 5
   Ala Glu Glu Glu Glu Asp Pro Ala Lys Ala Ala Phe Asn Ser Leu Gln
    1               5                  10                  15
   Ala Ser Ala Thr Glu Tyr Ile Gly Tyr Ala Trp Ala Met Val Val Val
               20                  25                  30
   Ile Val Gly Ala Thr Ile Gly Ile Lys Leu Phe Lys Lys Phe Thr Ser
                  35                  40                  45
   Lys Ala Ser
       50
<210> 6
   <211> 51
   <212> PRT
   <213> Artificial Sequence
<220>
   <223> M13 bacteriophage pVIII protein tailored for surface affinity to metal ions
<400> 6
   Ala Glu Glu Ala Glu Asp Pro Ala Lys Ala Ala Phe Asn Ser Leu Gln
    1               5                  10                  15
   Ala Ser Ala Thr Glu Tyr Ile Gly Tyr Ala Trp Ala Met Val Val Val
               20                  25                  30
   Ile Val Gly Ala Thr Ile Gly Ile Lys Leu Phe Lys Lys Phe Thr Ser
           35                  40                  45
   Lys Ala Ser
       50
<210> 7
   <211> 50
   <212> PRT
   <213> Artificial Sequence
<220>
   <223> M13 bacteriophage pVIII protein tailored for surface affinity to metal ions
<220>
   <221> VARIANT
   <222> 3
   <223> Xaa = Glu or Gly
<400> 7
   Glu Glu Xaa Glu Asp Pro Ala Lys Ala Ala Phe Asn Ser Leu Gln Ala
    1               5                  10                  15
   Ser Ala Thr Glu Tyr Ile Gly Tyr Ala Trp Ala Met Val Val Val Ile
               20                  25                  30
   Val Gly Ala Thr Ile Gly Ile Lys Leu Phe Lys Lys Phe Thr Ser Lys
           35                  40                  45
   Ala Ser
       50
<210> 8
   <211> 50
   <212> PRT
   <213> Artificial Sequence
<220>
   <223> M13 bacteriophage pVIII protein tailored for surface affinity to metal ions
<400> 8
   Ala Glu Asp Asp Asp Pro Ala Lys Ala Ala Phe Asn Ser Leu Gln Ala
    1               5                  10                  15
   Ser Ala Thr Glu Tyr Ile Gly Tyr Ala Trp Ala Met Val Val Val Ile
               20                  25                  30
   Val Gly Ala Thr Ile Gly Ile Lys Leu Phe Lys Lys Phe Thr Ser Lys
           35                  40                  45
   Ala Ser
       50
<210> 9
   <211> 55
   <212> PRT
   <213> Artificial Sequence
<220>
   <223> M13 bacteriophage pVIII protein tailored for surface affinity to metal ions
<400> 9
   Ala Val Ser Gly Ser Ser Pro Gly Asp Asp Pro Ala Lys Ala Ala Phe
    1               5                  10                  15
   Asn Ser Leu Gln Ala Ser Ala Thr Glu Tyr Ile Gly Tyr Ala Trp Ala
               20                  25                  30
   Met Val Val Val Ile Val Gly Ala Thr Ile Gly Ile Lys Leu Phe Lys
           35                  40                  45
   Lys Phe Thr Ser Lys Ala Ser
       50                  55
<210> 10
   <211> 55
   <212> PRT
   <213> Artificial Sequence
<220>
   <223> M13 bacteriophage pVIII protein tailored for surface affinity to metal ions
<400> 10
   Ala Val Ser Gly Ser Ser Pro Asp Ser Asp Pro Ala Lys Ala Ala Phe
    1               5                  10                  15
   Asn Ser Leu Gln Ala Ser Ala Thr Glu Tyr Ile Gly Tyr Ala Trp Ala
               20                  25                  30
   Met Val Val Val Ile Val Gly Ala Thr Ile Gly Ile Lys Leu Phe Lys
           35                  40                  45
   Lys Phe Thr Ser Lys Ala Ser
       50                  55
<210> 11
   <211> 55
   <212> PRT
   <213> Artificial Sequence
<220>
   <223> M13 bacteriophage pVIII protein tailored for surface affinity to metal ions
<400> 11
   Ala Gly Glu Thr Gln Gln Ala Met Glu Asp Pro Ala Lys Ala Ala Phe
    1               5                  10                  15
   Asn Ser Leu Gln Ala Ser Ala Thr Glu Tyr Ile Gly Tyr Ala Trp Ala
               20                  25                  30
   Met Val Val Val Ile Val Gly Ala Thr Ile Gly Ile Lys Leu Phe Lys
           35                  40                  45
   Lys Phe Thr Ser Lys Ala Ser
       50                  55
<210> 12
   <211> 55
   <212> PRT
   <213> Artificial Sequence
<220>
   <223> M13 bacteriophage pVIII protein tailored for surface affinity to metal ions
<400> 12
   Ala Ala Gly Glu Thr Gln Gln Ala Met Asp Pro Ala Lys Ala Ala Phe
    1               5                  10                  15
   Asn Ser Leu Gln Ala Ser Ala Thr Glu Tyr Ile Gly Tyr Ala Trp Ala
               20                  25                  30
   Met Val Val Val Ile Val Gly Ala Thr Ile Gly Ile Lys Leu Phe Lys
           35                  40                  45
   Lys Phe Thr Ser Lys Ala Ser
       50                  55
<210> 13
   <211> 56
   <212> PRT
   <213> Artificial Sequence
<220>
   <223> M13 bacteriophage pVIII protein tailored for surface affinity to metal ions
<400> 13
   Ala Glu Pro Gly His Asp Ala Val Pro Glu Asp Pro Ala Lys Ala Ala
    1               5                  10                  15
   Phe Asn Ser Leu Gln Ala Ser Ala Thr Glu Tyr Ile Gly Tyr Ala Trp
               20                  25                  30
   Ala Met Val Val Val Ile Val Gly Ala Thr Ile Gly Ile Lys Leu Phe
           35                  40                  45
   Lys Lys Phe Thr Ser Lys Ala Ser
       50                  55
<210> 14
   <211> 51
   <212> PRT
   <213> M13 Bacteriophage
<400> 14
   Ala Glu Gly Asp Asp Asp Pro Ala Lys Ala Ala Phe Asn Ser Leu Gln
    1               5                  10                  15
   Ala Ser Ala Thr Glu Tyr Ile Gly Tyr Ala Trp Ala Met Val Val Val
               20                  25                  30
   Ile Val Gly Ala Thr Ile Gly Ile Lys Leu Phe Lys Lys Phe Thr Ser
           35                  40                  45
   Lys Ala Ser
       50
<210> 15
   <211> 74
   <212> PRT
   <213> M13 Bacteriophage
<400> 15
   Met Lys Lys Ser Leu Val Leu Lys Ala Ser Val Ala Val Ala Thr Leu
    1               5                  10                  15
   Val Pro Met Leu Ser Phe Ala Ala Glu Gly Asp Asp Asp Pro Ala Lys
               20                  25                  30
   Ala Ala Phe Asn Ser Leu Gln Ala Ser Ala Thr Glu Tyr Ile Gly Tyr
           35                  40                  45
   Ala Trp Ala Met Val Val Val Ile Val Gly Ala Thr Ile Gly Ile Lys
       50                  55                  60
   Leu Phe Lys Lys Phe Thr Ser Lys Ala Ser
   65                  70